(19) Europäisches Patentamt
European Patent Office
Office européen des brevets

(11) EP 2 502 909 A1

(12) EUROPEAN PATENT APPLICATION
published in accordance with Art. 153(4) EPC

(43) Date of publication:
**26.09.2012 Bulletin 2012/39**

(21) Application number: **10831588.8**

(22) Date of filing: **17.11.2010**

(51) Int Cl.:
***C07D 213/74*** *(2006.01)* ***A61K 31/496*** *(2006.01)*
***A61K 31/5377*** *(2006.01)* ***A61K 31/551*** *(2006.01)*
***A61P 3/10*** *(2006.01)* ***A61P 9/00*** *(2006.01)*
***A61P 9/10*** *(2006.01)* ***A61P 13/00*** *(2006.01)*
***A61P 25/00*** *(2006.01)* ***A61P 25/14*** *(2006.01)*
***A61P 25/16*** *(2006.01)* ***A61P 25/18*** *(2006.01)*
***A61P 25/22*** *(2006.01)* ***A61P 25/24*** *(2006.01)*
***A61P 25/28*** *(2006.01)* ***A61P 43/00*** *(2006.01)*
***C07D 401/04*** *(2006.01)* ***C07D 405/04*** *(2006.01)*
***C07D 409/04*** *(2006.01)* ***C07D 413/04*** *(2006.01)*

(86) International application number:
**PCT/JP2010/070496**

(87) International publication number:
**WO 2011/062194 (26.05.2011 Gazette 2011/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.11.2009 JP 2009263003**

(71) Applicant: **Takeda Pharmaceutical Company Limited**
**Osaka-shi, Osaka 541-0045 (JP)**

(72) Inventors:
- **TSUKAMOTO Tetsuya**
  **Osaka-shi**
  **Osaka 532-8686 (JP)**
- **WAKABAYASHI Takeshi**
  **Osaka-shi**
  **Osaka 532-8686 (JP)**
- **TOKUNAGA Norihito**
  **Osaka-shi**
  **Osaka 532-8686 (JP)**
- **MIYANOHANA Yuhei**
  **Osaka-shi**
  **Osaka 532-8686 (JP)**

(74) Representative: **Held, Stephan et al**
**Meissner, Bolte & Partner GbR**
**Chemistry**
**Widenmayerstraße 47**
**80538 München (DE)**

# (54) AMINOPYRIDINE DERIVATIVE

(57) The present invention provides a novel aminopyridine derivative that is excellent in pharmaceutical effects such as a neuroprotective effect, a neurogenesis or nerve regeneration promoting effect, and a cognitive function improving effect and, preferably, is low toxic and delivered at a high rate to the central nervous system. Specifically, the present invention provides a compound represented by the formula (I), wherein ring A represents a monocyclic aromatic hydrocarbon ring or the like; ring B may further have a substituent(s); ring D represents a monocyclic aromatic hydrocarbon ring or the like; L represents a bond, or a methylene group or the like; X represents -NRa- or the like, wherein Ra represents a hydrogen atom or the like; n represents 1 or 2; and $R^1$ to $R^3$ each represent a hydrogen atom or the like, wherein ring A and ring D each may be condensed with another ring to form a condensed ring which may have a substituent(s), and ring A-L- in the formula (I) may be bonded to X, provided that when ring A is pyrazole which may be substituted and ring D is benzene which may be substituted, L is a bond; or a salt thereof.

EP 2 502 909 A1

## Description

Technical Field

The present invention relates to an aminopyridine derivative and a medicament comprising the same. More specifically, the present invention relates to a compound that is excellent in pharmaceutical effects such as a neurotrophic factor activating effect, a glucose metabolism improving effect, a neuronal death inhibitory effect, a neuronal differentiation promoting effect, a neurogenesis promoting effect, a nerve regeneration promoting effect, a beta (β) amyloid lowering effect, a tau/phosphorylated tau lowering effect, and a cognitive function improving effect and is particularly effective as a medicament for the prevention or treatment of a central nervous system disease or the like.

Background Art

Neurodegenerative diseases are progressive diseases that bring about destructive injury, i.e., neuronal death. For example, central nervous diseases such as Alzheimer's disease, Parkinson's disease, amyotrophic lateral sclerosis (ALS), and Huntington's disease, and peripheral neuropathy typified by diabetic neuropathy are mainly known as the neurodegenerative diseases. The majority of these diseases are related to aging. In fact, the incidence thereof increases with aging. However, the diseases sometimes affect middle-aged or younger persons.

Much remains to be revealed as to the cause of neurodegenerative diseases, though the roles, etc., of neurotransmitters or neurotrophic factors have been elucidated gradually as a result of research on the structure or functions of the brain. Parkinson's disease has been shown to be related to a particular neurotransmitter, i.e., dopamine. L-dopa, a dopamine precursor, is used as a drug that mitigates neurological symptoms and restores functions. Unfortunately, L-dopa does not prevent the progression of neurodegeneration, but gradually loses its effects along with the progression of the conditions, i.e., the degeneration or loss of dopaminergic nerves. Also, Alzheimer's disease is a disease that causes the deposition of senile plaques or the alteration of neurofibril by the degeneration or loss of many kinds of neurons, such as acetylcholinergic neurons or monoaminergic neurons. Choline esterase inhibitors or an NMDA antagonist memantine has been launched as drugs for Alzheimer's disease and however, is within the realm of symptomatic treatment, which temporarily improves neurological symptoms, as in L-dopa for Parkinson's disease. No drug has been reported so far to protect neurons from the toxicity of such factors causing cell death and prevent the progression of neurodegenerative diseases, including Alzheimer's disease and Parkinson's disease.

Moreover, cell death in neurodegenerative diseases is allegedly caused by the toxicity of a factor specific to each disease. For example, in Alzheimer's disease, endogenous β amyloid is regarded as a factor causing cell death. The β amyloid protein constitutes senile plaques, which are neurological characteristics observed in the brains of Alzheimer's disease patients, and consists of 40 to 43 amino acids. It has been revealed that the addition of this β amyloid to the primary culture system of hippocampal neurons causes neuronal death [see Non Patent Literature 1]. In addition, it has been shown that β amyloid aggregation is essential for the manifestation of its toxicity [see Non Patent Literatures 2 and 3]. As mechanisms underlying the manifestation of β amyloid toxicity, it is considered that, for example, 1] β amyloid forms an ion channel to cause calcium ion entry, 2] β amyloid promotes the formation of free radicals, 3] β amyloid activates tau protein kinase I (TPK-I)/glycogen synthase kinase 3 beta (GSK-3β) to accelerate tau phosphorylation, and 4] β amyloid activates microglia, which in turn secretes neurotoxins. In recent years, neurotrophic factors such as IGF-1 (insulin-like growth factor), NGF (nerve growth factor), BDNF (brain-derived neurotrophic factor), and GDNF (glial cell-derived neurotrophic factor) have been known to inhibit the apoptosis of neurons induced by β amyloid or the like, suggesting that the dysfunction of neurotrophic factor signal cascades leads to cell death (apoptosis) [see Non Patent Literature 4]. According to reports, particularly, IGF-1 (insulin-like growth factor 1) signals phosphorylate Akt, also called protein kinase B (PKB), via phosphatidylinositol-3'-kinase (PI3 kinase: PI3K), and this activated Akt phosphorylates its substrates such as Bad or glycogen synthase kinase-3β (GSK-3β), thereby inhibiting neuronal death. It has been demonstrated that the inhibition of GSK-3β by activated PI-3 kinase is involved in this mechanism [see Non Patent Literatures 5 to 7]. Once β amyloid inhibits PI-3 kinase to activate TPK-I/GSK-3β, inhibited pyruvate dehydrogenase (PDH) influences the acetylcholine synthesis reaction system, also decreasing the content of acetylcholine. This is also consistent with the decreased content of acetylcholine in the brains of Alzheimer's disease patients. On the contrary, the activation of PI-3 kinase is expected not only to prevent neuronal death but to increase the acetylcholine content in the brain, improving neurological symptoms. Furthermore, the inhibition of TPK-I/GSK-3β can also be expected to increase a brain glucose utilization rate reduced in Alzheimer's disease [see Non Patent Literatures 8 and 9]. The relation of glucose metabolism in the brain to cognitive functions has also been reported [see Non Patent Literature 10]. Thus, the improvement of glucose metabolism in the brain is expected to improve cognitive functions.

For example, aminopyridine compounds, aniline compounds, and benzofuran compounds are known in the art as compounds useful for the prevention or treatment of central nervous system diseases or brain diseases [see Patent Literatures 1 to 9 and Non Patent Literature 11].

Patent Literature 1 discloses the following compound [For the definitions of abbreviations in formulas representing the compounds of Patent Literatures 1 to 9, see the respective specifications of these patent literatures]:

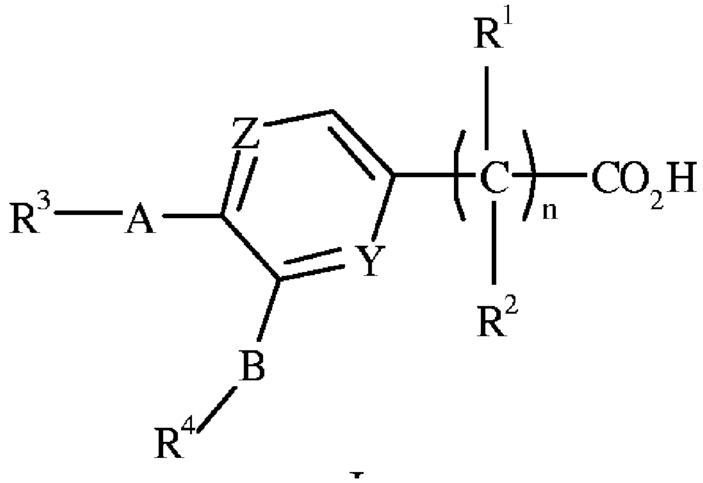

Patent Literature 2 discloses the following compound:

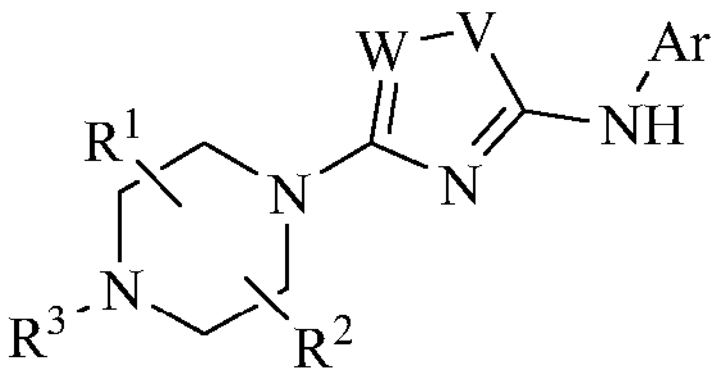

Patent Literature 3 discloses the following compound:

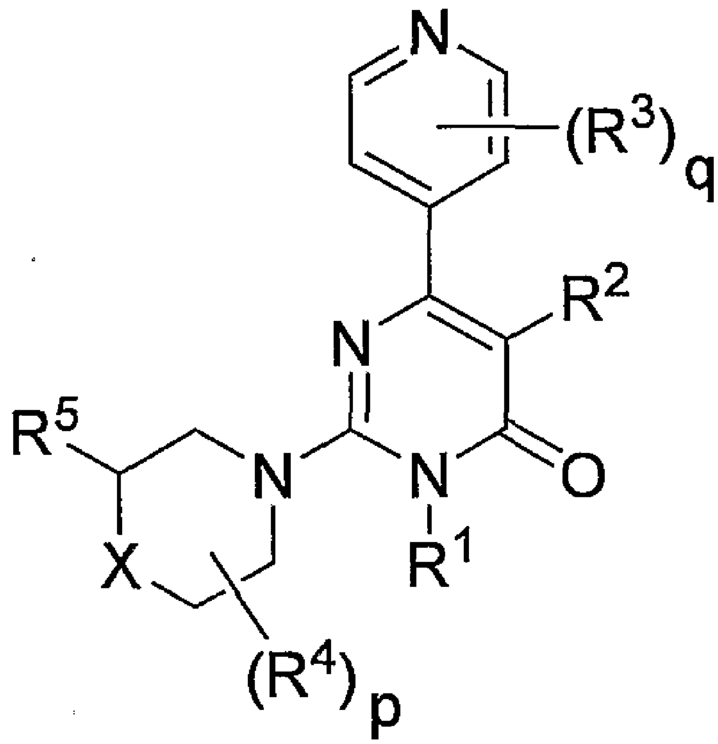

Patent Literature 4 discloses the following compound:

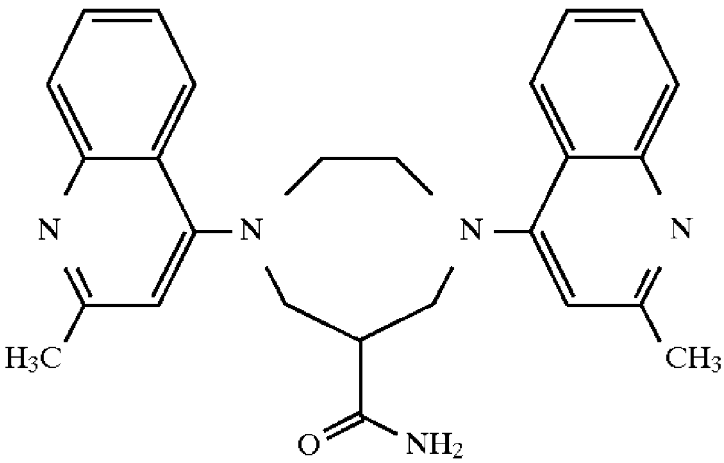

Patent Literature 5 discloses the following compound:

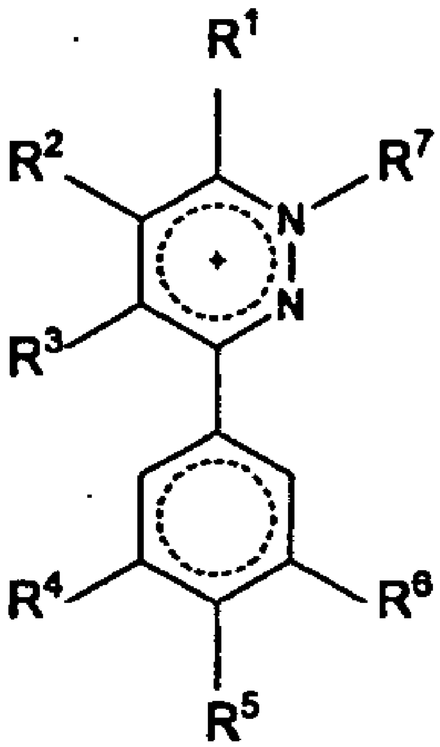

Patent Literature 6 discloses the following compound:

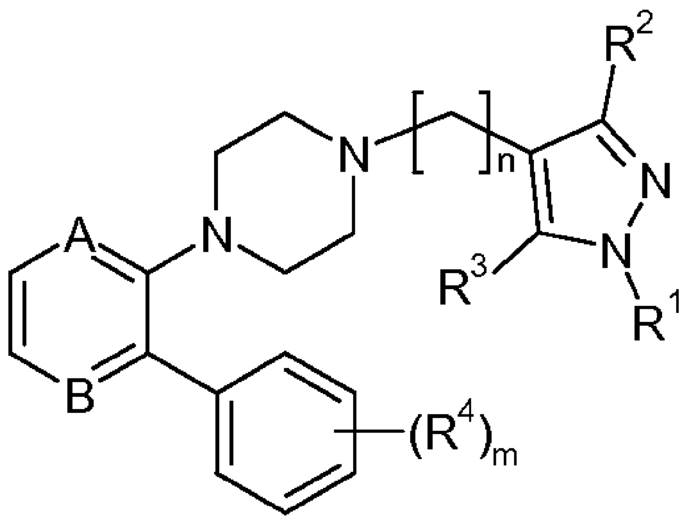

Patent Literature 7 discloses the following compound:

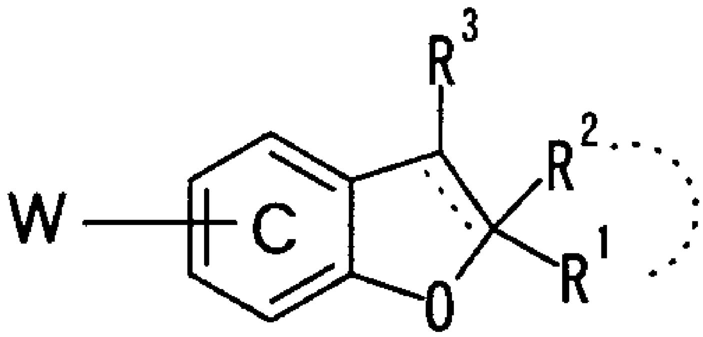

Patent Literature 8 discloses the following compound:

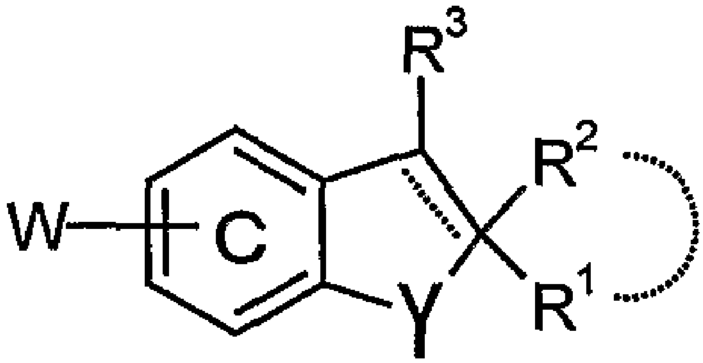

Patent Literature 9 discloses the following compound:

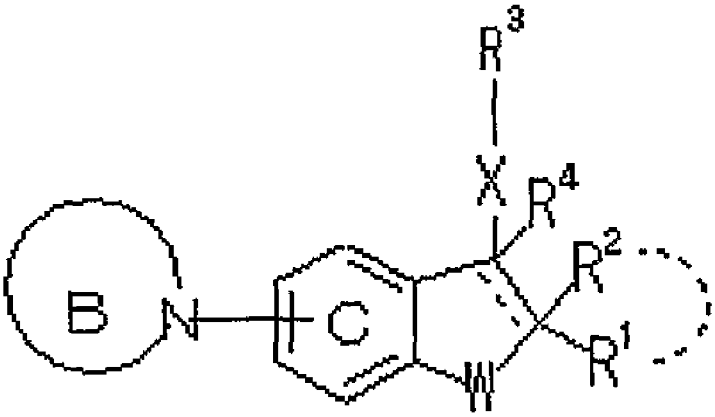

Patent Literature 10 discloses the following compound:

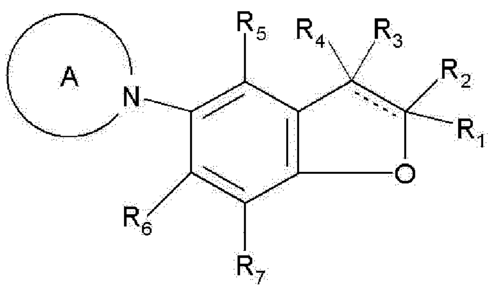

(I)

Non Patent Literature 11 discloses the following compound:

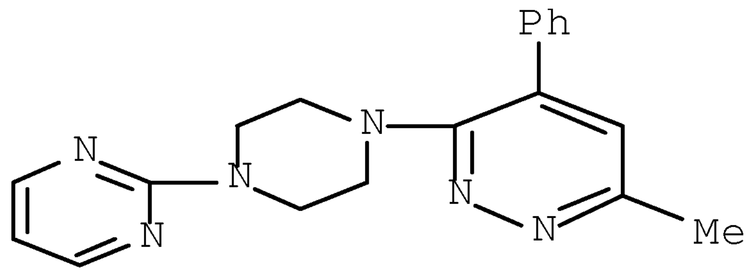

Citation List

Patent Literature

Patent Literature 1: International Publication No. W02006-008558 pumphlet
Patent Literature 2: International Publication No. W02008-099210 pumphlet
Patent Literature 3: International Publication No. W02006-036015 pumphlet
Patent Literature 4: U.S. Patent No. 5866562
Patent Literature 5: International Publication No. W02007-130383 pumphlet
Patent Literature 6: International Publication No. W02008-141020 pumphlet
Patent Literature 7: International Publication No. W02000-34262 pumphlet
Patent Literature 8: International Publication No. W02002-28850 pumphlet
Patent Literature 9: International Publication No. W02003-004485 pumphlet
Patent Literature 10: International Publication No. W02010-104194 pumphlet

Non Patent Literature

Non Patent Literature 1: Science, Vol. 245, p. 417-420, 1989
Non Patent Literature 2: Neurobiology of Aging, Vol. 13, p. 587-590, 1992
Non Patent Literature 3: Journal of Molecular Biology, Vol. 218, p. 149-163, 1991
Non Patent Literature 4: Cell, Vol. 91, p. 231-241, 1997
Non Patent Literature 5: J. Neurosci., Vol. 11, p. 2552-2563, 1991
Non Patent Literature 6: Science, Vol. 267, p. 2003-2006, 1995
Non Patent Literature 7: J. Biol. Chem., Vol. 272, p. 154-161, 1997
Non Patent Literature 8: J. Biol. Chem., Vol. 269, p. 3568-3573, 1994
Non Patent Literature 9: Endocrinology, Vol. 125, p. 314-320, 1989
Non Patent Literature 10: European Journal of Pharmacology, Vol. 490, p. 97-113, 2004
Non Patent Literature 11: Journal of Neuroscience, Vol. 26 (2), p. 662-670, 2006

Summary of Invention

Problem to be solved by the Invention

There is a demand for the provision of a medicament that is excellent in pharmaceutical effects such as a neuroprotective effect, a neuronal differentiation promoting effect, a neurogenesis or nerve regeneration promoting effect, and a cognitive function improving effect and, preferably, has improved safety, pharmacokinetics, etc.

Means for solving the Problem

The present inventors have conducted diligent studies in light of the problems described above and consequently completed the present invention by finding that an aminopyridine derivative, which is a compound represented by the formula (I) or a salt thereof [hereinafter, also abbreviated to a compound (I)], has an excellent neuroprotective effect, neuronal differentiation promoting effect, neurogenesis or nerve regeneration promoting effect, and cognitive function improving effect.

Specifically, the present invention provides:

[1] A compound represented by the formula (I):

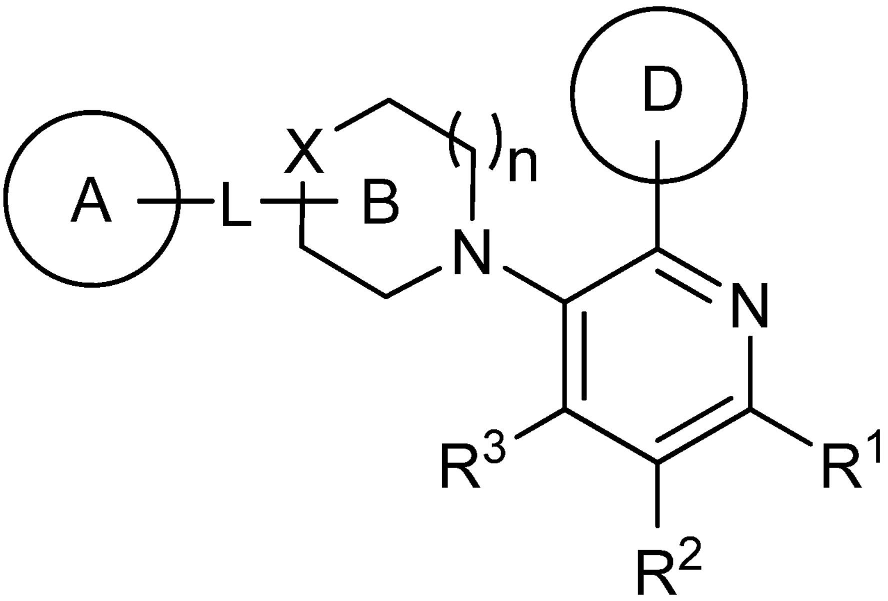

(I)

wherein

ring A represents a monocyclic aromatic hydrocarbon ring or a monocyclic aromatic heterocyclic ring, each of which may have a substituent(s)(s);
ring B may further have a substituent(s)(s);
ring D represents a monocyclic aromatic hydrocarbon ring or a monocyclic aromatic heterocyclic ring, each of which may have a substituent(s)(s);
L represents a bond, or a methylene group or an ethylene group, each of which may have a substituent(s)(s);
X represents -O-, -$NR^a$-, or a methylene group which may have a substituent(s), wherein $R^a$ represents a hydrogen atom or a substituent;
n represents 1 or 2; and
$R^1$ to $R^3$ are the same or different and each represent a hydrogen atom, a halogen atom, a cyano group, an acyl group, a $C_{1-6}$ alkyl group which may have a substituent(s), a $C_{1-6}$ alkoxy group which may have a substituent (s), a $C_{3-6}$ cycloalkyl group which may have a substituent(s), a $C_{3-6}$ cycloalkoxy group which may have a substituent(s), or an amino group which may have a substituent(s), wherein ring A and ring D each may be condensed with another ring to form a condensed ring which may have a substituent(s), and ring A-L- in the formula (I) may be bonded to X, provided that
when ring A is pyrazole which may be substituted and ring D is benzene which may be substituted, L is a bond;
or a salt thereof.

[2] The compound according to [1] or a salt thereof, wherein in the formula (I), the following substructural formula:

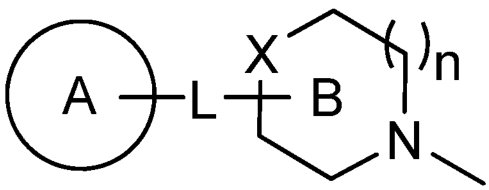

is

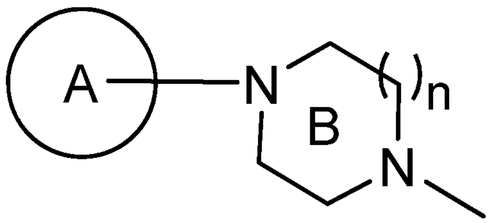

[3] The compound according to [2] or a salt thereof, wherein in the formula,
ring A is benzene having a substituent(s) or pyridine having a substituent(s);
ring B may further have a substituent(s); and n is 1 or 2.
[4] The compound according to [1] or a salt thereof, wherein in the formula,
ring A is benzene having a substituent(s) or pyridine having a substituent(s);
ring B may further have a substituent(s);
ring D is benzene having a substituent(s), pyrazole having a substituent(s), furan, pyridine having a substituent(s), or indazole having a substituent(s);
L is a bond or methylene;
X is

(1) -O-,
(2) -$NR^a$-, or
(3) methylene having a substituent(s), wherein

$R^a$ is a hydrogen atom or a substituent;
n is 1 or 2;
$R^1$ is a $C_{1-6}$ alkoxy group or a $C_{1-6}$ alkyl group having a substituent(s);
$R^2$ is a hydrogen atom; and
$R^3$ is a hydrogen atom or a $C_{1-6}$ alkyl group, wherein the substituent on ring D may be combined with ring D to form dihydrobenzofuran which may have a substituent(s), and ring A-L- in the formula (I) may be bonded to X.
[5] The compound according to [1] or a salt thereof, wherein in the formula,
ring A is benzene having a substituent(s) selected from a halogen atom, $C_{1-6}$ alkyl which may be halogenated, and $C_{1-6}$ alkoxy, or pyridine substituted by $C_{1-6}$ alkyl;
ring B may further have a substituent(s) selected from oxo and $C_{1-6}$ alkyl;
ring D is benzene having a substituent(s) selected from a halogen atom, $C_{1-6}$ alkyl, $C_{1-6}$ alkoxy which may be halogenated, morpholinyl, and di-$C_{1-6}$ alkylamino, pyrazole substituted by $C_{1-6}$ alkyl, furan, pyridine substituted by $C_{1-6}$ alkoxy, or indazole substituted by $C_{1-6}$ alkoxy;
L is a bond or methylene;
X is

(1) -O-,
(2) -$NR^a$-, or
(3) methylene bonded to ring A-L- in the formula (I) and further substituted by hydroxy, wherein

$R^a$ is a hydrogen atom, $C_{1-6}$ alkyl, $C_{1-6}$ alkoxycarbonyl, or ring A-L- in the formula (I);
n is 1 or 2;
$R^1$ is a $C_{1-6}$ alkoxy group or $C_{1-6}$ alkyl having hydroxy;
$R^2$ is a hydrogen atom; and
$R^3$ is a hydrogen atom or $C_{1-6}$ alkyl, wherein
the substituent on ring D may be combined with ring D to form dihydrobenzofuran substituted by $C_{1-6}$ alkyl.
[6] The compound according to [5] or a salt thereof, wherein in the formula,
ring A is benzene having a substituent(s) selected from a halogen atom, $C_{1-6}$ alkyl which may be halogenated, and $C_{1-6}$ alkoxy; and
ring D is benzene having a substituent(s) selected from a halogen atom, $C_{1-6}$ alkyl, $C_{1-6}$ alkoxy which may be halogenated, morpholinyl, and di-$C_{1-6}$ alkylamino.
[7] The compound according to [1] or a salt thereof, wherein in the formula,
ring A is benzene having a substituent(s) selected from a halogen atom, $C_{1-6}$ alkyl which may be halogenated, and $C_{1-6}$ alkoxy;

ring B may be further substituted by $C_{1-6}$ alkyl;
ring D is benzene having a substituent(s) selected from $C_{1-6}$ alkoxy and di-$C_{1-6}$ alkylamino;
L is a bond;
X is -O- or -$NR^a$-, wherein
$R^a$ is ring A-L- in the formula (I);
n is 1 or 2;
$R^1$ is a $C_{1-6}$ alkoxy group; and
both of $R^2$ and $R^3$ are a hydrogen atom.

[8] 4-{3-[4-(4-fluorophenyl)piperazin-1-yl]-6-methoxypyridin-2-yl}-N,N-dimethylaniline or a salt thereof.

[9] 4-{6-methoxy-3-[4-(4-methoxyphenyl)piperazin-1-yl]pyridin-2-yl}-N,N-dimethylaniline or a salt thereof.

[10] 4-{6-methoxy-3-[4-(4-methylphenyl)piperazin-1-yl]pyridin-2-yl}-N,N-dimethylaniline or a salt thereof.

[11] 1-[2-(4-ethoxyphenyl)-6-methoxypyridin-3-yl]-4-(4-methoxyphenyl)piperazine or a salt thereof.

[12] A medicament comprising a compound according to [1] or a salt thereof.

[13] The medicament according to [12], wherein the medicament is a neuronal differentiation promoter, an IGF-1 signal modulator, or a protein kinase B activator.

[14] A therapeutic or prophylactic agent for Alzheimer's disease comprising a compound represented by the formula ($I_0$) :

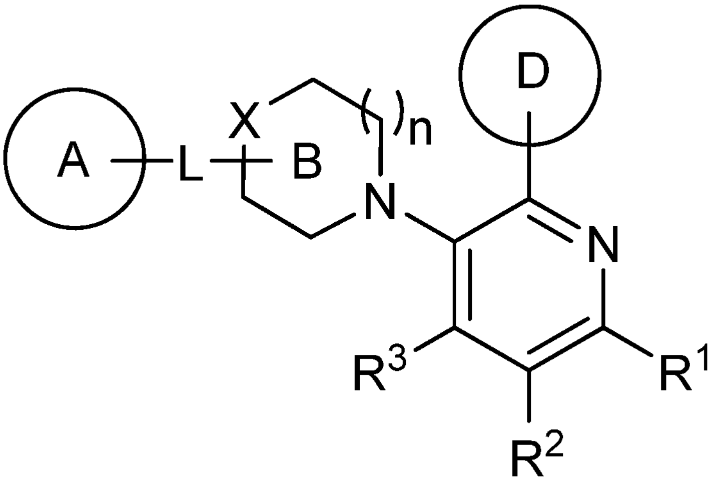

($I_0$)

wherein

ring A represents a monocyclic aromatic hydrocarbon ring or a monocyclic aromatic heterocyclic ring, each of which may have a substituent(s);
ring B may further have a substituent(s);
ring D represents a monocyclic aromatic hydrocarbon ring or a monocyclic aromatic heterocyclic ring, each of which may have a substituent(s);
L represents a bond, or a methylene group or an ethylene group, each of which may have a substituent(s);
X represents -O-, -$NR^a$-, or a methylene group which may have a substituent(s), wherein
$R^a$ represents a hydrogen atom or a substituent;
n represents 1 or 2; and
$R^1$ to $R^3$ are the same or different and each represent a hydrogen atom, a halogen atom, a cyano group, an acyl group, a $C_{1-6}$ alkyl group which may have a substituent(s), a $C_{1-6}$ alkoxy group which may have a substituent (s), a $C_{3-6}$ cycloalkyl group which may have a substituent(s), a $C_{3-6}$ cycloalkoxy group which may have a substituent(s), or an amino group which may have a substituent(s), wherein ring A and ring D each may be condensed with another ring to form a condensed ring which may have a substituent(s), and ring A-L- in the formula ($I_0$) may be bonded to X; or a salt thereof.

[15] The medicament according to [12], wherein the medicament is a therapeutic or prophylactic agent for Alzheimer's disease.

[16] A method for promoting neuronal differentiation, modulating an IGF-1 signal, or activating protein kinase B, comprising administering to a mammal an effective amount of a medicament according to [12].

[17] A method for preventing or treating Alzheimer's disease, comprising administering to a mammal an effective

amount of a compound represented by the formula ($I_0$) :

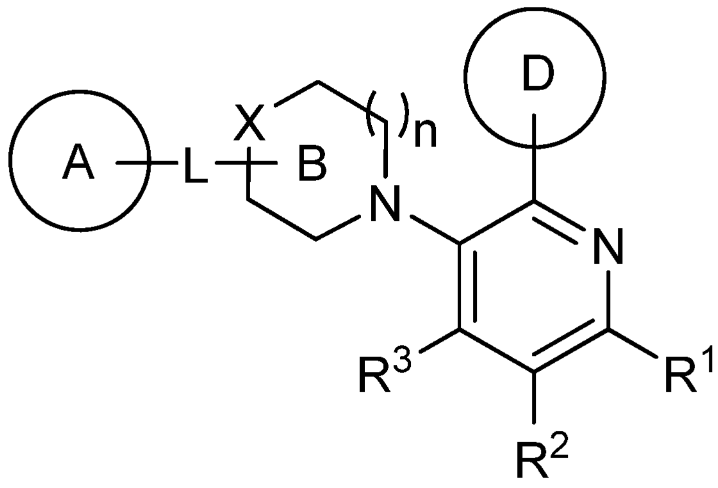

($I_0$)

wherein

ring A represents a monocyclic aromatic hydrocarbon ring or a monocyclic aromatic heterocyclic ring, each of which may have a substituent(s);
ring B may further have a substituent(s);
ring D represents a monocyclic aromatic hydrocarbon ring or a monocyclic aromatic heterocyclic ring, each of which may have a substituent(s);
L represents a bond, or a methylene group or an ethylene group, each of which may have a substituent(s);
X represents -O-, -$NR^a$-, or a methylene group which may have a substituent(s), wherein
$R^a$ represents a hydrogen atom or a substituent;
n represents 1 or 2; and
$R^1$ to $R^3$ are the same or different and each represent a hydrogen atom, a halogen atom, a cyano group, an acyl group, a $C_{1-6}$ alkyl group which may have a substituent(s), a $C_{1-6}$ alkoxy group which may have a substituent (s), a $C_{3-6}$ cycloalkyl group which may have a substituent(s), a $C_{3-6}$ cycloalkoxy group which may have a substituent(s), or an amino group which may have a substituent(s), wherein ring A and ring D each may be condensed with another ring to form a condensed ring which may have a substituent(s), and ring A-L- in the formula ($I_0$) may be bonded to X; or a salt thereof.

[18] A method for preventing or treating Alzheimer's disease, comprising administering to a mammal an effective amount of a compound according to [1] or a salt thereof.
[19] Use of a compound according to [1] or a salt thereof for manufacturing a neuronal differentiation promoter, an IGF-1 signal modulator, or a protein kinase B activator.
[20] Use of a compound represented by the formula ($I_0$):

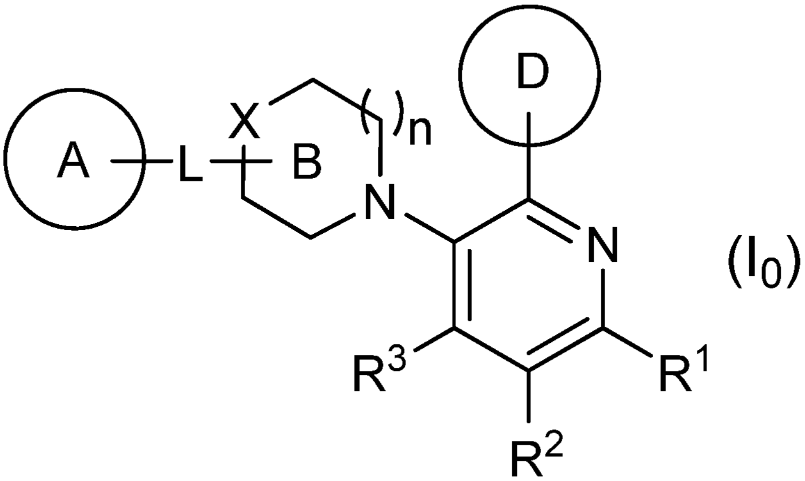

wherein

ring A represents a monocyclic aromatic hydrocarbon ring or a monocyclic aromatic heterocyclic ring, each of which may have a substituent(s);
ring B may further have a substituent(s);
ring D represents a monocyclic aromatic hydrocarbon ring or a monocyclic aromatic heterocyclic ring, each of which may have a substituent(s);
L represents a bond, or a methylene group or an ethylene group, each of which may have a substituent(s);
X represents -O-, $-NR^a-$, or a methylene group which may have a substituent(s), wherein
$R^a$ represents a hydrogen atom or a substituent;
n represents 1 or 2; and
$R^1$ to $R^3$ are the same or different and each represent a hydrogen atom, a halogen atom, a cyano group, an acyl group, a $C_{1-6}$ alkyl group which may have a substituent(s), a $C_{1-6}$ alkoxy group which may have a substituent (s), a $C_{3-6}$ cycloalkyl group which may have a substituent(s), a $C_{3-6}$ cycloalkoxy group which may have a substituent(s), or an amino group which may have a substituent(s), wherein ring A and ring D each may be condensed with another ring to form a condensed ring which may have a substituent(s), and ring A-L- in the formula ($I_0$) may be bonded to X; or a salt thereof for manufacturing a therapeutic or prophylactic agent for Alzheimer's disease.

[21] Use of a compound according to [1] or a salt thereof for manufacturing a therapeutic or prophylactic agent for Alzheimer's disease.
[22] A compound represented by the formula ($I_0$):

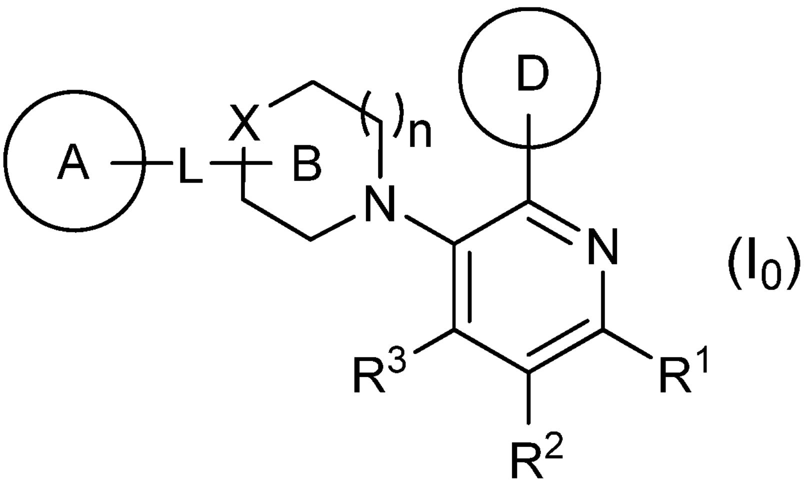

wherein

ring A represents a monocyclic aromatic hydrocarbon ring or a monocyclic aromatic heterocyclic ring, each of which may have a substituent(s);
ring B may further have a substituent(s);
ring D represents a monocyclic aromatic hydrocarbon ring or a monocyclic aromatic heterocyclic ring, each of which may have a substituent(s);
L represents a bond, or a methylene group or an ethylene group, each of which may have a substituent(s);
X represents -O-, -$NR^a$-, or a methylene group which may have a substituent(s), wherein
$R^a$ represents a hydrogen atom or a substituent;
n represents 1 or 2; and
$R^1$ to $R^3$ are the same or different and each represent a hydrogen atom, a halogen atom, a cyano group, an acyl group, a $C_{1-6}$ alkyl group which may have a substituent(s), a $C_{1-6}$ alkoxy group which may have a substituent (s), a $C_{3-6}$ cycloalkyl group which may have a substituent(s), a $C_{3-6}$ cycloalkoxy group which may have a substituent(s), or an amino group which may have a substituent(s), wherein ring A and ring D each may be condensed with another ring to form a condensed ring which may have a substituent(s), and ring A-L- in the formula ($I_0$) may be bonded to X; or a salt thereof for use in the prevention or treatment of Alzheimer's disease.

[23] The compound according to [1] or a salt thereof for use in the prevention or treatment of Alzheimer's disease.

Effects of Invention

A compound of the present invention or a salt thereof has an excellent neurogenesis promoting effect and neuronal regeneration promoting effect and is useful as, for example, an IGF-1 signal modulator, a protein kinase B activator, or an agent for the prevention or treatment of a central nervous system disease (e.g., Alzheimer's disease).

Preferred Embodiments

Hereinafter, the definitions of substituents in compounds of the formulas (I), (Ia), and ($I_0$) will be described.
In the present specification, examples of a "halogen atom" include fluorine, chlorine, bromine, and iodine.
In the present specification, examples of "$C_{1-6}$ alkyl" include methyl, ethyl, propyl, isopropyl, butyl, isobutyl, sec-butyl, tert-butyl, pentyl, and hexyl. $C_{1-3}$ alkyl is preferable, and methyl or ethyl is more preferable.
In the present specification, examples of a "substituent" in "$C_{1-6}$ alkyl which may have a substituent(s)" " represented by $R^1$ to $R^3$ include a substituent(s) selected from (1) to (62) shown below. The number of the substituent is, for example, 1 to 5, preferably 1 to 3.

(1) a halogen atom (e.g., fluorine, chlorine, bromine, and iodine),
(2) $C_{1-3}$ alkylenedioxy (e.g., methylenedioxy and ethylenedioxy),
(3) nitro,
(4) cyano,
(5) $C_{1-6}$ alkyl which may be halogenated (e.g., methyl, ethyl, propyl, isopropyl, butyl, isobutyl, sec-butyl, tert-butyl, pentyl, and hexyl which may have 1 to 5 (preferably 1 to 3) of fluorine, chlorine, bromine, and iodine atoms),
(6) $C_{2-6}$ alkenyl which may be halogenated (e.g., vinyl, propenyl, isopropenyl, 2-buten-1-yl, 4-penten-1-yl, and 5-hexen-1-yl which may have 1 to 5 (preferably 1 to 3) of fluorine, chlorine, bromine, and iodine atoms),
(7) carboxy-$C_{2-6}$ alkenyl (e.g., 2-carboxyethenyl and 2-carboxy-2-methylethenyl),
(8) $C_{2-6}$ alkynyl which may be halogenated (e.g., 2-butyn-1-yl, 4-pentyn-1-yl, and 5-hexyn-1-yl which may have 1 to 5 (preferably 1 to 3) of fluorine, chlorine, bromine, and iodine atoms),
(9) $C_{3-8}$ cycloalkyl which may be halogenated (e.g., cyclopropyl, cyclobutyl, cyclopentyl, cyclohexyl, cycloheptyl, and cyclooctyl which may have 1 to 5 (preferably 1 to 3) of fluorine, chlorine, bromine, and iodine atoms),
(10) $C_{6-14}$ aryl (e.g., phenyl, 1-naphthyl, 2-naphthyl, 2-biphenylyl, 3-biphenylyl, 4-biphenylyl, and 2-anthryl),
(11) $C_{1-6}$ alkoxy which may be halogenated (e.g., methoxy, ethoxy, propoxy, isopropoxy, butoxy, isobutoxy, sec-butoxy, tert-butoxy, pentoxy, and hexyloxy which may have 1 to 5 (preferably 1 to 3) of fluorine, chlorine, bromine, and iodine atoms),
(12) $C_{1-6}$ alkoxy-carbonyl-$C_{1-6}$ alkoxy (e.g., ethoxycarbonylmethyloxy),
(13) hydroxy,
(14) $C_{6-14}$ aryloxy (e.g., phenyloxy, 1-naphthyloxy, and 2-naphthyloxy),
(15) $C_{7-16}$ aralkyloxy (e.g., benzyloxy and phenethyloxy),
(16) mercapto,
(17) $C_{1-6}$ alkylthio which may be halogenated (e.g., methylthio, ethylthio, propylthio, isopropylthio, butylthio, isobutylthio, sec-butylthio, tert-butylthio, pentylthio, and hexylthio which may have 1 to 5 (preferably 1 to 3) of fluorine,

chlorine, bromine, and iodine atoms),
(18) $C_{6-14}$ arylthio (e.g., phenylthio, 1-naphthylthio, and 2-naphthylthio),
(19) $C_{7-16}$ aralkylthio (e.g., benzylthio and phenethylthio),
(20) amino which may have a substituent(s) (e.g., benzyl),
(21) mono-$C_{1-6}$ alkylamino (e.g., methylamino and ethylamino),
(22) mono-$C_{6-14}$ arylamino (e.g., phenylamino, 1-naphthylamino, and 2-naphthylamino),
(23) di-$C_{1-6}$ alkylamino (e.g., dimethylamino, diethylamino, and ethylmethylamino),
(24) di-$C_{6-14}$ arylamino (e.g., diphenylamino),
(25) formyl,
(26) carboxy,
(27) $C_{1-6}$ alkyl-carbonyl (e.g., acetyl and propionyl),
(28) $C_{3-8}$ cycloalkyl-carbonyl (e.g., cyclopropylcarbonyl, cyclopentylcarbonyl, cyclohexylcarbonyl, cycloheptylcarbonyl, and cyclooctylcarbonyl),
(29) $C_{1-6}$ alkoxy-carbonyl (e.g., methoxycarbonyl, ethoxycarbonyl, propoxycarbonyl, and tert-butoxycarbonyl),
(30) $C_{6-14}$ aryl-carbonyl (e.g., benzoyl, 1-naphthoyl, and 2-naphthoyl),
(31) $C_{7-16}$ aralkyl-carbonyl (e.g., phenylacetyl and 3-phenylpropionyl),
(32) $C_{6-14}$ aryloxy-carbonyl (e.g., phenoxycarbonyl),
(33) $C_{7-16}$ aralkyloxy-carbonyl (e.g., benzyloxycarbonyl and phenethyloxycarbonyl),
(34) 5- or 6-membered heterocyclic carbonyl (e.g., nicotinoyl, isonicotinoyl, thenoyl, furoyl, morpholinocarbonyl, thiomorpholinocarbonyl, piperazin-1-ylcarbonyl, and pyrrolidin-1-ylcarbonyl),
(35) carbamoyl,
(36) mono-$C_{1-6}$ alkyl-carbamoyl (e.g., methylcarbamoyl and ethylcarbamoyl),
(37) di-$C_{1-6}$ alkyl-carbamoyl (e.g., dimethylcarbamoyl, diethylcarbamoyl, and ethylmethylcarbamoyl),
(38) mono-$C_{6-14}$ aryl-carbamoyl (e.g., phenylcarbamoyl, 1-naphthylcarbamoyl, and 2-naphthylcarbamoyl),
(39) 5- or 6-membered heterocyclic carbamoyl (e.g., 2-pyridylcarbamoyl, 3-pyridylcarbamoyl, 4-pyridylcarbamoyl, 2-thienylcarbamoyl, and 3-thienylcarbamoyl),
(40) $C_{1-6}$ alkylsulfonyl (e.g., methylsulfonyl and ethylsulfonyl),
(41) $C_{6-14}$ arylsulfonyl (e.g., phenylsulfonyl, 1-naphthylsulfonyl, and 2-naphthylsulfonyl),
(42) formylamino,
(43) $C_{1-6}$ alkyl-carbonylamino (e.g., acetylamino),
(44) $C_{6-14}$ aryl-carbonylamino (e.g., benzoylamino and naphthoylamino),
(45) $C_{1-6}$ alkoxy-carbonylamino (e.g., methoxycarbonylamino, ethoxycarbonylamino, propoxycarbonylamino, and butoxycarbonylamino),
(46) $C_{1-6}$ alkylsulfonylamino (e.g., methylsulfonylamino and ethylsulfonylamino),
(47) $C_{6-14}$ arylsulfonylamino (e.g., phenylsulfonylamino, 2-naphthylsulfonylamino, and 1-naphthylsulfonylamino),
(48) $C_{1-6}$ alkyl-carbonyloxy (e.g., acetoxy and propionyloxy),
(49) $C_{6-14}$ aryl-carbonyloxy (e.g., benzoyloxy and naphthylcarbonyloxy),
(50) $C_{1-6}$ alkoxy-carbonyloxy (e.g., methoxycarbonyloxy, ethoxycarbonyloxy, propoxycarbonyloxy, and butoxycarbonyloxy),
(51) mono-$C_{1-6}$ alkyl-carbamoyloxy (e.g., methylcarbamoyloxy and ethylcarbamoyloxy),
(52) di-$C_{1-6}$ alkyl-carbamoyloxy (e.g., dimethylcarbamoyloxy and diethylcarbamoyloxy),
(53) mono-$C_{6-14}$ aryl-carbamoyloxy (e.g., phenylcarbamoyloxy and naphthylcarbamoyloxy),
(54) nicotinoyloxy,
(55) 5- to 7-membered saturated cyclic amino (e.g., piperidino and pyrrolidinyl which may be substituted by a substituent(s) (e.g., methyl, ethyl, and benzyl)),
(56) a 5- to 10-membered aromatic heterocyclic group (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, and 3-benzo[b]furanyl),
(57) sulfo,
(58) morpholino,
(59) thiomorpholino which may be oxygenated,
(60) pyrazolyl which may be substituted by a substituent(s) (e.g., methyl, ethyl, and benzyl),
(61) imidazolyl which may be substituted by a substituent(s) (e.g., methyl, ethyl, and benzyl), and
(62) a mono-spiro bicyclic group which may be substituted by a substituent(s) (e.g., methyl, ethyl, and benzyl).

Preferably, the substituent is a halogen atom, hydroxy, or the like.

In the present specification, examples of "$C_{1-6}$ alkoxy" specifically include methoxy, ethoxy, propoxy, isopropoxy,

butoxy, isobutoxy, sec-butoxy, pentyloxy, and hexyloxy). $C_{1-3}$ alkoxy is preferable.
In the present specification, examples of a "substituent" in "$C_{1-6}$ alkoxy which may have a substituent(s)" " represented by $R^1$ to $R^3$ include the same as the "substituent" in "$C_{1-6}$ alkyl which may have a substituent(s)". For example, 1 to 5, preferably 1 to 3 substituents may be substituted at possible positions. Preferably, the "substituent" is 1 to 3 halogen atoms.

In the present specification, examples of "$C_{3-6}$ cycloalkyl" specifically include cyclopropyl, cyclobutyl, cyclopentyl, and cyclohexyl. In the present specification, examples of a "substituent" in "$C_{3-6}$ cycloalkyl which may have a substituent(s)" represented by $R^1$ to $R^3$ include the same as the "substituent" in the "$C_{1-6}$ alkyl which may have a substituent(s)".
In the present specification, examples of "$C_{3-6}$ cycloalkoxy" specifically include cyclopropoxy, cyclobutoxy, cyclopentyloxy, and cyclohexyloxy. In the present specification, examples of a "substituent" in "$C_{3-6}$ cycloalkoxy which may have a substituent(s)" represented by $R^1$ to $R^3$ include the same as the "substituent" in the "$C_{1-6}$ alkyl which may have a substituent(s)". In the present specification, examples of "acyl" include formyl, carboxy, carbamoyl, $C_{1-6}$ alkyl-carbonyl (e.g., acetyl and propionyl), $C_{3-6}$ cycloalkyl-carbonyl (e.g., cyclopropylcarbonyl, cyclopentylcarbonyl, and cyclohexylcarbonyl), $C_{1-6}$ alkoxy-carbonyl (e.g., methoxycarbonyl, ethoxycarbonyl, propoxycarbonyl, and tert-butoxycarbonyl), $C_{6-14}$ aryl-carbonyl (e.g., benzoyl, 1-naphthoyl, and 2-naphthoyl), $C_{7-16}$ aralkyl-carbonyl (e.g., phenylacetyl and phenylpropionyl), $C_{6-14}$ aryloxy-carbonyl (e.g., phenoxycarbonyl), $C_{7-16}$ aralkyloxy-carbonyl (e.g., benzyloxycarbonyl and phenethyloxycarbonyl), 5- or 6-membered heterocyclic carbonyl (e.g., nicotinoyl, isonicotinoyl, 2-thenoyl, 3-thenoyl, 2-furoyl, 3-furoyl, morpholinocarbonyl, thiomorpholinocarbonyl, piperidinocarbonyl, and 1-pyrrolidinylcarbonyl), mono-$C_{1-6}$ alkyl-carbamoyl (e.g., methylcarbamoyl and ethylcarbamoyl), di-$C_{1-6}$ alkyl-carbamoyl (e.g., dimethylcarbamoyl, diethylcarbamoyl, and ethylmethylcarbamoyl), $C_{6-14}$ aryl-carbamoyl (e.g., phenylcarbamoyl, 1-naphthylcarbamoyl, and 2-naphthylcarbamoyl), thiocarbamoyl, 5- or 6-membered heterocyclic carbamoyl (e.g., 2-pyridylcarbamoyl, 3-pyridylcarbamoyl, 4-pyridylcarbamoyl, 2-thienylcarbamoyl, and 3-thienylcarbamoyl), $C_{1-6}$ alkylsulfonyl (e.g., methylsulfonyl and ethylsulfonyl), $C_{6-14}$ arylsulfonyl (e.g., phenylsulfonyl, 1-naphthylsulfonyl, and 2-naphthylsulfonyl), $C_{1-6}$ alkylsulfinyl (e.g., methylsulfinyl and ethylsulfinyl), and $C_{6-14}$ arylsulfinyl (e.g., phenylsulfinyl, 1-naphthylsulfinyl, and 2-naphthylsulfinyl).
Formyl, $C_{1-6}$ alkyl-carbonyl, $C_{3-6}$ cycloalkyl-carbonyl, $C_{6-14}$ aryl-carbonyl, $C_{7-16}$ aralkyl-carbonyl, 5- or 6- membered heterocyclic carbonyl, or the like is preferable.
Of them, acetyl or propionyl is particularly preferable.

In the present specification, examples of "amino which may have a substituent(s)" represented by $R^1$ to $R^3$ include amino, mono-$C_{1-6}$ alkylamino (e.g., methylamino and ethylamino), mono-$C_{6-14}$ arylamino (e.g., phenylamino, 1-naphthylamino, and 2-naphthylamino), mono-$C_{7-16}$ aralkylamino (e.g., benzylamino), di-$C_{1-6}$ alkylamino (e.g., dimethylamino and diethylamino), di-$C_{6-14}$ arylamino (e.g., diphenylamino), di-$C_{7-16}$ aralkylamino (e.g., dibenzylamino), and acylamino. Examples of the acylamino include formylamino, $C_{1-6}$ alkyl-carbonylamino (e.g., acetylamino), $C_{6-14}$ aryl-carbonylamino (e.g., phenylcarbonylamino and naphthylcarbonylamino), $C_{1-6}$ alkoxy-carbonylamino (e.g., methoxycarbonylamino, ethoxycarbonylamino, propoxycarbonylamino, and butoxycarbonylamino), $C_{1-6}$ alkylsulfonylamino (e.g., methylsulfonylamino and ethylsulfonylamino), and $C_{6-14}$ arylsulfonylamino (e.g., phenylsulfonylamino, 2-naphthylsulfonylamino, and 1-naphthylsulfonylamino). Amino, mono-$C_{1-6}$ alkylamino (e.g., methylamino and ethylamino), mono-$C_{6-14}$ arylamino (e.g., phenylamino, 1-naphthylamino, and 2-naphthylamino), di-$C_{1-6}$ alkylamino (e.g., dimethylamino and diethylamino), or di-$C_{6-14}$ arylamino (e.g., diphenylamino) is preferable.
Amino, mono-$C_{1-6}$ alkylamino, di-$C_{1-6}$ alkylamino, or the like is more preferable.

In the present specification, "methylene or ethylene which may have a substituent(s)" represented by L means a methylene group which may be mono- or di-substituted or ethylene which may be mono- to tetra-substituted by substituents selected from a halogen atom, cyano, hydroxy, oxo, $C_{1-6}$ alkyl which may be halogenated (e.g., methyl, ethyl, propyl, isopropyl, butyl, isobutyl, sec-butyl, tert-butyl, pentyl, and hexyl which may have 1 to 5 (preferably 1 to 3) of fluorine, chlorine, bromine, and iodine atoms), $C_{1-6}$ alkoxy which may be halogenated (e.g., methoxy, ethoxy, propoxy, isopropoxy, butoxy, isobutoxy, sec-butoxy, tert-butoxy, pentoxy, and hexyloxy which may have 1 to 5 (preferably 1 to 3) of fluorine, chlorine, bromine, and iodine atoms), amino which may be substituted by a substituent(s) (e.g., benzyl), mono-$C_{1-6}$ alkylamino (e.g., methylamino and ethylamino), di-$C_{1-6}$ alkylamino (e.g., dimethylamino, diethylamino, and ethylmethylamino), $C_{1-6}$ alkyl-carbonyl (e.g., acetyl and propionyl), $C_{1-6}$ alkyl which may be hydroxylated (e.g., methyl, ethyl, propyl, isopropyl, butyl, isobutyl, sec-butyl, tert-butyl, pentyl, and hexyl which may have 1 to 5 (preferably 1 to 3) -OH groups), $C_{1-6}$ alkyl which may be cyanated (e.g., methyl, ethyl, propyl, isopropyl, butyl, isobutyl, sec-butyl, tert-butyl, pentyl, and hexyl which may have 1 to 5 (preferably 1 to 3) -CN groups), etc.

In the present specification, $R^a$ in $-NR^a-$ is a hydrogen atom or a substituent(s). Examples of the substituent include cyano, hydroxy, $C_{1-6}$ alkyl which may be halogenated (e.g., methyl, ethyl, propyl, isopropyl, butyl, isobutyl, sec-butyl, tert-butyl, pentyl, and hexyl which may have 1 to 5 (preferably 1 to 3) of fluorine, chlorine, bromine, and iodine atoms), $C_{1-6}$ alkoxy which may be halogenated (e.g., methoxy, ethoxy, propoxy, isopropoxy, butoxy, isobutoxy, sec-butoxy, tert-butoxy, pentoxy, and hexyloxy which may have 1 to 5 (preferably 1 to 3) of fluorine, chlorine, bromine, and iodine atoms), amino which may be substituted by a substituent(s) (e.g., benzyl), mono-$C_{1-6}$ alkylamino (e.g., methylamino

and ethylamino), di-$C_{1-6}$ alkylamino (e.g., dimethylamino, diethylamino, and ethylmethylamino), $C_{1-6}$ alkyl-carbonyl (e.g., acetyl and propionyl), $C_{1-6}$ alkoxy-carbonyl (e.g., methoxycarbonyl, ethoxycarbonyl, propoxycarbonyl, and tert-butoxycarbonyl), $C_{1-6}$ alkyl which may be hydroxylated (e.g., methyl, ethyl, propyl, isopropyl, butyl, isobutyl, sec-butyl, tert-butyl, pentyl, and hexyl which may have 1 to 5 (preferably 1 to 3) -OH groups), and $C_{1-6}$ alkyl which may be cyanated (e.g., methyl, ethyl, propyl, isopropyl, butyl, isobutyl, sec-butyl, tert-butyl, pentyl, and hexyl which may have 1 to 5 (preferably 1 to 3) -CN groups). When X is $-NR^a-$ and is bonded to ring A-L- in the formula (I), $R^a$ is ring A-L- in the formula (I). $R^a$ is preferably ring A-L- in the formula (I), $C_{1-6}$ alkyl, $C_{1-6}$ alkoxy, $C_{1-6}$ alkyl-carbonyl, or $C_{1-6}$ alkoxy-carbonyl, or the like.

In the present specification, examples of "methylene which may have a substituent(s)" represented by X include a methylene group which may be mono- or di-substituted by substituents selected from a halogen atom, cyano, hydroxy, oxo, $C_{1-6}$ alkyl which may be halogenated (e.g., methyl, ethyl, propyl, isopropyl, butyl, isobutyl, sec-butyl, tert-butyl, pentyl, and hexyl which may have 1 to 5 (preferably 1 to 3) of fluorine, chlorine, bromine, and iodine atoms), $C_{1-6}$ alkoxy which may be halogenated (e.g., methoxy, ethoxy, propoxy, isopropoxy, butoxy, isobutoxy, sec-butoxy, tert-butoxy, pentoxy, and hexyloxy which may have 1 to 5 (preferably 1 to 3) of fluorine, chlorine, bromine, and iodine atoms), amino which may be substituted by a substituent(s) (e.g., benzyl), mono-$C_{1-6}$ alkylamino (e.g., methylamino and ethylamino), di-$C_{1-6}$ alkylamino (e.g., dimethylamino, diethylamino, and ethylmethylamino), $C_{1-6}$ alkyl-carbonyl (e.g., acetyl and propionyl), $C_{1-6}$ alkyl which may be hydroxylated (e.g., methyl, ethyl, propyl, isopropyl, butyl, isobutyl, sec-butyl, tert-butyl, pentyl, and hexyl which may have 1 to 5 (preferably 1 to 3) -OH groups), $C_{1-6}$ alkyl which may be cyanated (e.g., methyl, ethyl, propyl, isopropyl, butyl, isobutyl, sec-butyl, tert-butyl, pentyl, and hexyl which may have 1 to 5 (preferably 1 to 3) -CN groups), etc. Alternatively, only ring A-L- in the formula (I) or the ring A-L- together with a substituent(s) selected from those described above may be bonded to the methylene group.

Methylene bonded to ring A-L- in the formula (I) or methylene which may be substituted by oxo, hydroxy, or $C_{1-6}$ alkyl, or the like is preferable.

In the present specification, examples of a "monocyclic aromatic hydrocarbon ring" include 6-membered monocyclic aromatic hydrocarbon rings. Particularly, benzene is preferable.

In the present specification, examples of a "monocyclic aromatic heterocyclic ring" include 5- or 6- membered monocyclic aromatic heterocyclic rings containing one or more (e.g., 1 to 4) heteroatoms selected from a nitrogen atom, a sulfur atom, and an oxygen atom, in addition to carbon atoms.

Specific examples thereof include 5- to 6-membered monocyclic aromatic heterocyclic rings such as furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazan, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, and triazine. Furan, thiophene, thiazole, imidazole, pyrazole, pyridine, pyrimidine, or the like is preferable.

In the present specification, examples of a "substituent" in a "monocyclic aromatic hydrocarbon ring or a monocyclic aromatic heterocyclic ring, each of which may have a substituent(s)" represented by ring A and ring D include a substituent(s) selected from a halogen atom, cyano, hydroxy, oxo, $C_{1-6}$ alkyl which may be halogenated (e.g., methyl, ethyl, propyl, isopropyl, butyl, isobutyl, sec-butyl, tert-butyl, pentyl, and hexyl which may have 1 to 5 (preferably 1 to 3) of fluorine, chlorine, bromine, and iodine atoms), $C_{1-6}$ alkoxy which may be halogenated (e.g., methoxy, ethoxy, propoxy, isopropoxy, butoxy, isobutoxy, sec-butoxy, tert-butoxy, pentoxy, and hexyloxy which may have 1 to 5 (preferably 1 to 3) of fluorine, chlorine, bromine, and iodine atoms), amino which may be substituted by a substituent(s) (e.g., benzyl), mono-$C_{1-6}$ alkylamino (e.g., methylamino and ethylamino), di-$C_{1-6}$ alkylamino (e.g., dimethylamino, diethylamino, and ethylmethylamino), $C_{1-6}$ alkyl-carbonyl (e.g., acetyl and propionyl), $C_{1-6}$ alkyl which may be hydroxylated (e.g., methyl, ethyl, propyl, isopropyl, butyl, isobutyl, sec-butyl, tert-butyl, pentyl, and hexyl which may have 1 to 5 (preferably 1 to 3) -OH groups), $C_{1-6}$ alkyl which may be cyanated (e.g., methyl, ethyl, propyl, isopropyl, butyl, isobutyl, sec-butyl, tert-butyl, pentyl, and hexyl which may have 1 to 5 (preferably 1 to 3) -CN groups), etc. For example, 1 to 5, preferably 1 to 3 substituents may be substituted at possible positions.

A halogen atom, $C_{1-6}$ alkyl which may be substituted by halogen, hydroxy, or cyano, $C_{1-6}$ alkoxy which may be halogenated, mono-$C_{1-6}$ alkylamino, di-$C_{1-6}$ alkylamino, $C_{1-6}$ alkyl-carbonyl, or the like is preferable.

In this context, ring A and ring D each may be "condensed with another ring to form a condensed ring which may have a substituent(s)". The phrase "condensed with another ring" means that ring A and ring D are each further condensed with a carbocyclic ring or a heterocyclic ring at a possible position. Specifically, when each of ring A and ring D is a "monocyclic aromatic hydrocarbon ring" group, these rings form a "condensed ring" group in which a "carbocyclic ring is condensed with the monocyclic aromatic hydrocarbon ring group" or a "condensed ring" group in which a "heterocyclic ring is condensed with the monocyclic aromatic hydrocarbon ring group". When each of ring A and ring D is a "monocyclic aromatic heterocyclic ring" group, these rings form a "condensed ring" group in which a "carbocyclic ring is condensed with the monocyclic aromatic heterocyclic ring group" or a "condensed ring" group in which a "heterocyclic ring is condensed with the monocyclic aromatic heterocyclic ring group".

Examples of the "carbocyclic ring" include the monocyclic aromatic hydrocarbon rings exemplified above and 5- to 7-membered monocyclic saturated or unsaturated hydrocarbon rings. Also, examples of the "heterocyclic ring"

include the monocyclic aromatic heterocyclic rings exemplified above and 5- to 7-membered monocyclic saturated or unsaturated heterocyclic rings.

Examples of the "condensed ring" in which a "carbocyclic ring is condensed with the monocyclic aromatic hydrocarbon ring group" include indane, indene, naphthalene, dihydronaphthalene, 1,2,3,4-tetrahydronaphthalene, benzo[7]annulene, 6,7-dihydrobenzo[7]annulene, 6,9-dihydrobenzo[7]annulene, and 6,7,8,9-tetrahydrobenzo[7]annulene.

Examples of the "condensed ring" group in which a "carbocyclic ring is condensed with the monocyclic aromatic hydrocarbon ring group" specifically include indan-4-yl, indan-5-yl, 1H-inden-4-yl, 1H-inden-5-yl, naphthalen-1-yl, naphthalen-2-yl, 1,4-dihydronaphthalen-1-yl, 1,4-dihydronaphthalen-2-yl, 1,2-dihydronaphthalen-1-yl, 1,2-dihydronaphthalen-2-yl, 1,2-dihydronaphthalen-3-yl, 1,2-dihydronaphthalen-4-yl, 1,2,3,4-tetrahydronaphthalen-1-yl, 1,2,3,4-tetrahydronaphthalen-2-yl, 7H-benzo[7]annulen-1-yl, 7H-benzo[7]annulen-2-yl, 6,7-dihydro-5H-benzo[7]annulen-1-yl, 6,7-dihydro-5H-benzo[7]annulen-2-yl, 6,7-dihydro-5H-benzo[7]annulen-3-yl, 6,7-dihydro-5H-benzo[7]annulen-4-yl, 6,9-dihydro-5H-benzo[7]annulen-1-yl, 6,9-dihydro-5H-benzo[7]annulen-2-yl, 6,9-dihydro-5H-benzo[7]annulen-3-yl, 6,9-dihydro-5H-benzo[7]annulen-4-yl, 6,7,8,9-tetrahydro-5H-benzo[7]annulen-1-yl, and 6,7,8,9-tetrahydro-5H-benzo[7]annulen-2-yl.

Examples of the "condensed ring" in which a "heterocyclic ring is condensed with the monocyclic aromatic hydrocarbon ring group" include benzofuran, isobenzofuran, 2,3-dihydrobenzofuran, benzo[b]thiophene, indole, indoline, isoindole, isoindoline, indazole, indazoline, benzimidazole, benzoxazole, benzisoxazole, benzothiazole, benzisothiazole, benzotriazole, benzopyran, quinoline, isoquinoline, cinnoline, quinazoline, quinoxaline, and phthalazine.

Examples of the "condensed ring" group in which a "heterocyclic ring is condensed with the monocyclic aromatic hydrocarbon ring group" specifically include benzofuran-4-yl, benzofuran-5-yl, benzofuran-6-yl, benzofuran-7-yl, isobenzofuran-4-yl, isobenzofuran-5-yl, isobenzofuran-6-yl, isobenzofuran-7-yl, 2,3-dihydrobenzofuran-4-yl, 2,3-dihydrobenzofuran-5-yl, 2,3-dihydrobenzofuran-6-yl, 2,3-dihydrobenzofuran-7-yl, benzo[b]thiophen-4-yl, benzo[b]thiophen-5-yl, benzo[b]thiophen-6-yl, benzo[b]thiophen-7-yl, indol-4-yl, indol-5-yl, indol-6-yl, indol-7-yl, indolin-4-yl, indolin-5-yl, indolin-6-yl, indolin-7-yl, isoindol-4-yl, isoindol-5-yl, isoindol-6-yl, isoindol-7-yl, isoindolin-4-yl, isoindolin-5-yl, isoindolin-6-yl, isoindolin-7-yl, 1H-indazol-4-yl, 1H-indazol-5-yl, 1H-indazol-6-yl, 1H-indazol-7-yl, 1H-indazolin-4-yl, 1H-indazolin-5-yl, 1H-indazolin-6-yl, 1H-indazolin-7-yl, benzimidazol-4-yl, benzimidazol-5-yl, benzimidazol-6-yl, benzimidazol-7-yl, benzoxazol-4-yl, benzoxazol-5-yl, benzoxazol-6-yl, benzoxazol-7-yl, benzisoxazol-4-yl, benzisoxazol-5-yl, benzisoxazol-6-yl, benzisoxazol-7-yl, benzothiazol-4-yl, benzothiazol-5-yl, benzothiazol-6-yl, benzothiazol-7-yl, benzisothiazol-4-yl, benzisothiazol-5-yl, benzisothiazol-6-yl, benzisothiazol-7-yl, 1H-benzotriazol-4-yl, 1H-benzotriazol-5-yl, 1H-benzotriazol-6-yl, 1H-benzotriazol-7-yl, benzopyran-5-yl, benzopyran-6-yl, benzopyran-7-yl, benzopyran-8-yl, quinolin-5-yl, quinolin-6-yl, quinolin-7-yl, quinolin-8-yl, isoquinolin-5-yl, isoquinolin-6-yl, isoquinolin-7-yl, isoquinolin-8-yl, cinnolin-5-yl, cinnolin-6-yl, cinnolin-7-yl, cinnolin-8-yl, quinazolin-5-yl, quinazolin-6-yl, quinazolin-7-yl, quinazolin-8-yl, quinoxalin-5-yl, quinoxalin-6-yl, quinoxalin-7-yl, quinoxalin-8-yl, phthalazin-5-yl, phthalazin-6-yl, phthalazin-7-yl, and phthalazin-8-yl. Of them, benzofuran-4-yl, benzofuran-5-yl, benzofuran-6-yl, benzofuran-7-yl, 2,3-dihydrobenzofuran-4-yl, 2,3-dihydrobenzofuran-5-yl, 2,3-dihydrobenzofuran-6-yl, 2,3-dihydrobenzofuran-7-yl, benzo[b]thiophen-4-yl, benzo[b]thiophen-5-yl, benzo[b]thiophen-6-yl, benzo[b]thiophen-7-yl, benzimidazol-4-yl, benzimidazol-5-yl, benzimidazol-6-yl, benzimidazol-7-yl, indol-4-yl, indol-5-yl, indol-6-yl, indol-7-yl, indolin-4-yl, indolin-5-yl, indolin-6-yl, indolin-7-yl, or the like is particularly preferable.

Examples of the "condensed ring" in which a "carbocyclic ring is condensed with the monocyclic aromatic heterocyclic ring group" include benzofuran, isobenzofuran, benzo[b]thiophene, indole, isoindole, indazole, benzimidazole, benzoxazole, benzisoxazole, benzothiazole, benzisothiazole, benzotriazole, quinoline, isoquinoline, cinnoline, quinazoline, quinoxaline, phthalazine, 4,5,6,7-tetrahydrobenzofuran, 4,5,6,7-tetrahydroisobenzofuran, 4,5,6,7-tetrahydrobenzo[b]thiophene, 4,5,6,7-tetrahydroindole, 4,5,6,7-tetrahydroisoindole, 4,5,6,7-tetrahydroindazole, 4,5,6,7-tetrahydrobenzimidazole, 4,5,6,7-tetrahydrobenzoxazole, 4,5,6,7-tetrahydrobenzisoxazole, 4,5,6,7-tetrahydrobenzothiazole, 4,5,6,7-tetrahydrobenzisothiazole, 4,5,6,7-tetrahydrobenzotriazole, 5,6,7,8-tetrahydroquinoline, 5,6,7,8-tetrahydroisoquinoline, 5,6,7,8-tetrahydrocinnoline, 5,6,7,8-tetrahydroquinazoline, 5,6,7,8-tetrahydrophthalazine, 4,5-dihydrobenzofuran, 4,5-dihydroisobenzofuran, 4,5-dihydrobenzo[b]thiophene, 4,5-dihydroindole, 4,5-dihydroisoindole, 4,5-dihydroindazole, 4,5-dihydrobenzimidazole, 4,5-dihydrobenzoxazole, 4,5-dihydrobenzisoxazole, 4,5-dihydrobenzothiazole, 4,5-dihydrobenzothiazole, 4,5-dihydrobenzotriazole, 7,8-dihydroquinoline, 7,8-dihydroisoquinoline, 7,8-dihydrocinnoline, 7,8-dihydroquinazoline, 7,8-dihydroquinoxaline, and 7,8-dihydrophthalazine.

Examples of the "condensed ring" group in which a "carbocyclic ring is condensed with the monocyclic aromatic heterocyclic ring group" specifically include benzofuran-2-yl, benzofuran-3-yl, isobenzofuran-1-yl, isobenzofuran-3-yl, benzo[b]thiophen-2-yl, benzo[b]thiophen-3-yl, indol-1-yl, indol-2-yl, indol-3-yl, isoindol-1-yl, isoindol-2-yl, isoindol-3-yl, 1H-indazol-1-yl, 1H-indazol-3-yl, 1H-benzimidazol-1-yl, 1H-benzimidazol-2-yl, benzoxazol-2-yl, benzisoxazol-3-yl, benzothiazol-2-yl, benzothiazol-3-yl, benzisothiazol-3-yl, 1H-benzotriazol-1-yl, quinolin-2-yl, quinolin-3-yl, quinolin-4-yl, isoquinolin-1-yl, isoquinolin-3-yl, isoquinolin-4-yl, cinnolin-3-yl, cinnolin-4-yl, quinazolin-2-yl, quinazolin-4-yl, quinoxalin-2-yl, quinoxalin-3-yl, phthalazin-1-yl, phthalazin-4-yl, 4,5,6,7-tetrahydrobenzofuran-2-yl, 4,5,6,7-tetrahydrobenzofuran-3-yl, 4,5,6,7-tetrahydroisobenzofuran-1-yl, 4,5,6,7-tetrahydroisobenzofuran-3-yl, 4,5,6,7-tetrahydrobenzo[b]thiophen-2-yl, 4,5,6,7-tetrahydrobenzo[b]thiophen-3-yl, 4,5,6,7-tetrahydroindol-1-yl, 4,5,6,7-tetrahydroindol-2-yl, 4,5,6,7-tetrahydroindol-3-yl, 4,5,6,7-tetrahydroisoindol-1-yl, 4,5,6,7-tetrahydroisoindol-2-yl, 4,5,6,7-tetrahydroisoindol-3-yl, 4,5,6,7-tetrahydro-1H-indazol-1-yl, 4,5,6,7-tetrahydro-1H-indazol-3-yl, 4,5,6,7-tetrahydro-1H-benzimidazol-1-yl, 4,5,6,7-tetrahydro-1H-benzimidazol-2-yl, 4,5,6,7-tetrahydrobenzoxazol-2-yl, 4,5,6,7-tetrahydrobenzisoxazol-3-yl, 4,5,6,7-tetrahydrobenzothiazol-2-yl, 4,5,6,7-tetrahydrobenzothiazol-3-yl, 4,5,6,7-tetrahydrobenzisothiazol-3-yl, 4,5,6,7-tetrahydro-1H-benzotriazol-1-yl, 5,6,7,8-tetrahydroquinolin-2-yl, 5,6,7,8-tetrahydroquinolin-3-yl, 5,6,7,8-tetrahydroquinolin-4-yl, 5,6,7,8-tetrahydroisoquinolin-1-yl, 5,6,7,8-tetrahydroisoquinolin-3-yl, 5,6,7,8-tetrahydroisoquinolin-4-yl, 5,6,7,8-tetrahydrocinnolin-3-yl, 5,6,7,8-tetrahydrocinnolin-4-yl, 5,6,7,8-tetrahydroquinazolin-2-yl, 5,6,7,8-tetrahydroquinazolin-4-yl, 5,6,7,8-tetrahydroquinoxalin-2-yl, 5,6,7,8-tetrahydroquinoxalin-3-yl, 5,6,7,8-tetrahydrophthalazin-1-yl, 5,6,7,8-tetrahydrophthalazin-4-yl, 4,5-dihydrobenzofuran-2-yl, 4,5-dihydrobenzofuran-3-yl, 4,5-dihydroisobenzofuran-1-yl, 4,5-dihydroisobenzofuran-3-yl, 4,5-dihydrobenzo[b]thiophen-2-yl, 4,5-dihydrobenzo[b]thiophen-3-yl, 4,5-dihydroindol-1-yl, 4,5-dihydroindol-2-yl, 4,5-dihydroindol-3-yl, 4,5-dihydroisoindol-1-yl, 4,5-dihydroisoindol-2-yl, 4,5-dihydroisoindol-3-yl, 4,5-dihydro-1H-indazol-1-yl, 4,5-dihydro-1H-indazol-3-yl, 4,5-dihydro-1H-benzimidazol-1-yl, 4,5-dihydro-1H-benzimidazol-2-yl, 4,5-dihydrobenzoxazol-2-yl, 4,5-dihydrobenzisoxazol-3-yl, 4,5-dihydrobenzothiazol-2-yl, 4,5-dihydrobenzothiazol-3-yl, 4,5-dihydrobenzisothiazol-3-yl, 4,5-dihydro-1H-benzotriazol-1-yl, 7,8-dihydroquinolin-2-yl, 7,8-dihydroquinolin-3-yl, 7,8-dihydroquinolin-4-yl, 7,8-dihydroisoquinolin-1-yl, 7,8-dihydroisoquinolin-3-yl, 7,8-dihydroisoquinolin-4-yl, 7,8-dihydrocinnolin-3-yl, 7,8-dihydrocinnolin-4-yl, 7,8-dihydroquinazolin-2-yl, 7,8-dihydroquinazolin-4-yl, 7,8-dihydroquinoxalin-2-yl, 7,8-dihydroquinoxalin-3-yl, 7,8-dihydrophthalazin-1-yl, and 7,8-dihydrophthalazin-4-yl. Of them, benzofuran-2-yl, benzofuran-3-yl, benzo[b]thiophen-2-yl, benzo[b]thiophen-3-yl, indol-1-yl, indol-2-yl, indol-3-yl, 1H-benzimidazol-1-yl, 1H-benzimidazol-2-yl, or the like is particularly preferable.

Examples of the "condensed ring" group in which a "heterocyclic ring is condensed with the monocyclic aromatic heterocyclic ring group" include purine, pteridine, indolizine, pyrrolo[1,2-b]pyridazine, pyrazolo[1,5-a]pyridine, imidazo[1,2-a]pyridine, imidazo[1,5-a]pyridine, imidazo[1,2-b]pyridazine, imidazo[1,2-a]pyrimidine, [1,2,4]triazolo[4,3-a]pyridine, and [1,2,4]triazolo[4,3-b]pyridazine.

Examples of the "condensed ring" group in which a "heterocyclic ring is condensed with the monocyclic aromatic heterocyclic ring group" specifically include 8-to 11-membered condensed bicyclic aromatic heterocyclic group such as purin-2-yl, purin-6-yl, purin-7-yl, purin-8-yl, pteridin-2-yl, pteridin-4-yl, pteridin-6-yl, pteridin-7-yl, indolizin-1-yl, indolizin-2-yl, indolizin-3-yl, indolizin-5-yl, indolizin-6-yl, indolizin-7-yl, indolizin-8-yl, pyrrolo[1,2-b]pyridazin-2-yl, pyrrolo[1,2-b]pyridazin-3-yl, pyrrolo[1,2-b]pyridazin-5-yl, pyrrolo[1,2-b]pyridazin-6-yl, pyrrolo[1,2-b]pyridazin-7-yl, pyrazolo[1,5-a]pyridin-2-yl, pyrazolo[1,5-a]pyridin-3-yl, pyrazolo[1,5-a]pyridin-4-yl, pyrazolo[1,5-a]pyridin-5-yl, pyrazolo[1,5-a]pyridin-6-yl, pyrazolo[1,5-a]pyridin-7-yl, imidazo[1,2-a]pyridin-2-yl, imidazo[1,2-a]pyridin-3-yl, imidazo[1,2-a]pyridin-5-yl, imidazo[1,2-a]pyridin-6-yl, imidazo[1,2-a]pyridin-7-yl, imidazo[1,5-a]pyridin-1-yl, imidazo[1,5-a]pyridin-3-yl, imidazo[1,5-a]pyridin-5-yl, imidazo[1,5-a]pyridin-6-yl, imidazo[1,5-a]pyridin-7-yl, imidazo[1,5-a]pyridin-8-yl, imidazo[1,2-b]pyridazin-2-yl, imidazo[1,2-b]pyridazin-3-yl, imidazo[1,2-b]pyridazin-6-yl, imidazo[1,2-b]pyridazin-7-yl, imidazo[1,2-b]pyridazin-8-yl, imidazo[1,2-a]pyrimidin-1-yl, imidazo[1,2-a]pyrimidin-3-yl, imidazo[1,2-a]pyrimidin-5-yl, imidazo[1,2-a]pyrimidin-6-yl, imidazo[1,2-a]pyrimidin-7-yl, [1,2,4]triazolo[4,3-a]pyridin-3-yl, [1,2,4]triazolo[4,3-a]pyridin-5-yl, [1,2,4]triazolo[4,3-a]pyridin-6-yl, [1,2,4]triazolo[4,3-a]pyridin-7-yl, [1,2,4]triazolo[4,3-a]pyridin-8-yl, [1,2,4]triazolo[4,3-b]pyridazin-3-yl, [1,2,4]triazolo[4,3-b]pyridazin-6-yl, [1,2,4]triazolo[4,3-b]pyridazin-7-yl, and [1,2,4]triazolo[4,3-b]pyridazin-8-yl. Of them, imidazo[1,2-a]pyridin-2-yl, imidazo[1,2-a]pyridin-3-yl, imidazo[1,2-a]pyridin-5-yl, imidazo[1,2-a]pyridin-6-yl, imidazo[1,2-a]pyridin-7-yl, or the like is particularly preferable.

The "condensed ring" group in which a "carbocyclic ring is condensed with the monocyclic aromatic hydrocarbon ring group", the "condensed ring" group in which a "heterocyclic ring is condensed with the monocyclic aromatic hydrocarbon ring group", the "condensed ring" group in which a "carbocyclic ring is condensed with the monocyclic aromatic heterocyclic ring", or the "condensed ring" group in which a "heterocyclic ring is condensed with the monocyclic aromatic heterocyclic ring group" may be substituted by the same substituent as the "substituent" in the "monocyclic aromatic hydrocarbon ring or the monocyclic aromatic heterocyclic ring, each of which may have a substituent(s)". For example, 1 to 5, preferably 1 to 3 substituents may be substituted at possible positions.

In the present specification, the phrase "may further have a substituent(s)" in ring B means that in the formula (I), ring B may further have a substituent(s) at any possible position except for: the nitrogen atom bonded to the substituted pyridine group; the atom bonded at an arbitrary position to ring A-L-; and the moiety X. Examples of such a "substituent" include the same as the "substituent" in the "methylene which may have a substituent(s)". Ring B may have, for example, 1 to 5, preferably 1 to 3 substituents at possible positions.

The "substituent" is preferably hydroxy, oxo, $C_{1-6}$ alkyl (e.g., methyl, ethyl, propyl, isopropyl, butyl, isobutyl, sec-butyl, tert-butyl, pentyl, and hexyl), $C_{1-6}$ alkyl-carbonyl (e.g., acetyl and propionyl), or $C_{1-6}$ alkoxy-carbonyl (e.g., methoxycarbonyl, ethoxycarbonyl, propoxycarbonyl, and tert-butoxycarbonyl).

In the compound (I), ring A represents a monocyclic aromatic hydrocarbon ring or a monocyclic aromatic heterocyclic ring, each of which may have a substituent(s). Ring A is preferably a 6-membered monocyclic aromatic hydrocarbon ring which may have a substituent(s), or a 5 or 6-membered monocyclic aromatic heterocyclic ring which may have a substituent(s). Specifically, benzene, furan, thiophene, thiazole, pyrazole, imidazole, pyridine, or pyrimidine, each of which may have a substituent(s) is preferable. Ring A is particularly preferably benzene, thiazole, or pyridine,

each of which may have a substituent(s).

In the compound (I), ring B may further have a substituent(s). The substituent that may be further carried by ring B is preferably oxo, $C_{1-6}$ alkyl, or the like, particularly preferably oxo or methyl.

In the compound (I), ring D represents a monocyclic aromatic hydrocarbon ring or a monocyclic aromatic heterocyclic ring, each of which may have a substituent(s). Ring D is preferably a 6-membered monocyclic aromatic hydrocarbon ring which may have a substituent(s), or a 5 or 6-membered monocyclic aromatic heterocyclic ring which may have a substituent(s). Specifically, benzene, furan, thiophene, thiazole, pyrazole, imidazole, pyridine, or pyrimidine, each of which may have a substituent(s) is preferable. Ring D is particularly preferably benzene which may have a substituent(s).

In the compound (I), L represents a bond, or a methylene group or an ethylene group, each of which may have a substituent(s). L is preferably a bond, a methylene group, or an ethylene group.

In the compound (I), X represents -O-, -$NR^a$- ($R^a$ represents a hydrogen atom or a substituent), or a methylene group which may have a substituent(s). In the compound (I), ring A-L- is bonded to a replaceable carbon atom or the nitrogen atom on ring B. Specifically, when X means -O-, ring A-L- is bonded to a replaceable carbon atom on ring B. When X is a moiety other than -O-, ring A-L- in the formula (I) may be bonded to a replaceable carbon atom or X on ring B. Specifically, X is preferably (1) -O-, (2) -$NR^a$- ($R^a$ is a hydrogen atom, $C_{1-6}$ alkyl, $C_{1-6}$ alkoxy-carbonyl, or ring A-L- in the formula (I)), or (3) a methylene group which may have a substituent(s) selected from oxo and hydroxy or a methylene group which may be bonded to ring A-L- in the formula (I) and further substituted by hydroxy. X is particularly preferably (1) -O- or (2) - $NR^a$- ($R^a$ is a hydrogen atom, $C_{1-6}$ alkyl, $C_{1-6}$ alkoxy-carbonyl, or ring A-L- in the formula (I)).

In the compound (I), the following substructural formula:

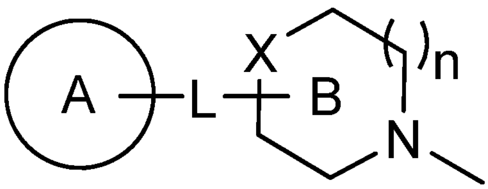

is preferably the following structure:

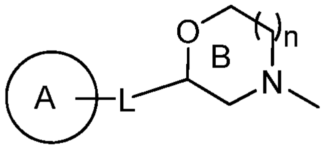

(B1)

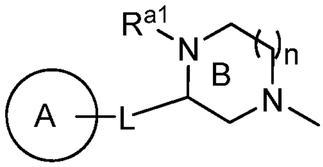

(B2)

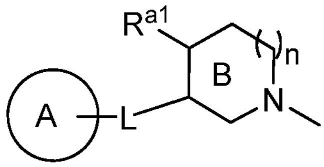

(B3)

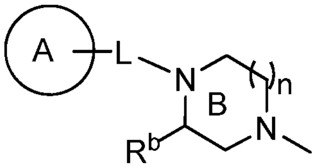

(B4)

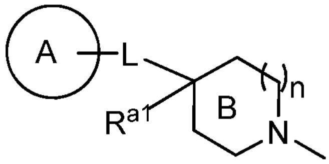

(B5)

wherein Ra1 represents a hydrogen atom or a group other than ring A-L- among the groups exemplified as the substituent in Ra; Rb represents a hydrogen atom, hydroxy, $C_{1-6}$ alkyl, $C_{1-6}$ alkyl-carbonyl, or $C_{1-6}$ alkoxy-carbonyl; and the other symbols are as defined above.

Preferably, in the formula, Ra1 is a hydrogen atom, hydroxy, carboxy, $C_{1-6}$ alkyl, $C_{1-6}$ alkyl-C(O)-, or $C_{1-6}$ alkoxy-carbonyl; Rb is a hydrogen atom, hydroxy, or $C_{1-6}$ alkyl; and the other symbols are as defined above.

A substructure represented by the formula (B1),(B2), or (B3) is more preferable.

The substructure represented by the following formula is particularly preferable:

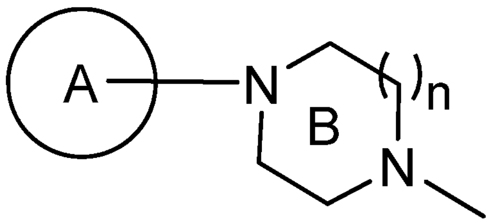

In the compound (I), n represents 1 or 2.

In the compound (I), $R^1$ to $R^3$ are the same or different and each represent a hydrogen atom, a halogen atom, a cyano group, an acyl group, a $C_{1-6}$ alkyl group which may have a substituent(s), a $C_{1-6}$ alkoxy group which may have a substituent(s), a $C_{3-6}$ cycloalkyl group which may have a substituent(s), a $C_{3-6}$ cycloalkoxy group which may have a substituent(s), or an amino group which may have a substituent(s). The "$C_{1-6}$ alkyl group which may have a substituent (s)" is preferably a $C_{1-6}$ alkyl group. The "$C_{1-6}$ alkoxy group which may have a substituent(s)" is preferably a $C_{1-6}$ alkoxy group. Also, the "amino group which may have a substituent(s)" is preferably an amino group which may have a benzyl group. $R^1$ is preferably a hydrogen atom, a halogen atom, an amino group which may have one benzyl group, a $C_{1-6}$ alkyl group, or a $C_{1-6}$ alkoxy group. $R^2$ is preferably a hydrogen atom, a $C_{1-6}$ alkyl group, or a $C_{1-6}$ alkoxy group. $R^3$ is preferably a hydrogen atom or a $C_{1-6}$ alkyl group.

In the compound (I), ring A and ring D each may be condensed with another ring to form a condensed ring which may have a substituent(s). The "condensed ring which may have a substituent(s)" is preferably benzofuran, dihydrobenzofuran, benzothiophene, benzimidazole, indole, or indoline, each of which may have a substituent(s), particularly preferably dihydrobenzofuran which may have a substituent(s).

In the compound (I), however, when ring A is pyrazole which may be substituted and ring D is benzene which may be substituted, L is a bond.

Examples of the compound (I) preferably include a compound or a salt thereof, wherein

ring A is benzene, furan, thiophene, thiazole, pyrazole, imidazole, pyridine, or pyrimidine, each of which may have a substituent(s);

ring B may be further substituted;

ring D is benzene, furan, thiophene, thiazole, pyrazole, imidazole, pyridine, or pyrimidine, each of which may have a substituent(s);

L is a bond, or methylene or ethylene, each of which may have a substituent(s);

X is -O-, -$NR^a$-, or a methylene group which may have a substituent(s), wherein $R^a$ is a hydrogen atom or a substituent;

n is 1 or 2;

$R^1$ is a hydrogen atom, a halogen atom, a cyano group, an acyl group, a $C_{1-6}$ alkyl group which may have a substituent (s), a$C_{1-6}$ alkoxy group which may have a substituent(s), or an amino group which may have a substituent(s);

$R^2$ is a hydrogen atom, a $C_{1-6}$ alkyl group which may have a substituent(s), or a $C_{1-6}$ alkoxy group which may have a substituent(s); and

$R^3$ is a hydrogen atom, a $C_{1-6}$ alkyl group which may have a substituent(s), or a $C_{1-6}$ alkoxy group which may have a substituent(s), wherein

ring A and ring D may be condensed with another ring to form benzofuran, dihydrobenzofuran, benzothiophene, benzimidazole, indole, or indoline, each of which may have a substituent(s), and ring A-L- in the formula (I) may be bonded to X, provided that

when ring A is pyrazole which may be substituted and ring D is benzene which may be substituted, L is a bond.

Examples of the compound (I) more preferably include a compound or a salt thereof, wherein

ring A is benzene which may have a substituent(s), thiazole which may have a substituent(s), pyridine which may have a substituent(s), or pyrazole which may have a substituent(s);

ring B may further have a substituent(s);

ring D is benzene which may have a substituent(s), pyrazole which may have a substituent(s), furan which may have a substituent(s), pyridine which may have a substituent(s), or indazole which may have a substituent(s);

L is a bond, methylene, or ethylene;

X is -O-, -$NR^a$-, or a methylene group which may have a substituent(s), wherein $R^a$ is a hydrogen atom or a substituent;

n is 1 or 2;

$R^1$ is a hydrogen atom, a halogen atom, a cyano group, an acyl group, a $C_{1-6}$ alkyl group which may have a substituent (s), a $C_{1-6}$ alkoxy group which may be substituted, or an amino group which may have a substituent(s);

$R^2$ is a hydrogen atom, a $C_{1-6}$ alkyl group which may have a substituent(s), or a $C_{1-6}$ alkoxy group which may have a substituent(s); and

$R^3$ is a hydrogen atom, a $C_{1-6}$ alkyl group which may have a substituent(s), or a $C_{1-6}$ alkoxy group which may have a

substituent(s), wherein
ring D may be condensed with another ring to form benzofuran or dihydrobenzofuran, each of which may have a substituent(s), and ring A-L- may be bonded to X, provided that in the compound (I),
when ring A is pyrazole which may be substituted and ring D is benzene which may be substituted, L is a bond.

Examples of the compound (I) even more preferably include a compound or a salt thereof, wherein ring A is benzene having a substituent(s), or pyridine having a substituent(s), more preferably wherein the number of the substituent is 1 or 2;
ring B may further have a substituent(s), more preferably wherein the number of the substituent is 0 to 2;
ring D is benzene having a substituent(s), pyrazole having a substituent(s), furan, pyridine having a substituent(s), or indazole having a substituent(s), more preferably wherein the number of the substituent is 1 or 2;
L is a bond or methylene;
X is -O-, -$NR^a$-, or a methylene group which may have a substituent(s), wherein the number of the substituent in the methylene group is preferably 1 or 2, and $R^a$ is a hydrogen atom or a substituent
n is 1 or 2;
$R^1$ is a $C_{1-6}$ alkoxy group or $C_{1-6}$ alkyl having a substituent(s), wherein the number of the substituent in the $C_{1-6}$ alkyl is preferably 1 or 2;
$R^2$ is a hydrogen atom; and
$R^3$ is a hydrogen atom or a $C_{1-6}$ alkyl group, wherein
the substituent on ring D may be combined with ring D to form dihydrobenzofuran which may have a substituent(s), and ring A-L- in the formula (I) may be bonded to X.
In this context, the substituent in dihydrobenzofuran which may have a substituent(s), formed by the substituent on ring D together with ring D may be, for example, the same substituent as the "substituent" in the "monocyclic aromatic hydrocarbon ring or the monocyclic aromatic heterocyclic ring which may have a substituent(s)". The dihydrobenzofuran may have, for example, 1 to 5, preferably 1 to 3 substituents at possible positions.

The compound (I) is further preferably a compound or a salt thereof, wherein
ring A is benzene having a substituent(s) selected from a halogen atom, cyano, $C_{1-6}$ alkyl which may be halogenated, and $C_{1-6}$ alkoxy, or pyridine substituted by $C_{1-6}$ alkyl, more preferably wherein the number of the substituent is 1 or 2;
ring B may further have a substituent(s) selected from oxo and $C_{1-6}$ alkyl, more preferably wherein the number of the substituent is 0 to 2;
ring D is benzene having a substituent(s) selected from a halogen atom, $C_{1-6}$ alkyl, $C_{1-6}$ alkoxy which may be halogenated, morpholinyl, and di-$C_{1-6}$ alkylamino, preferably wherein the number of the substituent is 1 or 2;
L is a bond or methylene;
X is (1) -O-, (2) -$NR^a$- ($R^a$ is a hydrogen atom, $C_{1-6}$ alkyl, $C_{1-6}$ alkyl-carbonyl, $C_{1-6}$ alkoxy-carbonyl, or ring A-L- in the formula (I)), or (3) a methylene group bonded to ring A-L- in the formula (I) and further substituted by hydroxy;
n is 1 or 2;
$R^1$ is a $C_{1-6}$ alkoxy group;
$R^2$ is a hydrogen atom; and
$R^3$ is a hydrogen atom or a $C_{1-6}$ alkyl group, wherein
the substituent on ring D may be combined with ring D to form dihydrobenzofuran substituted by a $C_{1-6}$ alkyl group.

Among them, the compound (I) is preferably a compound or a salt thereof, wherein
ring A is benzene having a substituent(s) selected from a halogen atom, cyano, $C_{1-6}$ alkyl which may be halogenated, and $C_{1-6}$ alkoxy, preferably benzene having 1 or 2 of these substituents; and
ring D is benzene which may have a substituent(s) selected from a halogen atom, $C_{1-6}$ alkyl which may be substituted by formyl, halogen, or hydroxy, $C_{1-6}$ alkoxy which may be halogenated, morpholinyl, di-$C_{1-6}$ alkylamino, and $C_{1-6}$ alkyl-carbonyl, preferably benzene having 1 or 2 of these substituents.
A compound is particularly preferable, wherein the following substructure:

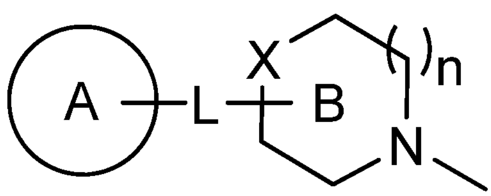

is preferably the structure represented by any of the formulas (B1) to (B5) described above, wherein Ra1 is a hydrogen atom; Rb represents a hydrogen atom, $C_{1-6}$ alkyl, $C_{1-6}$ alkoxy-carbonyl, or the ring A-L- group in the formula (I); and the other symbols are as defined above.

Among them, the compound (I) is more preferably a compound or a salt thereof, wherein
ring A is benzene having a substituent(s) selected from a halogen atom, $C_{1-6}$ alkyl which may be halogenated, and $C_{1-6}$ alkoxy, preferably benzene having 1 or 2 of these substituents;
ring B may be further substituted by $C_{1-6}$ alkyl, preferably, 1 to 3 $C_{1-6}$ alkyl groups;
ring D is benzene having a substituent(s) selected from a halogen atom, $C_{1-6}$ alkyl, $C_{1-6}$ alkoxy which may be halogenated, morpholinyl, di-$C_{1-6}$ alkylamino, and $C_{1-6}$ alkyl-carbonyl, preferably benzene having 1 or 2 of these substituents;
L is a bond;
X is -O- or -$NR^a$- ($R^a$ is ring A-L- in the formula (I)); n is 1 or 2;
$R^1$ is a $C_{1-6}$ alkoxy group; and
both of $R^2$ and $R^3$ are a hydrogen atom.

Of these compounds (I), a compound represented by the following formula (Ia) or a salt thereof is preferable:

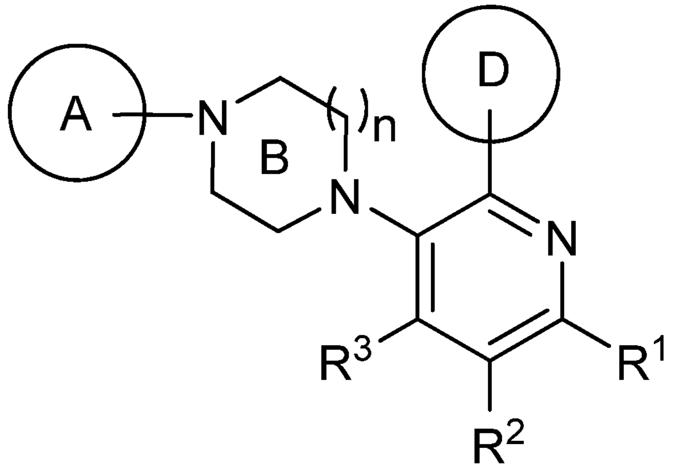

(Ia)

wherein

ring A is benzene having a substituent(s);
ring B may further have a substituent(s);
ring D is benzene having a substituent(s);
n is 1 or 2;
$R^1$ is a $C_{1-6}$ alkoxy group or $C_{1-6}$ alkyl having a substituent(s);
$R^2$ is a hydrogen atom; and
$R^3$ is a hydrogen atom or $C_{1-6}$ alkyl, wherein

the substituent on ring D may be combined with ring D to form dihydrobenzofuran which may be substituted.
A compound represented by the formula (Ia) or a salt thereof is more preferable, wherein
ring A is benzene substituted by a substituent(s) selected from a halogen atom, $C_{1-6}$ alkyl, and $C_{1-6}$ alkoxy, preferably benzene having 1 or 2 of these substituents;
ring B may further have a substituent(s) selected from oxo and $C_{1-6}$ alkyl, preferably wherein the number of the substituent is 0 to 2;
ring D is benzene having a substituent(s) selected from a halogen atom, $C_{1-6}$ alkyl, $C_{1-6}$ alkoxy which may be halogenated, morpholinyl, and di-$C_{1-6}$ alkylamino, preferably benzene having 1 or 2 of these substituents;
n is 1 or 2;
$R^1$ is $C_{1-6}$ alkoxy or $C_{1-6}$ alkyl having hydroxy;
$R^2$ is a hydrogen atom; and
$R^3$ is a hydrogen atom or $C_{1-6}$ alkyl, wherein
the substituent on ring D may be combined with ring D to form dihydrobenzofuran substituted by $C_{1-6}$ alkyl, preferably dihydrobenzofuran substituted by 1 or 2 $C_{1-6}$ alkyl groups.
More specifically, the compound (I) is preferably any of compounds described later in Examples or salts thereof.
4-{3-[4-(4-fluorophenyl)piperazin-1-yl]-6-methoxypyridin-2-yl}-N,N-dimethylaniline or a salt thereof; 4-{6-methoxy-3-[4-(4-methoxyphenyl)piperazin-1-yl]pyridin-2-yl}-N,N-dimethylaniline or a salt thereof; 4-{6-methoxy-3-[4-(4-methylphenyl)piperazin-1-yl]pyridin-2-yl}-N,N-dimethylaniline or a salt thereof; and 1-[2-(4-ethoxyphenyl)-6-methoxypyridin-3-yl]-4-(4-methoxyphenyl)piperazine or a salt thereof are particularly preferable.

Also, a medicament of the present invention is preferably a medicament comprising the compound represented

by the formula (I) or the salt thereof, more preferably a medicament which is a neuronal differentiation promoter, an IGF-1 signal modulator, or a protein kinase B activator, particularly preferably a therapeutic or prophylactic agent for Alzheimer's disease.

A therapeutic or prophylactic agent for Alzheimer's disease comprising a compound represented by the following formula ($I_0$) or a salt thereof is further preferable:

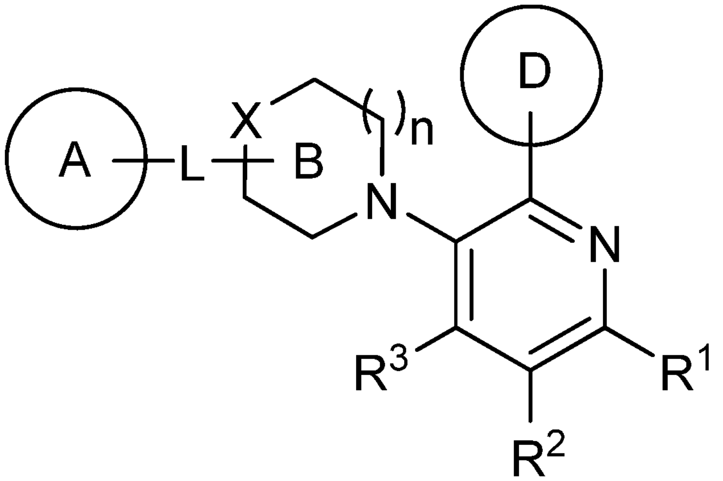

($I_0$)

wherein

ring A represents a monocyclic aromatic hydrocarbon ring or a monocyclic aromatic heterocyclic ring, each of which may have a substituent(s);

ring B may further have a substituent(s);

ring D represents a monocyclic aromatic hydrocarbon ring or a monocyclic aromatic heterocyclic ring, each of which may have a substituent(s);

L represents a bond, or a methylene group or an ethylene group, each of which may have a substituent(s);

X represents -O-, -$NR^a$-, or a methylene group which may have a substituent(s), wherein $R^a$ represents a hydrogen atom or a substituent;

n represents 1 or 2; and

$R^1$ to $R^3$ are the same or different and each represent a hydrogen atom, a halogen atom, a cyano group, an acyl group, a $C_{1-6}$ alkyl group which may have a substituent(s), a $C_{1-6}$ alkoxy group which may have a substituent(s), a $C_{3-6}$ cycloalkyl group which may have a substituent(s), a $C_{3-6}$ cycloalkoxy group which may have a substituent(s), or an amino group which may have a substituent(s), wherein ring A and ring D each may be condensed with another ring to form a condensed ring which may have a substituent(s), and ring A-L- in the formula ($I_0$) may be bonded to X.

The method comprising administering an effective amount of a compound represented by the formula ($I_0$) or a salt thereof is particularly preferable, wherein ring A is benzene having a substituent(s) selected from a halogen atom, $C_{1-6}$ alkyl which may be halogenated, and $C_{1-6}$ alkoxy, or pyridine substituted by $C_{1-6}$ alkyl;

ring B may further have a substituent(s) selected from oxo and $C_{1-6}$ alkyl;

ring D is benzene having a substituent(s) selected from a halogen atom, $C_{1-6}$ alkyl, $C_{1-6}$ alkoxy which may be halogenated, morpholinyl, and di-$C_{1-6}$ alkylamino, pyrazole substituted by $C_{1-6}$ alkyl, furan, pyridine substituted by $C_{1-6}$ alkoxy, or indazole substituted by $C_{1-6}$ alkoxy;

L is a bond or methylene;

X is

(1) -O-,
(2) -$NR^a$-, or
(3) methylene bonded to ring A-L- in the formula (I) and further substituted by hydroxy, wherein

$R^a$ is a hydrogen atom, $C_{1-6}$ alkyl, $C_{1-6}$ alkoxycarbonyl, or ring A-L- in the formula (I);

n is 1 or 2;

$R^1$ is a $C_{1-6}$ alkoxy group or $C_{1-6}$ alkyl having hydroxy;

$R^2$ is a hydrogen atom;

$R^3$ is a hydrogen atom or $C_{1-6}$ alkyl, wherein the substituent on ring D may be combined with ring D to form dihydrobenzofuran substituted by $C_{1-6}$ alkyl.

Hereinafter, processes for producing the compound (I) will be described. In this context, compounds (Ia') to (Ir) described later are all included in the compound (I). The compound (I) can be produced by use of general organic synthesis methods. In the simplified diagrams of reaction schemes below, each of symbols in compounds is as defined above. The compounds in the reaction schemes also encompass their salt forms. Examples of the salts include the same as salts of the compound (I) and its prodrug described later. Depending on the type of a functional group, the functional group in a starting material or an intermediate is protected with an appropriate protective group, which can be removed as appropriate after reaction to obtain the desired compound. Examples of such a functional group include a hydroxy group, a carboxy group, and an amino group. Examples of protective groups for these groups can include those described in Greene and Wuts, "Protective Groups in Organic Synthesis (third edition)". The protective groups can be selected appropriately according to reaction conditions.

Reaction Scheme 1

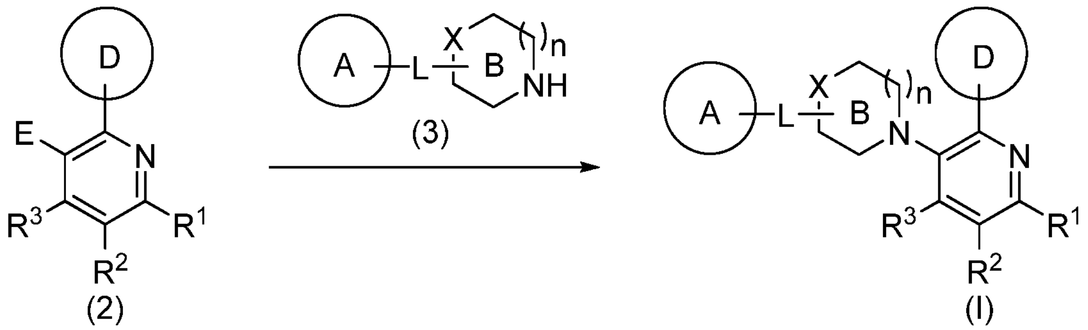

In Reaction Scheme 1, E represents a leaving group, and the other symbols are as defined above.
According to Reaction Scheme 1, the compound (I) is produced by reacting a compound (2) with a compound (3) represented by the formula:

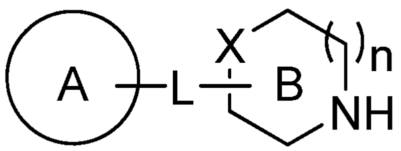

if desired, in the presence of a base. A transition metal such as copper or palladium and its ligand may be used, if necessary.
The compound (3) can be easily obtained as a commercially available product and is also produced by a method known per se in the art.
The amount of the compound (3) used is approximately 0.5 to approximately 10 mol, preferably approximately 1.0 to approximately 3.0 mol, with respect to 1 mol of the compound (2).
Examples of the "leaving group" represented by E include halogen atoms (e.g., fluorine, chlorine, bromine, and iodine), $C_{1-6}$ alkylsulfonyloxy which may be halogenated (e.g., methanesulfonyloxy, trifluoromethanesulfonyloxy, and trichloromethanesulfonyloxy), and $C_{6-10}$ arylsulfonyloxy which may have a substituent(s).
Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.
Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, N-ethyldiisopropylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, sodium tert-butoxide, and potassium tert-butoxide.
The amount of the base used is approximately 0.8 to approximately 10 mol, preferably approximately 1.0 to approximately 5.0 mol, with respect to 1 mol of the compound (2).
This reaction is advantageously performed using a solvent inert to the reaction. Such a solvent is not particularly limited

as long as the reaction proceeds. Preferable examples thereof include: alcohols such as methanol, ethanol, and propanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.
Copper, copper halide (e.g., copper chloride and copper bromide), copper oxide, or the like is used as the "copper".
The amount of the copper used is approximately 0.1 to approximately 10 mol, preferably approximately 0.5 to approximately 2.0 mol, with respect to 1 mol of the compound (2).
Palladium acetate, palladium chloride, tetrakis(triphenylphosphine)palladium, tris(dibenzylideneacetone)dipalladium, 1,1-bis(diphenylphosphino)ferrocenedichloropalladium, dichlorobis(triphenylphosphine)palladium, bis(tri(tert-butylphosphine))palladium, phenylallylchloro-[1,3-bis(diisopropylphenyl)imidazol-2-ylidene]palladium, phenylallylchloro-[1,3-bis(diisopropylphenyl)-2-imidazolidinylidene]palladium, or the like is used as the "palladium".
The amount of the palladium used is approximately 0.001 to approximately 5 mol, preferably approximately 0.02 to approximately 0.5 mol, with respect to 1 mol of the compound (2).
Triphenylphosphine, tri(tert-butyl)phosphine, 2,2'-bis(diphenylphosphino)-1,1'-binaphthyl, 2-(dicyclohexylphosphino)-2',4',6'-triisopropyl-1,1'-biphenyl, 4,5'-bis(diphenylphosphino)-9,9'-dimethylxanthene, or the like is used as the "ligand".
The amount of the ligand used is approximately 0.001 to approximately 5 mol, preferably approximately 0.05 to approximately 1 mol, with respect to 1 mol of the compound (2).
The reaction time is usually approximately 30 minutes to approximately 72 hours, preferably approximately 1 hour to approximately 48 hours. The reaction temperature is usually approximately -20 to approximately 200°C, preferably approximately 0 to approximately 150°C.
In this reaction, the reaction time can be shortened by use of a microwave reactor or the like.

Reaction Scheme 2

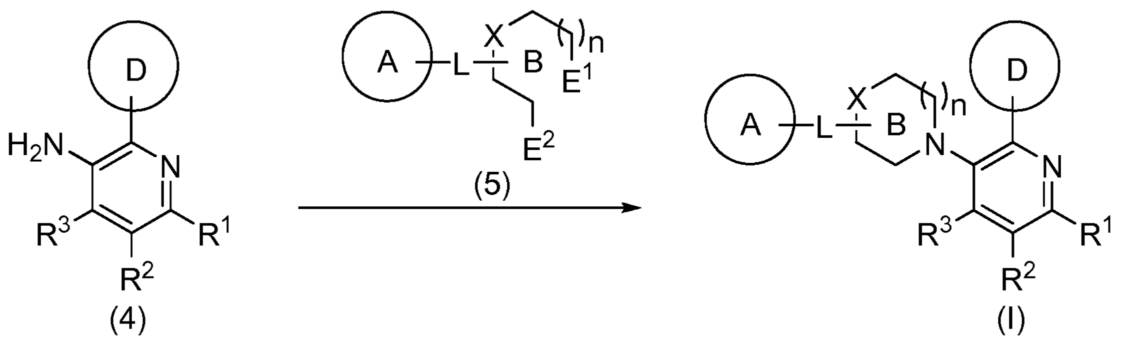

In Reaction Scheme 2, $E^1$ and $E^2$ represent the same or different leaving groups, and the other symbols are as defined above.
According to Reaction Scheme 2, the compound (I) is also produced by reacting a compound (4) with a compound (5) represented by the formula:

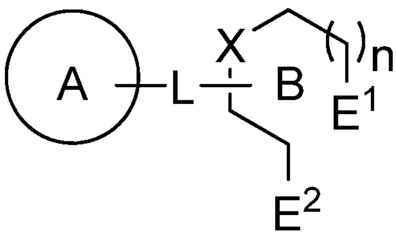

if desired, in the presence of a base. The newly formed bonds may be formed simultaneously or stepwise.
The compound (5) can be easily obtained as a commercially available product and is also produced by a method known per se in the art.
Examples of the "leaving group" represented by $E^1$ and $E^2$ include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).

Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.

The amount of the compound (5) used is approximately 0.8 to approximately 5.0 mol, preferably approximately 1.0 to approximately 2.0 mol, with respect to 1 mol of the compound (4).

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.

The amount of the base used is approximately 0.5 to approximately 10.0 mol, preferably approximately 1.0 to approximately 3.0 mol, with respect to 1 mol of the compound (4). The compound is also produced by performing the reaction, if desired, in the presence of quaternary ammonium salt or metal iodide, together with the base.

Examples of the "quaternary ammonium salt" include tetrabutyl ammonium iodide.

Examples of the "metal iodide" include sodium iodide and potassium iodide.

The amount of the quaternary ammonium salt used is approximately 0.1 to approximately 3.0 mol, preferably approximately 0.5 to approximately 1.0 mol, with respect to 1 mol of the compound (4).

The amount of the metal iodide used is approximately 0.1 to approximately 3.0 mol, preferably approximately 0.5 to approximately 1.0 mol, with respect to 1 mol of the compound (4).

This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.

Reaction Scheme 3

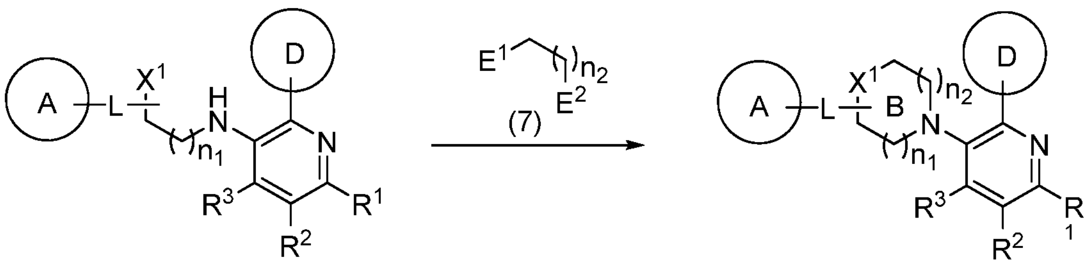

(6a) (Ia')

In Reaction Scheme 3, $E^1$ and $E^2$ represent the same or different leaving groups; $X^1$ represents oxygen or nitrogen (which appropriately has a hydrogen atom according to the binding manner); $n_1$ and $n_2$ each represent 1 or 2 (excluding, however, the cases where both of $n_1$ and $n_2$ are 2); and the other symbols are as defined above.

According to Reaction Scheme 3, the compound (Ia') is also produced by reacting a compound (6a) with a compound (7) represented by the formula:

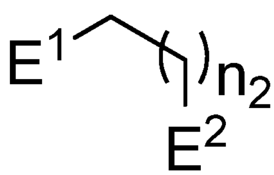

if desired, in the presence of a base. The newly formed bonds may be formed simultaneously or stepwise.

The compound (7) can be easily obtained as a commercially available product and is also produced by a method known per se in the art.

Examples of the "leaving group" represented by $E^1$ and $E^2$ include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).

Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.

The amount of the compound (7) used is approximately 0.8 to approximately 5.0 mol, preferably approximately 1.0 to approximately 2.0 mol, with respect to 1 mol of the compound (6a).

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.

The amount of the base used is approximately 0.5 to approximately 10.0 mol, preferably approximately 1.0 to approximately 3.0 mol, with respect to 1 mol of the compound (6a). The compound is also produced by performing the reaction, if desired, in the presence of quaternary ammonium salt or metal iodide, together with the base.

Examples of the "quaternary ammonium salt" include tetrabutyl ammonium iodide.

Examples of the "metal iodide" include sodium iodide and potassium iodide.

The amount of the quaternary ammonium salt used is approximately 0.1 to approximately 3.0 mol, preferably approximately 0.5 to approximately 1.0 mol, with respect to 1 mol of the compound (6a).

The amount of the metal iodide used is approximately 0.1 to approximately 3.0 mol, preferably approximately 0.5 to approximately 1.0 mol, with respect to 1 mol of the compound (6a).

This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.

Reaction Scheme 4

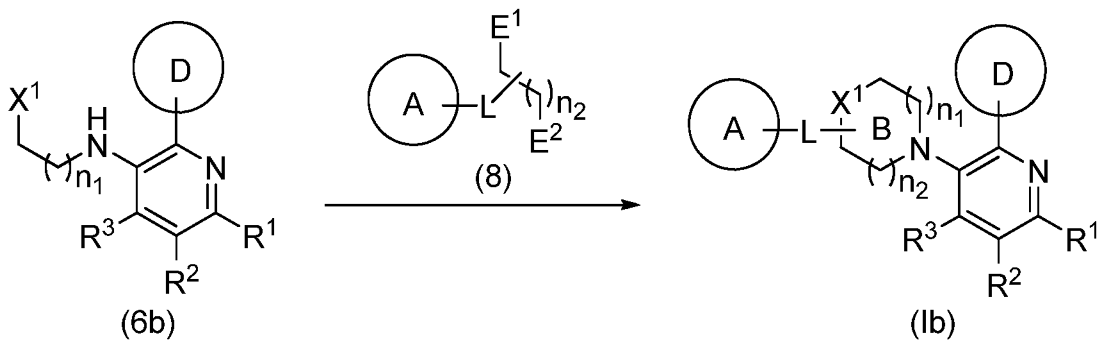

In Reaction Scheme 4, $E^1$ and $E^2$ represent the same or different leaving groups; $X^1$ represents oxygen or nitrogen (which appropriately has a hydrogen atom according to the binding manner); $n_1$ and $n_2$ each represent 1 or 2 (excluding, however, the cases where both of $n_1$ and $n_2$ are 2); and the other symbols are as defined above.

According to Reaction Scheme 4, the compound (Ib) is also produced by reacting a compound (6b) with a compound (8) represented by the formula:

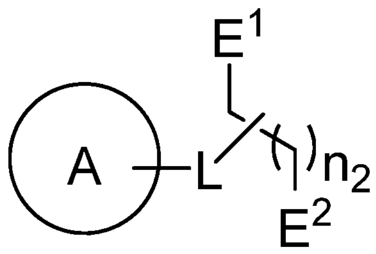

if desired, in the presence of a base. The newly formed bonds may be formed simultaneously or stepwise.

The compound (8) can be easily obtained as a commercially available product and is also produced by a method known per se in the art.

Examples of the "leaving group" represented by $E^1$ and $E^2$ include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).

Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.

The amount of the compound (8) used is approximately 0.8 to approximately 5.0 mol, preferably approximately 1.0 to approximately 2.0 mol, with respect to 1 mol of the compound (6b).

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.

The amount of the base used is approximately 0.5 to approximately 10.0 mol, preferably approximately 1.0 to approximately 3.0 mol, with respect to 1 mol of the compound (6b). The compound is also produced by performing the reaction, if desired, in the presence of quaternary ammonium salt or metal iodide, together with the base.

Examples of the "quaternary ammonium salt" include tetrabutyl ammonium iodide.

Examples of the "metal iodide" include sodium iodide and potassium iodide.

The amount of the quaternary ammonium salt used is approximately 0.1 to approximately 3.0 mol, preferably approximately 0.5 to approximately 1.0 mol, with respect to 1 mol of the compound (6b).

The amount of the metal iodide used is approximately 0.1 to approximately 3.0 mol, preferably approximately 0.5 to approximately 1.0 mol, with respect to 1 mol of the compound (6b).

This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.

Reaction Scheme 5

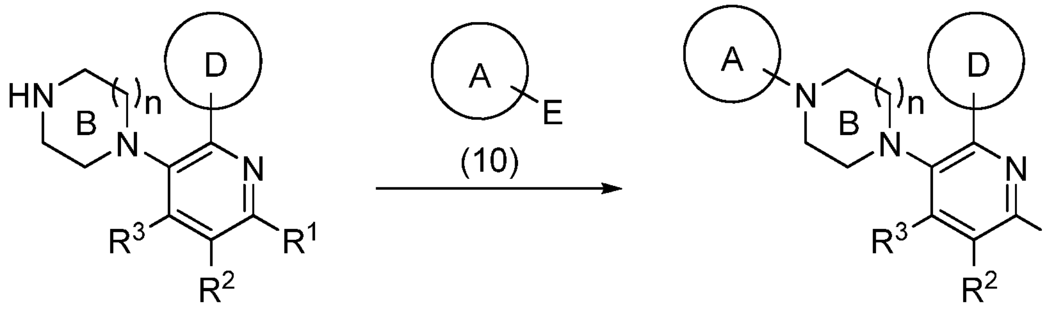
(10)

(9) (Ic)

In Reaction Scheme 5, E represents a leaving group, and the other symbols are as defined above.

According to Reaction Scheme 5, the compound (Ic) is produced by reacting a compound (9) with a compound (10) represented by the formula:

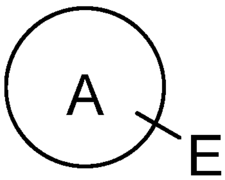

if desired, in the presence of a base. A transition metal such as copper or palladium and its ligand may be used, if necessary.

The compound (10) can be easily obtained as a commercially available product and is also produced by a method known per se in the art.

The amount of the compound (10) used is approximately 0.5 to approximately 10.0 mol, preferably approximately 1.0 to approximately 3.0 mol, with respect to 1 mol of the compound (9).

Examples of the "leaving group" represented by E include halogen atoms (e.g., fluorine, chlorine, bromine, and iodine), $C_{1-5}$ alkylsulfonyloxy which may be halogenated (e.g., methanesulfonyloxy, trifluoromethanesulfonyloxy, and trichloromethanesulfonyloxy), and $C_{6-10}$ arylsulfonyloxy which may have a substituent(s).

Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, N-ethyldiisopropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, sodium tert-butoxide, and potassium tert-butoxide.

The amount of the base used is approximately 0.8 to approximately 10.0 mol, preferably approximately 1.0 to approximately 5.0 mol, with respect to 1 mol of the compound (9).

This reaction is advantageously performed using a solvent inert to the reaction. Such a solvent is not particularly limited as long as the reaction proceeds. Preferable examples thereof include: alcohols such as methanol, ethanol, and propanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

Copper, copper halide (e.g., copper chloride, copper bromide, and copper iodide), copper oxide, or the like is used as the "copper".

The amount of the copper used is approximately 0.1 to approximately 10 mol, preferably approximately 0.5 to approximately 2.0 mol, with respect to 1 mol of the compound (2).

Palladium acetate, palladium chloride, tetrakis(triphenylphosphine)palladium, tris(dibenzylideneacetone)dipalladium, 1,1-bis(diphenylphosphino)ferrocenedichloropalladium, dichlorobis(triphenylphosphine)palladium, bis(tri(tert-butylphosphine))palladium, phenylallylchloro-[1,3-bis(diisopropylphenyl)imidazol-2-ylidene]palladium, phenylallylchloro-[1,3-bis(diisopropylphenyl)-2-imidazolidinylidene]palladium, or the like is used as the "palladium".

The amount of the palladium used is approximately 0.001 to approximately 5 mol, preferably approximately 0.02 to approximately 0.5 mol, with respect to 1 mol of the compound (2).

Triphenylphosphine, tri(tert-butyl)phosphine, 2,2'-bis(diphenylphosphino)-1,1'-binaphthyl, 2-(dicyclohexylphosphino)-2',4',6'-triisopropyl-1,1'-biphenyl, 4,5'-bis(diphenylphosphino)-9,9'-dimethylxanthene, N,N'-dimethylethylenediamine, 1,10-phenanthroline, or the like is used as the "ligand".

The amount of the ligand used is approximately 0.001 to approximately 5 mol, preferably approximately 0.05 to approximately 1 mol, with respect to 1 mol of the compound (2).

The reaction time is usually approximately 30 minutes to approximately 72 hours, preferably approximately 1 hour to approximately 48 hours. The reaction temperature is usually approximately -20 to approximately 200°C, preferably approximately 0 to approximately 150°C.

In this reaction, the reaction time can be shortened by use of a microwave reactor or the like.

Reaction Scheme 6

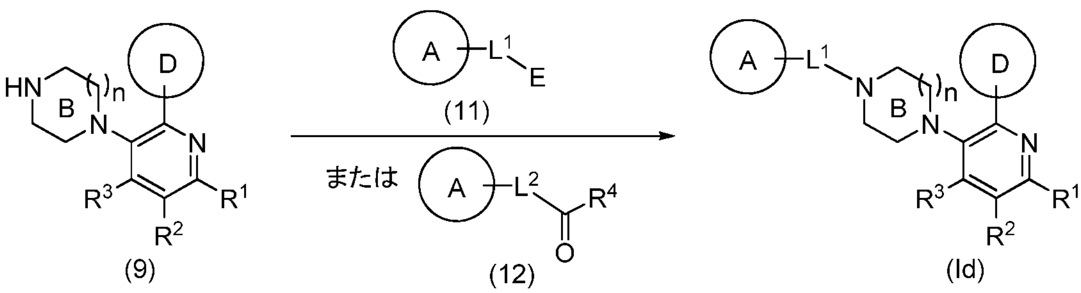


(9) (11) (12) (Id)

In Reaction Scheme 6, E represents a leaving group; $L^1$ represents methylene or ethylene which may be substituted; $R^4$ represents hydrogen or a lower alkyl group which may be substituted; $L^2$ represents a bond binding to $R^4$ and carbon having hydrogen as a substituent(s) to constitute $L^1$ in the compound (Id), or methylene which may be substituted; and the other symbols are as defined above.

According to Reaction Scheme 6, the compound (Id) is also produced by reacting a compound (9) with a compound (11) represented by the formula:

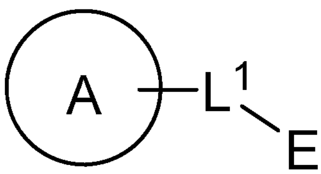

if desired, in the presence of a base.

The compound (11) can be easily obtained as a commercially available product and is also produced by a method known per se in the art.

Examples of the "leaving group" represented by E include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).

Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.

The amount of the compound (11) used is approximately 0.8 to approximately 5.0 mol, preferably approximately 1.0 to approximately 2.0 mol, with respect to 1 mol of the compound (9).

Examples of the "base" include: basic salts such as sodium carbonate, potassium carbonate, cesium carbonate, and

sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.

The amount of the base used is approximately 0.5 to approximately 10.0 mol, preferably approximately 1.0 to approximately 3.0 mol, with respect to 1 mol of the compound (9). The compound is also produced by performing the reaction, if desired, in the presence of quaternary ammonium salt or metal iodide, together with the base.

Examples of the "quaternary ammonium salt" include tetrabutyl ammonium iodide.

Examples of the "metal iodide" include sodium iodide and potassium iodide.

The amount of the quaternary ammonium salt used is approximately 0.1 to approximately 3.0 mol, preferably approximately 0.5 to approximately 1.0 mol, with respect to 1 mol of the compound (9).

The amount of the metal iodide used is approximately 0.1 to approximately 3.0 mol, preferably approximately 0.5 to approximately 1.0 mol, with respect to 1 mol of the compound (9).

This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.

According to Reaction Scheme 6, the compound (Id) is also produced by reductively alkylating the compound (9) and a compound (12) represented by the formula:

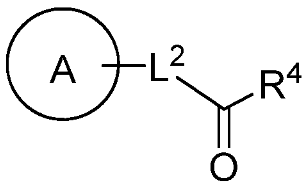

with a reducing agent.

The compound (12) can be easily obtained as a commercially available product and is also produced by a method known per se in the art.

For example, metal hydride such as sodium borohydride, lithium borohydride, sodium cyanoborohydride, sodium triacetoxyborohydride, lithium aluminum hydride, sodium bis(2-methoxyethoxy)aluminum hydride, a borane-tetrahydrofuran complex, or diisobutyl aluminum hydride is used as the "reducing agent". A mineral acid (e.g., hydrochloric acid and hydrobromic acid), an organic acid (e.g., acetic acid, p-toluenesulfonic acid, and trifluoromethanesulfonic acid), or a Lewis acid (e.g., titanium tetrachloride and ytterbium trifluoromethanesulfonate) may be added thereto, if desired.

The amount of the reducing agent used is approximately 0.8 to approximately 10.0 mol, preferably approximately 1.0 to approximately 5.0 mol, with respect to 1 mol of the compound (9).

The amount of the acid used is approximately 0.01 to approximately 10.0 mol, preferably approximately 0.1 to approximately 5.0 mol, with respect to 1 mol of the compound (9).

This reaction is advantageously performed using a solvent inert to the reaction. Such a solvent is not particularly limited as long as the reaction proceeds. Preferable examples thereof include: alcohols such as methanol, ethanol, and propanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

The reaction time is usually approximately 30 minutes to approximately 72 hours, preferably approximately 1 hour to approximately 48 hours. The reaction temperature is usually approximately -20 to approximately 200°C, preferably approximately 0 to approximately 120°C.

Reaction Scheme 7

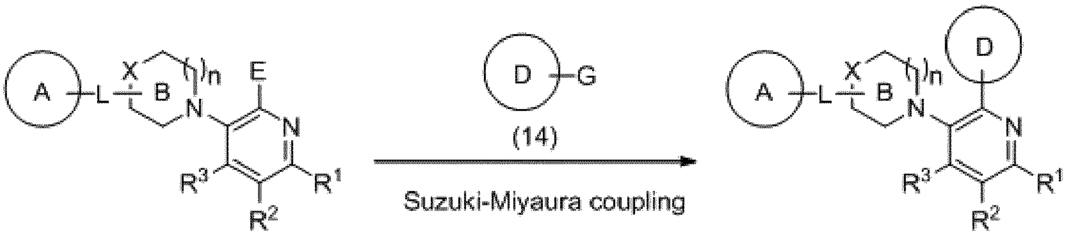


(13)                (I)

In Reaction Scheme 7, E represents a leaving group; G represents $B(OR^5)_2$ (wherein two $R^5$ moieties each independently represent a $C_{1-6}$ alkyl group or may be bonded to each other to form a $C_{2-6}$ alkylene chain); and the other symbols are as defined above.

Examples of the $C_{2-6}$ alkylene chain formed by two $R^5$ moieties together include $-CH_2-CH_2-$, $-C(CH_3)_2-C(CH_3)_2-$, $-CH_2-CH_2-CH_2-$, and $-CH_2-C(CH_3)_2-CH_2-$.

According to Reaction Scheme 7, the compound (I) is also produced by reacting a compound (13) with a compound (14) represented by the formula:

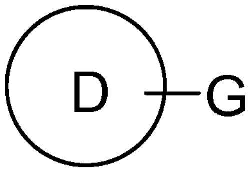

through Suzuki-Miyaura coupling reaction in the presence of a transition metal catalyst and a base.

The compound (14) can be easily obtained as a commercially available product and is also produced by a method known per se in the art.

Examples of the functional group represented by $B(OR^5)_2$ include boronic acid and boronic acid ester (e.g., 4,4,5,5-tetramethyl-1,3,2-dioxaborolan-2-yl).

The amount of the compound (14) used is approximately 0.5 to approximately 10.0 mol, preferably approximately 1.0 to approximately 3.0 mol, with respect to 1 mol of the compound (13).

Examples of the "leaving group" represented by E include halogen atoms (e.g., fluorine, chlorine, bromine, and iodine), $C_{1-5}$ alkylsulfonyloxy which may be halogenated (e.g., methanesulfonyloxy, trifluoromethanesulfonyloxy, and trichloromethanesulfonyloxy), and $C_{6-10}$ arylsulfonyloxy which may have a substituent(s).

Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro.

Specific examples thereof include benzenesulfonyloxy, m-nitrobenzenesulfonyloxy, and p-toluenesulfonyloxy.

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, N-ethyldiisopropylamine, tributylamine, cyclohexyldimethylamine, 4-dimethylaminopyridine, N,N-dimethylaniline, N-methylpiperidine, N-methylpyrrolidine, and N-methylmorpholine; and metal alkoxides such as sodium methoxide, sodium ethoxide, sodium tert-butoxide, and potassium tert-butoxide.

The amount of the base used is approximately 0.8 to approximately 10.0 mol, preferably approximately 1.0 to approximately 5.0 mol, with respect to 1 mol of the compound (13).

For example, a palladium catalyst such as palladium acetate, palladium chloride, tetrakis(triphenylphosphine)palladium, tris(dibenzylideneacetone)dipalladium, 1,1-bis(diphenylphosphino)ferrocenedichloropalladium, dichlorobis(triphenylphosphine)palladium, phenylallylchloro-[1,3-bis(diisopropylphenyl)imidazol-2-ylidene]palladium, or phenylallylchloro-[1,3-bis(diisopropylphenyl)-2-imidazolidinylidene]palladium is used as the "transition metal catalyst". A ligand such as triphenylphosphine, 2,2'-bis(diphenylphosphino)-1,1'-binaphthyl, or Xantphos may be added thereto, if desired.

The amount of the transition metal catalyst used is approximately 0.001 to approximately 3 mol, preferably approximately 0.02 to approximately 0.2 mol, with respect to 1 mol of the compound (13).

The amount of the ligand used is approximately 0.001 to approximately 3 mol, preferably approximately 0.05 to approximately 0.5 mol, with respect to 1 mol of the compound (13).

Examples of a solvent used include: ethers such as diethyl ether, diisopropyl ether, tetrahydrofuran, dioxane, and 1,2-dimethoxyethane; alcohols such as methanol, ethanol, and propanol; hydrocarbons such as benzene, toluene, carbon disulfide, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; water; and mixed solvents thereof.
The reaction temperature is usually 0 to 250°C, preferably 50 to 150°C. The reaction time is usually approximately 5 minutes to approximately 48 hours, preferably approximately 30 minutes to approximately 24 hours.
In this reaction, the reaction time can be shortened by use of a microwave reactor or the like.

Reaction Scheme 8

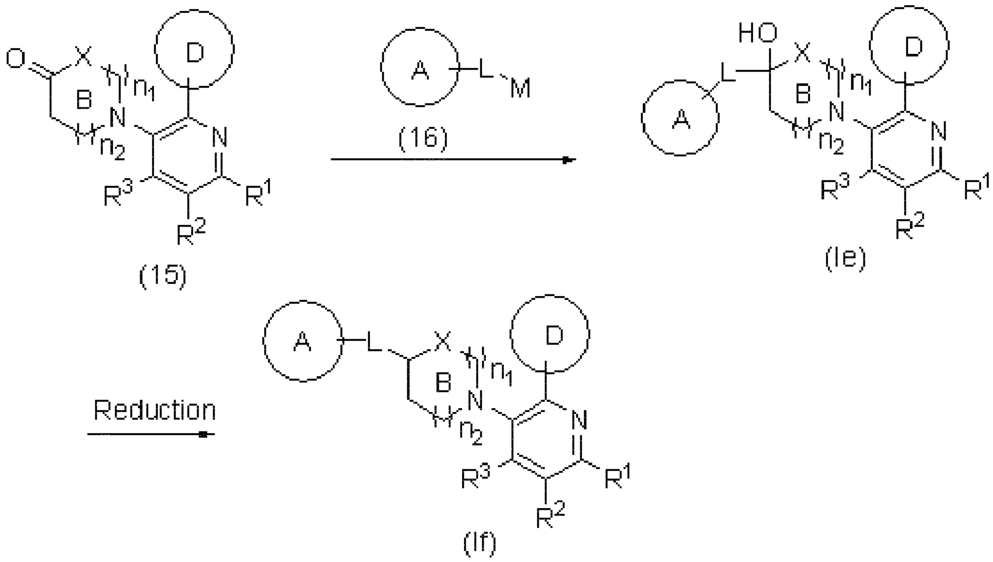


(15) (Ie) (If)

In Reaction Scheme 8, $n_1$ and $n_2$ each represent an integer of 0 to 3 for forming an atomic group constituting 6- or 7-membered ring B in the compounds (Ie) and (If); M represents a metal; and the other symbols are as defined above.
According to Reaction Scheme 8, the compound (Ie) can also be produced by reacting a compound (15) with an organic metal compound (16) represented by the formula:

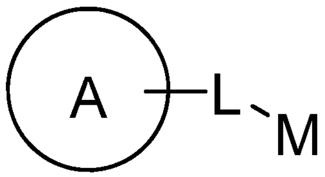

The organic metal compound (16) is preferably a corresponding Grignard reagent or organic lithium reagent. The organic metal compound (16) can be easily obtained as a commercially available product and is also produced by a method known per se in the art.
The amount of the organic metal compound (16) used is approximately 0.8 to approximately 30 mol, preferably approximately 1.0 to approximately 10 mol, with respect to 1 mol of the compound (15).
This reaction is advantageously performed without a solvent or using a solvent inert to the reaction. Such a solvent is not particularly limited as long as the reaction proceeds. Examples of a solvent used include: alcohols such as methanol, ethanol, and propanol; hydrocarbons such as hexane, cyclohexane, benzene, toluene, and xylene; ethers such as diethyl ether, diisopropyl ether, tetrahydrofuran, dioxane, and 1,2-dimethoxyethane; amides such as N,N-dimethylformamide, N,N-dimethylacetamide hexamethylphosphoric triamide; sulfoxides such as dimethyl sulfoxide; hydrogenated carbons such as dichloromethane, chloroform, carbon tetrachloride, and 1,2-dichloroethane; and mixed solvents thereof.
The reaction time is usually approximately 30 minutes to approximately 72 hours, preferably approximately 1 hour to approximately 48 hours. The reaction temperature is usually approximately -20 to approximately 200°C, preferably approximately 0 to approximately 100°C.
Furthermore, according to Reaction Scheme 8, the compound (If) can also be produced by reducing the compound (Ie) with a reducing agent.

For example, metal hydride such as sodium borohydride, lithium borohydride, sodium cyanoborohydride, sodium triacetoxyborohydride, lithium aluminum hydride, sodium bis(2-methoxyethoxy)aluminum hydride, a borane-tetrahydrofuran complex, or diisobutyl aluminum hydride, or a silane such as triethylsilane is used as the "reducing agent". A mineral acid (e.g., hydrochloric acid and hydrobromic acid), an organic acid (e.g., acetic acid, p-toluenesulfonic acid, and trifluoromethanesulfonic acid), or a Lewis acid (e.g., titanium tetrachloride and ytterbium trifluoromethanesulfonate) may be added thereto, if desired.
The amount of the reducing agent used is approximately 0.8 to approximately 20.0 mol, preferably approximately 1.0 to approximately 10.0 mol, with respect to 1 mol of the compound (Ie).
The amount of the acid used is approximately 0.1 to approximately 10000 mol, preferably approximately 1 to approximately 2000 mol, with respect to 1 mol of the compound (Ie).
This reaction is advantageously performed using a solvent inert to the reaction. Such a solvent is not particularly limited as long as the reaction proceeds. Preferable examples thereof include: alcohols such as methanol, ethanol, and propanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.
The reaction time is usually approximately 30 minutes to approximately 72 hours, preferably approximately 1 hour to approximately 48 hours. The reaction temperature is usually approximately -20 to approximately 200°C, preferably approximately 0 to approximately 120°C.

Reaction Scheme 9

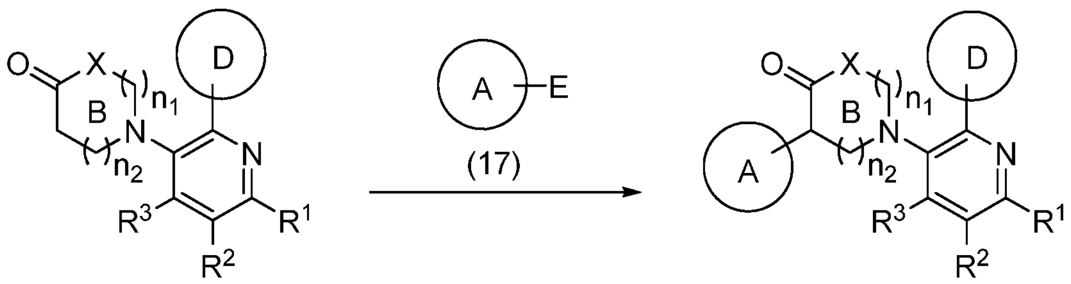
(17)

(15) (Ig)

In Reaction Scheme 9, $n_1$ and $n_2$ each represent an integer of 0 to 3 for forming an atomic group constituting 6- or 7-membered ring B in the compound (Ig); E represents a leaving group; and the other symbols are as defined above.
According to Reaction Scheme 9, the compound (Ig) is also produced by reacting a compound (15) with a compound (17) represented by the formula:

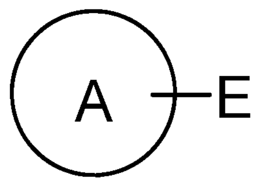

in the presence of a transition metal catalyst and a base.
The compound (17) can be easily obtained as a commercially available product and is also produced by a method known per se in the art.
Examples of the "leaving group" represented by E include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).
Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro.
Specific examples thereof include benzenesulfonyloxy, m-nitrobenzenesulfonyloxy, and p-toluenesulfonyloxy.
The amount of the compound (17) used is approximately 0.8 to approximately 10.0 mol, preferably approximately 1.0

to approximately 3.0 mol, with respect to 1 mol of the compound (15).
Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, N-ethyldiisopropylamine, tributylamine, cyclohexyldimethylamine, 4-dimethylaminopyridine, N,N-dimethylaniline, N-methylpiperidine, N-methylpyrrolidine, and N-methylmorpholine; and metal alkoxides such as sodium methoxide, sodium ethoxide, sodium tert-butoxide, and potassium tert-butoxide.
The amount of the base used is approximately 0.8 to approximately 10.0 mol, preferably approximately 1.0 to approximately 5.0 mol, with respect to 1 mol of the compound (15).
For example, a palladium catalyst such as palladium acetate, palladium chloride, tetrakis(triphenylphosphine)palladium, tris(dibenzylideneacetone)dipalladium, 1,1-bis(diphenylphosphino)ferrocenedichloropalladium, dichlorobis(triphenylphosphine)palladium, bis(tri(tert-butylphosphine))palladium, phenylallylchloro-[1,3-bis(diisopropylphenyl)imidazol-2-ylidene]palladium, or phenylallylchloro-[1,3-bis(diisopropylphenyl)-2-imidazolidinylidene]palladium is used as the "transition metal catalyst". A ligand such as triphenylphosphine, tri(tert-butyl)phosphine, 2,2'-bis(diphenylphosphino)-1,1'-binaphthyl, 2-(dicyclohexylphosphino)-2',4',6'-triisopropyl-1,1'-biphenyl, or 4,5'-bis(diphenylphosphino)-9,9'-dimethylxanthene may be added thereto, if desired.
The amount of the transition metal catalyst used is approximately 0.001 to approximately 3 mol, preferably approximately 0.02 to approximately 0.2 mol, with respect to 1 mol of the compound (15).
The amount of the ligand used is approximately 0.001 to approximately 3 mol, preferably approximately 0.05 to approximately 0.5 mol, with respect to 1 mol of the compound (15).
Examples of a solvent used include: ethers such as diethyl ether, diisopropyl ether, tetrahydrofuran, dioxane, and 1,2-dimethoxyethane; alcohols such as methanol, ethanol, and propanol; hydrocarbons such as benzene, toluene, carbon disulfide, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; water; and mixed solvents thereof.
The reaction temperature is usually approximately 0°C to 250°C, preferably approximately 50°C to 150°C. The reaction time is usually approximately 5 minutes to approximately 48 hours, preferably approximately 30 minutes to approximately 24 hours.
In this reaction, the reaction time can be shortened by use of a microwave reactor or the like.

Reaction Scheme 10

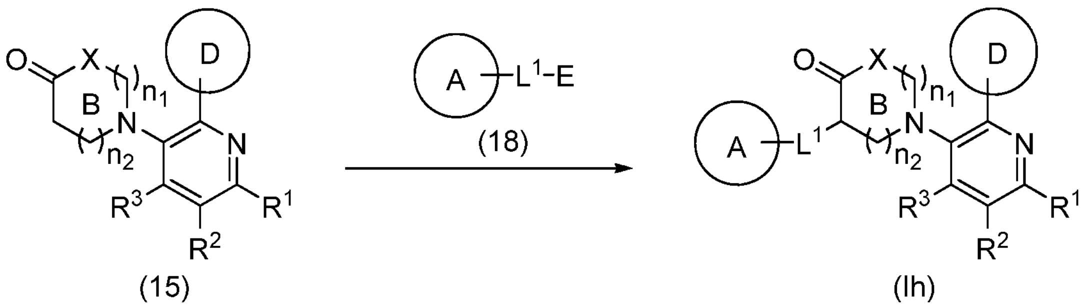

In Reaction Scheme 10, $n_1$ and $n_2$ each represent an integer of 0 to 3 for forming an atomic group constituting 6- or 7-membered ring B in the compound (Ih); E represents a leaving group; $L^1$ represents methylene or ethylene which may be substituted; and the other symbols are as defined above.
According to Reaction Scheme 10, the compound (Ih) is also produced by reacting a compound (15) with a compound (18) represented by the formula:

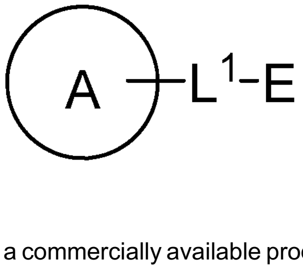

if desired, in the presence of a base.
The compound (18) can be easily obtained as a commercially available product and is also produced by a method known per se in the art.

Examples of the "leaving group" represented by E include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).

Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.

The amount of the compound (18) used is approximately 0.8 to approximately 5.0 mol, preferably approximately 1.0 to approximately 2.0 mol, with respect to 1 mol of the compound (15).

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.

The amount of the base used is approximately 0.5 to approximately 10.0 mol, preferably approximately 1.0 to approximately 3.0 mol, with respect to 1 mol of the compound (15). The compound is also produced by performing the reaction, if desired, in the presence of quaternary ammonium salt or metal iodide, together with the base.

Examples of the "quaternary ammonium salt" include tetrabutyl ammonium iodide.

Examples of the "metal iodide" include sodium iodide and potassium iodide.

The amount of the quaternary ammonium salt used is approximately 0.1 to approximately 3.0 mol, preferably approximately 0.5 to approximately 1.0 mol, with respect to 1 mol of the compound (15).

The amount of the metal iodide used is approximately 0.1 to approximately 3.0 mol, preferably approximately 0.5 to approximately 1.0 mol, with respect to 1 mol of the compound (15).

This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.

Reaction Scheme 11

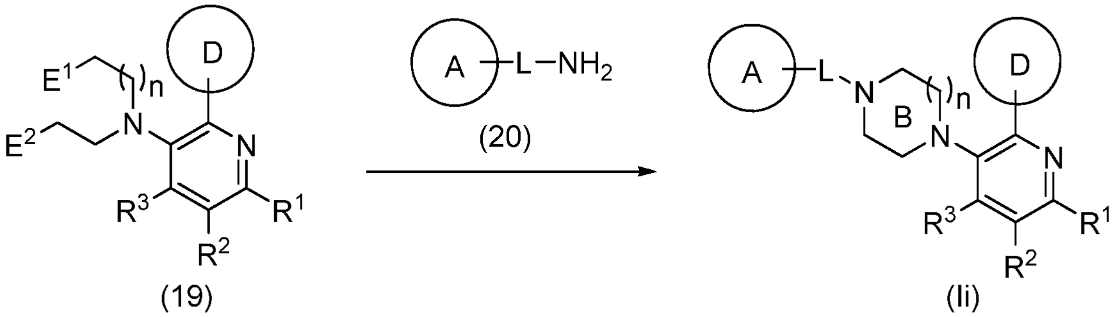

(19) (20) (Ii)

In Reaction Scheme 11, $E^1$ and $E^2$ represent the same or different leaving groups, and the other symbols are as defined above.

According to Reaction Scheme 11, the compound (Ii) is also produced by reacting a compound (19) with a compound (20) represented by the formula:

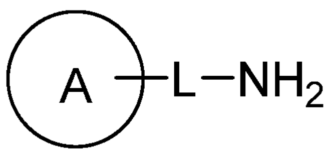

if desired, in the presence of a base. The newly formed bonds may be formed simultaneously or stepwise.
The compound (20) can be easily obtained as a commercially available product and is also produced by a method known per se in the art.
Examples of the "leaving group" represented by $E^1$ and $E^2$ include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).
Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.
The amount of the compound (20) used is approximately 0.8 to approximately 10.0 mol, preferably approximately 1.0 to approximately 5.0 mol, with respect to 1 mol of the compound (19).
Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.
The amount of the base used is approximately 0.5 to approximately 10.0 mol, preferably approximately 1.0 to approximately 3.0 mol, with respect to 1 mol of the compound (19). The compound is also produced by performing the reaction, if desired, in the presence of quaternary ammonium salt or metal iodide, together with the base.
Examples of the "quaternary ammonium salt" include tetrabutyl ammonium iodide.
Examples of the "metal iodide" include sodium iodide and potassium iodide.
The amount of the quaternary ammonium salt used is approximately 0.1 to approximately 3.0 mol, preferably approximately 0.5 to approximately 1.0 mol, with respect to 1 mol of the compound (19).
The amount of the metal iodide used is approximately 0.1 to approximately 3.0 mol, preferably approximately 0.5 to approximately 1.0 mol, with respect to 1 mol of the compound (19).
This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.
The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.

Reaction Scheme 12

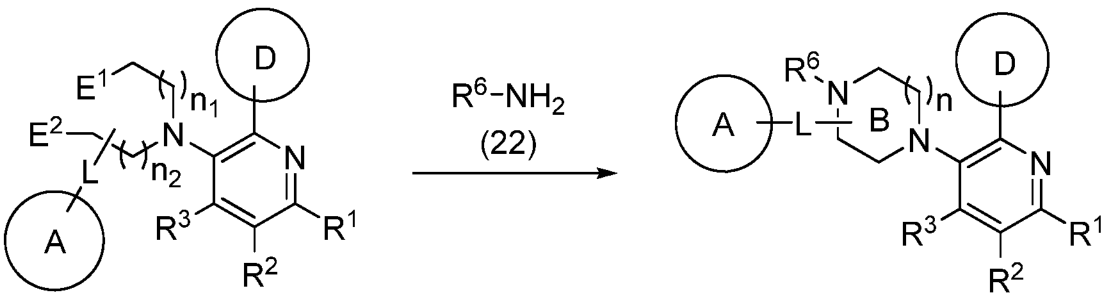


(21) (Ij)

In Reaction Scheme 12, $E^1$ and $E^2$ represent the same or different leaving groups; $n_1$ and $n_2$ each represent 1 or 2 (excluding, however, the cases where both of $n_1$ and $n_2$ are 2); $R^6$ represents an alkyl or aryl group which may be substituted; and the other symbols are as defined above.
According to Reaction Scheme 12, the compound (Ij) is also produced by reacting a compound (21) with a compound (22) represented by the formula: $R^6$-$NH_2$, if desired, in the presence of a base. The newly formed bonds may be formed

simultaneously or stepwise.

The compound (22) can be easily obtained as a commercially available product and is also produced by a method known per se in the art.

Examples of the "leaving group" represented by $E^1$ and $E^2$ include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).

Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.

The amount of the compound (22) used is approximately 0.8 to approximately 5.0 mol, preferably approximately 1.0 to approximately 2.0 mol, with respect to 1 mol of the compound (21).

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.

The amount of the base used is approximately 0.5 to approximately 10.0 mol, preferably approximately 1.0 to approximately 3.0 mol, with respect to 1 mol of the compound (21). The compound is also produced by performing the reaction, if desired, in the presence of quaternary ammonium salt or metal iodide, together with the base.

Examples of the "quaternary ammonium salt" include tetrabutyl ammonium iodide.

Examples of the "metal iodide" include sodium iodide and potassium iodide.

The amount of the quaternary ammonium salt used is approximately 0.1 to approximately 3.0 mol, preferably approximately 0.5 to approximately 1.0 mol, with respect to 1 mol of the compound (21).

The amount of the metal iodide used is approximately 0.1 to approximately 3.0 mol, preferably approximately 0.5 to approximately 1.0 mol, with respect to 1 mol of the compound (21).

This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.

Reaction Scheme 13

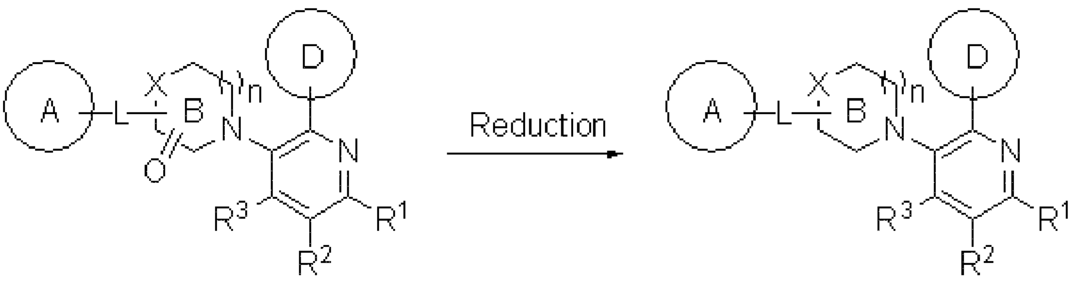


(Ik) (I)

In Reaction Scheme 13, the symbols are as defined above.

The compound (Ik) has an oxo group on ring B and is encompassed in the compound (I).

The compound (I) is also produced by reducing the compound (Ik) with a reducing agent.

For example, metal hydride such as thorium borohydride, lithium aluminum hydride, sodium bis(2-methoxyethoxy)aluminum hydride, a borane-tetrahydrofuran complex, or diisobutyl aluminum hydride is used as the "reducing agent". A Lewis acid such as titanium tetrachloride or aluminum chloride may be added thereto, if desired.

The amount of the reducing agent used is approximately 0.8 to approximately 10.0 mol, preferably approximately 1.0

to approximately 5.0 mol, with respect to 1 mol of the compound (Ik).

The amount of the Lewis acid used is approximately 0.8 to approximately 10.0 mol, preferably approximately 1.0 to approximately 5.0 mol, with respect to 1 mol of the compound (Ik).

This reaction is advantageously performed using a solvent inert to the reaction. Such a solvent is not particularly limited as long as the reaction proceeds. Preferable examples thereof include: alcohols such as methanol, ethanol, and propanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

The reaction time is usually approximately 30 minutes to approximately 72 hours, preferably approximately 1 hour to approximately 48 hours. The reaction temperature is usually approximately -20 to approximately 200°C, preferably approximately 0 to approximately 120°C.

A reaction solution of the product or its crude product can be used directly in the next reaction. Alternatively, the product may be isolated from the reaction mixture according to a routine method or may be easily purified by usual separation means (e.g., recrystallization, distillation, and chromatography).

Reaction Scheme 14

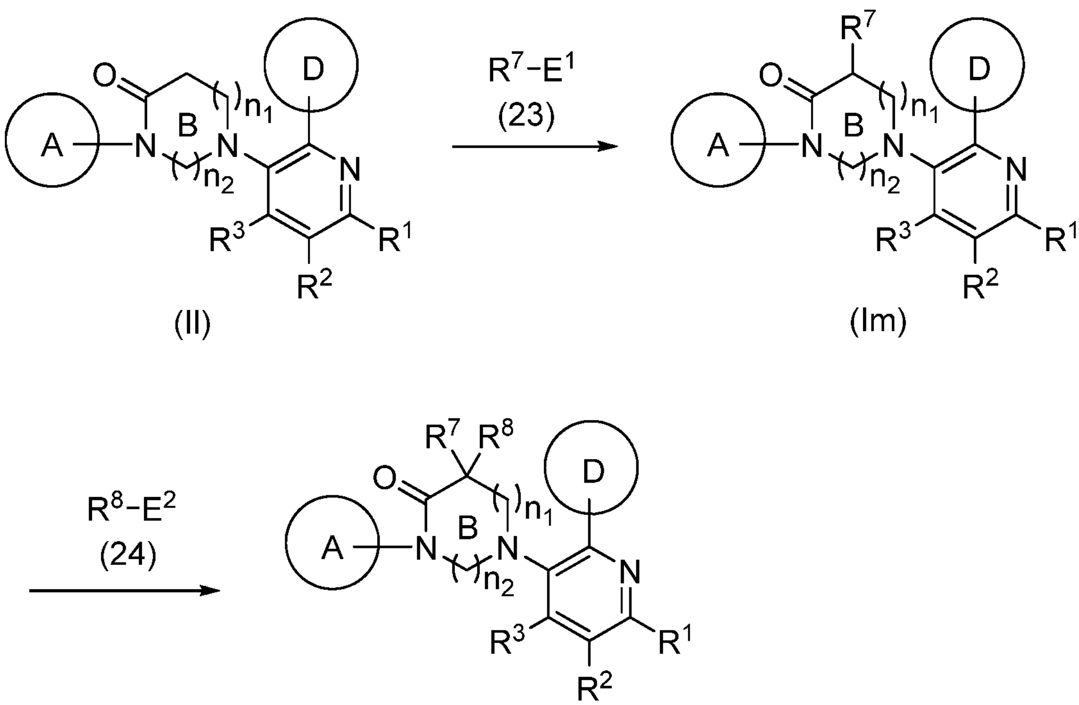


(Il) (Im) (In)

In Reaction Scheme 14, $E^1$ and $E^2$ represent the same or different leaving groups; $n_1$ and $n_2$ represent integers of 0 to 2 and 2 to 3, respectively, for forming an atomic group constituting 6- or 7-membered ring B in the compounds (I1), (Im), and (In); $R^7$ and $R^8$ each represent an alkyl group which may be substituted; and the other symbols are as defined above.

According to Reaction Scheme 14, the compound (Im) is also produced by reacting a compound (I1) with a compound (23) represented by the formula: $R^7$-$E^1$, if desired, in the presence of a base.

The compound (23) can be easily obtained as a commercially available product and is also produced by a method known per se in the art.

Examples of the "leaving group" represented by $E^1$ include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).

Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.
The amount of the compound (23) used is approximately 0.8 to approximately 5.0 mol, preferably approximately 1.0 to approximately 2.0 mol, with respect to 1 mol of the compound (I1).
Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.
The amount of the base used is approximately 0.5 to approximately 10.0 mol, preferably approximately 1.0 to approximately 3.0 mol, with respect to 1 mol of the compound (I1). The compound is also produced by performing the reaction, if desired, in the presence of quaternary ammonium salt or metal iodide, together with the base.
Examples of the "quaternary ammonium salt" include tetrabutyl ammonium iodide.
Examples of the "metal iodide" include sodium iodide and potassium iodide.
The amount of the quaternary ammonium salt used is approximately 0.1 to approximately 3.0 mol, preferably approximately 0.5 to approximately 1.0 mol, with respect to 1 mol of the compound (I1).
The amount of the metal iodide used is approximately 0.1 to approximately 3.0 mol, preferably approximately 0.5 to approximately 1.0 mol, with respect to 1 mol of the compound (I1).
This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.
The reaction time is usually approximately 30 minutes to approximately 72 hours, preferably approximately 3 hours to approximately 24 hours. The reaction temperature is usually approximately -100 to approximately 100°C, preferably approximately -80 to approximately 50°C.
A reaction solution of the product or its crude product can be used directly in the next reaction. Alternatively, the product may be isolated from the reaction mixture according to a routine method or may be easily purified by usual separation means (e.g., recrystallization, distillation, and chromatography).
Furthermore, according to Reaction Scheme 14, the compound (In) is also produced by reacting the compound (Im) with a compound (24) represented by the formula: $R^8$-$E^2$, if desired, in the presence of a base.
The compound (24) can be easily obtained as a commercially available product and is also produced by a method known per se in the art.
Examples of the "leaving group" represented by $E^2$ include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).
Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.
The amount of the compound (24) used is approximately 0.8 to approximately 5.0 mol, preferably approximately 1.0 to approximately 2.0 mol, with respect to 1 mol of the compound (Im).
Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.
The amount of the base used is approximately 0.5 to approximately 10.0 mol, preferably approximately 1.0 to approximately 3.0 mol, with respect to 1 mol of the compound (Im). The compound is also produced by performing the reaction, if desired, in the presence of quaternary ammonium salt or metal iodide, together with the base.
Examples of the "quaternary ammonium salt" include tetrabutyl ammonium iodide.
Examples of the "metal iodide" include sodium iodide and potassium iodide.
The amount of the quaternary ammonium salt used is approximately 0.1 to approximately 3.0 mol, preferably approximately 0.5 to approximately 1.0 mol, with respect to 1 mol of the compound (Im).
The amount of the metal iodide used is approximately 0.1 to approximately 3.0 mol, preferably approximately 0.5 to

approximately 1.0 mol, with respect to 1 mol of the compound (Im).
This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.
The reaction time is usually approximately 30 minutes to approximately 72 hours, preferably approximately 3 hours to approximately 24 hours. The reaction temperature is usually approximately -100 to approximately 100°C, preferably approximately -80 to approximately 50°C.
When $R^7$ and $R^8$ are the same as each other and $E^1$ and $E^2$ are the same as each other, the compound (In) is directly produced by reacting the compound (I1) with the compound (23) (= compound (24)) represented by the formula: $R^7$-$E^1$ (= $R^8$-$E^2$), if desired, in the presence of a base.
The compound (23) (= compound (24)) can be easily obtained as a commercially available product and is also produced by a method known per se in the art.
Examples of the "leaving group" represented by $E^1$ (= $E^2$) include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).
Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.
The amount of the compound (23) (= compound (24)) used is approximately 0.8 to approximately 10.0 mol, preferably approximately 2.0 to approximately 4.0 mol, with respect to 1 mol of the compound (I1).
Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.
The amount of the base used is approximately 0.5 to approximately 20.0 mol, preferably approximately 2.0 to approximately 6.0 mol, with respect to 1 mol of the compound (I1). The compound is also produced by performing the reaction, if desired, in the presence of quaternary ammonium salt or metal iodide, together with the base.
Examples of the "quaternary ammonium salt" include tetrabutyl ammonium iodide.
Examples of the "metal iodide" include sodium iodide and potassium iodide.
The amount of the quaternary ammonium salt used is approximately 0.1 to approximately 5.0 mol, preferably approximately 0.5 to approximately 2.0 mol, with respect to 1 mol of the compound (I1).
The amount of the metal iodide used is approximately 0.1 to approximately 5.0 mol, preferably approximately 0.5 to approximately 2.0 mol, with respect to 1 mol of the compound (I1).
This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.
The reaction time is usually approximately 30 minutes to approximately 72 hours, preferably approximately 3 hours to approximately 24 hours. The reaction temperature is usually approximately -100 to approximately 100°C, preferably approximately -80 to approximately 50°C.
A reaction solution of the product or its crude product can be used directly in the next reaction. Alternatively, the product may be isolated from the reaction mixture according to a routine method or may be easily purified by usual separation means (e.g., recrystallization, distillation, and chromatography).

Reaction Scheme 15

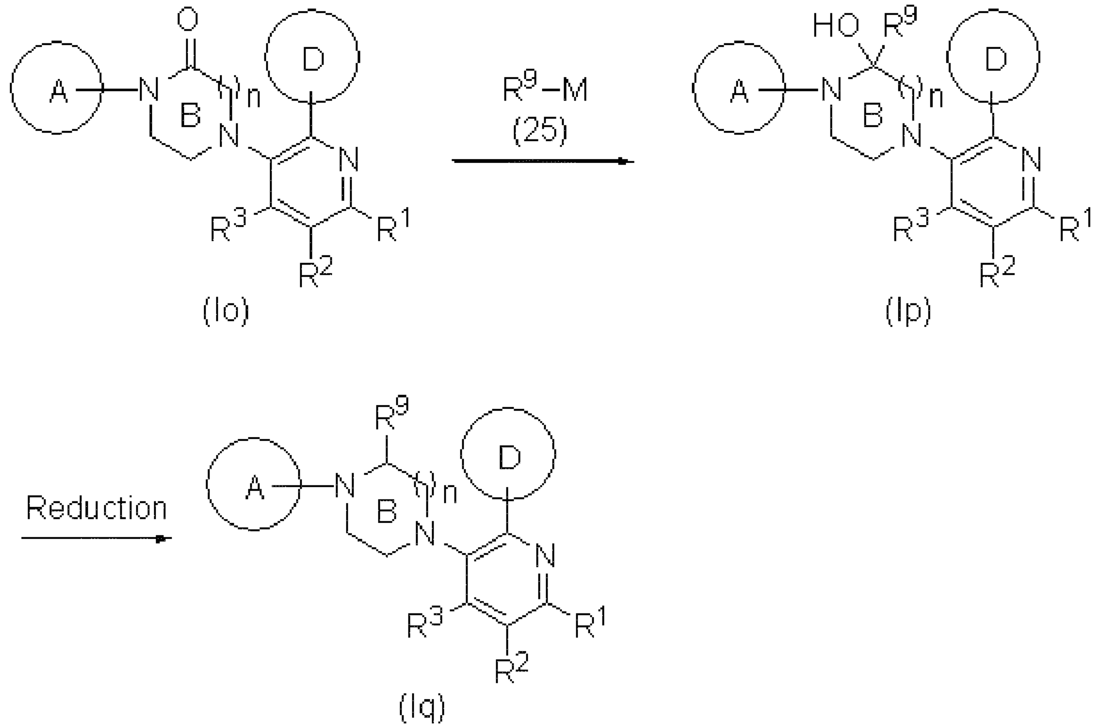


(Io) (Ip) (Iq)

In Reaction Scheme 15, n represents 1 or 2; $R^9$ represents an alkyl group or aryl group which may be substituted; M represents a metal; and the other symbols are as defined above.

According to Reaction Scheme 15, the compound (Ip) can also be produced by reacting a compound (Io) with an organic metal compound (25) represented by the formula: $R^9$-M.

The organic metal compound (25) is preferably a corresponding Grignard reagent or organic lithium reagent. The organic metal compound (25) can be easily obtained as a commercially available product and is also produced by a method known per se in the art.

The amount of the organic metal compound (25) used is approximately 0.8 to approximately 30 mol, preferably approximately 1.0 to approximately 10 mol, with respect to 1 mol of the compound (Io).

This reaction is advantageously performed without a solvent or using a solvent inert to the reaction. Such a solvent is not particularly limited as long as the reaction proceeds. Examples of a solvent used include: alcohols such as methanol, ethanol, and propanol; hydrocarbons such as hexane, cyclohexane, benzene, toluene, and xylene; ethers such as diethyl ether, diisopropyl ether, tetrahydrofuran, dioxane, and 1,2-dimethoxyethane; amides such as N,N-dimethylformamide, N,N-dimethylacetamide hexamethylphosphoric triamide; sulfoxides such as dimethyl sulfoxide; hydrogenated carbons such as dichloromethane, chloroform, carbon tetrachloride, and 1,2-dichloroethane; and mixed solvents thereof.

The reaction time is usually approximately 30 minutes to approximately 72 hours, preferably approximately 1 hour to approximately 48 hours. The reaction temperature is usually approximately -20 to approximately 200°C, preferably approximately 0 to approximately 100°C.

Furthermore, according to Reaction Scheme 15, the compound (Iq) can also be produced by reducing the compound (Ip) with a reducing agent.

For example, metal hydride such as sodium borohydride, lithium borohydride, sodium cyanoborohydride, sodium triacetoxyborohydride, lithium aluminum hydride, sodium bis(2-methoxyethoxy)aluminum hydride, a borane-tetrahydrofuran complex, or diisobutyl aluminum hydride, or a silane such as triethylsilane is used as the "reducing agent". A mineral acid (e.g., hydrochloric acid and hydrobromic acid), an organic acid (e.g., acetic acid, p-toluenesulfonic acid, and trifluoromethanesulfonic acid), or a Lewis acid (e.g., titanium tetrachloride and ytterbium trifluoromethanesulfonate) may be added thereto, if desired.

The amount of the reducing agent used is approximately 0.8 to approximately 20.0 mol, preferably approximately 1.0 to approximately 10.0 mol, with respect to 1 mol of the compound (Ip).

The amount of the acid used is approximately 0.1 to approximately 10000 mol, preferably approximately 1 to approximately 2000 mol, with respect to 1 mol of the compound (Ip).

This reaction is advantageously performed using a solvent inert to the reaction. Such a solvent is not particularly limited as long as the reaction proceeds. Preferable examples thereof include: alcohols such as methanol, ethanol, and propanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

The reaction time is usually approximately 30 minutes to approximately 72 hours, preferably approximately 1 hour to approximately 48 hours. The reaction temperature is usually approximately -20 to approximately 200°C, preferably approximately 0 to approximately 120°C.

Reaction Scheme 16

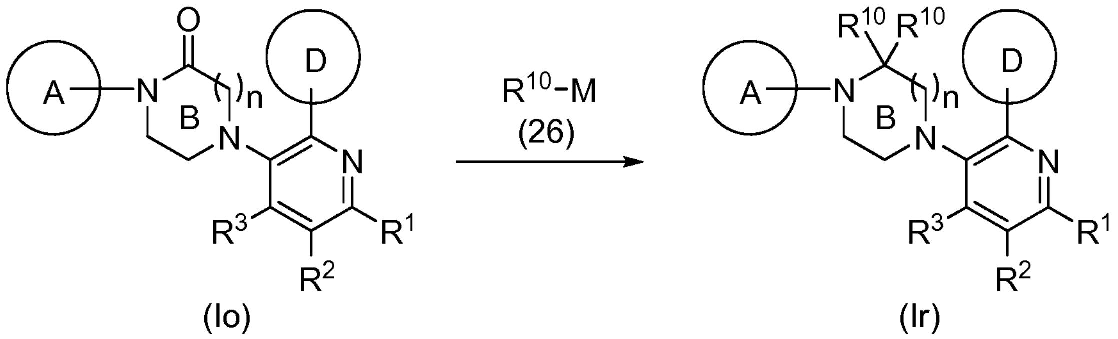


In Reaction Scheme 16, n represents 1 or 2; $R^{10}$ represents an alkyl or aryl group which may be substituted; M represents a metal; and the other symbols are as defined above.

According to Reaction Scheme 16, the compound (Ir) can also be produced by reacting a compound (Io) with an organic metal compound (26) represented by the formula: $R^{10}$-M, if desired, in the presence of a Lewis acid.

The organic metal compound (26) is preferably a corresponding Grignard reagent or organic lithium reagent. The organic metal compound (26) can be easily obtained as a commercially available product and is also produced by a method known per se in the art.

The amount of the organic metal compound (26) used is approximately 0.8 to approximately 30 mol, preferably approximately 1.0 to approximately 10 mol, with respect to 1 mol of the compound (Io).

Examples of the "Lewis acid" include titanium tetrachloride and aluminum chloride.

The amount of the Lewis acid used is approximately 0.8 to approximately 10.0 mol, preferably approximately 1.0 to approximately 5.0 mol, with respect to 1 mol of the compound (Io).

This reaction is advantageously performed using a solvent inert to the reaction. Such a solvent is not particularly limited as long as the reaction proceeds. Examples of a solvent used include: alcohols such as methanol, ethanol, and propanol; hydrocarbons such as hexane, cyclohexane, benzene, toluene, and xylene; ethers such as diethyl ether, diisopropyl ether, tetrahydrofuran, dioxane, and 1,2-dimethoxyethane,; amides such as N,N-dimethylformamide, N,N-dimethylacetamide hexamethylphosphoric triamide; sulfoxides such as dimethyl sulfoxide; hydrogenated carbons such as dichloromethane, chloroform, carbon tetrachloride, and 1,2-dichloroethane; and mixed solvents thereof.

The reaction time is usually approximately 30 minutes to approximately 72 hours, preferably approximately 1 hour to approximately 48 hours. The reaction temperature is usually approximately -100 to approximately 100°C, preferably approximately -80 to approximately 50°C.

In the compound (I) of the present invention represented by the formula:

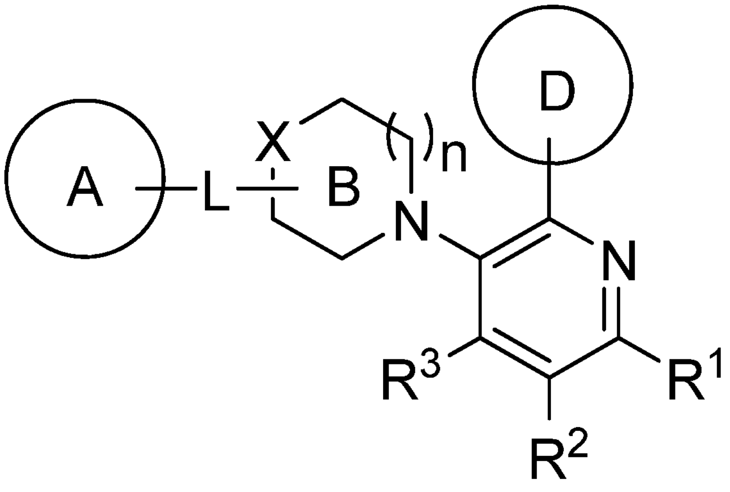

(I)

the substituents $R^1$, $R^2$, and $R^3$, a substituent(s) (other than ring A-L-) bonded to a replaceable atom constituting ring B bonded to the pyridine ring, a substituent(s) on ring A and ring D, and a substituent(s) bonded to carbon constituting the linker L (except that L is a bond) can be converted by usual organic reaction, for example, reduction reaction, oxidation reaction, substitution reaction, alkylation reaction, hydrolysis reaction, addition reaction with an alkyllithium reagent or a Grignard reagent, aldol reaction, coupling reaction using a palladium catalyst or the like such as Suzuki-Miyaura coupling reaction or Buchwald amination reaction, dehydration-condensation reaction such as esterification or amidation, and reductive alkylation reaction.

The product may be isolated from the reaction mixture according to a routine method or may be easily purified by usual separation means (e.g., recrystallization, distillation, and chromatography).

The compounds (2) and (4) are produced by a method known per se in the art or a method equivalent thereto, or the like. Moreover, the compound (2a) encompassed in the compound (2) and the compound (4) are also produced by a method described in the following reaction scheme:

Reaction Scheme 17

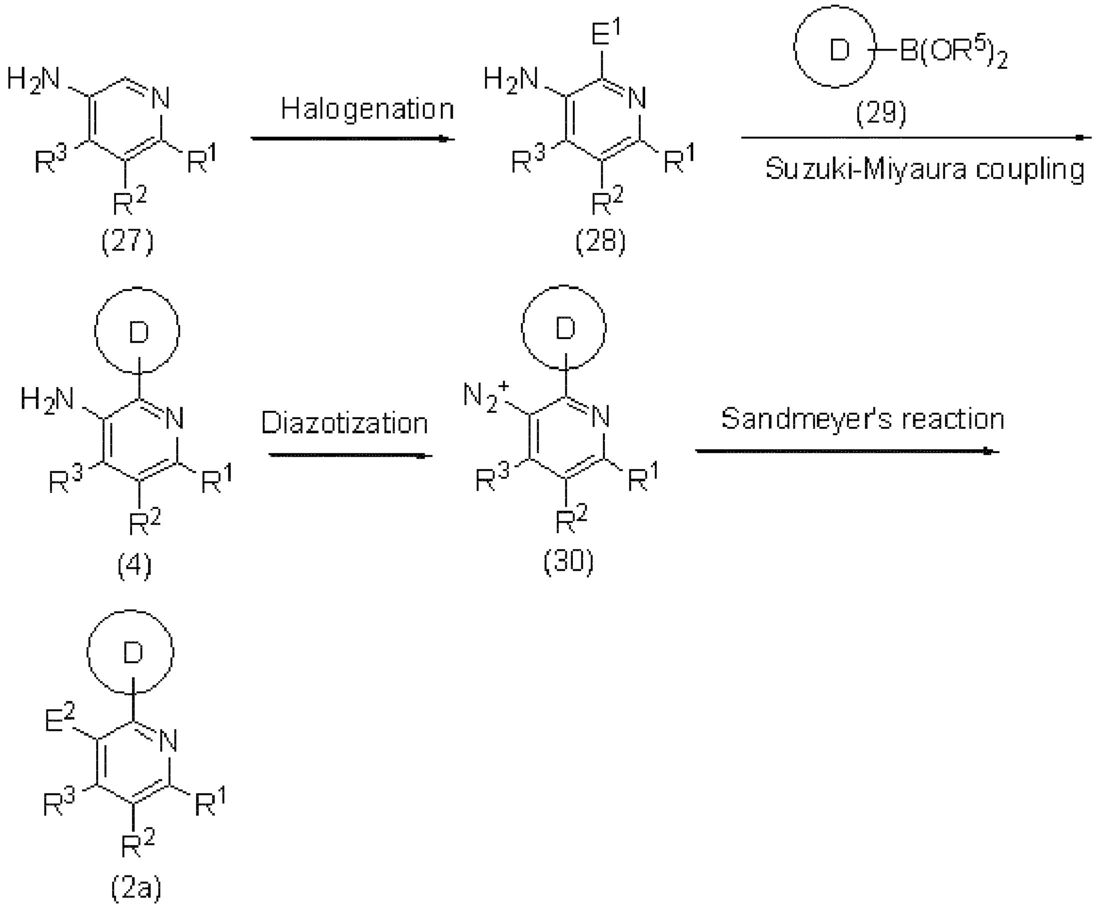


(27) (28) (4) (30) (2a)

In Reaction Scheme 17, $E^1$ and $E^2$ represent the same or different halogen atoms; the functional group represented by $B(OR^5)_2$ represents boronic acid or boronic acid ester (e.g., 4,4,5,5-tetramethyl-1,3,2-dioxaborolan-2-yl); and the other symbols are as defined above.

The compound (28) is produced by reacting a compound (27) with a halogenation reagent.

The compound (27) can be easily obtained as a commercially available product and is also produced by a method known per se in the art.

Chlorine, bromine, iodine, an imide (e.g., N-chlorosuccinimide and N-bromosuccinimide), a halogen adduct (e.g., benzyltrimethyl ammonium tribromide), or the like is used as the "halogenation reagent". The amount of the halogenation reagent used is approximately 0.8 to approximately 10.0 mol, preferably approximately 1.0 to approximately 5.0 mol, with respect to 1 mol of the compound (27).

This reaction is advantageously performed using a solvent inert to the reaction. Such a solvent is not particularly limited as long as the reaction proceeds. Examples of a solvent used include: ethers such as diethyl ether, diisopropyl ether, tetrahydrofuran, dioxane, and 1,2-dimethoxyethane; alcohols such as methanol, ethanol, and propanol; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; organic acids such as acetic acid and propionic acid; nitroalkanes such as nitromethane; aromatic amines such as pyridine, lutidine, and quinoline; and mixed solvents thereof.

This reaction is performed, if desired, in the presence of a base, an acid, or iron, or two or more of these three.

Examples of the "base" include: basic salts such as sodium carbonate, calcium carbonate, cesium carbonate, sodium bicarbonate, sodium acetate, sodium acetate, and potassium acetate; aromatic amines such as pyridine, and lutidine; and tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine. The amount of the base used is approximately 0.8 to approximately 10 mol with respect to 1 mol of the compound (27).

Examples of the "acid" include: organic acids such as acetic acid and p-toluenesulfonic acid; and Lewis acids such as iron chloride, aluminum chloride, and boron trifluoride. The amount of the acid used is approximately 0.01 to approximately 5 mol with respect to 1 mol of the compound (27).

The amount of the "iron" used is approximately 0.01 to approximately 5 mol with respect to 1 mol of the compound (27).

The reaction temperature is usually approximately - 50 to approximately 150°C, preferably approximately -20 to approximately 100°C. The reaction time is usually approximately 5 minutes to approximately 24 hours, preferably approximately 10 minutes to approximately 12 hours.

A reaction solution of the product or its crude product can be used directly in the next reaction. Alternatively, the product may be isolated from the reaction mixture according to a routine method or may be easily purified by usual separation means (e.g., recrystallization, distillation, and chromatography).

The compound (4) is produced by reacting the compound (28) with a compound (29) through Suzuki-Miyaura coupling reaction in the presence of a transition metal catalyst and a base.

The compound (29) can be easily obtained as a commercially available product and is also produced by a method known per se in the art.

The amount of the compound (29) used is approximately 0.5 to approximately 10 mol, preferably approximately 0.9 to approximately 3 mol, with respect to 1 mol of the compound (28).

For example, alkali metal or alkaline earth metal carbonate (e.g., sodium carbonate and potassium carbonate), alkali metal or alkaline earth metal bicarbonate (e.g., sodium bicarbonate and potassium bicarbonate), alkali metal or alkaline earth metal hydroxide (e.g., sodium hydroxide and potassium hydroxide), triethylamine, 4-dimethylaminopyridine, N-ethyldiisopropylamine, triethylenediamine, or 4-methylmorpholine is used as the "base". The amount of the base used is approximately 0.8 to approximately 10.0 mol, preferably approximately 1.0 to approximately 5.0 mol, with respect to 1 mol of the compound (28).

For example, a palladium catalyst such as palladium acetate, palladium chloride, tetrakis(triphenylphosphine)palladium, tris(dibenzylideneacetone)dipalladium, 1,1-bis(diphenylphosphino)ferrocenedichloropalladium, dichlorobis(triphenylphosphine)palladium, bis(tri(tert-butylphosphine))palladium, phenylallylchloro-[1,3-bis(diisopropylphenyl)imidazol-2-ylidene]palladium, phenylallylchloro-[1,3-bis(diisopropylphenyl)-2-imidazolidinylidene]palladium is used as the "transition metal catalyst". A ligand such as triphenylphosphine, tri(tert-butyl)phosphine, 2,2'-bis(diphenylphosphino)-1,1'-binaphthyl, 2-(dicyclohexylphosphino)-2',4',6'-triisopropyl-1,1'-biphenyl, or 4,5'-bis(diphenylphosphino)-9,9'-dimethylxanthene may be added thereto, if desired.

The amount of the transition metal catalyst used is approximately 0.001 to approximately 3 mol, preferably approximately 0.02 to approximately 0.2 mol, with respect to 1 mol of the compound (28).

The amount of the ligand used is approximately 0.001 to approximately 3 mol, preferably approximately 0.05 to approximately 0.5 mol, with respect to 1 mol of the compound (28).

Examples of a solvent used include: ethers such as diethyl ether, diisopropyl ether, tetrahydrofuran, dioxane, and 1,2-dimethoxyethane; alcohols such as methanol, ethanol, and propanol; hydrocarbons such as benzene, toluene, carbon disulfide, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; water: and mixed solvents thereof.

The reaction temperature is usually approximately 0°C to 250°C, preferably approximately 50°C to approximately 150°C. The reaction time is usually approximately 5 minutes to approximately 48 hours, preferably approximately 30 minutes to approximately 24 hours.

In this reaction, the reaction time can be shortened by use of a microwave reactor or the like.

A reaction solution of the product or its crude product can be used directly in the next reaction. Alternatively, the product may be isolated from the reaction mixture according to a routine method or may be easily purified by usual separation means (e.g., recrystallization, distillation, and chromatography).

The compound (30) is produced by reacting the compound (4) with a nitrous acid in an acidic solution.

Nitrous acid, sodium nitrite, ethyl nitrite, amyl nitrite, isoamyl nitrite, or the like is used as the "nitrous acid". The amount of the nitrous acid used is approximately 0.8 to approximately 10.0 mol, preferably approximately 1.0 to approximately 5.0 mol, with respect to 1 mol of the compound (4).

Examples of the "acid" include hydrochloric acid and hydrobromic acid. The amount of the acid used is approximately 1 to approximately 1000 mol with respect to 1 mol of the compound (4).

This reaction is advantageously performed using a solvent inert to the reaction. Such a solvent is not particularly limited as long as the reaction proceeds. Examples of a solvent used include: water; ethers such as diethyl ether, diisopropyl ether, dioxane, and 1,2-dimethoxyethane; alcohols such as methanol, ethanol, and propanol; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; organic acids such as acetic acid and propionic acid; nitroalkanes such as nitromethane; and mixed solvents thereof.

The reaction temperature is usually approximately - 50 to approximately 150°C, preferably approximately 0°C to approximately 100°C. The reaction time is usually approximately 5 minutes to approximately 24 hours, preferably approximately 10 minutes to approximately 12 hours.

A reaction solution of the product or its crude product can be used directly in the next reaction. Alternatively, the product

may be isolated from the reaction mixture according to a routine method or may be easily purified by usual separation means (e.g., recrystallization, distillation, and chromatography).

The compound (2a) is produced by reacting the compound (30) with copper halide through Sandmeyer's reaction.

Copper chloride, copper bromide, or the like is used as the "copper halide". The amount of the copper halide used is approximately 0.8 to approximately 20.0 mol, preferably approximately 1.0 to approximately 5.0 mol, with respect to 1 mol of the compound (30).

This reaction is performed, if desired, in the presence of an acid.

Examples of the "acid" include hydrochloric acid and hydrobromic acid. The amount of the acid used is approximately 1 to approximately 1000 mol with respect to 1 mol of the compound (30).

This reaction is advantageously performed using a solvent inert to the reaction. Such a solvent is not particularly limited as long as the reaction proceeds. Examples of a solvent used include: water; ethers such as diethyl ether, diisopropyl ether, dioxane, and 1,2-dimethoxyethane; alcohols such as methanol, ethanol, and propanol; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; organic acids such as acetic acid and propionic acid; nitroalkanes such as nitromethane; and mixed solvents thereof.

The reaction temperature is usually approximately - 50 to approximately 150°C, preferably approximately 0°C to approximately 100°C. The reaction time is usually approximately 5 minutes to approximately 48 hours, preferably approximately 10 minutes to approximately 24 hours.

A reaction solution of the product or its crude product can be used directly in the next reaction. Alternatively, the product may be isolated from the reaction mixture according to a routine method or may be easily purified by usual separation means (e.g., recrystallization, distillation, and chromatography).

The compounds (6a) and (6b) are produced by a method known per se in the art or a method equivalent thereto, or the like.

The compounds (6a) and (6b) are also produced by a method described in the following reaction scheme:

Reaction Scheme 18

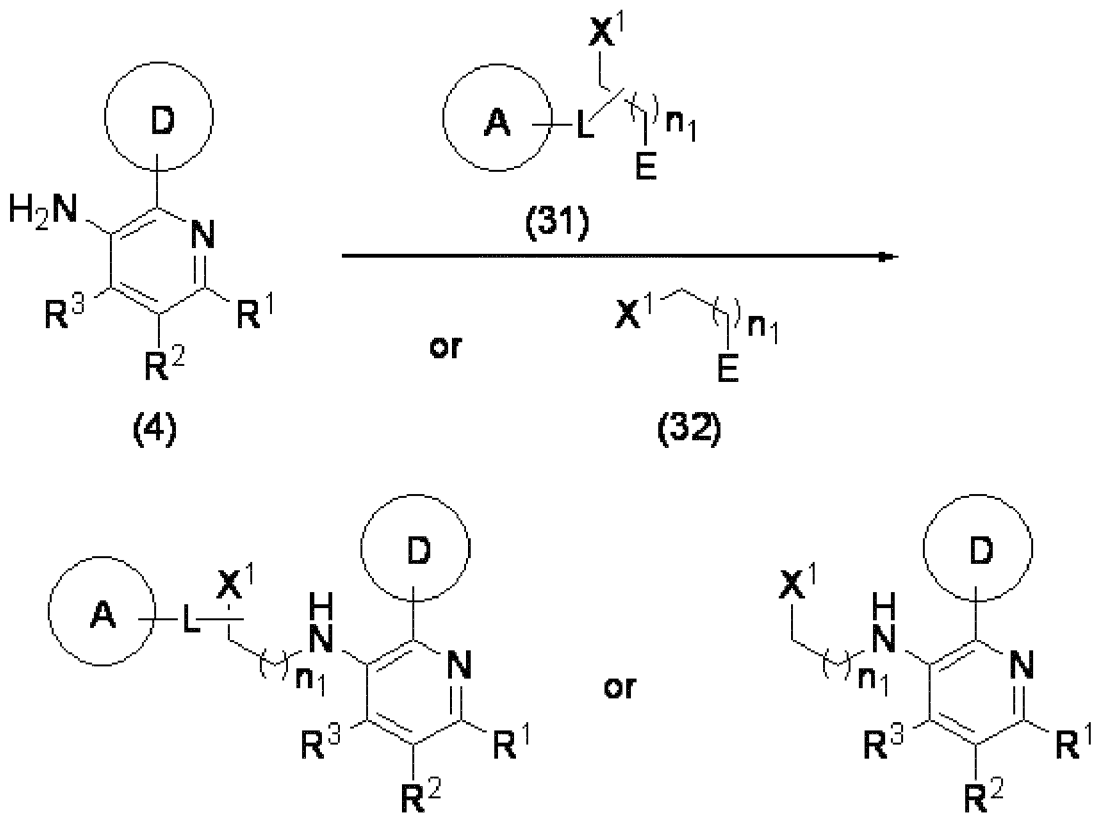

(31)

or

(4)

(32)

(6a)

or

(6b)

In Reaction Scheme 18, E represents a leaving group; $X^1$ represents oxygen or nitrogen which may be substituted

(which appropriately has a hydrogen atom according to the binding manner); $n_1$ represents 1 or 2; and the other symbols are as defined above.

The compounds (6a) and (6b) are produced by reacting compounds (31) and (32), respectively, with a compound (4), if desired, in the presence of a base or a condensing agent.

The compounds (31) and (32) can be easily obtained as a commercially available product and are also produced by a method known per se in the art.

Examples of the "leaving group" represented by E include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).

Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.

The amount of the compound (31) or (32) used is approximately 0.8 to approximately 10.0 mol, preferably approximately 1.0 to approximately 5.0 mol, with respect to 1 mol of the compound (4).

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.

The amount of the base used is approximately 0.5 to approximately 10.0 mol, preferably approximately 1.0 to approximately 3.0 mol, with respect to 1 mol of the compound (4). The compound is also produced by performing the reaction, if desired, in the presence of quaternary ammonium salt or metal iodide, together with the base.

Examples of the "quaternary ammonium salt" include tetrabutyl ammonium iodide.

Examples of the "metal iodide" include sodium iodide and potassium iodide.

The amount of the quaternary ammonium salt used is approximately 0.1 to approximately 3.0 mol, preferably approximately 0.5 to approximately 1.0 mol, with respect to 1 mol of the compound (4).

The amount of the metal iodide used is approximately 0.1 to approximately 3.0 mol, preferably approximately 0.5 to approximately 1.0 mol, with respect to 1 mol of the compound (4).

Examples of the "condensing agent" include: N,N'-carbodiimides such as N,N'-dicyclohexylcarbodiimide and 1-ethyl-3-(3-dimethylaminopropyl)carbodiimide hydrochloride (WSC); azolides such as N,N'-carbonylimidazole; 2-halogenopyridinium salts such as 2-chloro-1-methylpyridinium iodide and 2-fluoro-1-methylpyridinium iodide; and N-ethoxycarbonyl-2-ethoxy-1,2-dihydroquinoline, 2-(7-aza-1H-benzotriazol-1-yl)-1,1,3,3-tetramethyluronium hexafluorophosphate (HATU), 4-(4,6-dimethoxy-1,3,5-triazin-2-yl)-4-methylmorpholinium chloride n-hydrate (DMTMM), diethyl cyanophosphate, phosphorus oxychloride, and acetic anhydride.

The amount of the "condensing agent" used is usually approximately 0.8 to approximately 5 mol, preferably approximately 1 to approximately 3 mol, with respect to 1 mol of the compound (4).

The reaction may be carried out, if desired, in the presence of a base. Examples of the "base" include: basic salts such as potassium acetate and sodium acetate; and tertiary amines such as triethylamine, tripropylamine, tributylamine, cyclohexyldimethylamine, 4-dimethylaminopyridine, N-methylpiperidine, N-methylpyrrolidine, and N-methylmorpholine. An additive such as 1-hydroxy-1H-benzotriazole (HOBt) may be allowed to coexist therewith, if desired.

The amount of the "base" used is usually approximately 0.5 to approximately 5 mol, preferably approximately 2 to approximately 3 mol, with respect to 1 mol of the compound (4).

The amount of the "additive" used is usually approximately 0.5 to approximately 5 mol, preferably approximately 1.5 to approximately 3 mol, with respect to 1 mol of the compound (4).

This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.

A reaction solution of the product or its crude product can be used directly in the next reaction. Alternatively, the product may be isolated from the reaction mixture according to a routine method or may be easily purified by usual separation

means (e.g., recrystallization, distillation, and chromatography).

Moreover, the compounds (6a1) and (6b2) encompassed in the compounds (6a) and (6b), respectively, are also produced by a method described in the following reaction scheme:

Reaction Scheme 19

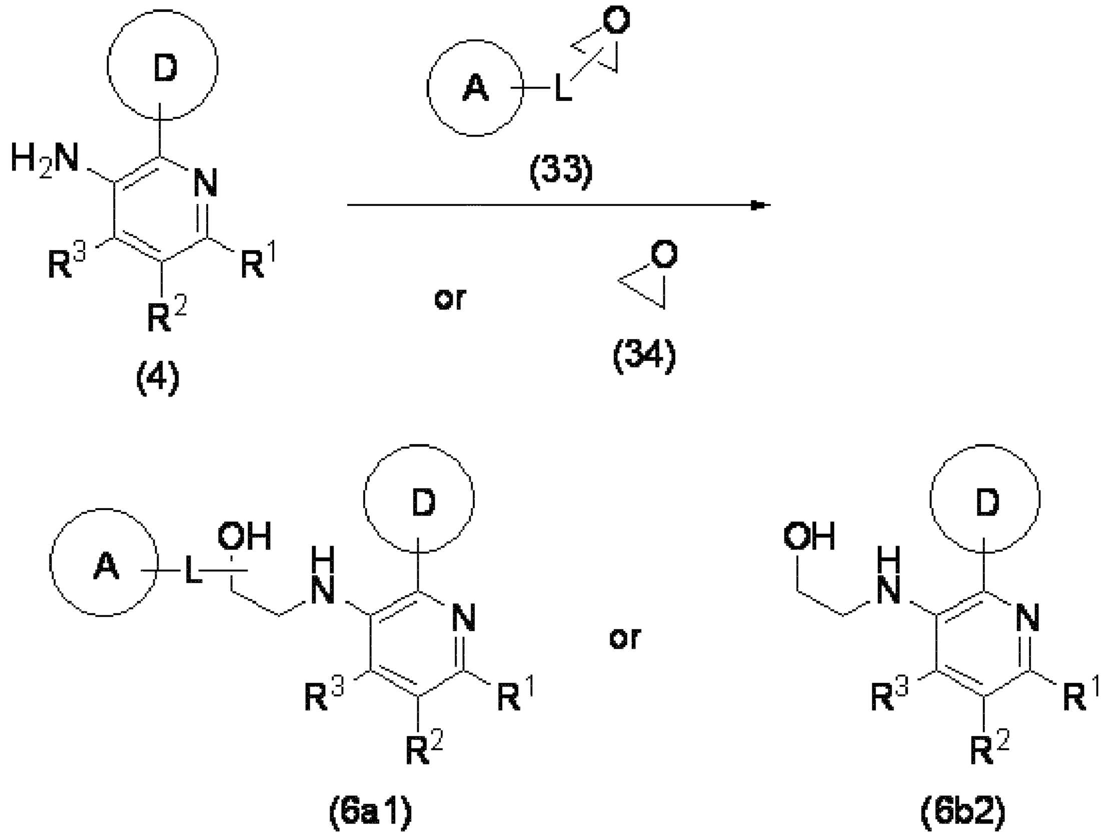

(4) (33) or (34)

(6a1) or (6b2)

In Reaction Scheme 19, the symbols are as defined above.

The compounds (6a1) and (6b2) are produced by reacting compounds (33) and (34), respectively, with a compound (4), if desired, in the presence of an acid.

The compounds (33) and (34) can be easily obtained as a commercially available product and are also produced by a method known per se in the art.

The amount of the compound (33) or (34) used is approximately 0.8 to approximately 10.0 mol, preferably approximately 1.0 to approximately 5.0 mol, with respect to 1 mol of the compound (4).

Examples of the "acid" include: protonic acids such as p-toluenesulfonic acid and camphor sulfonic acid; and Lewis acids such as cobalt chloride and tetraisopropoxytitanium.

The amount of the acid used is approximately 0.01 to approximately 10.0 mol, preferably approximately 0.05 to approximately 2.0 mol, with respect to 1 mol of the compound (4).

This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.

A reaction solution of the product or its crude product can be used directly in the next reaction. Alternatively, the product may be isolated from the reaction mixture according to a routine method or may be easily purified by usual separation means (e.g., recrystallization, distillation, and chromatography).

The compound (9) is produced by a method known per se in the art or a method equivalent thereto, or the like.

The compound (9) is also produced by a method described in the following reaction scheme:

Reaction Scheme 20

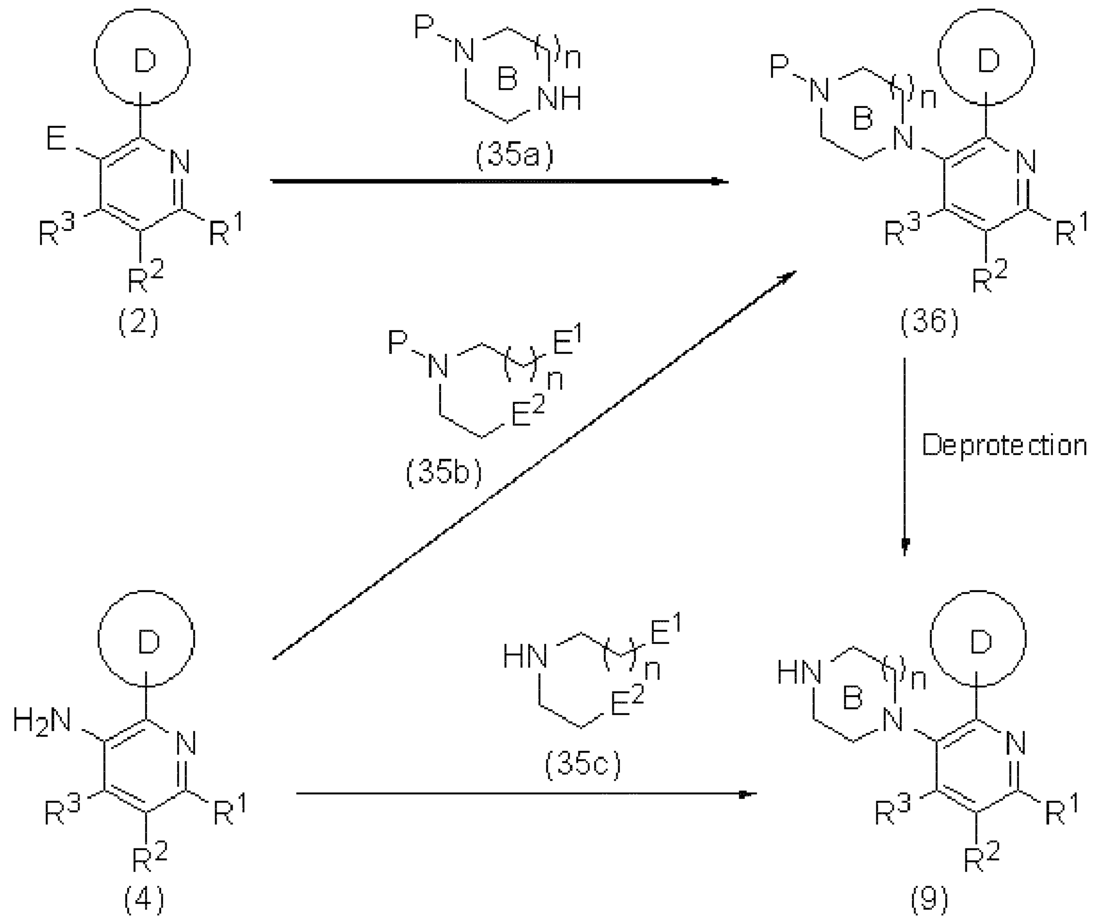


In Reaction Scheme 20, E, $E^1$, and $E^2$ represent the same or different leaving groups; P represents a protective group for the amino group; and the other symbols are as defined above.

The compound (36) is produced by reacting a compound (2) with a compound (35a) in the presence of a base. A transition metal such as copper or palladium and a ligand may be used, if necessary.

The compound (35a) can be easily obtained as a commercially available product and is also produced by a method known per se in the art.

The amount of the compound (35a) used is approximately 0.5 to approximately 20 mol, preferably approximately 1.0 to approximately 5.0 mol, with respect to 1 mol of the compound (2).

Examples of the "leaving group" represented by E include halogen atoms (e.g., fluorine, chlorine, bromine, and iodine), $C_{1-6}$ alkylsulfonyloxy which may be halogenated (e.g., methanesulfonyloxy, trifluoromethanesulfonyloxy, and trichloromethanesulfonyloxy), and $C_{6-10}$ arylsulfonyloxy which may have a substituent(s).

Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.

For example, formyl, or $C_{1-6}$ alkyl-carbonyl (e.g., acetyl and propionyl), phenylcarbonyl, $C_{1-6}$ alkoxy-carbonyl (e.g., methoxycarbonyl, ethoxycarbonyl, and tert-butoxycarbonyl), phenyloxycarbonyl, $C_{7-10}$ aralkyloxy-carbonyl (e.g., benzyloxycarbonyl), or trityl, each of which may have a substituent(s) is used as the "protective group" represented by P. A halogen atom (e.g., fluorine, chlorine, bromine, and iodine), $C_{1-6}$ alkyl-carbonyl (e.g., acetyl, propionyl, and valeryl), nitro, or the like is used as the substituent. The number of the substituent is approximately 1 to 3.

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, N-ethyldiisopropylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, sodium tert-butoxide, and potassium tert-butoxide.
The amount of the base used is approximately 0.8 to approximately 10 mol, preferably approximately 1.0 to approximately 5.0 mol, with respect to 1 mol of the compound (2).
Copper, copper halide (e.g., copper chloride and copper bromide), copper oxide, or the like is used as the "copper".
The amount of the copper used is approximately 0.1 to approximately 10 mol, preferably approximately 0.5 to approximately 2.0 mol, with respect to 1 mol of the compound (2).
Palladium acetate, palladium chloride, tetrakis(triphenylphosphine)palladium, tris(dibenzylideneacetone)dipalladium, 1,1-bis(diphenylphosphino)ferrocenedichloropalladium, dichlorobis(triphenylphosphine)palladium, bis(tri(tert-butylphosphine))palladium, phenylallylchloro-[1,3-bis(diisopropylphenyl)imidazol-2-ylidene]palladium, phenylallylchloro-[1,3-bis(diisopropylphenyl)-2-imidazolidinylidene]palladium, or the like is used as the "palladium".
The amount of the palladium used is approximately 0.001 to approximately 5 mol, preferably approximately 0.02 to approximately 0.5 mol, with respect to 1 mol of the compound (2).
Triphenylphosphine, tri(tert-butyl)phosphine, 2,2'-bis(diphenylphosphino)-1,1'-binaphthyl, 2-(dicyclohexylphosphino)-2',4',6'-triisopropyl-1,1'-biphenyl, 4,5'-bis(diphenylphosphino)-9,9'-dimethylxanthene, or the like is used as the "ligand".
The amount of the ligand used is approximately 0.001 to approximately 5 mol, preferably approximately 0.05 to approximately 1 mol, with respect to 1 mol of the compound (2).
This reaction is advantageously performed using a solvent inert to the reaction. Such a solvent is not particularly limited as long as the reaction proceeds. Preferable examples thereof include: alcohols such as methanol, ethanol, and propanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.
The reaction time is usually approximately 30 minutes to approximately 72 hours, preferably approximately 1 hour to approximately 48 hours. The reaction temperature is usually approximately -20 to approximately 200°C, preferably approximately 0 to approximately 150°C.
In this reaction, the reaction time can be shortened by use of a microwave reactor or the like.
A reaction solution of the product or its crude product can be used directly in the next reaction. Alternatively, the product may be isolated from the reaction mixture according to a routine method or may be easily purified by usual separation means (e.g., recrystallization, distillation, and chromatography).
The compound (36) is also produced by reacting a compound (4) with a compound (35b), if desired, in the presence of a base. The newly formed bonds may be formed simultaneously or stepwise.
The compound (35b) can be easily obtained as a commercially available product and is also produced by a method known per se in the art.
For example, formyl, or $C_{1-6}$ alkyl-carbonyl (e.g., acetyl and propionyl), phenylcarbonyl, $C_{1-6}$ alkoxy-carbonyl (e.g., methoxycarbonyl, ethoxycarbonyl, and tert-butoxycarbonyl), phenyloxycarbonyl, $C_{7-10}$ aralkyloxy-carbonyl (e.g., benzyloxycarbonyl), or trityl, each of which may have a substituent(s) is used as the "protective group" represented by P. A halogen atom (e.g., fluorine, chlorine, bromine, and iodine), $C_{1-6}$ alkyl-carbonyl (e.g., acetyl, propionyl, and valeryl), nitro, or the like is used as the substituent. The number of the substituent is approximately 1 to 3.
Examples of the "leaving group" include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).
Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.
The amount of the compound (35b) used is approximately 0.8 to approximately 10.0 mol, preferably approximately 1.0 to approximately 5.0 mol, with respect to 1 mol of the compound (4).
Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium

methoxide, sodium ethoxide, and potassium tert-butoxide.
The amount of the base used is approximately 0.5 to approximately 10.0 mol, preferably approximately 1.0 to approximately 5.0 mol, with respect to 1 mol of the compound (4). The compound is also produced by performing the reaction, if desired, in the presence of quaternary ammonium salt or metal iodide, together with the base.
Examples of the "quaternary ammonium salt" include tetrabutyl ammonium iodide.
Examples of the "metal iodide" include sodium iodide and potassium iodide.
The amount of the quaternary ammonium salt used is approximately 0.1 to approximately 3.0 mol, preferably approximately 0.5 to approximately 1.0 mol, with respect to 1 mol of the compound (4).
The amount of the metal iodide used is approximately 0.1 to approximately 3.0 mol, preferably approximately 0.5 to approximately 1.0 mol, with respect to 1 mol of the compound (4).
This reaction is advantageously performed using a solvent inert to the reaction. Such a solvent is not particularly limited as long as the reaction proceeds. Preferable examples thereof include: water; alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.
The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.
A reaction solution of the product or its crude product can be used directly in the next reaction. Alternatively, the product may be isolated from the reaction mixture according to a routine method or may be easily purified by usual separation means (e.g., recrystallization, distillation, and chromatography).
The compound (9) is produced by removing the protective group in the compound (36).
A method known per se in the art or a method equivalent thereto is used as a method for removing the protective group. For example, a treatment method with an acid, a base, UV light, hydrazine, phenylhydrazine, sodium N-methyldithiocarbamate, tetrabutyl ammonium fluoride, palladium acetate, or the like, or reduction reaction is used.
The reaction time is usually approximately 10 minutes to approximately 72 hours, preferably 1 hour to approximately 24 hours. The reaction temperature is usually approximately -20 to approximately 200°C, preferably approximately 20 to approximately 150°C.
A reaction solution of the product or its crude product can be used directly in the next reaction. Alternatively, the product may be isolated from the reaction mixture according to a routine method or may be easily purified by usual separation means (e.g., recrystallization, distillation, and chromatography).
The compound (9) is also produced by reacting the compound (4) with a compound (35c), if desired, in the presence of a base. The newly formed bonds may be formed simultaneously or stepwise.
The compound (35c) can be easily obtained as a commercially available product and is also produced by a method known per se in the art.
Examples of the "leaving group" include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).
Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.
The amount of the compound (35c) used is approximately 0.8 to approximately 10.0 mol, preferably approximately 1.0 to approximately 5.0 mol, with respect to 1 mol of the compound (4).
Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.
The amount of the base used is approximately 0.5 to approximately 10.0 mol, preferably approximately 1.0 to approximately 5.0 mol, with respect to 1 mol of the compound (4). The compound is also produced by performing the reaction, if desired, in the presence of quaternary ammonium salt or metal iodide, together with the base.
Examples of the "quaternary ammonium salt" include tetrabutyl ammonium iodide.
Examples of the "metal iodide" include sodium iodide and potassium iodide.
The amount of the quaternary ammonium salt used is approximately 0.1 to approximately 3.0 mol, preferably approximately 0.5 to approximately 1.0 mol, with respect to 1 mol of the compound (4).
The amount of the metal iodide used is approximately 0.1 to approximately 3.0 mol, preferably approximately 0.5 to approximately 1.0 mol, with respect to 1 mol of the compound (4).
This reaction is advantageously performed using a solvent inert to the reaction. Such a solvent is not particularly limited as long as the reaction proceeds. Preferable examples thereof include: water; alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.
The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.
A reaction solution of the product or its crude product can be used directly in the next reaction. Alternatively, the product may be isolated from the reaction mixture according to a routine method or may be easily purified by usual separation means (e.g., recrystallization, distillation, and chromatography).
The compound (13) is produced by a method known per se in the art or a method equivalent thereto, or the like.
The compound (13a) encompassed in the compound (13) is also produced by a method described in the following reaction scheme:

Reaction Scheme 21

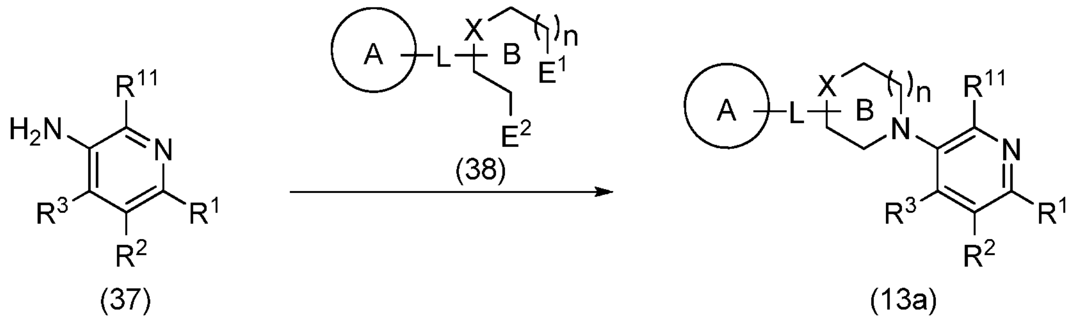

In Reaction Scheme 21, $E^1$ and $E^2$ represent the same or different leaving groups; $R^{11}$ represents a halogen atom (e.g., fluorine, chlorine, bromine, and iodine), $C_{1-5}$ alkylsulfonyloxy which may be halogenated (e.g., methanesulfonyloxy and trifluoromethanesulfonyloxy), or $C_{6-10}$ arylsulfonyloxy which may have a substituent(s) (e.g., benzenesulfonyloxy and p-toluenesulfonyloxy); and the other symbols are as defined above.
The compound (13a) is produced by reacting a compound (37) with a compound (38), if desired, in the presence of a base. The newly formed bonds may be formed simultaneously or stepwise.
The compounds (37) and (38) can be easily obtained as a commercially available product and are also produced by a method known per se in the art.
Examples of the "leaving group" include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).
Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.
The amount of the compound (38) used is approximately 0.8 to approximately 10.0 mol, preferably approximately 1.0 to approximately 5.0 mol, with respect to 1 mol of the compound (37).
Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.
The amount of the base used is approximately 0.5 to approximately 10.0 mol, preferably approximately 1.0 to approximately 5.0 mol, with respect to 1 mol of the compound (37). The compound is also produced by performing the reaction, if desired, in the presence of quaternary ammonium salt or metal iodide, together with the base.
Examples of the "quaternary ammonium salt" include tetrabutyl ammonium iodide.
Examples of the "metal iodide" include sodium iodide and potassium iodide.
The amount of the quaternary ammonium salt used is approximately 0.1 to approximately 3.0 mol, preferably approximately 0.5 to approximately 1.0 mol, with respect to 1 mol of the compound (37).
The amount of the metal iodide used is approximately 0.1 to approximately 3.0 mol, preferably approximately 0.5 to approximately 1.0 mol, with respect to 1 mol of the compound (37).
This reaction is advantageously performed using a solvent inert to the reaction. Such a solvent is not particularly limited as long as the reaction proceeds. Preferable examples thereof include: water; alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.
The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.
A reaction solution of the product or its crude product can be used directly in the next reaction. Alternatively, the product may be isolated from the reaction mixture according to a routine method or may be easily purified by usual separation means (e.g., recrystallization, distillation, and chromatography).
The compound (15) is produced by a method known per se in the art or a method equivalent thereto, or the like.
The compound (15a) encompassed in the compound (15) is also produced by a method described in the following reaction scheme:

Reaction Scheme 22

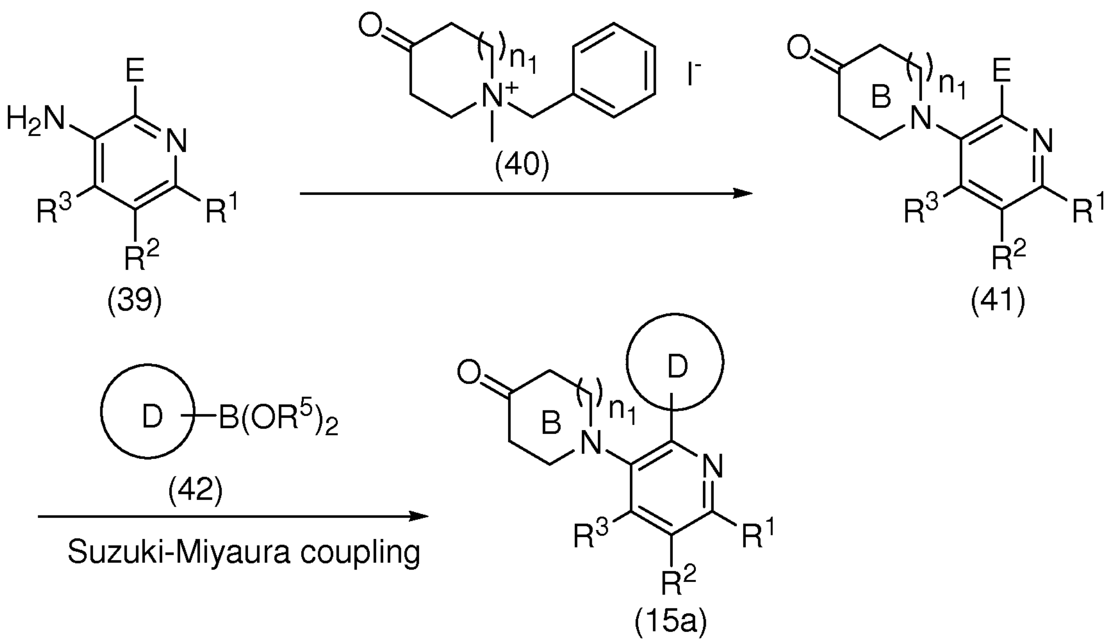

(39) (40) (41) (42) (15a)

In Reaction Scheme 22, E represents a leaving group; $n_1$ represents 0 to 2; the functional group represented by $B(OR^5)_2$ represents boronic acid or boronic acid ester (e.g., 4,4,5,5-tetramethyl-1,3,2-dioxaborolan-2-yl); and the other symbols are as defined above.
The compound (41) is produced by reacting a compound (39) with a compound (40), if desired, in the presence of a base. The compounds (39) and (40) can be easily obtained as a commercially available product and are also produced by a method known per se in the art.
Examples of the "leaving group" include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).
Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl,

pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.

The amount of the compound (40) used is approximately 0.8 to approximately 10.0 mol, preferably approximately 0.1 to approximately 3.0 mol, with respect to 1 mol of the compound (39).

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.

The amount of the base used is approximately 0.5 to approximately 10.0 mol, preferably approximately 1.0 to approximately 3.0 mol, with respect to 1 mol of the compound (39).

The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.

A reaction solution of the product or its crude product can be used directly in the next reaction. Alternatively, the product may be isolated from the reaction mixture according to a routine method or may be easily purified by usual separation means (e.g., recrystallization, distillation, and chromatography).

The compound (15a) is produced by reacting the compound (41) with a compound (42) through Suzuki-Miyaura coupling reaction in the presence of a transition metal catalyst and a base.

The compound (42) can be easily obtained as a commercially available product and is also produced by a method known per se in the art.

The amount of the compound (42) used is approximately 0.5 to approximately 10 mol, preferably approximately 0.9 to approximately 3 mol, with respect to 1 mol of the compound (41).

For example, alkali metal or alkaline earth metal carbonate (e.g., sodium carbonate and potassium carbonate), alkali metal or alkaline earth metal bicarbonate (e.g., sodium bicarbonate and potassium bicarbonate), alkali metal or alkaline earth metal hydroxide (e.g., sodium hydroxide and potassium hydroxide), triethylamine, 4-dimethylaminopyridine, N-ethyldiisopropylamine, triethylenediamine, or 4-methylmorpholine is used as the "base". The amount of the base used is approximately 0.8 to approximately 10.0 mol, preferably approximately 1.0 to approximately 5.0 mol, with respect to 1 mol of the compound (41).

For example, a palladium catalyst such as palladium acetate, palladium chloride, tetrakis(triphenylphosphine)palladium, tris(dibenzylideneacetone)dipalladium, 1,1-bis(diphenylphosphino)ferrocenedichloropalladium, dichlorobis(triphenylphosphine)palladium, bis(tri(tert-butylphosphine))palladium, phenylallylchloro-[1,3-bis(diisopropylphenyl)imidazol-2-ylidene]palladium, or phenylallylchloro-[1,3-bis(diisopropylphenyl)-2-imidazolidinylidene]palladium is used as the "transition metal catalyst". A ligand such as triphenylphosphine, tri(tert-butyl)phosphine, 2,2'-bis(diphenylphosphino)-1,1'-binaphthyl, 2-(dicyclohexylphosphino)-2',4',6'-triisopropyl-1,1'-biphenyl, or 4,5'-bis(diphenylphosphino)-9,9'-dimethylxanthene may be added thereto, if desired.

The amount of the transition metal catalyst used is approximately 0.001 to approximately 3 mol, preferably approximately 0.02 to approximately 0.2 mol, with respect to 1 mol of the compound (41).

The amount of the ligand used is approximately 0.001 to approximately 3 mol, preferably approximately 0.05 to approximately 0.5 mol, with respect to 1 mol of the compound (41).

Examples of a solvent used include: ethers such as diethyl ether, diisopropyl ether, tetrahydrofuran, dioxane, and 1,2-dimethoxyethane; alcohols such as methanol, ethanol, and propanol; hydrocarbons such as benzene, toluene, carbon disulfide, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; water; and mixed solvents thereof.

The reaction temperature is usually 0 to 250°C, preferably 50 to 150°C. The reaction time is usually approximately 5 minutes to approximately 48 hours, preferably approximately 30 minutes to approximately 24 hours.

In this reaction, the reaction time can be shortened by use of a microwave reactor or the like.

A reaction solution of the product or its crude product can be used directly in the next reaction. Alternatively, the product may be isolated from the reaction mixture according to a routine method or may be easily purified by usual separation means (e.g., recrystallization, distillation, and chromatography).

The compound (9a) encompassed in the compound (9) and the compound (15b) encompassed in the compound (15) are also produced by a method described in following reaction scheme:

Reaction Scheme 23

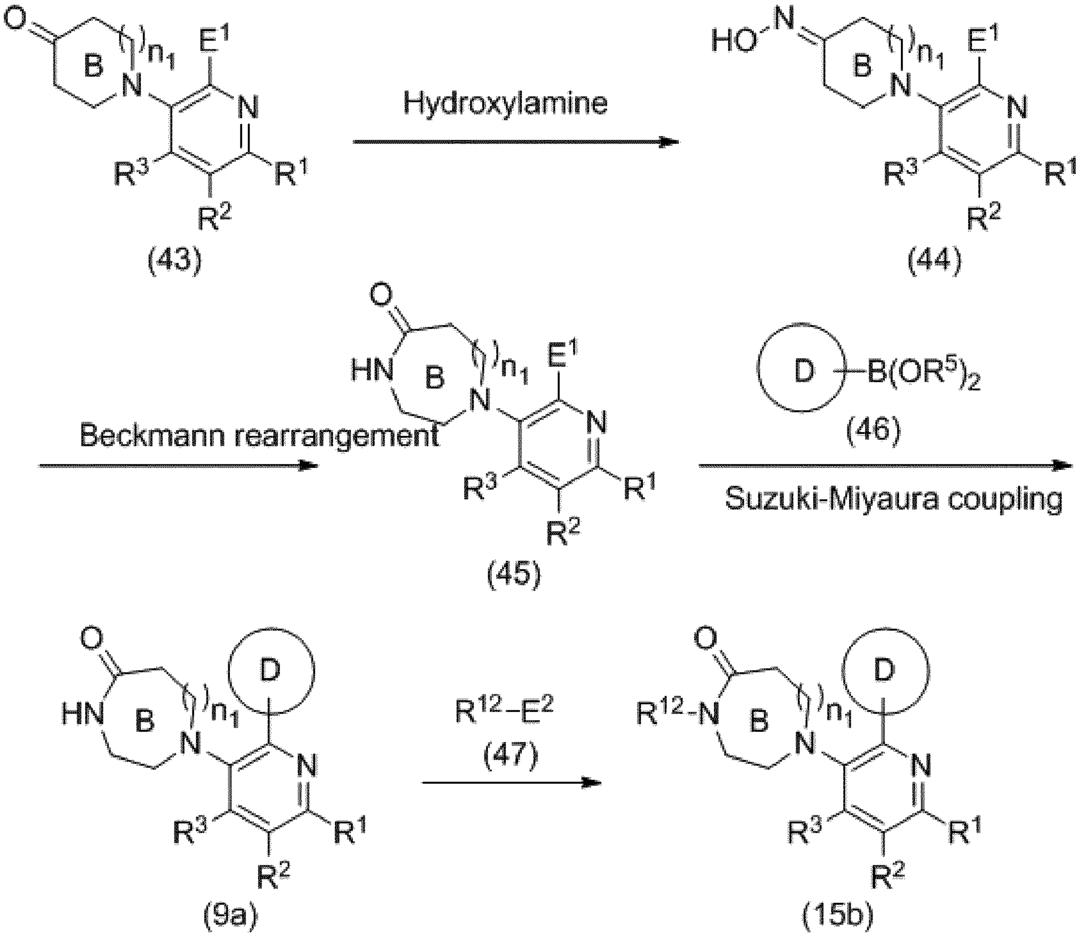


In Reaction Scheme 23, $E^1$ and $E^2$ represent the same or different leaving groups; $n_1$ represents 0 or 1; $R^{12}$ represents an alkyl group which may be substituted; the functional group represented by $B(OR^5)_2$ represents boronic acid or boronic acid ester (e.g., 4,4,5,5-tetramethyl-1,3,2-dioxaborolan-2-yl); and the other symbols are as defined above.

Examples of the "leaving group" include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).

Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.

The compound (44) is produced by reacting a compound (43) with hydroxylamine, if desired, in the presence of an acid or a base.

The compound (43) can be easily obtained as a commercially available product and is also produced by a method known per se in the art.

The amount of the hydroxylamine used is approximately 0.8 to approximately 10.0 mol, preferably approximately 1.0 to approximately 3.0 mol, with respect to 1 mol of the compound (43).

Examples of the "base" include: basic salts such as sodium carbonate, potassium carbonate, cesium carbonate, and sodium bicarbonate; aromatic amines such as pyridine and lutidine; and tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine.

The amount of the base used is approximately 0.5 to approximately 10.0 mol, preferably approximately 1.0 to approximately 3.0 mol, with respect to 1 mol of the compound (43).

Examples of the "acid" include; protonic acids such as p-toluenesulfonic acid and camphor sulfonic acid; and Lewis acids such as titanium tetrachloride and ytterbium trifluoromethanesulfonate.

The amount of the acid used is approximately 0.01 to approximately 10.0 mol, preferably approximately 0.05 to approximately 3.0 mol, with respect to 1 mol of the compound (43).

The reaction time is usually approximately 30 minutes to approximately 72 hours, preferably approximately 1 hour to approximately 24 hours. The reaction temperature is usually approximately -20 to approximately 200°C, preferably approximately 20 to approximately 150°C.
A reaction solution of the product or its crude product can be used directly in the next reaction. Alternatively, the product may be isolated from the reaction mixture according to a routine method or may be easily purified by usual separation means (e.g., recrystallization, distillation, and chromatography).
The compound (45) is produced by subjecting the compound (44) to Beckmann rearrangement, if desired, in the presence of an additive.
An acid, phosphorus pentachloride, or thionyl chloride is used as the "additive". Also, p-toluenesulfonyl chloride, trifluoromethanesulfonic anhydride, or the like may be used, if desired, in the presence of a base.
The amount of the phosphorus pentachloride or thionyl chloride used is approximately 0.5 to approximately 10.0 mol, preferably approximately 1.0 to approximately 3.0 mol, with respect to 1 mol of the compound (44).
The amount of the p-toluenesulfonyl chloride or trifluoromethanesulfonic anhydride used is approximately 0.8 to approximately 10.0 mol, preferably approximately 1.0 to approximately 3.0 mol, with respect to 1 mol of the compound (44).
Examples of the "acid" include hydrochloric aid, sulfuric acid, polyphosphoric acid, and p-toluenesulfonic acid. The amount of the acid used is approximately 0.01 to approximately 100.0 mol, preferably approximately 0.1 to approximately 10.0 mol, with respect to 1 mol of the compound (44).
Examples of the "base" include: basic salts such as sodium carbonate, potassium carbonate, cesium carbonate, and sodium bicarbonate; aromatic amines such as pyridine and lutidine; and tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine. The amount of the base used is approximately 0.8 to approximately 30.0 mol, preferably approximately 1.0 to approximately 10.0 mol, with respect to 1 mol of the compound (44).
The reaction time is usually approximately 30 minutes to approximately 72 hours, preferably approximately 1 hour to approximately 24 hours. The reaction temperature is usually approximately -20 to approximately 200°C, preferably approximately 20 to approximately 150°C.
A reaction solution of the product or its crude product can be used directly in the next reaction. Alternatively, the product may be isolated from the reaction mixture according to a routine method or may be easily purified by usual separation means (e.g., recrystallization, distillation, and chromatography).
The compound (9a) is produced by reacting the compound (45) with a compound (46) through Suzuki-Miyaura coupling reaction in the presence of a transition metal catalyst and a base.
The compound (46) can be easily obtained as a commercially available product and is also produced by a method known per se in the art.
The amount of the compound (46) used is approximately 0.5 to approximately 10 mol, preferably approximately 0.9 to approximately 3 mol, with respect to 1 mol of the compound (45).
For example, alkali metal or alkaline earth metal carbonate (e.g., sodium carbonate and potassium carbonate), alkali metal or alkaline earth metal bicarbonate (e.g., sodium bicarbonate and potassium bicarbonate), alkali metal or alkaline earth metal hydroxide (e.g., sodium hydroxide and potassium hydroxide), triethylamine, 4-dimethylaminopyridine, N-ethyldiisopropylamine, triethylenediamine, or 4-methylmorpholine is used as the "base". The amount of the base used is approximately 0.8 to approximately 10.0 mol, preferably approximately 1.0 to approximately 5.0 mol, with respect to 1 mol of the compound (45).
For example, a palladium catalyst such as palladium acetate, palladium chloride, tetrakis(triphenylphosphine)palladium, tris(dibenzylideneacetone)dipalladium, 1,1-bis(diphenylphosphino)ferrocenedichloropalladium, dichlorobis(triphenylphosphine)palladium, bis(tri(tert-butylphosphine))palladium, phenylallylchloro-[1,3-bis(diisopropylphenyl)imidazol-2-ylidene]palladium, or phenylallylchloro-[1,3-bis(diisopropylphenyl)-2-imidazolidinylidene]palladium is used as the "transition metal catalyst". A ligand such as triphenylphosphine, tri(tert-butyl)phosphine, 2,2'-bis(diphenylphosphino)-1,1'-binaphthyl, 2-(dicyclohexylphosphino)-2',4',6'-triisopropyl-1,1'-biphenyl, or 4,5'-bis(diphenylphosphino)-9,9'-dimethylxanthene may be added thereto, if desired.
The amount of the transition metal catalyst used is approximately 0.001 to approximately 3 mol, preferably approximately 0.02 to approximately 0.2 mol, with respect to 1 mol of the compound (45).
The amount of the ligand used is approximately 0.001 to approximately 3 mol, preferably approximately 0.05 to approximately 0.5 mol, with respect to 1 mol of the compound (45).
Examples of a solvent used include: ethers such as diethyl ether, diisopropyl ether, tetrahydrofuran, dioxane, and 1,2-dimethoxyethane; alcohols such as methanol, ethanol, and propanol; hydrocarbons such as benzene, toluene, carbon disulfide, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; water; and mixed solvents thereof.
The reaction temperature is usually approximately 0°C to 250°C, preferably approximately 50°C to approximately 150°C.
The reaction time is usually approximately 5 minutes to approximately 48 hours, preferably approximately 30 minutes

to approximately 24 hours.
In this reaction, the reaction time can be shortened by use of a microwave reactor or the like.
A reaction solution of the product or its crude product can be used directly in the next reaction. Alternatively, the product may be isolated from the reaction mixture according to a routine method or may be easily purified by usual separation means (e.g., recrystallization, distillation, and chromatography).
The compound (15b) is produced by reacting the compound (9a) with a compound (47), if desired, in the presence of a base.
The compound (47) can be easily obtained as a commercially available product and is also produced by a method known per se in the art.
The amount of the compound (47) used is approximately 0.8 to approximately 10.0 mol, preferably approximately 1.0 to approximately 5.0 mol, with respect to 1 mol of the compound (9a).
Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.
The amount of the base used is approximately 0.5 to approximately 10.0 mol, preferably approximately 1.0 to approximately 5.0 mol, with respect to 1 mol of the compound (9a). The compound is also produced by performing the reaction, if desired, in the presence of quaternary ammonium salt or metal iodide, together with the base.
Examples of the "quaternary ammonium salt" include tetrabutyl ammonium iodide.
Examples of the "metal iodide" include sodium iodide and potassium iodide.
The amount of the quaternary ammonium salt used is approximately 0.1 to approximately 3.0 mol, preferably approximately 0.5 to approximately 1.0 mol, with respect to 1 mol of the compound (9a).
The amount of the metal iodide used is approximately 0.1 to approximately 3.0 mol, preferably approximately 0.5 to approximately 1.0 mol, with respect to 1 mol of the compound (9a).
This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.
The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.
A reaction solution of the product or its crude product can be used directly in the next reaction. Alternatively, the product may be isolated from the reaction mixture according to a routine method or may be easily purified by usual separation means (e.g., recrystallization, distillation, and chromatography).
The compound (19) is produced by a method known per se in the art or a method equivalent thereto, or the like.
The compounds (19a) and (19b) encompassed in the compound (19) are also produced by a method described in following reaction scheme:

Reaction Scheme 24

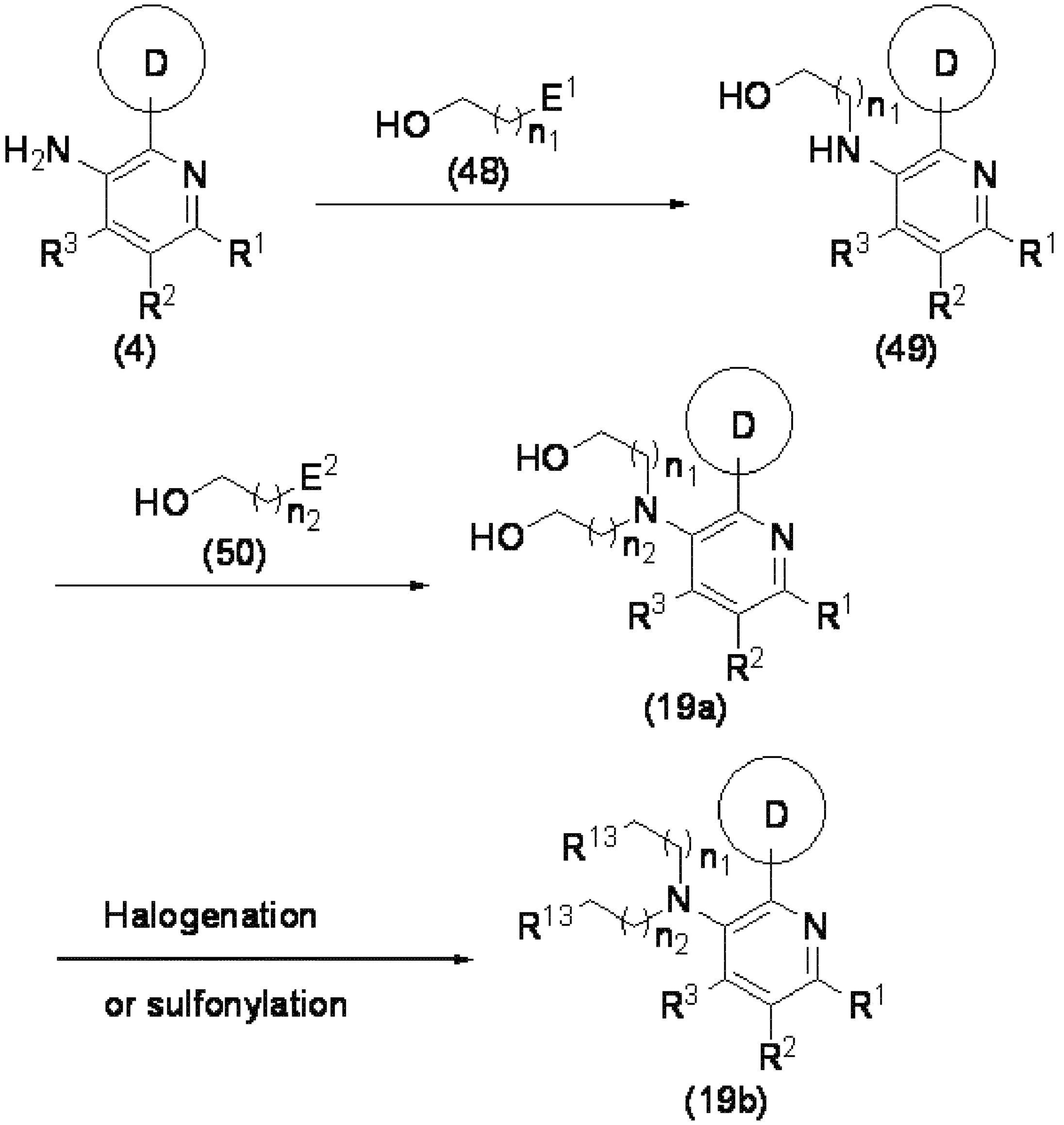


In Reaction Scheme 24, $E^1$ and $E^2$ represent the same or different leaving groups; $R^{13}$ represents halogen (e.g., chlorine, bromine, and iodine) or a sulfonyloxy group (e.g., methanesulfonyloxy, p-toluenesulfonyloxy, and trifluoromethanesulfonyloxy groups); $n_1$ and $n_2$ each represent 1 or 2 (excluding, however, the cases where both of $n_1$ and $n_2$ are 2); and the other symbols are as defined above.

Examples of the "leaving group" include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).

Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.

The compound (49) is produced by reacting a compound (4) with a compound (48), if desired, in the presence of a base.

The compound (48) can be easily obtained as a commercially available product and is also produced by a method known per se in the art.

The amount of the compound (48) used is approximately 0.8 to approximately 10.0 mol, preferably approximately 1.0 to approximately 5.0 mol, with respect to 1 mol of the compound (4).

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.
The amount of the base used is approximately 0.5 to approximately 10.0 mol, preferably approximately 1.0 to approximately 3.0 mol, with respect to 1 mol of the compound (4). The compound is also produced by performing the reaction, if desired, in the presence of quaternary ammonium salt or metal iodide, together with the base.
Examples of the "quaternary ammonium salt" include tetrabutyl ammonium iodide.
Examples of the "metal iodide" include sodium iodide and potassium iodide.
The amount of the quaternary ammonium salt used is approximately 0.1 to approximately 3.0 mol, preferably approximately 0.5 to approximately 1.0 mol, with respect to 1 mol of the compound (4).
The amount of the metal iodide used is approximately 0.1 to approximately 3.0 mol, preferably approximately 0.5 to approximately 1.0 mol, with respect to 1 mol of the compound (4).
This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.
The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.
A reaction solution of the product or its crude product can be used directly in the next reaction. Alternatively, the product may be isolated from the reaction mixture according to a routine method or may be easily purified by usual separation means (e.g., recrystallization, distillation, and chromatography).
The compound (19a) is produced by reacting the compound (49) with a compound (50), if desired, in the presence of a base.
The compound (50) can be easily obtained as a commercially available product and is also produced by a method known per se in the art.
The amount of the compound (50) used is approximately 0.8 to approximately 10.0 mol, preferably approximately 1.0 to approximately 5.0 mol, with respect to 1 mol of the compound (49).
Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.
The amount of the base used is approximately 0.5 to approximately 10.0 mol, preferably approximately 1.0 to approximately 3.0 mol, with respect to 1 mol of the compound (49). The compound is also produced by performing the reaction, if desired, in the presence of quaternary ammonium salt or metal iodide, together with the base.
Examples of the "quaternary ammonium salt" include tetrabutyl ammonium iodide.
Examples of the "metal iodide" include sodium iodide and potassium iodide.
The amount of the quaternary ammonium salt used is approximately 0.1 to approximately 3.0 mol, preferably approximately 0.5 to approximately 1.0 mol, with respect to 1 mol of the compound (49).
The amount of the metal iodide used is approximately 0.1 to approximately 3.0 mol, preferably approximately 0.5 to approximately 1.0 mol, with respect to 1 mol of the compound (49).
This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.
The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.
A reaction solution of the product or its crude product can be used directly in the next reaction. Alternatively, the product

may be isolated from the reaction mixture according to a routine method or may be easily purified by usual separation means (e.g., recrystallization, distillation, and chromatography).

When both of $n_1$ and $n_2$ in the compound (19a) are 1, the compound (19a) can be synthesized in one step by reacting the compound (4) with the compound (48), if desired, in the presence of a base.

The amount of the compound (48) used is approximately 1.5 to approximately 20.0 mol, preferably approximately 2.0 to approximately 10.0 mol, with respect to 1 mol of the compound (4).

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.

The amount of the base used is approximately 1.5 to approximately 20.0 mol, preferably approximately 2.0 to approximately 10.0 mol, with respect to 1 mol of the compound (4). The compound is also produced by performing the reaction, if desired, in the presence of quaternary ammonium salt or metal iodide, together with the base.

Examples of the "quaternary ammonium salt" include tetrabutyl ammonium iodide.

Examples of the "metal iodide" include sodium iodide and potassium iodide.

The amount of the quaternary ammonium salt used is approximately 0.1 to approximately 3.0 mol, preferably approximately 0.5 to approximately 1.0 mol, with respect to 1 mol of the compound (4).

The amount of the metal iodide used is approximately 0.1 to approximately 3.0 mol, preferably approximately 0.5 to approximately 1.0 mol, with respect to 1 mol of the compound (4).

This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.

A reaction solution of the product or its crude product can be used directly in the next reaction. Alternatively, the product may be isolated from the reaction mixture according to a routine method or may be easily purified by usual separation means (e.g., recrystallization, distillation, and chromatography).

The compound (19b) is produced by halogenating the compound (19a) with a halogenation reagent or sulfonylating it with a sulfonylation reagent, if desired, in the presence of a base.

A halogen molecule (e.g., chlorine, bromine, and iodine), thionyl chloride, phosphorus pentachloride, an imide (e.g., N-chlorosuccinimide and N-bromosuccinimide), a halogen adduct (e.g., benzyltrimethyl ammonium tribromide and triphenylphosphine dibromide), carbon tetrachloride, carbon tetrabromide, or the like is used as the "halogenation reagent". Triphenylphosphine or the like may be added thereto, if desired.

The amount of the halogenation reagent used is approximately 0.8 to approximately 100.0 mol, preferably approximately 1.0 to approximately 10.0 mol, with respect to 1 mol of the compound (19a).

The amount of the triphenylphosphine used is approximately 0.8 to approximately 10.0 mol, preferably approximately 1.0 to approximately 5.0 mol, with respect to 1 mol of the compound (19a).

Methanesulfonyl chloride, p-toluenesulfonyl chloride, trifluoromethanesulfonic anhydride, or the like is used as the "sulfonylation reagent". The amount of the sulfonylation reagent used is approximately 0.8 to approximately 10.0 mol, preferably approximately 1.0 to approximately 5.0 mol, with respect to 1 mol of the compound (19a).

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.

The amount of the base used is approximately 0.5 to approximately 10.0 mol, preferably approximately 1.0 to approximately 3.0 mol, with respect to 1 mol of the compound (19a).

This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide;

halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.
The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.
A reaction solution of the product or its crude product can be used directly in the next reaction. Alternatively, the product may be isolated from the reaction mixture according to a routine method or may be easily purified by usual separation means (e.g., recrystallization, distillation, and chromatography).
The compound (21) is produced by a method known per se in the art or a method equivalent thereto, or the like.
The compound (21a) encompassed in the compound (21) is also produced by a method described in following reaction scheme:

Reaction Scheme 25

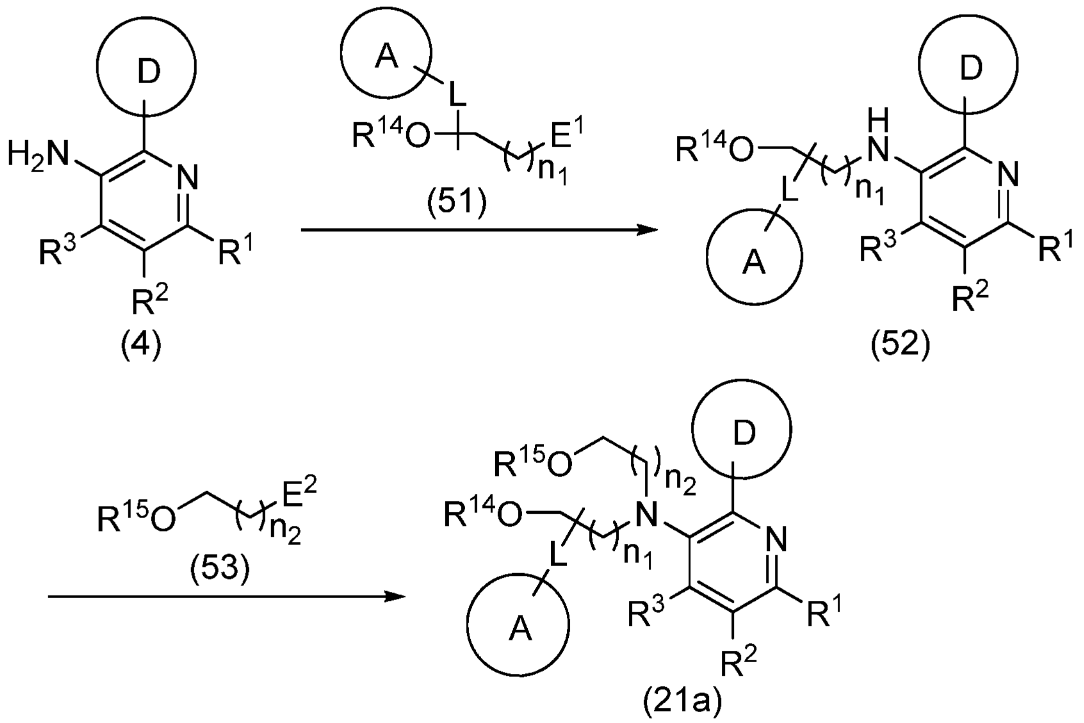

(4) (51) (52) (53) (21a)

In Reaction Scheme 25, $E^1$ and $E^2$ represent the same or different leaving groups; $n_1$ and $n_2$ each represent 1 or 2 (excluding, however, the cases where both of $n_1$ and $n_2$ are 2); $R^{14}$ and $R^{15}$ are the same or different and each represent hydrogen, lower alkyl (e.g., methyl and allyl), formyl, or $C_{1-6}$ alkyl-carbonyl (e.g., acetyl and propionyl), $C_{1-6}$ alkoxy-carbonyl (e.g., methoxycarbonyl, ethoxycarbonyl, tert-butoxycarbonyl and allylcarbonyl), or $C_{7-10}$ aralkyloxy-carbonyl (e.g., benzyloxycarbonyl), each of which may have a substituent(s), benzyl which may be substituted, trityl, or the like; and the other symbols are as defined above.
Examples of the "leaving group" include hydroxy, halogen atoms (e.g., fluorine, chlorine, bromine, and iodine), $C_{1-5}$ alkylsulfonyloxy which may be halogenated (e.g., methanesulfonyloxy, ethanesulfonyloxy, and trichloromethanesulfonyloxy), and $C_{6-10}$ arylsulfonyloxy which may have a substituent(s).
Examples of the "$C_{6-10}$ arylsulfonyloxy which may have a substituent(s)" include $C_{6-10}$ arylsulfonyloxy which may have 1 to 3 substituents selected from $C_{1-6}$ alkyl (e.g., methyl, ethyl, propyl, isopropyl, butyl, isobutyl, sec-butyl, tert-butyl, pentyl, and hexyl), $C_{1-6}$ alkoxy (e.g., methoxy, ethoxy, propoxy, isopropoxy, butoxy, isobutoxy, sec-butoxy, pentyloxy, and hexyloxy), and nitro. Specific examples thereof include benzenesulfonyloxy, m-nitrobenzenesulfonyloxy, and p-toluenesulfonyloxy.
The compound (52) is produced by reacting a compound (4) with a compound (51), if desired, in the presence of a base. The compound (51) can be easily obtained as a commercially available product and is also produced by a method known per se in the art.
The amount of the compound (51) used is approximately 0.8 to approximately 10.0 mol, preferably approximately 1.0

to approximately 5.0 mol, with respect to 1 mol of the compound (4).

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.

The amount of the base used is approximately 0.5 to approximately 10.0 mol, preferably approximately 1.0 to approximately 3.0 mol, with respect to 1 mol of the compound (4). The compound is also produced by performing the reaction, if desired, in the presence of quaternary ammonium salt or metal iodide, together with the base.

Examples of the "quaternary ammonium salt" include tetrabutyl ammonium iodide.

Examples of the "metal iodide" include sodium iodide and potassium iodide.

The amount of the quaternary ammonium salt used is approximately 0.1 to approximately 3.0 mol, preferably approximately 0.5 to approximately 1.0 mol, with respect to 1 mol of the compound (4).

The amount of the metal iodide used is approximately 0.1 to approximately 3.0 mol, preferably approximately 0.5 to approximately 1.0 mol, with respect to 1 mol of the compound (4).

This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.

A reaction solution of the product or its crude product can be used directly in the next reaction. Alternatively, the product may be isolated from the reaction mixture according to a routine method or may be easily purified by usual separation means (e.g., recrystallization, distillation, and chromatography).

The compound (21a) is produced by reacting the compound (52) with a compound (53), if desired, in the presence of a base.

The compound (53) can be easily obtained as a commercially available product and is also produced by a method known per se in the art.

The amount of the compound (53) used is approximately 0.8 to approximately 10.0 mol, preferably approximately 1.0 to approximately 5.0 mol, with respect to 1 mol of the compound (52).

Examples of the "base" include: basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tert-butoxide.

The amount of the base used is approximately 0.5 to approximately 10.0 mol, preferably approximately 1.0 to approximately 3.0 mol, with respect to 1 mol of the compound (52). The compound is also produced by performing the reaction, if desired, in the presence of quaternary ammonium salt or metal iodide, together with the base.

Examples of the "quaternary ammonium salt" include tetrabutyl ammonium iodide.

Examples of the "metal iodide" include sodium iodide and potassium iodide.

The amount of the quaternary ammonium salt used is approximately 0.1 to approximately 3.0 mol, preferably approximately 0.5 to approximately 1.0 mol, with respect to 1 mol of the compound (52).

The amount of the metal iodide used is approximately 0.1 to approximately 3.0 mol, preferably approximately 0.5 to approximately 1.0 mol, with respect to 1 mol of the compound (52).

This reaction is advantageously performed using a solvent inert to the reaction. Such a solvent is not particularly limited as long as the reaction proceeds. Preferable examples thereof include: alcohols such as methanol, ethanol, propanol, and butanol; ethers such as diethyl ether, tetrahydrofuran, dioxane, and 1,2-dimethoxyethane; hydrocarbons such as benzene, toluene, cyclohexane, and hexane; amides such as N,N-dimethylformamide and N,N-dimethylacetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and mixed solvents thereof.

The reaction time is usually approximately 30 minutes to approximately 72 hours, preferably approximately 3 hours to approximately 24 hours. The reaction temperature is usually approximately -20 to approximately 200°C, preferably approximately 20 to approximately 150°C.

Moreover, the compound (21a) can also be synthesized from the compound (4) under the same conditions as above by changing places between the reactions with the compounds (51) and (53).
A reaction solution of the product or its crude product can be used directly in the next reaction. Alternatively, the product may be isolated from the reaction mixture according to a routine method or may be easily purified by usual separation means (e.g., recrystallization, distillation, and chromatography).
When the compound (I) has optical isomers, stereoisomers, positional isomers, and rotational isomers, these isomers are also included in the compound (I) and can be obtained as single products by a synthesis approach or a separation approach (e.g., concentration, solvent extraction, column chromatography, and recrystallization) known per se in the art. For example, when the compound (I) has optical isomers, the optical isomers resolved from the compound (I) are also encompassed in the compound (I).

The optical isomers can be produced by a method known per se in the art. Specifically, the optical isomers are obtained by use of an optically active synthetic intermediate or optical resolution of a racemate of a final product according to a routine method.
A method known per se in the art, for example, a fractional crystallization method, a chiral column method, or a diastereomer method, is used as an optical resolution method.

1) Fractional crystallization method

In this method, a racemate and an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, and brucine) are allowed to form a salt, which is resolved by the fractional crystallization technique and subjected, if desired, to a neutralization step to obtain a free optical isomer.

2) Chiral column method

In this method, a racemate or a salt thereof is resolved by application to a column for optical isomer resolution (chiral column). For example, in the case of liquid chromatography, an optical isomer mixture is added to a chiral column such as ENANTIO-OVM (manufactured by Tosoh Corp.) or CHIRAL series (manufactured by Daicel Corp.), on which water, various buffer solutions (e.g., a phosphate buffer solution), or organic solvents (e.g., ethanol, methanol, isopropanol, acetonitrile, trifluoroacetic acid, and diethylamine) are then developed alone or as a mixed solution to thereby resolve the optical isomers. Alternatively, for example, in the case of gas chromatography, a chiral column such as CP-Chirasil-DeX CB (manufactured by GL Sciences) is used for resolution.

3) Diastereomer method

In this method, a racemic mixture is converted by chemical reaction with an optically active reagent to a diastereomeric mixture, which is then converted through usual separation means (e.g., fractional crystallization and chromatography) or the like to single substances, followed by the cleave-off of the optically active reagent site by chemical treatment such as hydrolysis reaction to thereby obtain an optical isomer. For example, when the compound (I) has hydroxy or primary/secondary amino in its molecule, the compound and an optically active organic acid (e.g., MTPA [$\alpha$-methoxy-$\alpha$-(trifluoromethyl)phenylacetic acid] and (-)-menthoxyacetic acid) or the like are subjected to condensation reaction to thereby obtain their diastereomeric ester or amide forms. On the other hand, when the compound (I) has a carboxylic acid group, the compound and an optically active amine or alcohol reagent are subjected to condensation reaction to thereby obtain their diastereomeric amide or ester forms. The resolved diastereomers are converted to optical isomers of the original compound through acid hydrolysis or basic hydrolysis reaction.

The compound (I) may be in a crystalline form. Both of single crystal forms or crystal form mixtures are encompassed in the compound (I). The crystals of the compound (I) can be produced by crystallizing the compound (I) by use of a crystallization method known per se in the art.
The compound (I) may be in a pharmaceutically acceptable co-crystal or co-crystal salt form. In this context, the co-crystal or co-crystal salt means a crystalline substance composed of, at room temperature, two or more unique solids differing in physical properties (e.g., structure, melting point, heat of fusion, hygroscopicity, solubility, and stability). The co-crystal or co-crystal salt can be produced according to a co-crystallization method known per se in the art.
The compound (I) may be in a solvate (e.g., hydrate) or non-solvate (e.g., anhydride) form. Both of these forms are encompassed in the compound (I).
Examples of the crystallization method include a solution-assisted crystallization method, a steam-assisted crystallization method, and a melt-assisted crystallization method.

The "solution-assisted crystallization method" generally involves changing a factor related to the solubility of a compound (solvent composition, pH, temperature, ion strength, and redox state) or the amount of a solvent to thereby

shift a unsaturated state to a supersaturated state. Examples thereof specifically include a concentration method, a slow cooling method, reaction methods (diffusion and electrolysis methods), a hydrothermal growth method, and a flux method. Examples of the solvent used include aromatic hydrocarbons (e.g., benzene, toluene, and xylene), halogenated hydrocarbons (e.g., dichloromethane and chloroform), saturated hydrocarbons (e.g., hexane, heptane, and cyclohexane), ethers (e.g., diethyl ether, diisopropyl ether, tetrahydrofuran, and dioxane), nitriles (e.g., acetonitrile), ketones (e.g., acetone), sulfoxides (e.g., dimethyl sulfoxide), acid amides (e.g., N,N-dimethylformamide), esters (e.g., ethyl acetate), alcohols (e.g., methanol, ethanol, and isopropyl alcohol), and water. These solvents are used alone or as a mixture of two or more thereof at an appropriate ratio (e.g., 1:1 to 1:100 (volume ratio)). Seed crystals can also be used, if necessary.

Examples of the "steam-assisted crystallization method" include vaporization methods (sealed tube and air flow methods), a gas-phase reaction method, and a chemical transport reaction.

Examples of the "melt-assisted crystallization method" include normal freezing methods (pulling, temperature gradient, and Bridgman methods), zone melting methods (zone leveling and float zone methods), and special growth methods (VLS and liquid-phase epitaxy methods).

Preferable examples of the crystallization method include methods involving dissolving the compound (I) in an appropriate solvent (e.g., alcohols such as methanol and ethanol) at a temperature of 20°C to 120°C and cooling the obtained solution to a temperature equal to or lower than the dissolution temperature (e.g. 0°C to 50°C, preferably 0°C to 20°C). The crystals of the present invention thus obtained can be isolated by, for example, filtration. The obtained crystals are generally analyzed by a crystal analysis method based on powder X-ray diffraction. Furthermore, examples of methods for determining the orientation of the crystals include both mechanical and optical methods.

The crystals of the compound (I) (hereinafter, abbreviated to "crystals of the present invention") obtained by the production process have high purity, high quality, and low hygroscopicity and are very excellent in stability because they are not deteriorated even after being stored for a long period under usual conditions. The crystals are also excellent in biological properties (e.g., biodynamics (absorbability, distribution, metabolism, and excretion) and manifestation of pharmacological efficacy) and are thus very useful as a medicament.

In the present specification, a specific rotation ($[\alpha]_D$) means that measured using, for example, a polarimeter (JASCO polarimeter model P-1030 (No. AP-2)). In the present specification, a melting point means that measured using, for example, a melting point apparatus (Stanford Research Systems, Inc., OptiMelt), a micro melting point apparatus (Yanaco New Science Inc., model MP-500V), or a DSC (differential scanning calorimetry) apparatus (SEIKO, EXSTAR6000).

The prodrug of the compound (I) refers to a compound that is converted to the compound (I) through reaction with an enzyme, gastric acid, or the like under physiological conditions *in vivo,* i.e., a compound that is converted to the compound (I) through enzymatic oxidation, reduction, hydrolysis, or the like, or a compound that is converted to the compound (I) through hydrolysis or the like caused by gastric acid or the like. A compound (I) containing acylated, alkylated, or phosphorylated amino (e.g., a compound (I) containing eicosanoylated, alanylated, pentylamino-carbonylated, (5-methyl-2-oxo-1,3-dioxolen-4-yl)methoxy-carbonylated, tetrahydrofuranylated, pyrrolidyl-methylated pivaloyloxy-methylated, or tert-butylated amino), a compound (I) containing acylated, alkylated, phosphorylated, or borated hydroxy (e.g., a compound (I) containing acetylated, palmitoylated, propanoylated, pivaloylated, succinylated, fumarylated, alanylated, or dimethylaminomethyl-carbonylated hydroxy), a compound (I) containing esterified or amidated carboxyl (e.g., a compound (I) containing ethyl-esterified, phenyl-esterified, carboxymethyl-esterified, dimethylaminomethyl-esterified, pivaloyloxymethyl-esterified, ethoxycarbonyloxyethyl-esterified, phthalidyl-esterified, (5-methyl-2-oxo-1,3-dioxolen-4-yl)methyl-esterified, cyclohexyloxycarbonylethyl-esterified, or methyl-amidated carboxyl), or the like is used as the prodrug of the compound (I). These compounds can be produced from the compound (I) by a method known per se in the art.

Also, the prodrug of the compound (I) may be a compound that is converted to the compound (I) under physiological conditions as described in Hirokawa Shoten Co., Ltd., "Development of Medicines" published in 1990, Vol. 7, Molecular Design, p. 163-198.

The compound (I) and the prodrug thereof may form a salt. The salt of the compound is not particularly limited, and, for example, a salt with an inorganic base, an ammonium salt, a salt with an organic base, a salt with an inorganic acid, a salt with an organic acid, or a salt with an amino acid is used. Preferable examples of the salt with an inorganic base used include: alkali metal salts such as sodium salt and potassium salt; alkaline earth metal salts such as calcium salt and magnesium salt; and aluminum salt and ammonium salt. Preferable examples of the salt with an organic base used include salts with trimethylamine, triethylamine, pyridine, picoline, 2,6-lutidine, ethanolamine, diethanolamine, triethanolamine, cyclohexylamine, dicyclohexylamine, or N,N'-dibenzylethylenediamine. Preferable examples of the salt with an inorganic acid used include salts with hydrochloric acid, hydrobromic acid, nitric acid, sulfuric acid, or phosphoric acid. Preferable examples of the salt with an organic acid used include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, or p-toluenesulfonic acid. Preferable examples of the salt with a basic amino acid used include salts with arginine, lysine, or ornithine. Preferable examples of the salt with an acidic amino acid used include

salts with aspartic acid or glutamic acid.
Of them, a pharmaceutically acceptable salt is preferable. For example, when the compound contains an acidic functional group, examples of the pharmaceutically acceptable salt include: inorganic salts such as alkali metal salts (e.g., sodium salt and potassium salt) and alkaline earth metal salts (e.g., calcium salt, magnesium salt, and barium salt); and ammonium salt. Alternatively, when the compound contains a basic functional group, examples thereof include salts with inorganic acids such as hydrobromic acid, nitric acid, sulfuric acid, and phosphoric acid or with organic acids such as acetic acid, phthalic acid, fumaric acid, oxalic acid, tartaric acid, maleic acid, citric acid, succinic acid, methanesulfonic acid, and p-toluenesulfonic acid.
When the compound (I) is obtained as a mixture of optically active forms (racemate), the mixture can be resolved into the (R) or (S) form of interest by optical resolution means known per se in the art.
A compound labeled or substituted with an isotopic element (e.g., $^{2}H$, $^{3}H$, $^{11}C$, $^{14}C$, $^{18}F$, $^{35}S$, and $^{125}I$) is also encompassed in the compound (I) or the like.
The compound (I) of the present invention have an excellent neuroprotective effect, neurogenesis promoting effect, nerve regeneration promoting effect, cognitive function improving effect, etc. Moreover, the compound (I) has low toxicity (e.g., acute toxicity, chronic toxicity, genetic toxicity, reproductive toxicity, cardiotoxicity, drug interaction, and cancer toxicity), safety, and excellent biodynamics (e.g., drug half-life in blood) and is delivered at a high rate to the central nervous system. Thus, the compound (I) is useful as a medicament. Thus, the compound can be administered as a medicament with safe either directly or as a mixture with a pharmaceutically acceptable carrier or the like through an oral or parenteral route to mammals (e.g., mice, rats, hamsters, rabbits, cats, dogs, cow, horses, pigs, sheep, goats, monkeys, and humans). The "parenteral" route includes intravenous, intramuscular, hypodermic, intraorgan, intranasal, intradermal, eye drop, intracerebral, intrarectal, intravaginal, or intraperitoneal administration or the like, and direct administration to lesions.
The compound (I) is useful as an IGF-1 signal modulator, a stem cell growth/differentiation promoter, a neuronal precursor growth/differentiation promoter, a protein kinase B activator, a neurogenesis promoter, or a nerve regeneration promoter. The compound of the present invention is particularly useful as a neuronal differentiation promoter, an IGF-1 signal modulator, or a protein kinase B activator.
Also, the compound (I) is useful as a therapeutic or prophylactic agent for central nervous system diseases, for example, the following diseases:

(1) neuropsychiatric diseases (e.g., depression, anxiety, manic-depression, schizophrenia, anxiety neurosis, obsessive-compulsive neurosis, and hyperkinetic syndrome),
(2) neurodegenerative diseases (e.g., Alzheimer's disease, Parkinson's disease, amyotrophic lateral sclerosis, Huntington's disease, spinocerebellar degeneration, multiple sclerosis (MS), and Pick disease),
(3) memory disorders (e.g., senile dementia, mild cognitive impairment, and mild memory impairment),
(4) cerebrovascular disorders (e.g., cerebral infarction, stroke, and multiinfarct dementia),
(5) head trauma and spinal cord injury,
(6) ischemic diseases (e.g., angina pectoris and myocardial infarction),
(7) cerebral ischemic diseases (e.g., cerebral infarction),
(8) metabolic diseases (e.g., diabetes mellitus and hypertension),
(9) peripheral neurological diseases (e.g., diabetic neuropathy and urinary tract/bladder dysfunction), and
(10) circulatory diseases (e.g., arteriosclerosis).

The compound (I) is particularly useful as a therapeutic or prophylactic agent for of a neurodegenerative disease, specifically, Alzheimer's disease.
Furthermore, the compound (I) can be used either directly or as a mixture with a pharmaceutically acceptable carrier or the like as a drug improving quality of life in a patient with hear failure after myocardial infarction, a drug improving quality of life in a patient after cerebral infarction, a hypoglycemic drug, an insulin resistance improving drug, or a blood triglyceride lowering drug.
In addition, the compound (I) of the present invention is effective as stem cell, iPS cell, and/or neuronal precursor growth/differentiation promoters for, for example, neurodegenerative diseases (e.g., Alzheimer's disease, Parkinson's disease, amyotrophic lateral sclerosis (ALS), Huntington's disease, and spinocerebellar degeneration), neuropsychiatric diseases (e.g., schizophrenia), head trauma, spinal cord injury, cerebrovascular disorders, and multiinfarct dementia, and is used as a therapeutic or prophylactic agent for these central nervous system diseases.
The medicament comprising the compound (I) of the present invention can be used as a pharmaceutical composition comprising the compound (I) alone or in combination with a pharmaceutically acceptable carrier according to a method for producing a pharmaceutical preparation known per se in the art (e.g., a method described in Japanese Pharmacopoeia). The medicament comprising the compound (I) can be administered with safe as, for example, tablets (including sugar-coated tablets, film-coated tablets, sublingual tablets, orally disintegrating tablets, buccal tablets, etc.),

pills, powders, granules, capsules (including soft capsules and microcapsules), troches, syrups, solutions, emulsions, suspensions, release-controlled preparations (e.g., quick-release preparations, sustained-release preparations, and sustained-release microcapsules), aerosols, films (e.g., orally disintegrating films and oral mucosal patch films), injections (e.g., hypodermic injections, intravenous injections, intramuscular injections, and intraperitoneal injections), drips, transdermally absorbable preparations, ointments, lotions, patches, suppositories (e.g., rectal suppositories and vaginal suppositories), pellets, transnasal agents, transpulmonary agents (inhalants), or eye droppers, through an oral or parenteral (e.g., intravenous, intramuscular, hypodermic, intraorgan, intranasal, intradermal, eye drop, intracerebral, intrarectal, intravaginal, intraperitoneal, or to lesions) route.

Various organic or inorganic carrier substances routinely used as pharmaceutical materials are used as the pharmaceutically acceptable carrier. An excipient, a lubricant, a binder, and a disintegrant, etc. are used, for example, in solid preparations. A solvent, a solubilizer, a suspending agent, a tonicity agent, a buffer, and a soothing agent, etc., are used in liquid preparations. Pharmaceutical additives such as antiseptics, antioxidants, coloring agents, and sweeteners can also be used, if necessary.

Preferable examples of the excipient used include lactose, saccharose, D-mannitol, starch, crystalline cellulose, and light anhydrous silicic acid.

Preferable examples of the lubricant used include magnesium stearate, calcium stearate, talc, and colloidal silica.

Preferable examples of the binder used include crystalline cellulose, saccharose, D-mannitol, dextrin, hydroxypropylcellulose, hydroxypropylmethylcellulose, and polyvinylpyrrolidone.

Preferable examples of the disintegrant used include starch, carboxymethylcellulose, carboxymethylcellulose calcium, croscarmellose sodium, and carboxymethyl starch sodium.

Preferable examples of the solvent used include injectable water, alcohols, propylene glycol, macrogol, sesame oil, corn oil, and olive oil.

Preferable examples of the solubilizer used include polyethylene glycol, propylene glycol, D-mannitol, benzyl benzoate, ethanol, trisaminomethane, hydrophilic surfactants such as Tween (registered trademark) 80, cyclodextrin (e.g., α-, β-, or γ-cyclodextrin, 2-hydroxypropyl-β-cyclodextrin, or methyl-β-cyclodextrin), cholesterol, triethanolamine, sodium carbonate, and sodium citrate.

Preferable examples of the suspending agent used include: surfactants such as stearyltriethanolamine, sodium lauryl sulfate, laurylaminopropionic acid, lecithin, benzalkonium chloride, benzethonium chloride, and monostearic acid glycerin; and hydrophilic polymers such as polyvinyl alcohol, polyvinylpyrrolidone, carboxymethylcellulose sodium, methylcellulose, hydroxymethylcellulose, hydroxyethylcellulose, and hydroxypropylcellulose.

Preferable examples of the tonicity agent used include glucose, D-sorbitol, sodium chloride, glycerin, and D-mannitol.

Preferable examples of the buffer used include phosphate, acetate, carbonate, and citrate buffer solutions.

Preferable examples of the soothing agent used include benzyl alcohol.

Preferable examples of the antiseptic used include parahydroxybenzoic acid esters, chlorobutanol, benzyl alcohol, phenethyl alcohol, dehydroacetic acid, and sorbic acid.

Preferable examples of the antioxidant used include sulfite, ascorbic acid, and α-tocopherol.

The pharmaceutical composition can be produced according to a standard method by allowing it to contain usually 0.01 to 100% (w/w), preferably 0.1 to 95% (w/w) of the compound of the present invention with respect to the whole amount of the preparation, though this approach differs depending on dosage forms, administration methods, carriers, etc.

The dose of the compound (I) differs depending on administration routes, conditions, the age of a patient, etc. For example, when the compound (I) is orally administered as a drug for the treatment of Alzheimer's disease to an Alzheimer's disease patient (body weight: 40 to 80 kg), the dose is, for example, 0.1 to 200 mg/kg body weight/day, preferably 1 to 100 mg/kg body weight/day, more preferably 1 to 50 mg/kg body weight/day. This daily dose can be administered once a day or two or three times a day.

The compound (I) may be used as a medicament in combination with an additional drug.

Examples of the drug that can be used in combination with the compound (I) (hereinafter, abbreviated to a concomitant drug) include the followings:

(1) Preventive or therapeutic drug for central nervous disease

Examples thereof include therapeutic drugs for depression, therapeutic drugs for anxiety (e.g., benzodiazepines such as chlordiazepoxide, diazepam, potassium clorazepate, lorazepam, clonazepam, and alprazolam), mood stabilizing drugs (e.g., lithium carbonate), 5-HT2 antagonists (e.g., nefazodone), 5-HT1A agonists (e.g., tandospirone, buspirone, and gepirone), CRF antagonists (e.g., pexacerfont), β3 agonists (e.g., amibegron), melatonin agonists (e.g., ramelteon and agomelatine), α2 antagonists (e.g., mirtazapine and setiptiline), NK2 antagonists (e.g., saredutant), GR antagonists (e.g., mifepristone), NK-1 antagonists (e.g., casopitant and orvepitant), therapeutic drugs for schizophrenia (e.g., chlorpromazine, haloperidol, sulpiride, clozapine, aripiprazole, quetiapine, olanzapine, and risperidone), acetylcholine esterase inhibitors (e.g., donepezil, rivastigmine, galanthamine, and zanapezil), NMDA antagonists (e.g., memantine), β

amyloid protein production, secretion, accumulation, aggregation, and/or deposition inhibitors [e.g., β secretase inhibitors, agents having a γ secretase inhibitory effect (e.g., LY-450139, E-2012, and E-2212), agents having β amyloid protein aggregation inhibitory effect (e.g., PTI-00703, ALZHEMED (NC-531), PPI-368 (National Publication of International Patent Application No. 1999-514333), PPI-558 (National Publication of International Patent Application No. 2001-500852), and SKF-74652 (Biochem. J. (1999), 340 (1), 283-289)), β amyloid vaccines, antibodies against β amyloid (e.g., AAB-001), and β amyloid-degrading enzymes], tau aggregation inhibitors (e.g., Rember), brain function enhancers (e.g., aniracetam and nicergoline), therapeutic drugs for Parkinson's disease [e.g., dopamine receptor agonists (e.g., L-dopa, bromocriptine, pergolide, talipexole, pramipexole, cabergoline, and amantadine), and COMT inhibitors (e.g., entacapone)], therapeutic drugs for attention deficit hyperactivity disorder (e.g., modafinil), therapeutic drugs for amyotrophic lateral sclerosis (e.g., riluzole and neurotrophic factors), therapeutic drugs for insomnia (e.g., etizolam, zopiclone, triazolam, zolpidem, and indiplon), therapeutic drugs for hypersomnia (e.g., modafinil), therapeutic drugs for cerebrovascular disorder (edaravone and tPA), anti-cytokine drugs (TNF inhibitors and MAP kinase inhibitors), and steroid drugs (e.g., dexamethasone, hexestrol, and cortisone acetate).

(2) Preventive or therapeutic drug for urinary incontinence

Examples thereof include adrenaline α1 receptor agonists (e.g., ephedrine hydrochloride and midodrine hydrochloride), adrenaline β2 receptor agonists (e.g., clenbuterol), substances inhibiting norepinephrine uptake, substances inhibiting norepinephrine and serotonin uptakes (e.g., duloxetine), tricyclic antidepressants (e.g., imipramine hydrochloride), anticholinergics or smooth muscle stimulants (e.g., oxybutynin hydrochloride, propiverine hydrochloride, and celimeverine hydrochloride), and female hormone drugs (e.g., conjugated estrogen (Premarin) and estriol).

(3) Therapeutic drugs for diabetes mellitus

Examples thereof include insulin preparations [e.g., animal insulin preparations extracted from bovine or porcine pancreas; human insulin preparations synthesized in a genetic engineering manner using E. *coli* or yeast; insulin zinc; protamine insulin zinc; and insulin fragments or derivatives (e.g., INS-1)], insulin sensitivity enhancers (e.g., pioglitazone hydrochloride, troglitazone, rosiglitazone or maleate thereof, JTT-501, MCC-555, YM-440, GI-262570, KRP-297, FK-614, and CS-011), α-glucosidase inhibitors (e.g., voglibose, acarbose, miglitol, and emiglitate), biguanides (e.g., phenformin, metformin, and buformin), sulfonylurea agents (e.g., tolbutamide, glibenclamide, gliclazide, chlorpropamide, tolazamide, acetohexamide, glyclopyramide, and glimepiride) or other insulin secretion promoters (e.g., repaglinide, senaglinide, mitiglinide or calcium salt hydrate thereof, GLP-1, and nateglinide), dipeptidyl peptidase IV inhibitors (e.g., vildagliptin, sitagliptin, saxagliptin, alogliptin, NVP-DPP-728, PT-100, and P32/98), GPR40 agonists (e.g., TAK-875), GPR119 agonists (e.g., PSN-821 and AR-231453), β3 agonists (e.g., CL-316243, SR-58611-A, UL-TG-307, AJ-9677, and AZ40140), amylin agonists (e.g., pramlintide), phosphotyrosine phosphatase inhibitors (e.g., vanadic acid), gluconeogenesis inhibitors (e.g., glycogen phosphorylase inhibitors, glucose-6-phosphatase inhibitors, and glucagon antagonists), glucokinase activators, and SGLT (sodium-glucose cotransporter) inhibitors (e.g., T-1095).

(4) Therapeutic drug for diabetic complication

Examples thereof include aldose reductase inhibitors (e.g., tolrestat, epalrestat, zenarestat, zopolrestat, fidarestat (SNK-860), minalrestat (ARI-509), and CT-112), neurotrophic factors (e.g., NGF and NT-3), AGE inhibitors (e.g., ALT-945, pimagedine, pyratoxanthine, N-phenacylthiazolium bromide (ALT-766), and EXO-226), active oxygen scavengers (e.g., thioctic acid), and cerebral vasodilators (e.g., tiapride).

(5) Anti-hyperlipidemia drug

Examples thereof include statin compounds as cholesterol synthesis inhibitors (e.g., pravastatin, simvastatin, lovastatin, atorvastatin, fluvastatin, cerivastatin, and salts thereof (e.g., sodium salt)), and squalene synthase inhibitors or fibrate compounds having a triglyceride lowering effect (e.g., bezafibrate, clofibrate, simfibrate, and clinofibrate).

(6) Hypotensive drug

Examples thereof include angiotensin converting enzyme inhibitors (e.g., captopril, enalapril, and delapril), angiotensin II antagonists (e.g., losartan and candesartan cilexetil), calcium antagonists (e.g., manidipine, nifedipine, amlodipine, efonidipine, and nicardipine), and clonidine.

(7) Antiobesity drug

Examples thereof include central antiobesity drugs (e.g., dexfenfluramine, fenfluramine, phentermine, sibutramine, amfepramone, dexamfetamine, mazindol, phenylpropanolamine, and clobenzorex), pancreatic lipase inhibitors (e.g., orlistat), β3 agonists (e.g., CL-316243, SR-58611-A, UL-TG-307, AJ-9677, and AZ40140), serotonin 5-HT2C agonists (e.g., lorcaserin), peptidic anorectics (e.g., leptin and CNTF (ciliary neurotrophic factor)), and cholecystokinin agonists (e.g., lintitript and FPL-15849).

(8) Diuretic

Examples thereof include xanthine derivatives (e.g., theobromine and sodium salicylate and theobromine and calcium salicylate), thiazide preparations (e.g., ethiazide, cyclopentiazide, trichlormethiazide, hydrochlorothiazide, hydroflumethiazide, benzylhydrochlorothiazide, penflutizide, polythiazide, and methyclothiazide), anti-aldosterone preparations (e.g., spironolactone and triamterene), carbonic anhydrase inhibitors (e.g., acetazolamide), chlorobenzenesulfonamide preparations (e.g., chlorthalidone, mefruside, and indapamide), azosemide, isosorbide, ethacrynic acid, piretanide, bumetanide, and furosemide.

(9) Chemotherapeutic

Examples thereof include alkylating agents (e.g., cyclophosphamide and ifosfamide), antimetabolites (e.g., methotrexate and 5-fluorouracil), anticancer antibiotics (e.g., mitomycin and adriamycin), plant-derived anticancer drugs (e.g., vincristine, vindesine, and taxol), cisplatin, carboplatin, and etoposide, and, particularly, a 5-fluorouracil derivative Furtulon or Neo-Furtulon.

(10) Immunotherapeutic

Examples thereof include microbial or bacterial components (e.g., muramyl dipeptide derivatives and picibanil), polysaccharides having an immunopotentiating activity (e.g., lentinan, sizofiran, and Krestin), cytokines obtained by a genetic engineering approach (e.g., interferon and interleukin (IL)), and colony stimulating factors (e.g., granulocyte colony stimulating factors and erythropoietin), and, particularly, IL-1, IL-2, and IL-12.

(11) Drug confirmed to have cachexia improving effect in animal models or clinical trials

Examples thereof include progesterone derivatives (e.g., megestrol acetate) [Journal of Clinical Oncology, Vol. 12, p. 213-225, 1994], metoclopramide drugs, tetrahydrocannabinol drugs (all, idem), lipid metabolism improving drugs (e.g., eicosapentaenoic acid) [British Journal of Cancer, Vol. 68, p. 314-318, 1993], growth hormones, IGF-1, and antibodies against cachexia-inducing factors TNF-α, LIF, IL-6, and oncostatin M.

(12) Anti-inflammatory agent

Examples thereof include steroid drugs (e.g., dexamethasone), sodium hyaluronate, and cyclooxygenase inhibitors (e.g., indomethacin, ketoprofen, loxoprofen, meloxicam, ampiroxicam, celecoxib, and rofecoxib).

(13) Others

Examples thereof include saccharification inhibitors (e.g., ALT-711), nerve regeneration promoters (e.g., Y-128, VX853, and Prosaptide), central nervous system drugs (e.g., antidepressants such as desipramine, amitriptyline, imipramine, fluoxetine, paroxetine, and doxepin), antiepileptic drugs (e.g., lamotrigine and carbamazepine), antiarrhythmic drugs (e.g., mexiletine), acetylcholine receptor ligands (e.g., ABT-594), endothelin receptor antagonists (e.g., ABT-627), monoamine uptake inhibitors (e.g., tramadol), indolamine uptake inhibitors (e.g., fluoxetine and paroxetine), narcotic analgesics (e.g., morphine), GABA receptor agonists (e.g., gabapentin), GABA uptake inhibitors (e.g., tiagabine), α2 receptor agonists (e.g., clonidine), local analgesics (e.g., capsaicin), protein kinase C inhibitors (e.g., LY-333531), antianxiety drugs (e.g., benzodiazepines), phosphodiesterase inhibitors (e.g., sildenafil), dopamine receptor agonists (e.g., apomorphine), dopamine receptor antagonists (e.g., haloperidol), serotonin receptor agonists (e.g., tandospirone citrate and sumatriptan), serotonin receptor antagonists (e.g., cyproheptadine hydrochloride and ondansetron), serotonin uptake inhibitors (e.g., fluvoxamine maleate, fluoxetine, and paroxetine), sleep inducing drugs (e.g., triazolam and zolpidem), anticholinergics, α1 receptor blockers (e.g., tamsulosin, silodosin, and naftopidil), muscle relaxants (e.g., baclofen), potassium channel openers (e.g., nicorandil), calcium channel blockers (e.g., nifedipine), preventive or therapeutic drugs

for Alzheimer's disease (e.g., donepezil, rivastigmine, and galantamine), therapeutic drugs for Parkinson's disease (e.g., L-dopa), preventive or therapeutic drugs for multiple sclerosis (e.g., interferon β-1a), histamine H1 receptor inhibitors (e.g., promethazine hydrochloride), proton pump inhibitors (e.g., lansoprazole and omeprazole), antithrombotic drugs (e.g., aspirin and cilostazol), NK-2 receptor antagonists, therapeutic drugs for HIV infection (saquinavir, zidovudine, lamivudine, and nevirapine), and therapeutic drugs for chronic obstructive pulmonary disease (salmeterol, tiotropium bromide, and cilomilast).

For example, atropine, scopolamine, homatropine, tropicamide, cyclopentolate, butylscopolamine bromide, propantheline bromide, methylbenactyzium bromide, mepenzolate bromide, flavoxate, pirenzepine, ipratropium bromide, trihexyphenidyl, oxybutynin, propiverine, darifenacin, tolterodine, temiverine, trospium chloride, or a salt thereof (e.g., atropine sulfate, scopolamine hydrobromide, homatropine hydrobromide, cyclopentolate hydrochloride, flavoxate hydrochloride, pirenzepine hydrochloride, trihexyphenidyl hydrochloride, oxybutynin hydrochloride, and tolterodine tartrate) is used as an anticholinergic. Among them, oxybutynin, propiverine, darifenacin, tolterodine, temiverine, trospium chloride, or a salt thereof (e.g., oxybutynin hydrochloride and tolterodine tartrate) is preferable. Also, an acetylcholine esterase inhibitor (e.g., distigmine) or the like can be used.

Examples of NK-2 receptor antagonists include: piperidine derivatives such as GR159897, GR149861, SR48968 (saredutant), SR144190, YM35375, YM38336, ZD7944, L-743986, MDL105212A, ZD6021, MDL105172A, SCH205528, SCH62373, and R-113281; perhydroisoindole derivatives such as RPR-106145; quinoline derivatives such as SB-414240; pyrrolopyrimidine derivatives such as ZM-253270; pseudopeptide derivatives such as MEN11420 (nepadutant), SCH217048, L-659877, PD-147714 (CAM-2291), MEN10376, and S16474; and GR100679, DNK333, GR94800, UK-224671, MEN10376, and MEN10627; and salts thereof.

The combination of the compound (I) with the concomitant drug can produce such excellent effects that:

(1) the dose thereof can be reduced compared with the single administration of the compound (I) or the concomitant drug,
(2) the drug to be used in combination with the compound (I) can be selected according to the symptoms (mild case, serious case, etc.) of a patient,
(3) the period of treatment can be set to a long duration by selecting a concomitant drug differing in the mechanism of action from the compound (I),
(4) therapeutic effects can be sustained by selecting a concomitant drug differing in the mechanism of action from the compound (I), and
(5) the combination use of the compound (I) with the concomitant drug produces synergistic effects.

For the combination use, the administration timings of the compound (I) and the concomitant drug are not limited. The compound (I) or its pharmaceutical composition and the concomitant drug or its pharmaceutical composition may be administered to a recipient simultaneously or at time intervals. The dose of the concomitant drug can follow doses clinically used and can be selected appropriately according to recipients, administration routes, diseases, combinations, etc. When these drugs are administered at time intervals, the time intervals differ depending on administered active ingredients, dosage forms, and administration methods. For example, when the concomitant drug is administered first, examples thereof include a method involving administering the compound (I) within 1 minute to 3 days, preferably within 10 minutes to 1 day, more preferably within 15 minutes to 1 hour, after the administration of the concomitant drug. When the compound (I) is administered first, examples thereof include a method involving administering the concomitant drug within 1 minute to 1 day, preferably within 10 minutes to 6 hours, more preferably within 15 minutes to 1 hour, after the administration of the compound (I).

For the combination use, dosage forms are not particularly limited. It is only required that the compound (I) and the concomitant drug should be combined during the administration period. Examples of such dosage forms include

(1) administration of a single preparation formulated to contain the compound (I) or its pharmaceutical composition with the concomitant drug,
(2) simultaneous administration through the same administration route of two preparations respectively formulated from the compound (I) or its pharmaceutical composition and the concomitant drug or its pharmaceutical composition,
(3) administration at time intervals through the same administration route of two preparations respectively formulated from the compound (I) or its pharmaceutical composition and the concomitant drug or its pharmaceutical composition,
(4) simultaneous administration through different administration routes of two preparations respectively formulated from the compound (I) or its pharmaceutical composition and the concomitant drug or its pharmaceutical composition, and
(5) administration at time intervals through different administration routes of two preparations respectively formulated from the compound (I) or its pharmaceutical composition and the concomitant drug or its pharmaceutical composition (e.g., administration of the compound (I) or its pharmaceutical composition and the concomitant drug or its pharmaceutical composition in this order, or vice versa). The combination formulation of the compound (I) can be used as a pharmaceutical composition comprising the compound (I) or the concomitant drug in combination with a pharmaceutically acceptable carrier according to a method for producing a pharmaceutical preparation known per se in the art (e.g., a method described in Japanese Pharmacopoeia). The combination formulation can be administered with safe as, for example, tablets (including sugar-coated tablets, film-coated tablets, sublingual tablets, orally disintegrating tablets, buccal tablets, etc.), pills, powders, granules, capsules (including soft capsules and microcapsules), troches, syrups, solutions, emulsions, suspensions, release-controlled preparations (e.g., quick-release preparations, sustained-release preparations, and sustained-release microcapsules), aerosols, films (e.g., orally disintegrating films and oral mucosal patch films), injections (e.g., hypodermic injections, intravenous injections, intramuscular injections, and intraperitoneal injections), drips, transdermally absorbable preparations, ointments, lotions, patches, suppositories (e.g., rectal suppositories and vaginal suppositories), pellets, transnasal agents, transpulmonary agents (inhalants), or eye droppers, through an oral or parenteral (e.g., intravenous, intramuscular, hypodermic, intraorgan, intranasal, intradermal, eye drop, intracerebral, intrarectal, intravaginal, intraperitoneal, or to lesions) route.

The combination formulation of the compound (I) may also be prepared as a sustained-release preparation comprising the active ingredients and a biodegradable polymer compound. The sustained-release preparation can be produced according to a method described in Japanese Patent Laid-Open No. 9-263545.
Various organic or inorganic carrier substances routinely used as pharmaceutical materials are used as the pharmaceutically acceptable carrier. An excipient, a lubricant, a binder, and a disintegrant, etc. are used, for example, in solid preparations. A solvent, a solubilizer, a suspending agent, a tonicity agent, a buffer, and a soothing agent, etc., are used in liquid preparations. Pharmaceutical additives such as antiseptics, antioxidants, coloring agents, and sweeteners can also be used, if necessary.
Preferable examples of the excipient used include lactose, saccharose, D-mannitol, starch, crystalline cellulose, and light anhydrous silicic acid.
Preferable examples of the lubricant used include magnesium stearate, calcium stearate, talc, and colloidal silica.
Preferable examples of the binder used include crystalline cellulose, saccharose, D-mannitol, dextrin, hydroxypropylcellulose, hydroxypropylmethylcellulose, and polyvinylpyrrolidone.
Preferable examples of the disintegrant used include starch, carboxymethylcellulose, carboxymethylcellulose calcium, croscarmellose sodium, and carboxymethyl starch sodium.
Preferable examples of the solvent used include injectable water, alcohols, propylene glycol, macrogol, sesame oil, corn oil, and olive oil.
Preferable examples of the solubilizer used include polyethylene glycol, propylene glycol, D-mannitol, benzyl benzoate, ethanol, trisaminomethane, hydrophilic surfactants such as Tween (registered trademark) 80, cyclodextrin (e.g., α-, β-, or γ-cyclodextrin, 2-hydroxypropyl-β-cyclodextrin, or methyl-β-cyclodextrin), cholesterol, triethanolamine, sodium carbonate, and sodium citrate.
Preferable examples of the suspending agent used include: surfactants such as stearyltriethanolamine, sodium lauryl sulfate, laurylaminopropionic acid, lecithin, benzalkonium chloride, benzethonium chloride, and monostearic acid glycerin; and hydrophilic polymers such as polyvinyl alcohol, polyvinylpyrrolidone, carboxymethylcellulose sodium, methylcellulose, hydroxymethylcellulose, hydroxyethylcellulose, and hydroxypropylcellulose.
Preferable examples of the tonicity agent used include glucose, D-sorbitol, sodium chloride, glycerin, and D-mannitol.
Preferable examples of the buffer used include phosphate, acetate, carbonate, and citrate buffer solutions.
Preferable examples of the soothing agent used include benzyl alcohol.
Preferable examples of the antiseptic used include parahydroxybenzoic acid esters, chlorobutanol, benzyl alcohol, phenethyl alcohol, dehydroacetic acid, and sorbic acid.
Preferable examples of the antioxidant used include sulfite, ascorbic acid, and α-tocopherol.
The mixing ratio between the compound (I) and the concomitant drug in the combination formulation of the present invention can be selected appropriately according to recipients, administration routes, diseases, etc.
For example, the content of the compound (I) in the combination formulation of the present invention is usually approximately 0.01 to 100% by weight, preferably approximately 0.1 to 50% by weight, more preferably approximately 0.5 to 20% by weight, with respect to the whole preparation, though it differs depending on the form of the preparation.
The content of the concomitant drug in the combination formulation of the present invention is usually approximately 0.01 to 100% by weight, preferably approximately 0.1 to 50% by weight, more preferably approximately 0.5 to 20% by weight, with respect to the whole preparation, though it differs depending on the form of the preparation.
The content of additives such as the carrier in the combination formulation of the present invention is usually approximately 1 to 99.99% by weight, preferably approximately 10 to 90% by weight, with respect to the whole preparation, though it differs depending on the form of the preparation.
When preparations are respectively formulated from the compound (I) and the concomitant drug, the same contents

can be used.

The dose of the compound (I) differs depending on administration routes, conditions, the age of a patient, etc. For example, when the compound (I) is orally administered as a drug for the treatment of Alzheimer's disease to an Alzheimer's disease patient (body weight: 40 to 80 kg), the dose is, for example, 0.1 to 200 mg/kg body weight/day, preferably 1 to 100 mg/kg body weight/day, more preferably 1 to 50 mg/kg body weight/day. This daily dose can be administered once a day or two or three times a day.

When the medicament comprising the compound (I) is a sustained-release preparation, the dose of the compound (I) applied to, for example, parenteral administration can be 1 to 100 mg/week of the compound (I) in terms of an amount released from the administered preparation.

Any dose of the concomitant drug may be set within a range free from adverse reaction. The daily dose of the concomitant drug differs depending on severity, the age, sex, and body weight of a recipient, difference in sensitivity, administration timings, intervals, the properties, formulation, and type of the pharmaceutical preparation, the type of the active ingredient, etc., and is not particularly limited. The amount of the drug in, for example, oral administration is usually approximately 0.001 to 2000 mg, preferably approximately 0.01 to 500 mg, more preferably approximately 0.1 to 100 mg per kg body weight of a mammal. This daily dose is usually administered once to four times a day.

When preparations are respectively formulated from the compound of the present invention and the concomitant drug, the same contents can be used.

The prodrug of the compound (I), the compound (Ia), and the compound ($I_0$) can be used as medicaments in the same way as in the compound (I).

## Examples

Hereinafter, the present invention will be described further specifically with reference to Reference Examples, Examples, Experimental Examples, and Preparation Examples. However, the present invention is not intended to be limited to them. In the chemical formulas of Reference Examples and Examples, the abbreviation "Me" represents a methyl group and may be omitted in some cases. Also, in Examples, a solvent (e.g., ethyl acetate-hexane or hexane) described within parentheses next to the melting point of a compound means a solvent used for the recrystallization of the compound in melting point measurement.

In Reference Examples below, "room temperature" usually represents approximately 10°C to approximately 35°C. "%" represents percent by weight unless otherwise specified. However, a yield is indicated in mol/mol%.

NMR spectra represent proton NMR. These spectra were measured in a 300 MHz-, 400 MHz-, or 600 MHz-type spectrometer using tetramethylsilane as an internal standard and indicated in ppm of δ values. In $^1$H-NMR, very gentle peaks of protons in a hydroxy group, an amino group, a carboxyl group, or the like may not be described in some cases.

The other abbreviations used herein represent the following meanings:

J: coupling constant
s: singlet
brs: broad singlet
d: doublet
dd: double doublet
t: triplet
dt: double triplet
q: quartet
m: multiplet
$CDCl_3$: deuterated chloroform
DMSO-d6: deuterated dimethyl sulfoxide
$^1$H-NMR: proton nuclear magnetic resonance
THF: tetrahydrofuran
DMSO: dimethyl sulfoxide
DMF: N,N-dimethylformamide
TFA: trifluoroacetic acid
WSC: 1-ethyl-3-(3'-dimethylaminopropyl)carbodiimide hydrochloride
HOBt: 1-hydroxybenzotriazole
BINAP: 2,2'-bis(diphenylphosphino)-1,1'-binaphthyl
Xantphos: 4,5-bis(diphenylphosphino)-9,9-dimethylxanthene
rhIGF-1: recombinant human IGF-1
HRP: horseradish peroxidase

Reference Example 1

2-bromo-6-methoxypyridin-3-amine

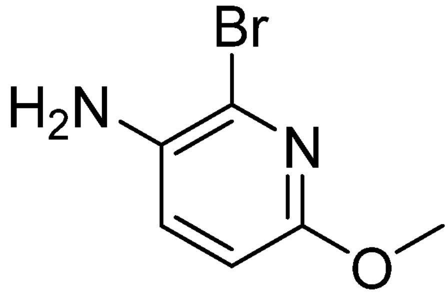

To an acetic acid (100 mL) solution of 6-methoxypyridin-3-amine (13.5 g, 109 mmol), sodium acetate (8.94 g, 109 mmol) was added, and the mixture was stirred at room temperature for 30 minutes. To the reaction solution, bromine (5.58 mL, 109 mmol) was added dropwise, and the mixture was stirred at room temperature for 15 hours. The reaction solution was neutralized by dropwise addition of a 10% aqueous sodium hydroxide solution, followed by extraction with ethyl acetate. The extracts were mixed, washed with a saturated aqueous solution of sodium disulfite, and then dried over anhydrous magnesium sulfate. The solvent was distilled off under reduced pressure, and the obtained residue was purified by silica gel column chromatography (hexane-ethyl acetate 100:0-85:15) to obtain 14.5 g of the title compound (yield: 66%).
$^1$H-NMR ($CDCl_3$): δ 3.72 (2H, brs), 3.86 (3H, s), 6.58 (1H, d, J = 8.7 Hz), 7.04 (1H, d, J = 8.7 Hz).

Reference Example 2

2-[4-(dimethylamino)phenyl]-6-methoxypyridin-3-amine

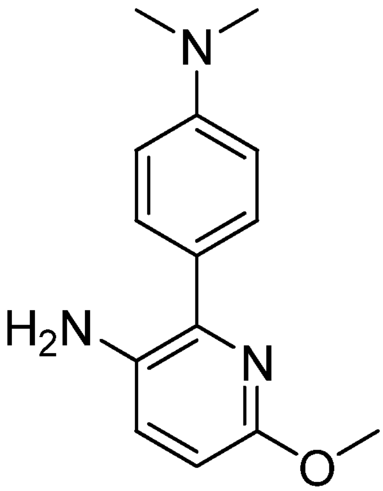

To a toluene (80 mL)-ethanol (40 mL)-water (24 mL) mixed solution of 2-bromo-6-methoxypyridin-3-amine (12.0 g, 59.1 mmol) synthesized in Reference Example 1, [4-(dimethylamino)phenyl]boronic acid (11.7 g, 70.9 mmol), and sodium carbonate (12.5 g, 118 mmol), tetrakis(triphenylphosphine)palladium (0) (4.78 g, 4.14 mmol) was added, and the mixture was stirred at 105°C for 10 hours. To the reaction solution, saturated brine was added, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 100:0-80:20) to obtain 14.2 g of the title compound (yield: 99%).
$^1$H-NMR ($CDCl_3$): δ 3.00 (6H, s), 3.55 (2H, brs), 3.90 (3H, s), 6.51 (1H, d, J = 8.7 Hz), 6.81 (2H, d, J = 9.1 Hz), 7.04 (1H,

d, J = 8.3 Hz), 7.63-7.73 (2H, m).

Reference Example 3

4-(3-bromo-6-methoxypyridin-2-yl)-N,N-dimethylaniline

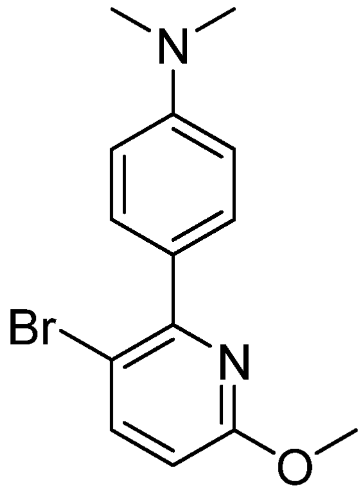

To a 48% hydrobromic acid (58.8 mL, 349 mmol) solution of copper bromide (16.5 g, 115 mmol), 2-[4-(dimethylamino) phenyl]-6-methoxypyridin-3-amine (7.00 g, 28.8 mmol) synthesized in Reference Example 2 was added, and an aqueous (70 mL) solution of sodium nitrite (3.97 g, 57.6 mmol) was then added dropwise thereto under ice cooling. The reaction mixture was stirred for 30 minutes under ice cooling, heated to room temperature, and stirred for 16 hours. To the reaction solution, a saturated aqueous solution of sodium disulfite was added, and the mixture was neutralized with an 8 N aqueous sodium hydroxide solution, followed by extraction with ethyl acetate. The extracts were mixed, washed with a saturated aqueous solution of sodium disulfite, and then dried over anhydrous magnesium sulfate. The solvent was distilled off under reduced pressure, and the obtained residue was purified by silica gel column chromatography (hexane-ethyl acetate 100:0-85:15) to obtain 7.16 g of the title compound (yield: 81%).
$^1$H-NMR ($CDCl_3$) : δ 3.02 (6H, s), 3.94 (3H, s), 6.51 (1H, d, J = 8.7 Hz), 6.72-6.83 (2H, m), 7.69-7.81 (3H, m).

Reference Example 4

Tert-butyl 4-{2-[4-(dimethylamino)phenyl]-6-methoxypyridin-3-yl}piperazine-1-carboxylate

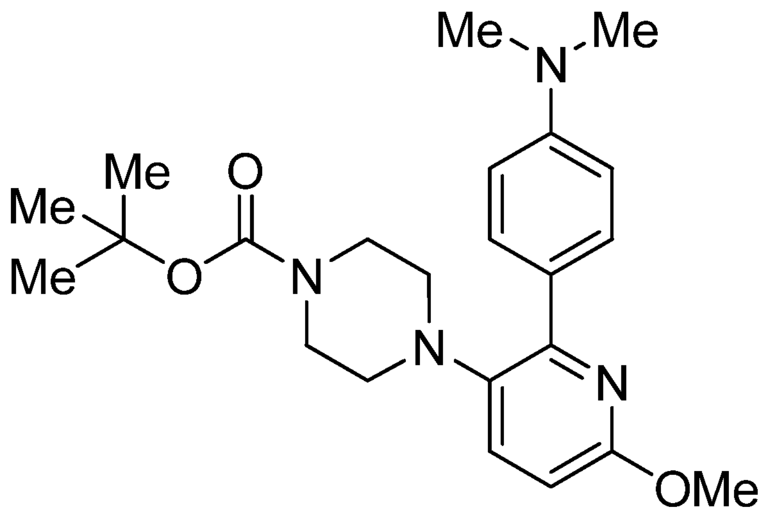

To a mixed solution of 4-(3-bromo-6-methoxypyridin-2-yl)-N,N-dimethylaniline (2.00 g, 6.51 mmol) synthesized in Reference Example 3, tert-butyl piperazine-1-carboxylate (2.43 g, 13.0 mmol), palladium acetate (73.0 mg, 0.330 mmol), and BINAP (608 mg, 0.980 mmol) in toluene (14 mL), sodium tert-butoxide (1.88 g, 19.5 mmol) was added, and the mixture was stirred at 160°C for 20 minutes under microwave irradiation. After cooling to room temperature, the reaction solution was partitioned with water and ethyl acetate, and the organic phase was dried over anhydrous magnesium sulfate. The solvent was distilled off under reduced pressure, and the obtained residue was purified by silica gel column chromatography (hexane-ethyl acetate 100:1-85:15) to obtain 1.35 g of the title compound (yield: 50%).
$^1$H-NMR ($CDCl_3$) : δ 1.46 (9H, s), 2.66-2.86 (4H, m), 3.01 (6H, s), 3.40-3.57 (4H, m), 3.95 (3H, s), 6.56 (1H, d, J = 8.5 Hz), 6.66-6.82 (2H, m), 7.31 (1H, d, J = 8.7 Hz), 8.14 (2H, d, J = 8.9 Hz).

Reference Example 5

4-(6-methoxy-3-piperazin-1-ylpyridin-2-yl)-N,N-dimethylaniline

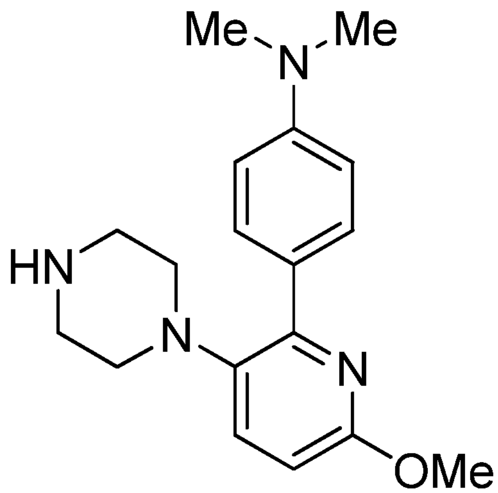

To an ethyl acetate (40 mL) solution of tert-butyl 4-{2-[4-(dimethylamino)phenyl]-6-methoxypyridin-3-yl}piperazine-1-carboxylate (16.1 g, 44.7 mmol) synthesized in Reference Example 4, a 4 N solution of hydrogen chloride in ethyl acetate (23.2 mL, 92.4 mmol) was added, and the mixture was stirred at room temperature for 36 hours. The reaction was stopped by addition of sodium bicarbonate. The mixed solution was partitioned with ethyl acetate and water, and the organic phase was dried over anhydrous magnesium sulfate. The solvent was distilled off under reduced pressure, and the obtained residue was purified by basic silica gel column chromatography (hexane-ethyl acetate 95:5-40:60) to obtain 879 mg of the title compound (yield: 85%).

$^1$H-NMR ($CDCl_3$): δ 2.77-2.85 (4H, m), 2.90-2.96 (4H, m), 3.01 (6H, s), 3.94 (3H, s), 6.56 (1H, d, J = 8.7 Hz), 6.76 (2H, d, J = 9.1 Hz), 7.35 (1H, d, J = 8.7 Hz), 8.18 (2H, d, J = 9.1 Hz).

Reference Example 6

6-methoxy-2-(4-methylphenyl)pyridin-3-amine

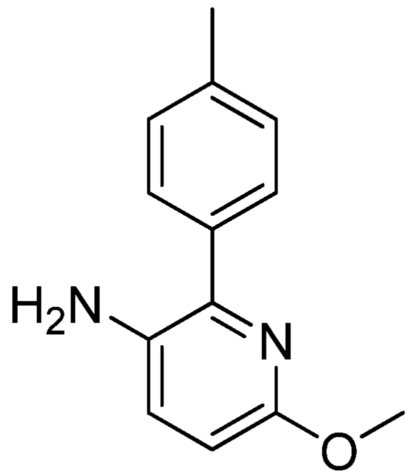

The title compound was obtained in the same way as in Reference Example 2 using 2-bromo-6-methoxypyridin-3-amine (10.0 g, 49.3 mmol) synthesized in Reference Example 1 and (4-methylphenyl)boronic acid (18.4 g, 61.6 mmol). Yield: 71%.
$^1$H-NMR ($CDCl_3$): δ 2.40 (3H, s), 3.55 (2H, brs), 3.90 (3H, s), 6.56 (1H, d, J = 8.4 Hz), 7.08 (1H, d, J = 8.8 Hz), 7.28 (2H, d, J = 7.6 Hz), 7.65 (2H, d, J = 8.4 Hz).

Reference Example 7

3-bromo-6-methoxy-2-(4-methylphenyl)pyridine

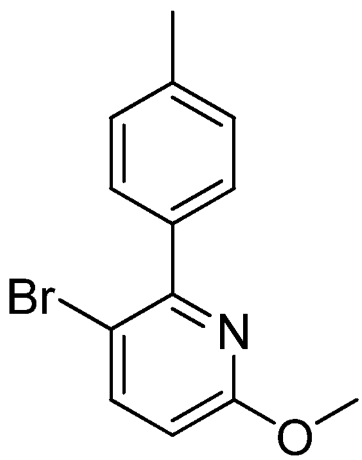

The title compound was obtained in the same way as in Reference Example 3 using 6-methoxy-2-(4-methylphenyl) pyridin-3-amine (10.0 g, 46.7 mmol) synthesized in Reference Example 6. Yield: 49%.
$^1$H-NMR ($CDCl_3$) : δ 2.41 (3H, s), 3.93 (3H, s), 6.58 (1H, d, J = 8.8 Hz), 7.26 (2H, d, J = 8.0 Hz), 7.56 (2H, d, J = 8.0 Hz), 7.77 (1H, d, J = 8.8 Hz).

Reference Example 8

2-(4-ethoxyphenyl)-6-methoxypyridin-3-amine

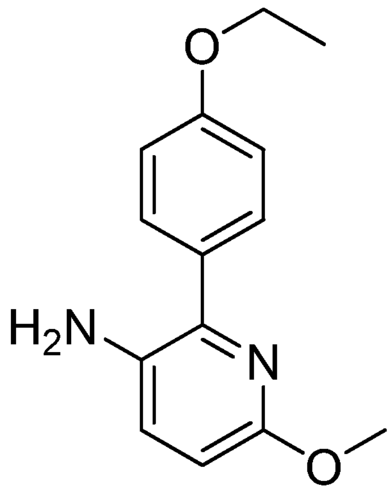

The title compound was obtained in the same way as in Reference Example 2 using 2-bromo-6-methoxypyridin-3-amine (3.05 g, 15.0 mmol) synthesized in Reference Example 1 and (4-ethoxyphenyl)boronic acid (2.99 g, 18.0 mmol). Yield: 99%.
$^1$H-NMR ($CDCl_3$) : δ 1.44 (3H, t, J = 7.0 Hz), 3.54 (2H, brs), 3.90 (3H, s), 4.09 (2H, q, J = 6.9 Hz), 6.56 (1H, d, J = 8.3 Hz), 6.98 (2H, d, J = 9.0 Hz), 7.07 (1H, d, J = 8.3 Hz), 7.70 (2H, d, J = 8.7 Hz).

Reference Example 9

3-bromo-2-(4-ethoxyphenyl)-6-methoxypyridine

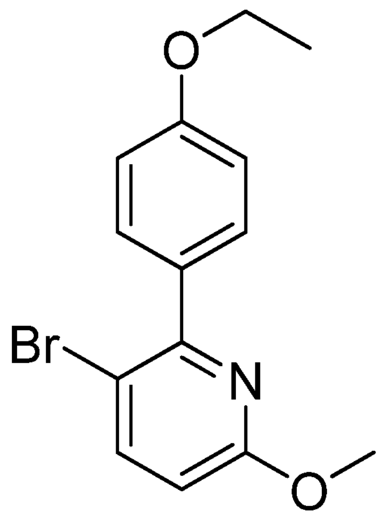

The title compound was obtained in the same way as in Reference Example 3 using 2-(4-ethoxyphenyl)-6-methoxypyridin-3-amine (3.64 g, 14.9 mmol) synthesized in Reference Example 8. Yield: 25%.
$^1$H-NMR ($CDCl_3$) : δ 1.45 (3H, t, J = 7.0 Hz), 3.94 (3H, s), 4.10 (2H, q, J = 6.8 Hz), 6.56 (1H, d, J = 8.7 Hz), 6.92-7.02 (2H, m), 7.71-7.80 (2H, m), 7.99 (1H, d, J = 9.0 Hz).

Reference Example 10

6-methoxy-2-[4-(1-methylethyl)phenyl]pyridin-3-amine

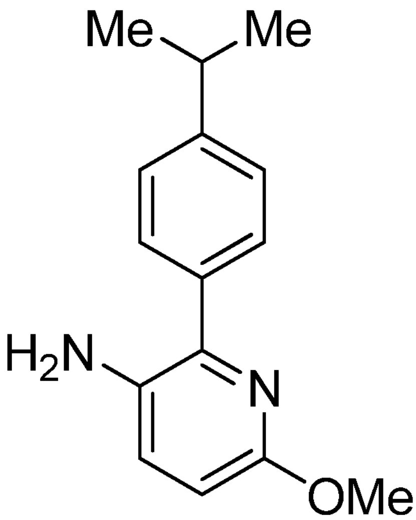

The title compound was obtained in the same way as in Reference Example 2 using 2-bromo-6-methoxypyridin-3-amine (10.0 g, 49.3 mmol) synthesized in Reference Example 1 and p-isopropylphenylboronic acid (10.5 g, 64.0 mmol). Yield: 67%.
$^1$H-NMR ($CDCl_3$) : δ 1.29 (6H, t, J = 6.9 Hz), 2.88-3.05 (1H, m), 3.56 (2H, brs), 3.91 (3H, s), 6.57 (1H, d, J = 8.7 Hz), 7.07 (1H, d, J = 8.7 Hz), 7.29-7.38 (2H, m), 7.65-7.74 (2H, m).

Reference Example 11

3-bromo-6-methoxy-2-[4-(1-methylethyl)phenyl]pyridine

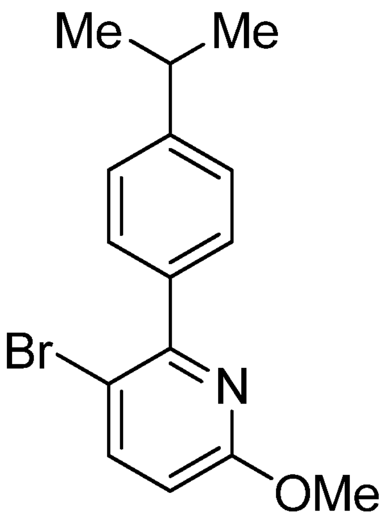

The title compound was obtained in the same way as in Reference Example 3 using 6-methoxy-2-[4-(1-methylethyl) phenyl]pyridin-3-amine (5.00 g, 20.6 mmol) synthesized in Reference Example 10. Yield: 17%.
$^1$H-NMR ($CDCl_3$) : δ 1.30 (6H, d, J = 6.6 Hz), 2.88-3.07 (1H, m), 3.94 (3H, s), 6.58 (1H, d, J = 8.7 Hz), 7.27-7.35 (2H, m), 7.66-7.74 (2H, m), 7.77 (1H, d, J = 8.7 Hz).

Reference Example 12

2-[N-(4-methoxyphenyl)amino]-N-{2-[4-(dimethylamino)phenyl]-6-methoxypyridin-3-yl}acetamide

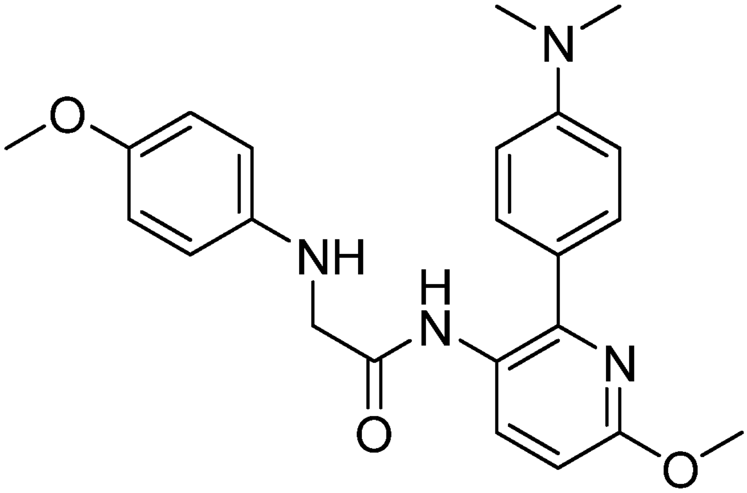

To a DMF (15 mL) solution of 2-[4-(dimethylamino)phenyl]-6-methoxypyridin-3-amine (2.01 g, 8.28 mmol) synthesized in Reference Example 2, WSC (1.91 g, 9.94 mmol), and HOBt (1.34 g, 9.94 mmol), N-(4-methoxyphenyl)glycine (1.50 g, 8.28 mmol) was added, and the mixture was stirred at room temperature for 6 hours. To the reaction solution, a saturated aqueous solution of sodium bicarbonate was added, followed by extraction with ethyl acetate. The extracts were mixed, washed with water, and then dried over anhydrous magnesium sulfate. The solvent was distilled off under reduced pressure, and the obtained residue was subjected to collection by filtration with diethyl ether to obtain 1.20 g of the title compound (yield: 36%).
$^1$H-NMR ($CDCl_3$) : δ 2.92 (6H, s), 3.78 (3H, s), 3.81 (2H, d, J = 5.3 Hz), 3.91 (3H, s), 3.95-4.04 (1H, m), 6.39 (2H, d, J = 9.1 Hz), 6.58 (2H, d, J = 9.1 Hz), 6.66 (1H, d, J = 9.1 Hz), 6.83 (2H, d, J = 8.7 Hz), 7.21-7.37 (2H, m), 8.59 (1H, d, J = 8.7 Hz), 9.03 (1H, brs).

Reference Example 13

2-[N-(bromoacetyl)-N-(4-methoxyphenyl)amino]-N-{2-[4-(dimethylamino)phenyl]-6-methoxypyridin-3-yl}acetamide

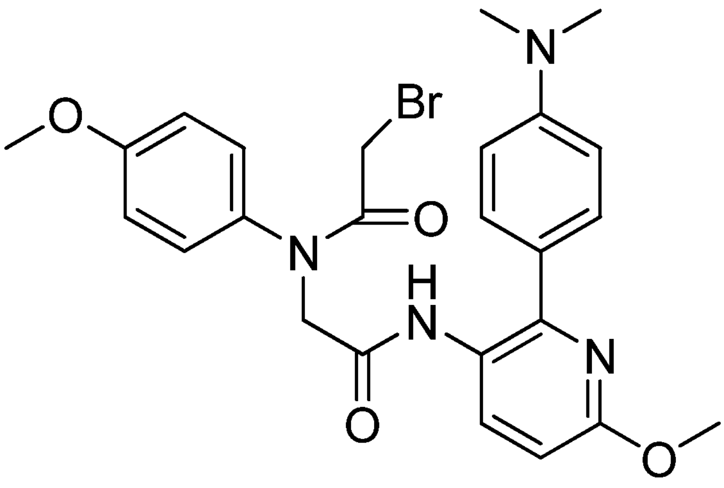

To a mixture of 2-[N-(4-methoxyphenyl)amino]-N-{2-[4-(dimethylamino)phenyl]-6-methoxypyridin-3-yl}acetamide (300

mg, 0.74 mmol) synthesized in Reference Example 12, ethyl acetate (3 mL), and a saturated aqueous solution of sodium bicarbonate (1.5 mL), bromoacetyl bromide (0.128 mL, 1.48 mmol) was added dropwise, and the mixture was stirred at room temperature for 16 hours. To the reaction solution, water was added, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 95:5-60: 40) to obtain 241 mg of the title compound (yield: 62%).
$^1$H-NMR ($CDCl_3$): δ 3.01 (6H, s), 3.66 (2H, s), 3.81 (3H, s), 3.93 (3H, s), 4.35 (2H, s), 6.66 (1H, d, J = 8.7 Hz), 6.74-6.91 (4H, m), 6.95-7.10 (2H, m), 7.51 (2H, d, J = 8.7 Hz), 7.85 (1H, brs), 8.29 (1H, d, J = 8.7 Hz).

Reference Example 14

2-bromo-N-[2-({2-[4-(dimethylamino)phenyl]-6-methoxypyridin-3-yl}amino)-2-oxoethyl]-N-(4-methoxyphenyl)propanamide

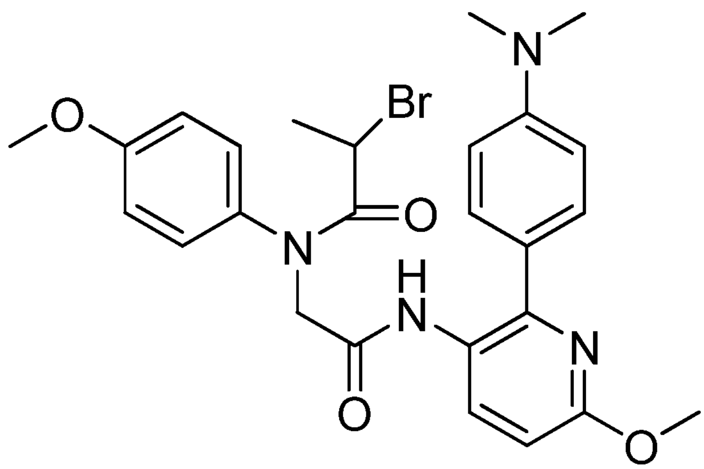

To a mixture of 2-[N-(4-methoxyphenyl)amino]-N-{2-[4-(dimethylamino)phenyl]-6-methoxypyridin-3-yl}acetamide (300 mg, 0.74 mmol) synthesized in Reference Example 12, ethyl acetate (3 mL), and a saturated aqueous solution of sodium bicarbonate (1.5 mL), 2-bromopropionyl chloride (0.152 mL, 1.48 mmol) was added dropwise, and the mixture was stirred at room temperature for 3 hours. To the reaction solution, water was added, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 100:0-50:50) to obtain 270 mg of the title compound (yield: 67%).
$^1$H-NMR ($CDCl_3$): δ 1.71 (3H, d, J = 6.4 Hz), 3.02 (6H, s), 3.83 (3H, s), 3.93 (3H, s), 4.19 (1H, d, J = 15.8 Hz), 4.28 (1H, q, J = 6.5 Hz), 4.47 (1H, d, J = 15.4 Hz), 6.65 (1H, d, J = 8.7 Hz), 6.80 (2H, d, J = 8.7 Hz), 6.89 (2H, d, J = 9.0 Hz), 7.20 (2H, d, J = 8.7 Hz), 7.52 (2H, d, J = 9.0 Hz), 7.70 (1H, s), 8.26 (1H, d, J = 8.7 Hz).

Reference Example 15

Methyl 2-[N-(4-methoxyphenyl)amino]propionate

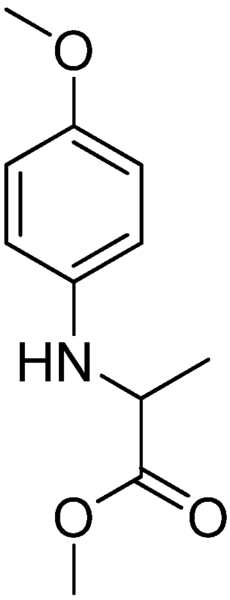

A mixture of 4-methoxyaniline (2.00 g, 16.2 mmol), methyl 2-bromopropanoate (2.16 mL, 19.4 mmol), potassium carbonate (3.36 g, 24.3 mmol), and DMF (20 mL) was stirred at 100°C for 12 hours. To the reaction solution, water was added, followed by extraction with ethyl acetate. The extracts were mixed, washed with water, and then dried over anhydrous magnesium sulfate. The solvent was distilled off under reduced pressure, and the obtained residue was purified by silica gel column chromatography (hexane-ethyl acetate 100:0-80:20) to obtain 3.10 g of the title compound (yield: 91%).
$^1$H-NMR ($CDCl_3$): δ 1.45 (3H, d, J = 7.2 Hz), 3.71 (3H, s), 3.73 (3H, s), 3.86 (1H, brs), 4.00-4.12 (1H, m), 6.54-6.63 (2H, m), 6.72-6.81 (2H, m).

Reference Example 16

2-[N-(4-methoxyphenyl)amino]propionic acid

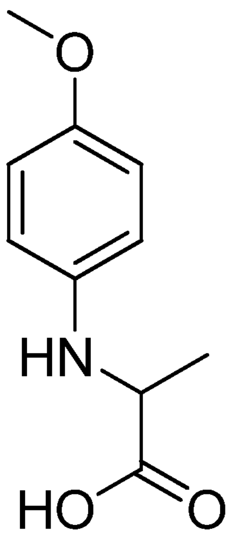

To a THF (30 mL)-methanol (15 mL) mixed solution of methyl 2-[N-(4-methoxyphenyl)amino]propionate (3.10 g, 14.8 mmol) synthesized in Reference Example 15, a 2 N aqueous sodium hydroxide solution (14.8 mL, 29.6 mmol) was added, and the mixture was stirred at room temperature for 2 hours. The reaction solution was neutralized with 6 N hydrochloric acid and diluted with water, followed by extraction with ethyl acetate. The extracts were mixed, washed

with water, and then dried over anhydrous magnesium sulfate. The solvent was distilled off under reduced pressure, and the obtained residue was subjected to collection by filtration with diethyl ether to obtain 2.22 g of the title compound (yield: 77%).
$^1$H-NMR ($CDCl_3$): δ 1.33 (3H, d, J = 7.2 Hz), 3.63 (3H, s), 3.86 (1H, q, J = 6.9 Hz), 6.44-6.60 (2H, m), 6.64-6.80 (2H, m), 8.88 (2H, brs).

Reference Example 17

2-[N-(4-methoxyphenyl)amino]-N-{2-[4-(dimethylamino)phenyl]-6-methoxypyridin-3-yl}propanamide

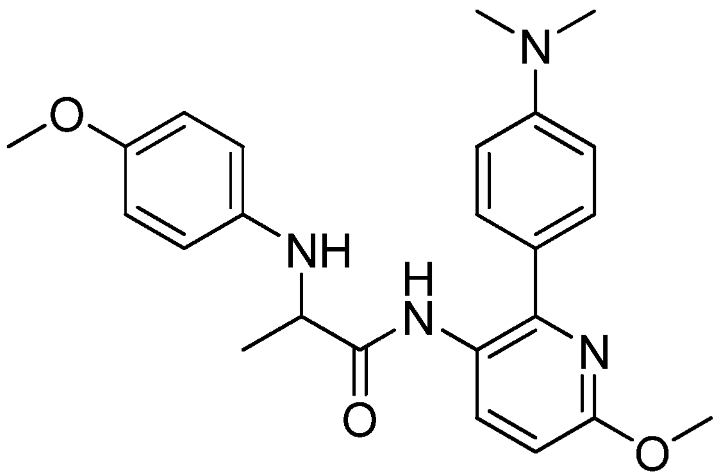

The title compound was obtained in the same way as in Reference Example 12 using 2-[4-(dimethylamino)phenyl]-6-methoxypyridin-3-amine (1.00 g, 4.11 mmol) synthesized in Reference Example 2 and 2-[N-(4-methoxyphenyl)amino] propionic acid (962 mg, 4.93 mmol) synthesized in Reference Example 16. Yield: 75%.
$^1$H-NMR ($CDCl_3$) : δ 1.52 (3H, d, J = 7.2 Hz), 2.93 (6H, s), 3.59 (1H, s), 3.69-3.85 (4H, m), 3.91 (3H, s), 6.39 (2H, d, J = 9.1 Hz), 6.57 (2H, d, J = 9.1 Hz), 6.65 (1H, d, J = 8.7 Hz), 6.78-6.90 (2H, m), 7.21-7.33 (2H, m), 8.55 (1H, d, J = 9.1 Hz), 9.02 (1H, s).

Reference Example 18

2-[N-(bromoacetyl)-N-(4-methoxyphenyl)amino]-N-{2-[4-(dimethylamino)phenyl]-6-methoxypyridin-3-yl}propanamide

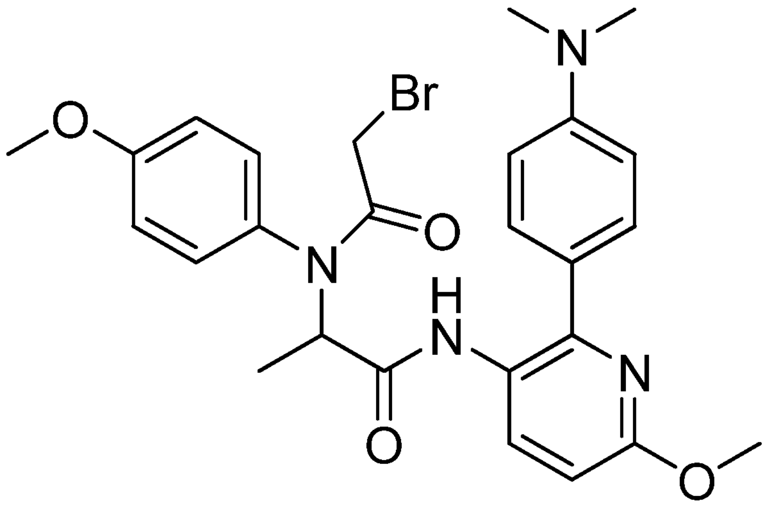

The title compound was obtained in the same way as in Reference Example 13 using 2-[N-(4-methoxyphenyl)amino]-N-{2-[4-(dimethylamino)phenyl]-6-methoxypyridin-3-yl}propanamide (300 mg, 0.71 mmol) synthesized in Reference Example 17 and bromoacetyl bromide (0.246 mL, 2.84 mmol). Yield: 65%.
$^1$H-NMR ($CDCl_3$): δ 1.15 (3H, d, J = 7.2 Hz), 3.00 (6H, s), 3.57 (2H, d, J = 4.5 Hz), 3.82 (3H, s), 3.94 (3H, s), 5.14 (1H, q, J = 7.2 Hz), 6.66 (1H, d, J = 8.7 Hz), 6.81 (2H, d, J = 9.1 Hz), 6.86 (2H, d, J = 8.7 Hz), 7.04 (2H, d, J = 7.6 Hz), 7.54 (2H, d, J = 9.1 Hz), 8.08 (1H, s), 8.17 (1H, d, J = 8.7 Hz).

Reference Example 19

2,2'-[(2-bromo-6-methoxypyridin-3-yl)imino]diethanol

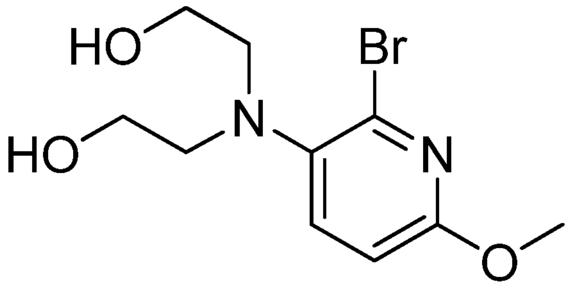

2-bromo-6-methoxypyridin-3-amine (5.00 g, 30.0 mmol) synthesized in Reference Example 1 was dissolved in 2-chloroethanol (39.7 g, 493 mmol). To the solution, N-ethyldiisopropylamine (9.54 g, 7.38 mmol) and potassium iodide (4.08 g, 24.6 mmol) were added, and the mixture was stirred at 120°C for 13 hours. The reaction solution was cooled, then diluted with ethyl acetate, and washed with water. The organic phase was dried over magnesium sulfate, and the solvent was then distilled off under reduced pressure. The obtained residue was purified by silica gel column chromatography (hexane-ethyl acetate 50:50-20:80) to obtain 1.49 g of the title compound (yield: 21%).
$^1$H-NMR ($CDCl_3$): δ 2.53 (2H, brs), 3.13-3.25 (4H, m), 3.52-3.65 (4H, m), 3.93 (3H, s), 6.74 (1H, d, J = 8.7 Hz), 7.54 (1H, d, J = 8.7 Hz).

Reference Example 20

2-bromo-N,N-bis(2-bromoethyl)-6-methoxypyridin-3-amine

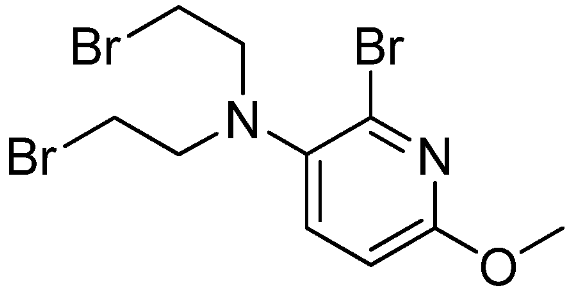

To an acetonitrile (40 mL) solution of 2,2'-[(2-bromo-6-methoxypyridin-3-yl)imino]diethanol (1.49 g, 5.14 mmol) synthesized in Reference Example 19, triphenylphosphine (5.06 g, 19.3 mmol) and tetrabromomethane (5.63 g, 17.0 mmol) were added, and the mixture was stirred at room temperature for 12 hours.
The reaction solution was cooled, then diluted with ethyl acetate, and washed with water. The organic phase was dried over magnesium sulfate, and the solvent was then distilled off under reduced pressure. The obtained residue was purified by silica gel column chromatography (hexane-ethyl acetate 100:0-50:50) to obtain 1.73 g of the title compound (yield: 81%).
$^1$H-NMR ($CDCl_3$): δ 3.25-3.39 (4H, m), 3.43-3.63 (4H, m), 3.92 (3H, s), 6.69 (1H, d, J = 8.3 Hz), 7.51 (1H, d, J = 8.3 Hz).

Reference Example 21

1-(2-bromo-6-methoxypyridin-3-yl)-4-(4-methoxyphenyl)piperazine

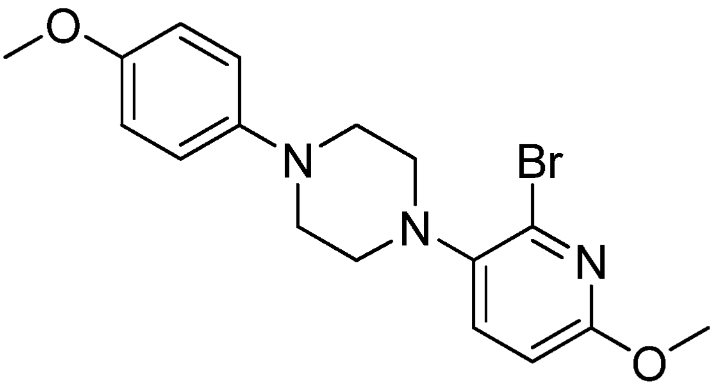

To a DMF (25 mL) solution of 2-bromo-N,N-bis(2-bromoethyl)-6-methoxypyridin-3-amine (1.73 g, 4.15 mmol) synthesized in Reference Example 20, 4-methoxyaniline (613 mg, 4.98 mmol) and sodium bicarbonate (767 mg, 9.13 mmol) were added, and the mixture was stirred at 100°C for 12 hours. After cooling to room temperature, water was added to the mixed solution, followed by extraction with ethyl acetate. The organic phase was dried over anhydrous magnesium sulfate. Then, the solvent was distilled off under reduced pressure, and the obtained residue was purified by silica gel column chromatography (hexane-ethyl acetate 100:0-93:17) to obtain 852 mg of the title compound (yield: 54%).
$^1$H-NMR ($CDCl_3$) : δ 3.07-3.16 (4H, m), 3.22-3.29 (4H, m), 3.78 (3H, s), 3.92 (3H, s), 6.70 (1H, d, J = 8.7 Hz), 6.86 (2H, d, J = 9.0 Hz), 6.96 (2H, d, J = 9.0 Hz), 7.38 (1H, d, J = 8.7 Hz).

Reference Example 22

1-(2-bromopyridin-3-yl)-4-(4-methoxyphenyl)piperazine

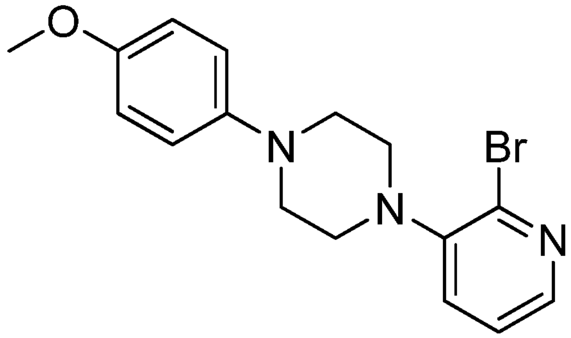

2-bromopyridin-3-amine (2.00 g, 11.6 mmol) was dissolved in 1-methylpyrrolidin-2-one (20 mL). To the solution, N,N-bis(2-chloroethyl)-4-methoxyaniline (5.74 g, 23.1 mmol), potassium carbonate (2.40 g, 17.4 mmol), sodium iodide (1.74 g, 11.6 mmol), and water (2 mL) were added, and the mixture was stirred at 90°C for 48 hours. The reaction solution was cooled, then diluted with ethyl acetate, and washed with water. The organic phase was dried over magnesium sulfate, and the solvent was then distilled off under reduced pressure. The obtained residue was purified by silica gel column chromatography (hexane-ethyl acetate 100:0-20:80) to obtain 273 mg of the title compound (yield: 7%).
$^1$H-NMR ($CDCl_3$): δ 3.18-3.33 (8H, m), 3.79 (3H, s), 6.81-6.91 (2H, m), 6.92-7.02 (2H, m), 7.19-7.26 (1H, m), 7.32-7.39 (1H, m), 8.08 (1H, dd, J = 4.5, 1.9 Hz).

Reference Example 23

2-(4-methylphenyl)pyridin-3-amine

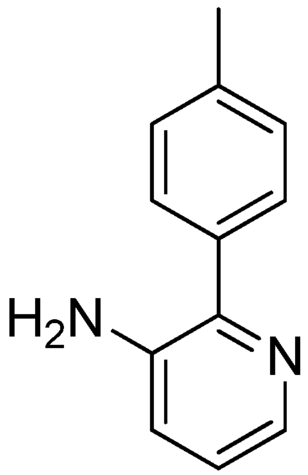

The title compound was obtained in the same way as in Reference Example 2 using 2-bromopyridin-3-amine (15.0 g, 87.0 mmol) and (4-methylphenyl)boronic acid (14.7 g, 108 mmol). Yield: 97%.
$^1$H-NMR ($CDCl_3$) : δ 2.40 (3H, s), 3.84 (2H, brs), 7.01-7.04 (2H, m), 7.26-7.28 (2H, m), 7.56 (2H, dd, J = 6.2, 1.8 Hz), 8.11 (1H, dd, J = 3.8, 2.2 Hz).

Reference Example 24

3-bromo-2-(4-methylphenyl)pyridine

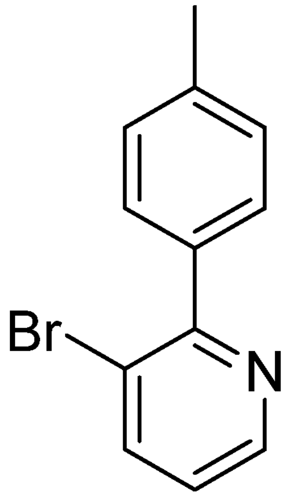

The title compound was obtained in the same way as in Reference Example 3 using 2-(4-methylphenyl)pyridin-3-amine (15.5 g, 84.0 mmol) synthesized in Reference Example 23. Yield: 86%.
$^1$H-NMR ($CDCl_3$) : δ 2.42 (3H, s), 7.56 (1H, dd, J = 8.0, 4.4 Hz), 7.26-7.28 (2H, m), 7.58 (2H, dd, J = 6.2, 1.8 Hz), 7.98 (1H, dd, J = 8.0, 1.6 Hz), 8.61 (1H, dd, J = 4.4, 1.6 Hz).

Reference Example 25

2-(4-methoxyphenyl)pyridin-3-amine

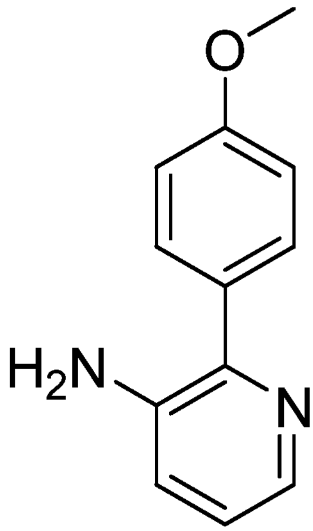

The title compound was obtained in the same way as in Reference Example 2 using 2-bromopyridin-3-amine (10.0 g, 57.8 mmol) and (4-methoxyphenyl)boronic acid (10.9 g, 72.2 mmol). Yield: 98%.
$^1$H-NMR ($CDCl_3$): δ 3.82 (2H, brs), 3.86 (3H, s), 7.01 (2H, dd, J = 6.6, 2.2 Hz), 7.04 (2H, d, J = 3.2 Hz), 7.22 (2H, dd, J = 6.6, 2.2 Hz), 8.12 (1H, t, J = 3.2 Hz).

Reference Example 26

3-bromo-2-(4-methoxyphenyl)pyridine

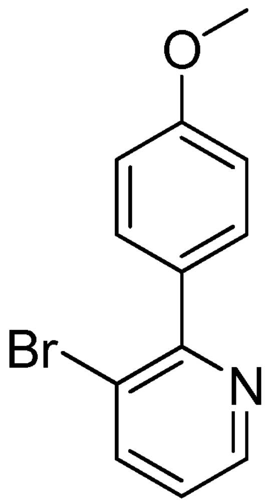

The title compound was obtained in the same way as in Reference Example 3 using 2-(4-methoxyphenyl)pyridin-3-amine (12.0 g, 59.9 mmol) synthesized in Reference Example 25. Yield: 42%.
$^1$H-NMR ($CDCl_3$): δ 3.86 (3H, s), 6.98 (2H, dd, J = 7.6 Hz, 2.4 Hz), 7.09 (1H, dd, J = 8.0 Hz, 4.4 Hz), 7.67 (2H, dd, J = 10.2, 3.4 Hz), 7.92-8.00 (1H, m), 8.57-8.63 (1H, m).

Reference Example 27

2-[4-(dimethylamino)phenyl]pyridin-3-amine

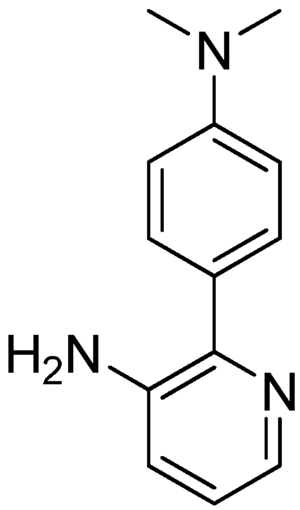

The title compound was obtained in the same way as in Reference Example 2 using 2-bromopyridin-3-amine (15.0 g, 87.0 mmol) and [4-(dimethylamino)phenyl]boronic acid (17.8 g, 108 mmol). Yield: 95%.
$^1$H-NMR ($CDCl_3$): δ 3.00 (6H, s), 3.84 (2H, brs), 6.81 (2H, dd, J = 2.0, 6.8 Hz), 7.01 (2H, d, J = 2.0 Hz), 7.59 (2H, dd, J = 2.0, 6.8 Hz), 8.11 (1H, dd, J = 1.8, 4.2 Hz).

Reference Example 28

4-(3-bromopyridin-2-yl)-N,N-dimethylaniline

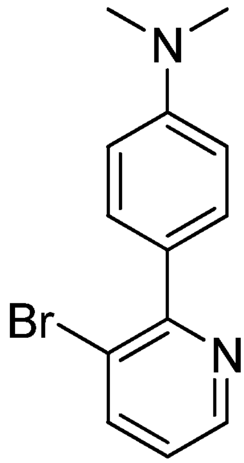

The title compound was obtained in the same way as in Reference Example 3 using 2-[4-(dimethylamino)phenyl]pyridin-3-amine (10.0 g, 46.9 mmol) synthesized in Reference Example 27. Yield: 97%.
$^1$H-NMR ($CDCl_3$): δ 3.02 (6H, s), 6.77 (2H, dd, J = 7.0, 2.2 Hz), 7.03 (1H, dd, J = 8.0, 4.8 Hz), 7.62-7.72 (2H, m), 7.94 (1H, dd, J = 8.0, 1.2 Hz), 8.57 (1H, dd, J = 4.8, 1.6 Hz).

Reference Example 29

4-benzyl-2-(4-methoxybenzyl)-1-methylpiperazine

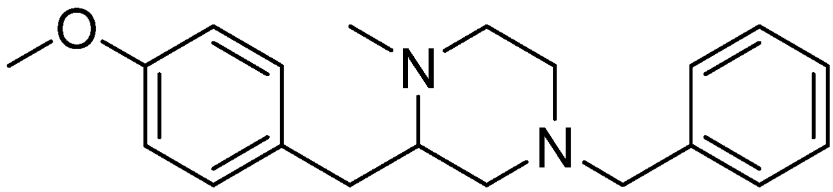

To a DMF (100 mL) solution of N-(tert-butoxycarbonyl)-O-methyltyrosine (5.00 g, 17.9 mmol) and ethyl N-benzylglycinate (3.53 g, 18.3 mmol), 1-ethyl-3-(3-dimethylaminopropyl)carbodiimide hydrochloride (4.11 g, 21.5 mmol) and 1-hydroxybenzotriazole (2.66 g, 19.7 mmol) were added, and the mixture was stirred at room temperature for 20 hours. The reaction solution was added to water, followed by extraction with ethyl acetate. The organic phase was washed with water, a 10% aqueous citric acid solution, a saturated aqueous solution of sodium bicarbonate, and saturated brine and then dried over anhydrous magnesium sulfate to obtain 8.42 g of ethyl N-(tert-butoxycarbonyl)-O-methyltyrosyl-N-benzylglycinate (yield: 100%).
$^1$H-NMR ($CDCl_3$): δ 1.14-1.46 (12H, m), 2.85-3.15 (2H, m), 3.69-3.85 (5H, m), 4.04-4.27 (2H, m), 4.42-4.78 (2H, m), 4.83-5.35 (2H, m), 6.67-7.40 (9H, m).
To a toluene (10 mL) solution of the obtained ethyl N-(tert-butoxycarbonyl)-O-methyltyrosyl-N-benzylglycinate (8.42 g, 17.9 mmol), trifluoroacetic acid (30 mL) was added, and the mixture was stirred at room temperature for 3.5 hours. The solvent was distilled off under reduced pressure, and toluene was added to the obtained residue. The solvent was distilled off again under reduced pressure. To a toluene (30 mL) solution of the obtained oil, triethylamine (10 mL) was added, and the mixture was stirred at room temperature for 3.5 hours. The solvent was distilled off under reduced pressure, and the residue was dissolved in an ethyl acetate-THF (2:1) mixed solution. The organic phase was washed with water, a 10% aqueous citric acid solution, a saturated aqueous solution of sodium bicarbonate, and saturated brine and then dried over anhydrous magnesium sulfate to obtain 4.56 g of 1-benzyl-3-(4-methoxybenzyl)piperazine-2,5-dione (yield: 78%).
$^1$H-NMR ($CDCl_3$): δ 2.86-3.24 (3H, m), 3.42-3.58 (1H, m), 3.75 (3H, s), 4.23-4.62 (4H, m), 6.66-7.37 (9H, m).
To a THF (150 mL) suspension of lithium aluminum hydride (2.14 g, 56.4 mmol), a THF (50 mL) solution of 1-benzyl-3-(4-methoxybenzyl)piperazine-2,5-dione (4.56 g, 14.1 mmol) was added dropwise at 0°C, and the mixture was then stirred at room temperature for 2 hours and at 60°C for 12 hours. The reaction solution was cooled to -78°C. An ethanol-ethyl acetate mixed solution (1:1) and a 1 N aqueous sodium hydroxide solution were added dropwise thereto, and the

mixture was stirred at room temperature for 2 hours. The deposited solid was filtered off. The obtained solution was washed with saturated brine and then dried over anhydrous magnesium sulfate, and the solvent was distilled off under reduced pressure. The obtained residue was purified by basic silica gel column chromatography (hexane-ethyl acetate 90:10-50:50) to obtain 3.44 g of 1-benzyl-3-(4-methoxybenzyl)piperazine (yield: 82%).
$^1$H-NMR ($CDCl_3$): δ 1.87 (1H, t, J = 10.2 Hz), 2.07 (1H, td, J = 3.9, 11.4 Hz), 2.47 (1H, dd, J = 9.3, 13.5 Hz), 2.65 (1H, dd, J = 4.8, 13.5 Hz), 2.70-3.00 (5H, m), 3.46 (1H, d, J = 12.9 Hz), 3.54 (1H, d, J = 12.9 Hz), 3.79 (3H, s), 6.83 (2H, d, J = 8.4 Hz), 7.11 (2H, d, J = 8.4 Hz), 7.21-7.40 (5H, m).
To a THF (10 mL) solution of 1-benzyl-3-(4-methoxybenzyl)piperazine (1.00 g, 3.37 mmol) and a 38% aqueous formalin solution (0.400 mL, 5.06 mmol), sodium triacetoxyborohydride (1.43 g, 6.74 mmol) was added, and the mixture was stirred at room temperature for 12 hours. To the reaction solution, ethyl acetate and water were added to separate an organic phase. The organic phase was dried over anhydrous magnesium sulfate. The solvent was distilled off under reduced pressure, and the obtained residue was purified by silica gel column chromatography (hexane-ethyl acetate 100:0-70:30) to obtain 1.01 g of the title compound (yield: 96%).
$^1$H-NMR ($CDCl_3$): δ 1.95-2.09 (1H, m), 2.13-2.29 (1H, m), 2.30-2.65 (8H, m), 2.70-2.83 (1H, m), 2.95-3.08 (1H, m), 3.26 (1H, d, J = 13.1 Hz), 3.54 (1H, d, J = 13.1 Hz), 3.78 (3H, s), 6.79 (2H, d, J = 8.7 Hz), 7.04 (2H, d, J = 8.7 Hz), 7.17-7.40 (5H, m).

Reference Example 30

2-(4-methoxybenzyl)-1-methylpiperazine

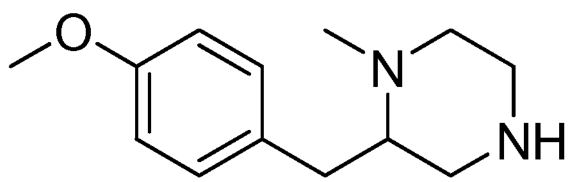

To an ethyl acetate (80 mL) solution of 4-benzyl-2-(4-methoxybenzyl)-1-methylpiperazine (1.01 g, 3.25 mmol) synthesized in Reference Example 29, 10% palladium carbon (50% hydrated, 500 mg) was added, and the mixture was stirred at room temperature for 24 hours in a hydrogen atmosphere. Palladium carbon was filtered off, and the solvent was then distilled off under reduced pressure to obtain 631 mg of the title compound (yield: 88%).
$^1$H-NMR ($CDCl_3$): δ 2.11-2.29 (2H, m), 2.31-2.50 (5H, m), 2.69-2.84 (2H, m), 2.84-2.94 (2H, m), 3.06-3.16 (1H, m), 3.79 (3H, s), 6.82 (2H, d, J = 8.7 Hz), 7.07 (2H, d, J = 8.7 Hz).

Reference Example 31

1-(2-chloro-5-methoxypyridin-3-yl)-4-(4-methylphenyl)piperazine

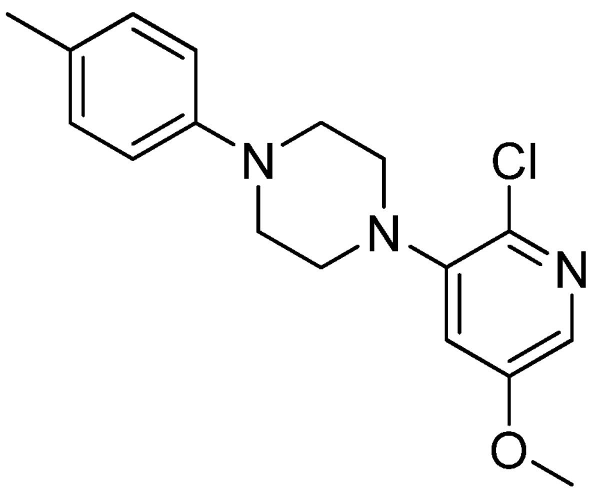

A mixture of 3-bromo-2-chloro-5-methoxypyridine (200 mg, 0.899 mmol), 1-(4-methylphenyl)piperazine (190 mg, 1.08 mmol), palladium acetate (10.1 mg, 0.0450 mmol), BINAP (84.0 mg, 0.135 mmol), sodium tert-butoxide (259 mg, 2.70 mmol), and toluene (6.0 mL) was stirred at 100°C for 4 hours in an argon atmosphere. After cooling to room temperature, the reaction solution was partitioned with water and ethyl acetate, and the organic phase was dried over anhydrous magnesium sulfate. The solvent was distilled off under reduced pressure, and the obtained residue was purified by silica gel column chromatography (hexane-ethyl acetate 100:0-85:15) to obtain 192 mg of the title compound (yield: 75%).
$^1$H-NMR ($CDCl_3$): δ 2.29 (3H, s), 3.18-3.36 (8H, m), 3.87 (3H, s), 6.87-6.95 (3H, m), 7.06-7.14 (2H, m), 7.76 (1H, d, J = 3.0 Hz).

Reference Example 32

1-(2-bromo-6-methoxypyridin-3-yl)piperidin-4-one

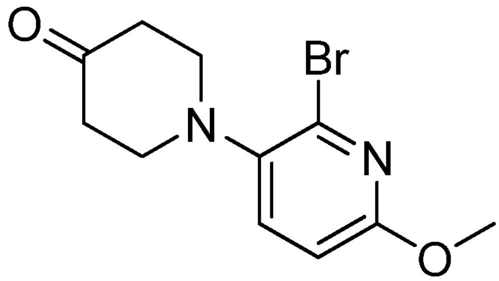

To a mixture of 2-bromo-6-methoxypyridin-3-amine (6.60 g, 32.5 mmol) synthesized in Reference Example 1, potassium carbonate (89.8 mg, 0.65 mmol), ethanol (60 mL), and water (30 mL), 1-benzyl-1-methyl-4-oxopiperidinium iodide (43.1 g, 130 mmol) was added, and the mixture was refluxed for 7 hours. After standing to cool to room temperature, the reaction solution was concentrated under reduced pressure, and water was added to the obtained residue, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 100:0-80:20) to obtain 6.07 g of the title compound (yield: 66%).
$^1$H-NMR ($CDCl_3$) :δ 2.65 (4H, t, J = 6.0 Hz), 3.26 (4H, t, J = 6.0 Hz), 3.92 (3H, s), 6.69 (1H, d, J = 8.7 Hz), 7.34 (1H, d, J = 8.3 Hz).

Reference Example 33

1-(2-bromo-6-methoxypyridin-3-yl)-N-hydroxypiperidin-4-imine

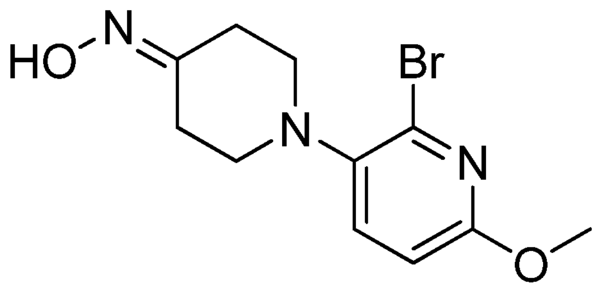

To a methanol (80 mL)-water (8 mL) mixed solution of 1-(2-bromo-6-methoxypyridin-3-yl)piperidin-4-one (6.07 g, 21.3 mmol) synthesized in Reference Example 32, potassium carbonate (4.42 g, 32.0 mmol) and hydroxyl ammonium chloride (5.92 g, 85.2 mmol) were added, and the mixture was refluxed for 2 hours. After standing to cool to room temperature, the reaction solution was poured into water, and the precipitate was collected by filtration to obtain 5.30 g of the title compound (yield: 83%).
$^1$H-NMR ($CDCl_3$): δ 2.49-2.57 (2H, m), 2.78-2.87 (2H, m), 3.02 (2H, t, J = 6.0 Hz), 3.07 (2H, t, J = 5.8 Hz), 3.91 (3H, s),

6.67 (1H, d, J = 8.3 Hz), 7.06 (1H, s), 7.30 (1H, d, J = 8.7 Hz).

Reference Example 34

1-(2-bromo-6-methoxypyridin-3-yl)-1,4-diazepan-5-one

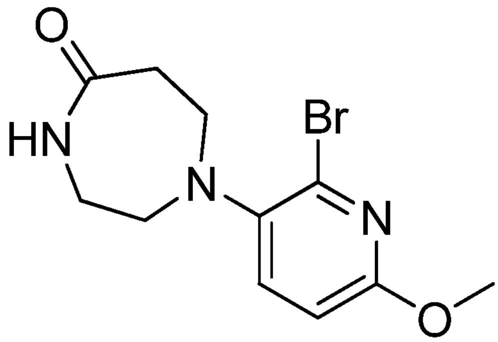

To an acetone (80 mL) solution of 1-(2-bromo-6-methoxypyridin-3-yl)-N-hydroxypiperidin-4-imine (5.30 g, 17.7 mmol) synthesized in Reference Example 33, an aqueous (50 mL) solution of sodium carbonate (5.63 g, 53.1 mmol) was added, and the mixture was stirred at room temperature for 5 minutes. To the reaction solution, an acetone (20 mL) solution of p-toluenesulfonyl chloride (5.07 g, 26.6 mmol) was added dropwise, and the mixture was then stirred at room temperature for 2 hours and at 50°C for 3 hours. After standing to cool to room temperature, acetone was distilled off under reduced pressure, followed by extraction with ethyl acetate. The extracts were mixed and dried over anhydrous magnesium sulfate. The solvent was distilled off under reduced pressure, and the obtained residue was subjected to collection by filtration with diethyl ether to obtain 4.90 g of the title compound (yield: 92%).
$^1$H-NMR ($CDCl_3$): δ 2.77-2.87 (2H, m), 3.02-3.13 (4H, m), 3.48 (2H, dd, J = 5.9, 8.9 Hz), 3.91 (3H, s), 6.27 (1H, brs), 6.67 (1H, d, J = 8.3 Hz), 7.31 (1H, d, J = 8.7 Hz).

Reference Example 35

1-{2-[4-(dimethylamino)phenyl]-6-methoxypyridin-3-yl}-1,4-diazepan-5-one

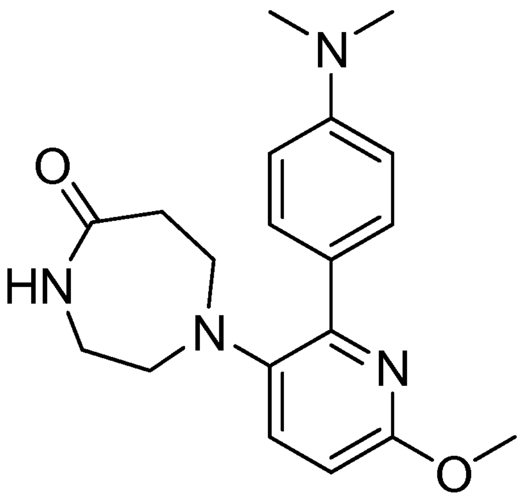

To a mixture of 1-(2-bromo-6-methoxypyridin-3-yl)-1,4-diazepan-5-one (1.00 g, 3.33 mmol) synthesized in Reference Example 34, [4-(dimethylamino)phenyl]boronic acid (660 mg, 4.00 mmol), sodium carbonate (706 mg, 6.66 mmol), toluene (10 mL), ethanol (5 mL), and water (3 mL), tetrakis(triphenylphosphine)palladium (0) (197 mg, 0.17 mmol) was added, and the mixture was stirred at 105°C for 4 hours in a nitrogen atmosphere. After standing to cool to room

temperature, saturated brine was added to the reaction solution, followed by extraction with ethyl acetate. The extracts were mixed and dried over anhydrous magnesium sulfate. The solvent was distilled off under reduced pressure, and the obtained residue was crystallized from THF-hexane to obtain 1.09 g of the title compound (yield: 92%).
$^1$H-NMR ($CDCl_3$): δ 2.64-2.73 (2H, m), 2.94-3.00 (2H, m), 3.02 (6H, s), 3.03-3.09 (2H, m), 3.26-3.35 (2H, m), 3.95 (3H, s), 5.83 (1H, brs), 6.57 (1H, d, J = 8.7 Hz), 6.76 (2H, d, J = 9.0 Hz), 7.35 (1H, d, J = 8.7 Hz), 8.00-8.11 (2H, m).

Reference Example 36

1-{6-methoxy-2-[4-(1-methylethyl)phenyl]pyridin-3-yl}-1,4-diazepan-5-one

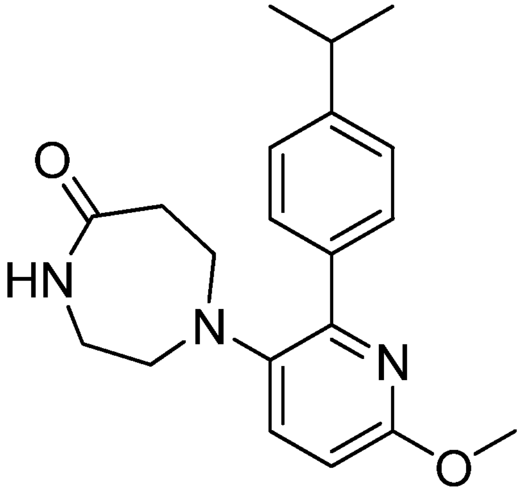

The title compound was obtained in the same way as in Reference Example 35 using 1-(2-bromo-6-methoxypyridin-3-yl)-1,4-diazepan-5-one (1.00 g, 3.33 mmol) synthesized in Reference Example 34 and [4-(1-methylethyl)phenyl]boronic acid (656 mg, 4.00 mmol). Yield: 65%.
$^1$H-NMR ($CDCl_3$): δ 1.29 (6H, d, J = 6.8 Hz), 2.56-2.67 (2H, m), 2.91-2.98 (3H, m), 2.99-3.06 (2H, m), 3.25 (2H, dd, J = 9.1, 5.7 Hz), 3.94 (3H, s), 5.87 (1H, brs), 6.65 (1H, d, J = 8.3 Hz), 7.28 (2H, d, J = 8.3 Hz), 7.38 (1H, d, J = 8.3 Hz), 7.93 (2H, d, J = 8.3 Hz).

Reference Example 37

1-[2-(4-ethoxyphenyl)-6-methoxypyridin-3-yl]-1,4-diazepan-5-one

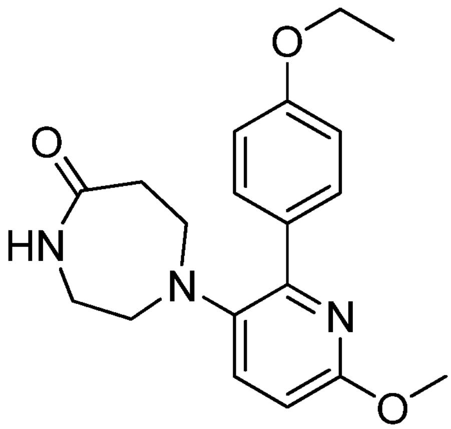

The title compound was obtained in the same way as in Reference Example 35 using 1-(2-bromo-6-methoxypyridin-3-yl)-1,4-diazepan-5-one (5.00 g, 16.7 mmol) synthesized in Reference Example 34 and (4-ethoxyphenyl)boronic acid (3.32 g, 20.0 mmol). Yield: 77%.
$^1$H-NMR ($CDCl_3$): δ 1.45 (3H, t, J = 7.0 Hz), 2.57-2.72 (2H, m), 2.90-3.00 (2H, m), 3.00-3.10 (2H, m), 3.22-3.36 (2H, m), 3.94 (3H, s), 4.09 (2H, q, J = 7.2 Hz), 5.99 (1H, brs), 6.62 (1H, d, J = 8.7 Hz), 6.94 (2H, d, J = 8.7 Hz), 7.37 (1H, d, J = 8.7 Hz), 8.00 (2H, d, J = 8.7 Hz).

Reference Example 38

4-[3-(1,4-diazepan-1-yl)-6-methoxypyridin-2-yl]-N,N-dimethylaniline

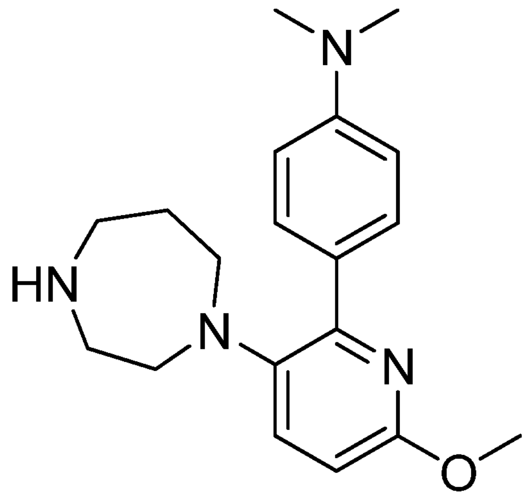

To a THF (40 mL) suspension of lithium aluminum hydride (448 mg, 11.8 mmol), 1-{2-[4-(dimethylamino)phenyl]-6-methoxypyridin-3-yl}-1,4-diazepan-5-one (2.00 g, 5.88 mmol) synthesized in Reference Example 35 was carefully added under ice cooling, and the mixture was then refluxed for 2 hours. After cooling to room temperature, water (0.45 mL), a 15% aqueous sodium hydroxide solution (0.45 mL), and water (1.35 mL) were added dropwise in this order to the reaction solution, and the obtained suspension was filtered through celite. The filtrate was concentrated under reduced pressure, and the obtained residue was purified by basic silica gel column chromatography (hexane-ethyl acetate 60:40-0:100) to obtain 1.89 g of the title compound (yield: 98%) as an oil.
$^1$H-NMR ($CDCl_3$): δ 1. 73 (2H, t, J = 5.9 Hz), 2.81-2.88 (2H, m), 2.93-2.99 (2H, m), 3.00 (6H, s), 3.01-3.12 (4H, m), 3.93 (3H, s), 6.55 (1H, d, J = 8.7 Hz), 6.76 (2H, d, J = 9.1 Hz), 7.43 (1H, d, J = 8.7 Hz), 7.94-8.04 (2H, m).

Reference Example 39

1-{6-methoxy-2-[4-(1-methylethyl)phenyl]pyridin-3-yl}-1,4-diazepane

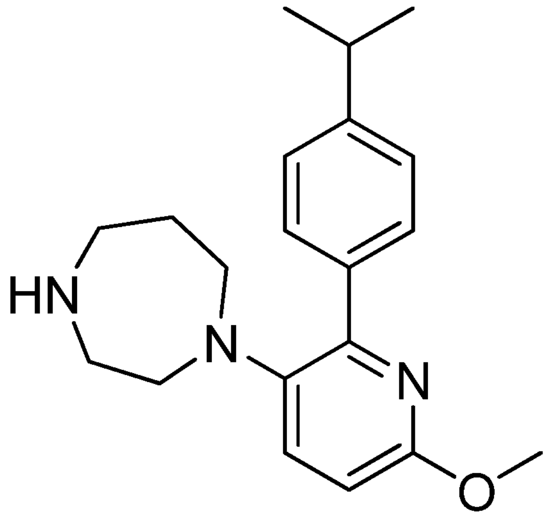

The title compound was obtained in the same way as in Reference Example 38 using 1-{6-methoxy-2-[4-(1-methylethyl) phenyl]pyridin-3-yl}-1,4-diazepan-5-one (0.74 g, 2.18 mmol) synthesized in Reference Example 36.
Yield: 85%.
$^1$H-NMR ($CDCl_3$): δ 1.28 (6H, d, J = 6.8 Hz), 1.60-1.69 (3H, m), 2.71-2.81 (2H, m), 2.88-2.98 (3H, m), 2.98-3.12 (4H, m), 3.93 (3H, s), 6.62 (1H, d, J = 8.7 Hz), 7.23-7.30 (2H, m), 7.43-7.48 (1H, m), 7.87 (2H, d, J = 8.3 Hz).

Reference Example 40

6-(3,5-dimethoxyphenyl)morpholin-3-one

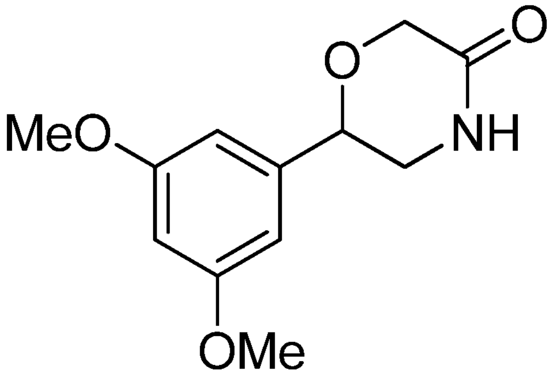

To a THF (30 mL) solution of 2-amino-1-(3,5-dimethoxyphenyl)ethanol (1.50 g, 7.60 mmol), triethylamine (1.15 g, 11.4 mmol) and chloroacetyl chloride (901 mg, 7.98 mmol) were added in this order under ice cooling, and the mixture was stirred at 0°C for 30 minutes. The reaction solution was partitioned with ethyl acetate and water, and the organic phase was washed with water and saturated brine and then dried over anhydrous magnesium sulfate. The solvent was distilled off under reduced pressure, and the obtained residue was dissolved in tert-butanol (30 mL). To the solution, potassium tert-butoxide (1.02 g, 9.12 mmol) was added, and the mixture was stirred at room temperature for 15 hours. To the reaction solution, a saturated aqueous solution of ammonium chloride was added, followed by extraction with ethyl acetate. The extracts were washed with saturated brine and dried over anhydrous magnesium sulfate, and the solvent was then distilled off under reduced pressure. The obtained residue was purified by silica gel column chromatography (hexane-ethyl acetate 3:2-1:9) to obtain 1.01 g of the title compound (yield: 56%).
$^1$H-NMR ($CDCl_3$): δ 3.43-3.60 (2H, m), 3.80 (6H, s), 4.33 (1H, d, J = 16.8 Hz), 4.45 (1H, d, J = 16.8 Hz), 4.70 (1H, d, J = 4.5, 9.6 Hz), 6.25 (1H, brs), 6.43 (1H, t, J = 2.4 Hz), 6.53 (1H, d, J = 2.4 Hz).

Reference Example 41

2-(3,5-dimethoxyphenyl)morpholine

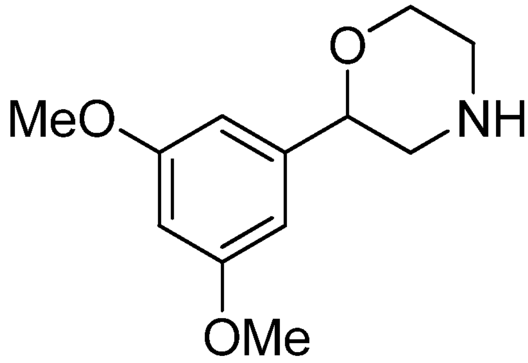

To a toluene (18 mL) solution of 6-(3,5-dimethoxyphenyl)morpholin-3-one (1.01 g, 4.26 mmol) synthesized in Reference Example 40, a 70% solution of sodium bis(2-methoxyethoxy)aluminum hydride in toluene (3.70 g, 12.8 mmol) was added, and the mixture was stirred at room temperature for 15 hours. The reaction solution was cooled to 0°C, and the reaction was stopped by addition of a 1 N aqueous sodium hydroxide solution, followed by a total of two extractions with isopropyl ether and with a THF-ethyl acetate (1:2) mixed solvent. The extracts were mixed, washed with saturated brine, and then dried over anhydrous magnesium sulfate. The solvent was distilled off under reduced pressure, and the obtained residue was purified by silica gel column chromatography (hexane-ethyl acetate 7:3-1:4) to obtain 550 mg of the title compound (yield: 58%).
$^1$H-NMR ($CDCl_3$): δ 1.79 (1H, brs), 2.77 (1H, d, J = 10.2, 12.3 Hz), 2.83-2.92 (1H, m), 2.92-3.01 (1H, m), 3.05 (1H, dd, J = 2.7, 12.3 Hz), 3.69-3.83 (7H, m), 3.98-4.07 (1H, m), 4.42 (1H, dd, J = 2.7, 10.2 Hz), 6.38 (1H, t, J = 2.1 Hz), 6.51 (2H, d, J = 2.1 Hz).

Reference Example 42

2-amino-1-(2-methyl-1,3-thiazol-4-yl)ethanol

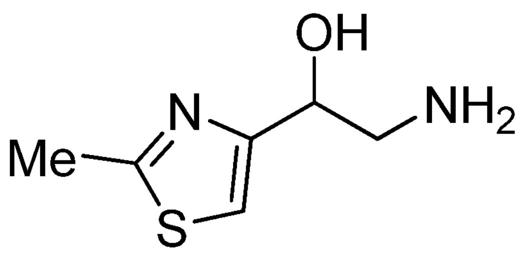

To a toluene (30 mL) solution of 2-methyl-1,3-thiazole-4-carbaldehyde (3.19 g, 25.1 mmol) and trimethylsilyl cyanide (2.74 g, 27.6 mmol), zinc iodide (401 mg, 1.26 mmol) was added, and the mixture was stirred at room temperature for 1 hour. Then, the reaction solution was concentrated under reduced pressure, and the residue was dissolved in THF (30 mL). This solution was cooled to 0°C. Lithium aluminum lithium hydride (1.91 g, 50.2 mmol) was added thereto, and the mixture was heated to room temperature and stirred for 15 hours. The reaction solution was cooled to 0°C, and the reaction was stopped by addition of water (2 g), a 1 N aqueous sodium hydroxide solution (2 g), and water (6 g) in this order. Then, the mixed solution was filtered through celite. The filtrate was dried over anhydrous magnesium sulfate, and the solvent was distilled off under reduced pressure to obtain 1.67 g of the title compound (yield: 42%).
$^1$H-NMR ($CDCl_3$): δ 2.43 (3H, d, J = 1.2 Hz), 3.11 (1H, dd, J = 5.1, 12.9 Hz), 3.19 (1H, d, J = 4.5, 12.9 Hz), 4.88 (1H, dd, J = 4.5, 5.1 Hz), 6.81-6.86 (1H, m).

Reference Example 43

6-(2-methyl-1,3-thiazol-4-yl)morpholin-3-one

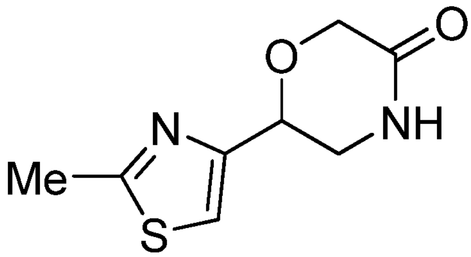

The title compound was obtained in the same way as in Reference Example 40 using 2-amino-1-(2-methyl-1,3-thiazol-4-yl)ethanol (1.67 g, 10.6 mmol) synthesized in Reference Example 42. Yield: 13%.
$^1$H-NMR ($CDCl_3$): δ 2.44 (3H, d, J = 0.6 Hz), 3.69 (1H, dd, J = 9.9, 12.3 Hz), 3.79-3.90 (1H, m), 4.39 (1H, d, J = 16.8 Hz), 5.05 (1H, dd, J = 3.3, 9.9 Hz), 6.51 (1H, brs), 6.90-6.95 (1H, m).

Reference Example 44

2-(2-methyl-1,3-thiazol-4-yl)morpholine

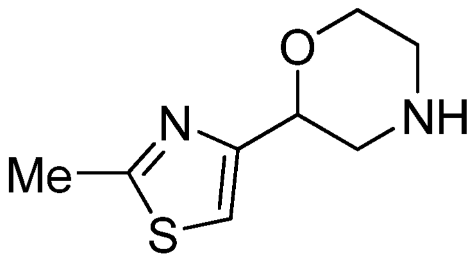

The title compound was obtained in the same way as in Reference Example 41 using 6-(2-methyl-1,3-thiazol-4-yl) morpholin-3-one (280 mg, 1.41 mmol) synthesized in Reference Example 43. Yield: 23%.
$^1$H-NMR ($CDCl_3$): δ 2.43 (3H, s), 2.85-3.05 (3H, m), 3.38 (1H, dd, J = 2.7, 12.3 Hz), 3.72-3.84 (1H, m), 3.98-4.08 (1H, m), 4.77 (1H, dd, J = 2.7, 9.9 Hz), 6.82-6.88 (1H, m).

Reference Example 45

Tert-butyl 3-(4-methoxyphenyl)piperazine-1-carboxylate

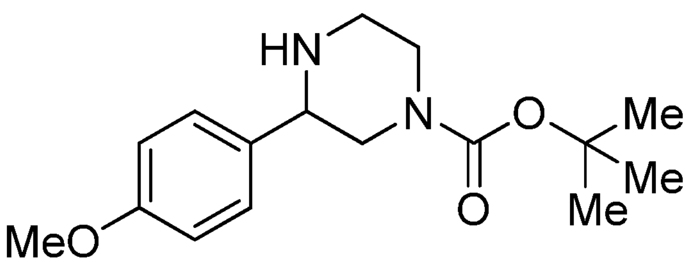

To a THF (30 mL)-toluene (180 mL) solution of 2-(4-methoxyphenyl)piperazine (17.0 g, 88.0 mmol), di-tert-butyl dicarbonate (6.91 g, 31.6 mmol) was added, and the mixture was stirred at room temperature for 2 hours and then further stirred at 80°C for 2 hours. After cooling, the reaction solution was concentrated under reduced pressure, and the residue was purified by silica gel column chromatography (hexane-ethyl acetate 6:1) to obtain 15.5 g of the title compound (yield: 60%).
$^1$H-NMR ($CDCl_3$): δ 1.46 (9H, s), 1.62 (1H, s), 2.72 (1H, brs), 2.84-2.89 (2H, m), 3.06 (1H, d, J = 8.4 Hz), 3.64 (1H, d, J = 8.4 Hz), 3.80 (3H, s), 4.04 (2H, brs), 6.87 (2H, d, J = 8.8 Hz), 7.31 (2H, d, J = 8.4 Hz).

Reference Example 46

4-tert-butyl 1-ethyl 2-(4-methoxyphenyl)piperazine-1,4-dicarboxylate

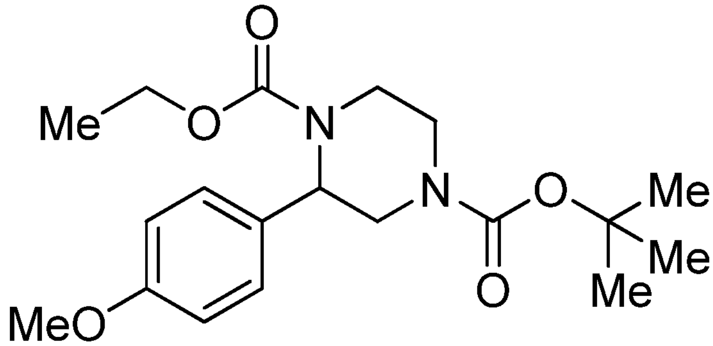

To a THF (10 mL) suspension of tert-butyl 3-(4-methoxyphenyl)piperazine-1-carboxylate (1.00 g, 3.42 mmol) synthesized in Reference Example 45 and potassium carbonate (945 mg, 6.84 mmol), ethyl chloroformate (445 mg, 4.10 mmol) was added, and the mixture was stirred at room temperature for 4 hours. The reaction solution was partitioned with ethyl acetate and water, and the organic phase was washed with saturated brine and then dried over anhydrous magnesium sulfate. The solvent was distilled off under reduced pressure, and the obtained residue was purified by silica gel column chromatography (hexane-ethyl acetate 92:8-75:25) to obtain 1.15 g of the title compound (yield: 92%).
$^1$H-NMR ($CDCl_3$): δ 1.27 (3H, t, J = 7.2 Hz), 1.44 (9H, s), 2.81-3.11 (2H, m), 3.21-3.34 (1H, m), 3.79 (3H, s), 3.91-4.04 (2H, m), 4.15-4.26 (2H, m), 4.42-4.55 (1H, m), 5.29 (1H, brs), 6.82-6.90 (2H, m), 7.19-7.29 (2H, m).

Reference Example 47

Ethyl 2-(4-methoxyphenyl)piperazine-1-carboxylate

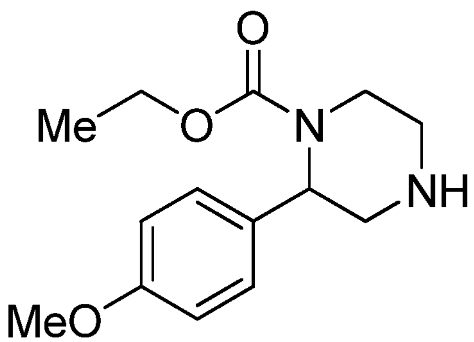

4-tert-butyl 1-ethyl 2-(4-methoxyphenyl)piperazine-1,4-dicarboxylate (1.15 g, 3.16 mmol) synthesized in Reference Example 46 and a 4 N hydrochloric acid-ethyl acetate solution (10 mL) were mixed and stirred at room temperature for 4 hours. The reaction solution was poured into a saturated aqueous solution of sodium bicarbonate, followed by extraction with ethyl acetate. The extracts were washed with saturated brine and dried over anhydrous magnesium sulfate, and

the solvent was then distilled off under reduced pressure to obtain 700 mg of the title compound (yield: 84%).
$^1$H-NMR ($CDCl_3$): δ 1.26 (3H, t, J = 7.2 Hz), 2.76-3.05 (3H, m), 3.14 (1H, dd, J = 4.2, 12.9 Hz), 3.47-3.58 (1H, m), 3.80 (3H, s), 3.92-4.02 (1H, m), 4.19 (2H, q, J = 7.2 Hz), 5.18-5.25 (1H, m), 6.85-6.94 (2H, m), 7.22-7.32 (2H, m).

Reference Example 48

1-{6-methoxy-2-[4-(trifluoromethoxy)phenyl]pyridin-3-yl}piperidin-4-one

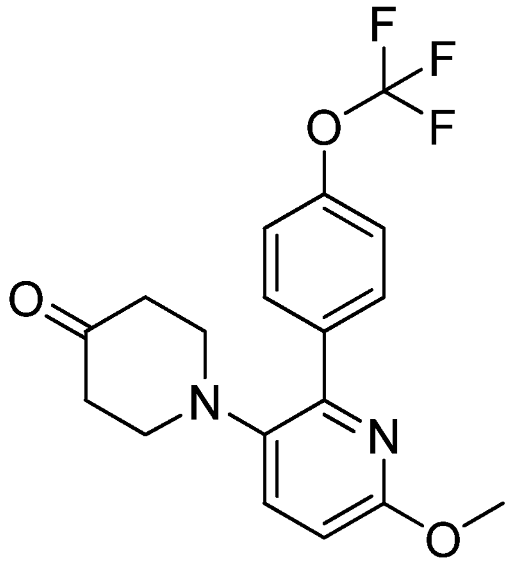

The title compound was obtained in the same way as in Reference Example 2 using 1-(2-bromo-6-methoxypyridin-3-yl)piperidin-4-one (3.00 g, 10.6 mmol) synthesized in Reference Example 32 and [4-(trifluoromethoxy)phenyl]boronic acid (3.26 g, 15.8 mmol). Yield: 98%.
$^1$H-NMR ($CDCl_3$): δ 2.40-2.55 (4H, m), 3.08-3.18 (4H, m), 3.96 (3H, s), 6.71 (1H, d, J = 8.7 Hz), 7.26-7.33 (2H, m), 7.44 (1H, d, J = 9.0 Hz), 8.17 (2H, d, J = 9.0 Hz).

Reference Example 49

1-{6-methoxy-2-[4-(1-methylethyl)phenyl]pyridin-3-yl}piperidin-4-one

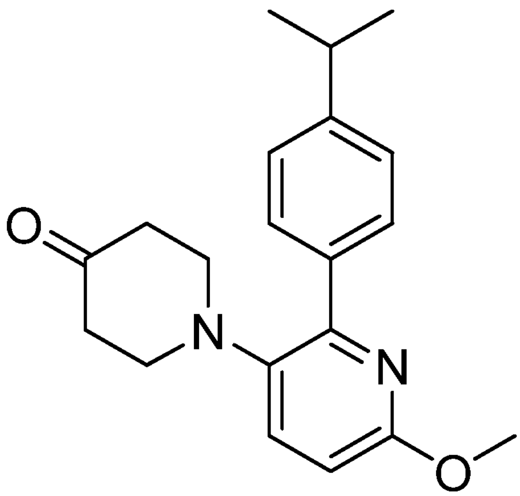

The title compound was obtained in the same way as in Reference Example 2 using 1-(2-bromo-6-methoxypyridin-3-yl)piperidin-4-one (1.10 g, 3.86 mmol) synthesized in Reference Example 32 and [4-(1-methylethyl)phenyl]boronic acid (950 mg, 5.79 mmol). Yield: 90%.
$^1$H-NMR ($CDCl_3$): δ 1.29 (6H, d, J = 6.8 Hz), 2.37-2.52 (4H, m), 2.85-3.05 (1H, m), 3.06-3.18 (4H, m), 3.96 (3H, s), 6.65 (1H, d, J = 8.3 Hz), 7.30 (2H, d, J = 8.3 Hz), 7.39 (1H, d, J = 8.7 Hz), 8.05 (2H, d, J = 8.3 Hz).

Reference Example 50

1-{2-[4-(dimethylamino)phenyl]-6-methoxypyridin-3-yl}-4-methyl-1,4-diazepan-5-one

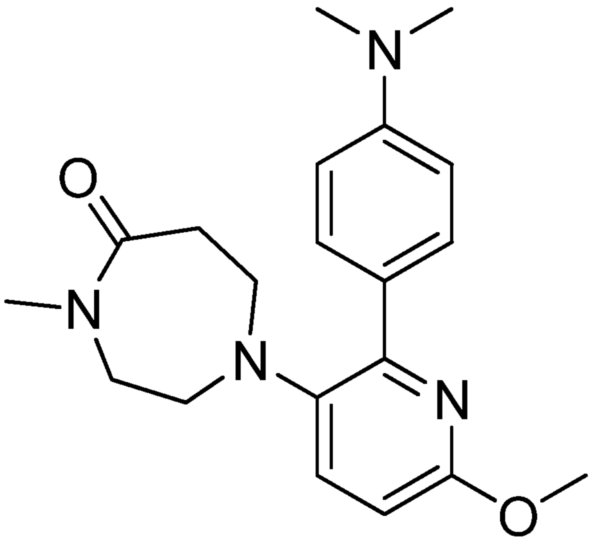

To a DMF (5 mL) suspension of 1-{2-[4-(dimethylamino)phenyl]-6-methoxypyridin-3-yl}-1,4-diazepan-5-one (500 mg, 1.47 mmol) synthesized in Reference Example 35, 60% mineral oil-mixed sodium hydride (118 mg, 2.94 mmol) was added, and the mixture was stirred for 30 minutes. To the reaction solution, iodomethane (0.183 mL, 2.94 mmol) was subsequently added dropwise, and the mixture was stirred for 1 hour. To the reaction solution, water was added, followed by extraction with ethyl acetate. The extracts were mixed, washed with water, and then dried over anhydrous magnesium sulfate. The solvent was distilled off under reduced pressure, and the obtained residue was purified by silica gel column chromatography (hexane-ethyl acetate 80:20-0:100) to obtain 438 mg of the title compound (yield: 84%).
$^1$H-NMR ($CDCl_3$): δ 2.70-2.77 (2H, m), 2.91-2.97 (2H, m), 2.99 (3H, s), 3.00-3.06 (8H, m), 3.40-3.49 (2H, m), 3.95 (3H, s), 6.56 (1H, d, J = 8.7 Hz), 6.75 (2H, d, J = 9.1 Hz), 7.33 (1H, d, J = 8.7 Hz), 8.06 (2H, d, J = 9.1 Hz).

Reference Example 51

1-[2-(4-ethoxyphenyl)-6-methoxypyridin-3-yl]-4-methyl-1,4-diazepan-5-one

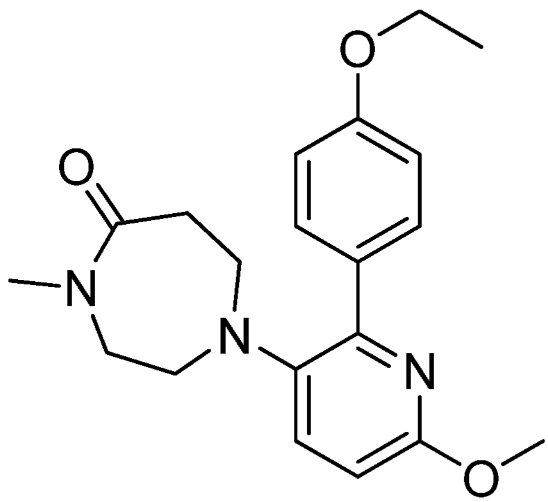

The title compound was obtained in the same way as in Reference Example 50 using 1-[2-(4-ethoxyphenyl)-6-methoxypyridin-3-yl]-1,4-diazepan-5-one (2.00 g, 5.86 mmol) synthesized in Reference Example 37. Yield: 88%.
$^1$H-NMR ($CDCl_3$): δ 1.45 (3H, t, J = 7.0 Hz), 2.65-2.75 (2H, m), 2.88-2.96 (2H, m), 2.96-3.05 (5H, m), 3.37-3.46 (2H, m), 3.94 (3H, s), 4.09 (2H, q, J = 7.2 Hz), 6.62 (1H, d, J = 8.3 Hz), 6.88-6.99 (2H, m), 7.36 (1H, d, J = 8.7 Hz), 7.96-8.06 (2H, m).

Reference Example 52

1-{2-[4-(dimethylamino)phenyl]-6-methoxypyridin-3-yl}-4-ethyl-1,4-diazepan-5-one

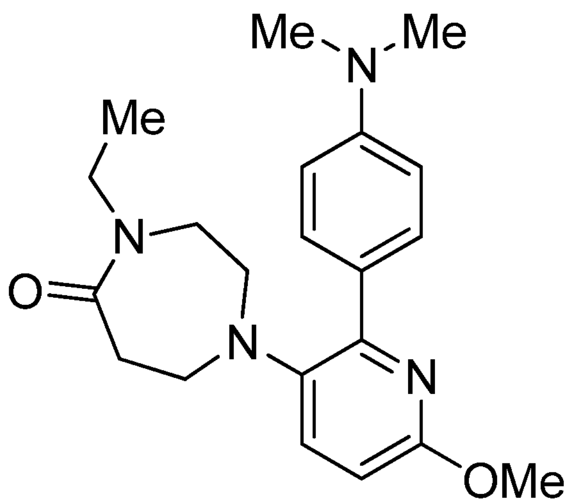

The title compound was obtained in the same way as in Reference Example 50 using 1-{2-[4-(dimethylamino)phenyl]-6-methoxypyridin-3-yl}-1,4-diazepan-5-one (2.00 g, 5.86 mmol) synthesized in Reference Example 35 and iodoethane (1.84 g, 11.8 mmol). Yield: 74%.
$^1$H-NMR ($CDCl_3$): δ 1.10 (3H, t, J = 7.2 Hz) 2.69-2.77 (2H, m), 2.89-2.99 (2H, m), 2.98-3.07 (8H, m), 3.38-3.48 (4H, m), 3.95 (3H, s), 6.56 (1H, d, J = 8.7 Hz), 6.76 (2H, d, J = 9.0 Hz), 7.34 (1H, d, J = 8.3 Hz) 8.07 (2H, d, J = 9.0 Hz).

Reference Example 53

1-[2-(4-ethoxyphenyl)-6-methoxypyridin-3-yl]-4-ethyl-1,4-diazepan-5-one

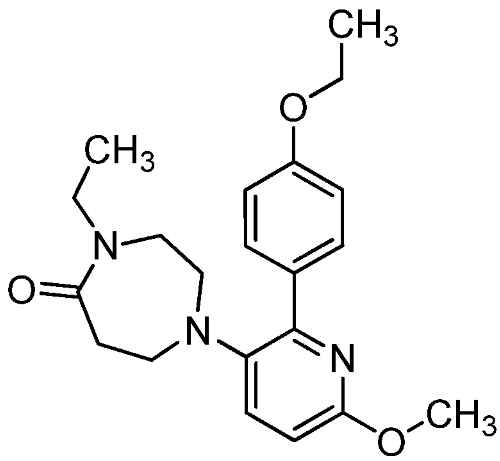

The title compound was obtained in the same way as in Reference Example 50 using 1-[2-(4-ethoxyphenyl)-6-methoxypyridin-3-yl]-1,4-diazepan-5-one (2.00 g, 5.86 mmol) synthesized in Reference Example 37 and iodoethane (1.84 g, 11.8 mmol). Yield: 95%.
$^1$H-NMR ($CDCl_3$): δ 1.09 (3H, t, J = 7.2 Hz), 1.45 (3H, t, J = 7.0 Hz), 2.63-2.77 (2H, m), 2.87-2.96 (2H, m), 2.96-3.07 (2H, m), 3.33-3.48 (4H, m), 3.94 (3H, s), 4.09 (2H, q, J = 6.9 Hz), 6.61 (1H, d, J = 8.7 Hz), 6.94 (2H, d, J = 8.7 Hz), 7.36 (1H, d, J = 8.7 Hz), 8.02 (2H, d, J = 9.1 Hz).

Reference Example 54

2-(4-ethoxy-2-fluorophenyl)-4,4,5,5-tetramethyl-1,3,2-dioxaborolane

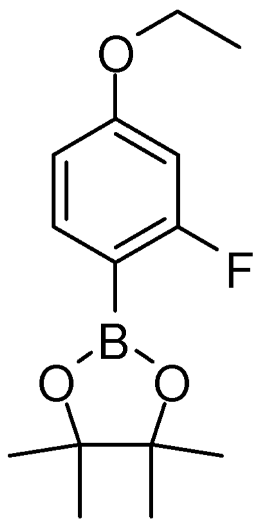

To a mixed solution of 1-bromo-4-ethoxy-2-fluorobenzene (5.70 g, 26.0 mmol), 4,4,4',4',5,5,5',5'-octamethyl-2,2'-bi-1,3,2-dioxaborolane (9.91 g, 39 mmol), and potassium acetate (5.10 g, 52.0 mmol) in DMF (50 mL), a 1,1'-bis(diphenylphosphino)ferrocene-palladium (II) dichloride-dichloromethane complex (1.06 g, 1.30 mmol) was added, and the mixture was stirred at 90°C for 12 hours in a nitrogen atmosphere. To the reaction solution, water was added, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 100:0-90:10) to obtain 6.92 g of the title compound (yield: 100%).
$^1$H-NMR ($CDCl_3$): δ 1.34 (12H, s), 1.41 (3H, t, J = 7.0 Hz), 4.04 (2H, q, J = 7.2 Hz), 6.55 (1H, dd, J = 11.7, 2.3 Hz), 6.67 (1H, dd, J = 8.3, 2.3 Hz), 7.59-7.69 (1H, m).

Reference Example 55

1-{6-methoxy-2-[4-(trifluoromethoxy)phenyl]pyridin-3-yl}-1,4-diazepan-5-one

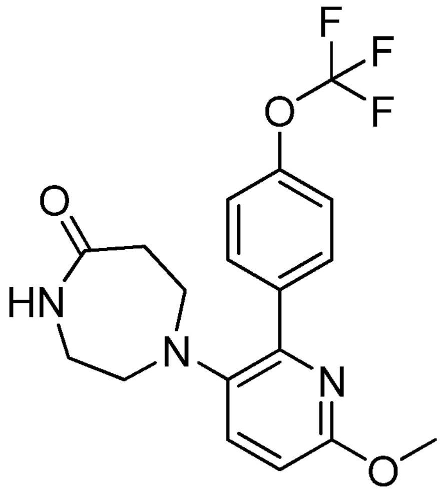

The title compound was obtained in the same way as in Reference Example 35 using 1-(2-bromo-6-methoxypyridin-3-yl)-1,4-diazepan-5-one (3.70 g, 12.3 mmol) synthesized in Reference Example 34 and [4-(trifluoromethoxy)phenyl]boronic acid (3.17 g, 15.4 mmol). Yield: 73%.
$^1$H-NMR ($CDCl_3$): δ 2.60-2.63 (2H, m), 2.94-2.96 (2H, m), 3.01-3.04 (2H, m), 3.23-3.27 (2H, m), 3.95 (3H, s), 6.01 (1H, brs), 6.70 (1H, d, J = 8.8 Hz), 7.47 (2H, d, J = 8.8 Hz), 7.43 (1H, d, J = 8.4 Hz), 8.06 (2H, d, J = 8.8 Hz).

Reference Example 56

1-{6-methoxy-2-[4-(trifluoromethoxy)phenyl]pyridin-3-yl}-4-methyl-1,4-diazepan-5-one

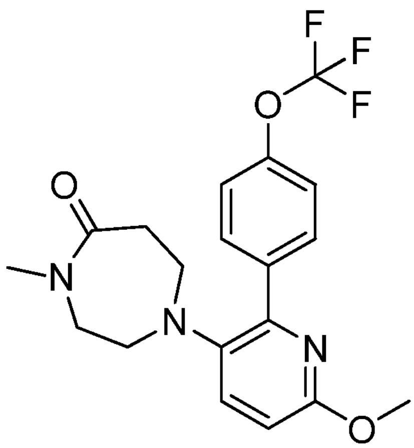

The title compound was obtained in the same way as in Reference Example 50 using 1-{6-methoxy-2-[4-(trifluoromethoxy)phenyl]pyridin-3-yl}-1,4-diazepan-5-one (1.50 g, 3.93 mmol) synthesized in Reference Example 55. Yield: 95%.
$^1$H-NMR ($CDCl_3$): δ 2.64-2.72 (2H, m), 2.87-2.94 (2H, m), 2.97 (3H, s), 2.98-3.04 (2H, m), 3.35-3.43 (2H, m), 3.94 (3H, s), 6.69 (1H, d, J = 8.7 Hz), 7.22-7.32 (2H, m), 7.42 (1H, d, J = 9.0 Hz), 8.01-8.12 (2H, m).

Reference Example 57

2-[4-(1-methylethyl)phenyl]pyridin-3-amine

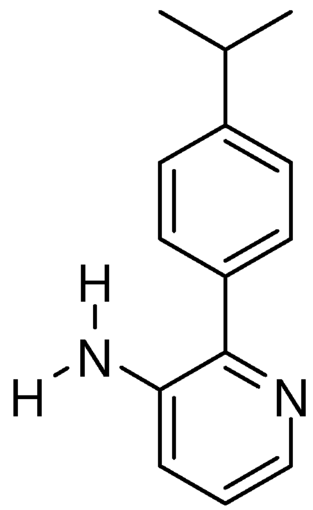

The title compound was obtained in the same way as in Reference Example 2 using 2-bromopyridin-3-amine (10.0 g, 57.8 mmol) and [4-(1-methylethyl)phenyl]boronic acid (11.8 g, 71.7 mmol). Yield: 83%.
$^1$H-NMR ($CDCl_3$): δ 1.28 (6H, d, J = 6.8 Hz), 2.96 (1H, septet, J = 6.8 Hz), 3.84 (2H, brs), 7.04 (2H, dd J = 3.2, 2.8 Hz), 7.32-7.34 (2H, m), 7.59-7.61 (2H, m), 8.12 (1H, dd, J = 3.2, 2.8 Hz).

Reference Example 58

3-bromo-2-[4-(1-methylethyl)phenyl]pyridine

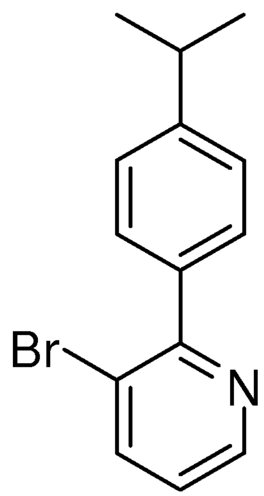

The title compound was obtained in the same way as in Reference Example 3 using 2-[4-(1-methylethyl)phenyl]pyridin-3-amine (5.00 g, 23.6 mmol) synthesized in Reference Example 57. Yield: 78%. $^1$H-NMR ($CDCl_3$): δ 1.29 (6H, d, J = 6.8 Hz), 2.97 (1H, septet, J = 6.8 Hz), 7.12 (1H, dd, J = 8.0, 4.4 Hz), 7.61-7.64 (2H, m), 7.97-7.99 (2H, m), 7.98 (1H, dd, J = 8.4, 1.6 Hz), 8.61 (1H, dd, J = 4.4, 1.6 Hz).

Reference Example 59

1-{6-methoxy-2-[4-(1-methylethyl)phenyl]pyridin-3-yl}-4-methyl-1,4-diazepan-5-one

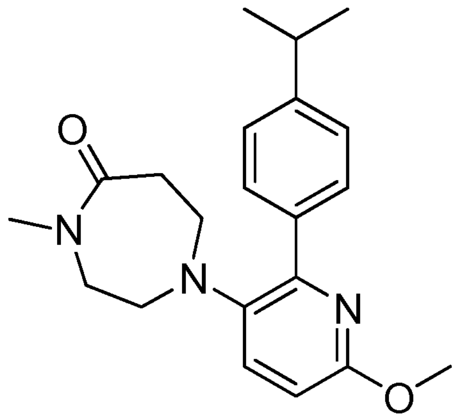

To a DMF (15 mL) suspension of 1-{6-methoxy-2-[4-(1-methylethyl)phenyl]pyridin-3-yl}-1,4-diazepan-5-one (1.50 g, 4.42 mmol) synthesized in Reference Example 36, 60% mineral oil-mixed sodium hydride (354 mg, 8.84 mmol) was added, and the mixture was stirred for 1 hour. To the reaction solution, iodomethane (0.550 mL, 8.84 mmol) was subsequently added dropwise, and the mixture was stirred for 5 hours. To the reaction solution, 60% mineral oil-mixed sodium hydride (176 mg, 4.42 mmol) and iodomethane (0.275 mL, 4.42 mmol) were subsequently added, and the mixture was stirred for 14 hours. To the reaction solution, water was added, followed by extraction with ethyl acetate. The extracts were mixed, washed with water, and then dried over anhydrous magnesium sulfate. The solvent was distilled off under reduced pressure, and the obtained residue was purified by silica gel column chromatography (hexane-ethyl acetate 100:0-60:40) and then crystallized from diethyl ether to obtain 830 mg of the title compound (yield: 53%).
$^1$H-NMR ($CDCl_3$): $\delta$ 1.29 (6H, d, J = 6.8 Hz), 2.64-2.73 (2H, m), 2.87-3.05 (8H, m), 3.34-3.43 (2H, m), 3.94 (3H, s), 6.64 (1H, d, J = 8.7 Hz), 7.23-7.31 (2H, m), 7.37 (1H, d, J = 8.3 Hz), 7.94 (2H, d, J = 8.3 Hz).

Reference Example 60

1-[6-methoxy-2-(4-methoxyphenyl)pyridin-3-yl]-1,4-diazepan-5-one

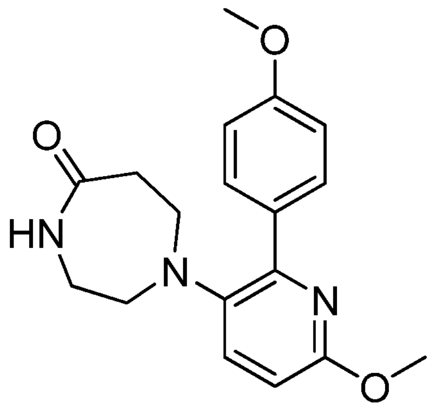

The title compound was obtained in the same way as in Reference Example 35 using 1-(2-bromo-6-methoxypyridin-3-yl)-1,4-diazepan-5-one (5.00 g, 16.7 mmol) synthesized in Reference Example 34 and (4-methoxyphenyl)boronic acid (3.16 g, 20.8 mmol). Yield: 99%.
$^1$H-NMR ($CDCl_3$): $\delta$ 2.63-2.65 (2H, m), 2.94-2.97 (2H, m), 3.01-3.04 (2H, m), 3.25-3.29 (2H, m), 3.87 (3H, s), 3.94 (3H, s), 6.01 (1H, brs), 6.63 (1H, d, J = 8.4 Hz), 6.94-6.97 (2H, m), 7.38 (1H, d, J = 8.4 Hz), 8.00-8.04 (2H, m).

Reference Example 61

1-[6-methoxy-2-(4-methoxyphenyl)pyridin-3-yl]-4-methyl-1,4-diazepan-5-one

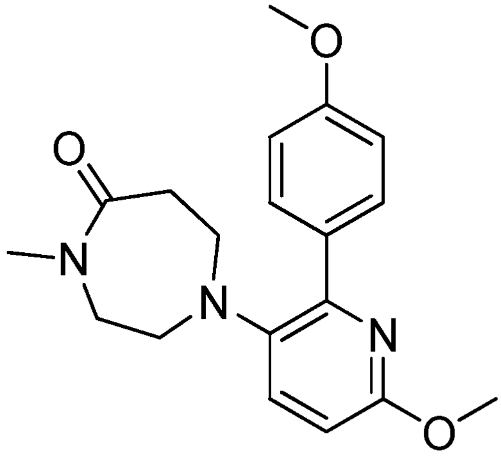

The title compound was obtained in the same way as in Reference Example 59 using 1-[6-methoxy-2-(4-methoxyphenyl) pyridin-3-yl]-1,4-diazepan-5-one (1.50 g, 4.58 mmol) synthesized in Reference Example 60. Yield: 71%.
$^1$H-NMR ($CDCl_3$): δ 2.66-2.74 (2H, m), 2.89-2.95 (2H, m), 2.98 (3H, s), 2.98-3.04 (2H, m), 3.37-3.44 (2H, m), 3.86 (3H, s), 3.94 (3H, s), 6.62 (1H, d, J = 8.3 Hz), 6.95 (2H, d, J = 8.7 Hz), 7.37 (1H, d, J = 8.7 Hz), 7.97-8.08 (2H, m).

Reference Example 62

1-[2-(4-ethoxyphenyl)-6-methoxypyridin-3-yl]-1,4-diazepane

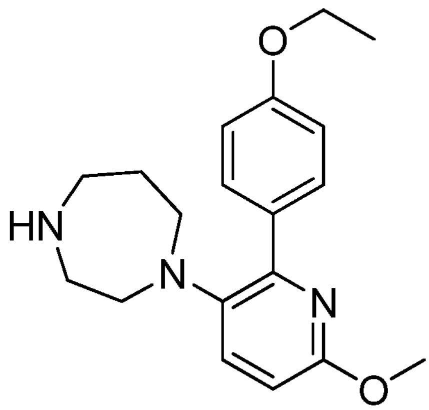

The title compound was obtained in the same way as in Reference Example 38 using 1-[2-(4-ethoxyphenyl)-6-methoxypyridin-3-yl]-1,4-diazepan-5-one (1.58 g, 4.63 mmol) synthesized in Reference Example 37. Yield: 47%.
$^1$H-NMR ($CDCl_3$): δ 1.44 (3H, t, J = 7.0 Hz), 1.64-1.73 (2H, m), 2.78-2.85 (2H, m), 2.90-2.98 (2H, m), 2.99-3.11 (4H, m), 3.93 (3H, s), 4.03-4.15 (2H, m), 6.60 (1H, d, J = 8.7 Hz), 6.89-6.98 (2H, m), 7.41-7.48 (1H, m), 7.91-8.00 (2H, m).

Reference Example 63

2-bromo-6-ethoxypyridin-3-amine

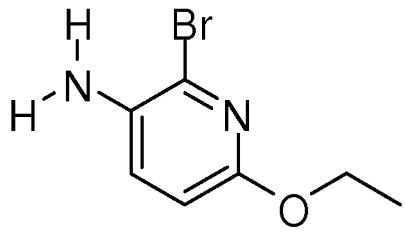

The title compound was obtained in the same way as in Reference Example 1 using 6-ethoxypyridin-3-amine (25.0 g, 181 mmol). Yield: 79%.
$^1$H-NMR ($CDCl_3$): δ 1.35 (3H, t, J = 7.2 Hz), 3.60-3.80 (2H, m), 4.26 (2H, q, J = 7.0 Hz), 6.56 (1H, d, J = 8.3 Hz), 7.03 (1H, d, J = 8.7 Hz).

Reference Example 64

1-(2-bromo-6-ethoxypyridin-3-yl)-4-(4-methoxyphenyl)piperazine

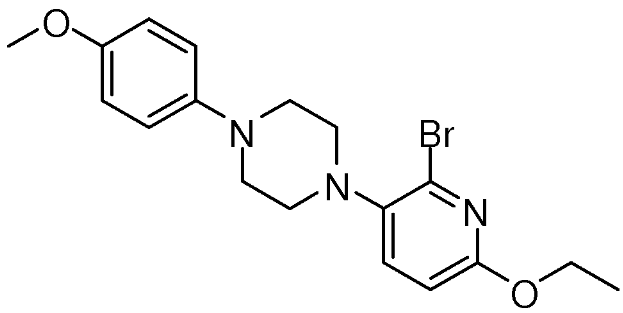

The title compound was obtained in the same way as in Reference Example 22 using 2-bromo-6-ethoxypyridin-3-amine (10.7 g, 49.2 mmol) synthesized in Reference Example 63. Yield: 78%.
$^1$H-NMR ($CDCl_3$): δ 1.38 (3H, t, J = 7.0 Hz), 3.04-3.17 (4H, m), 3.18-3.36 (4H, m), 3.78 (3H, s), 4.33 (2H, q, J = 6.9 Hz), 6.68 (1H, d, J = 8.7 Hz), 6.81-6.91 (2H, m), 6.92-7.00 (2H, m), 7.38 (1H, d, J = 8.3 Hz).

Reference Example 65

1-[2-(4-ethoxy-3-fluorophenyl)-6-methoxypyridin-3-yl]-1,4-diazepan-5-one

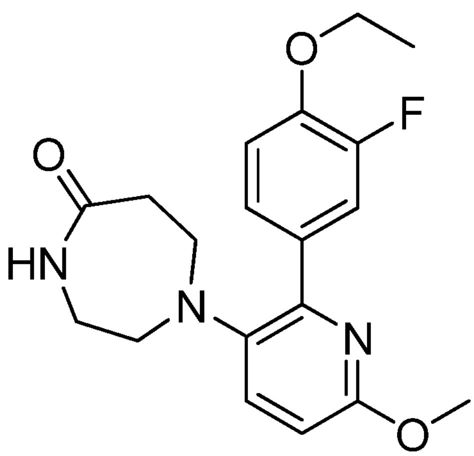

The title compound was obtained in the same way as in Reference Example 35 using 1-(2-bromo-6-methoxypyridin-3-yl)-1,4-diazepan-5-one (1.00 g, 3.33 mmol) synthesized in Reference Example 34 and (4-ethoxy-3-fluorophenyl)boronic acid (736 mg, 4.00 mmol). Yield: 55%.
$^1$H-NMR ($CDCl_3$): δ 1.49 (3H, t, J = 7.0 Hz), 2.62-2.71 (2H, m), 2.93-3.00 (2H, m), 3.00-3.07 (2H, m), 3.26-3.35 (2H, m), 3.95 (3H, s), 4.17 (2H, q, J = 7.2 Hz), 5.87 (1H, brs), 6.65 (1H, d, J = 8.3 Hz), 7.00 (1H, t, J = 8.7 Hz), 7.40 (1H, d, J = 8.7 Hz), 7.85 (1H, d, J = 8.7 Hz), 7.93 (1H, dd, J = 13.2, 2.3 Hz).

Reference Example 66

1-[2-(4-ethoxy-3-fluorophenyl)-6-methoxypyridin-3-yl]-1,4-diazepane

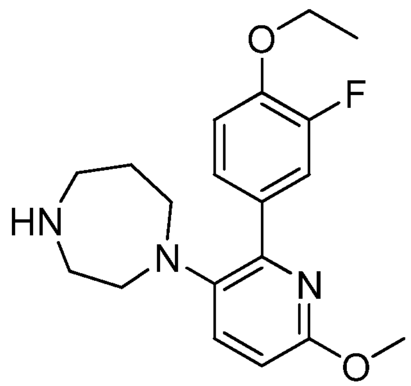

The title compound was obtained in the same way as in Reference Example 38 using 1-[2-(4-ethoxy-3-fluorophenyl)-6-methoxypyridin-3-yl]-1,4-diazepan-5-one (656 mg, 1.83 mmol) synthesized in Reference Example 65. Yield: 82%.
$^1$H-NMR ($CDCl_3$): δ 1.48 (3H, t, J = 7.0 Hz), 1.65-1.78 (2H, m), 2.82-2.90 (2H, m), 2.93-3.00 (2H, m), 3.00-3.11 (4H, m), 3.93 (3H, s), 4.16 (2H, q, J = 6.8 Hz), 6.63 (1H, d, J = 8.7 Hz), 6.99 (1H, t, J = 8.7 Hz), 7.47 (1H, d, J = 8.7 Hz), 7.76-7.83 (1H, m), 7. 89 (1H, dd, J = 13.3, 1.9 Hz).

Reference Example 67

1-[2-(4-ethoxy-3-methylphenyl)-6-methoxypyridin-3-yl]-1,4-diazepan-5-one

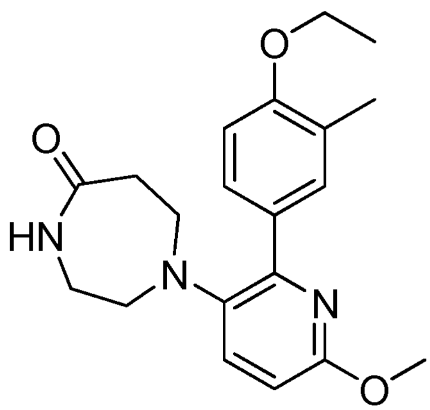

The title compound was obtained in the same way as in Reference Example 35 using 1-(2-bromo-6-methoxypyridin-3-yl)-1,4-diazepan-5-one (500 mg, 1.67 mmol) synthesized in Reference Example 34 and (4-ethoxy-3-methylphenyl) boronic acid (360 mg, 2.00 mmol). Yield: quantitative.
$^1$H-NMR ($CDCl_3$): δ 1.46 (3H, t, J = 7.0 Hz), 2.28 (3H, s), 2.59-2.68 (2H, m), 2.91-2.99 (2H, m), 2.99-3.09 (2H, m), 3.21-3.32 (2H, m), 3.95 (3H, s), 4.09 (2H, q, J = 6.9 Hz), 5.91 (1H, t, J = 5.3 Hz), 6.61 (1H, d, J = 8.7 Hz), 6.82-6.89 (1H,

m), 7.36 (1H, d, J = 8.7 Hz), 7.81-7.90 (2H, m).

Reference Example 68

1-[2-(4-ethoxy-3-methylphenyl)-6-methoxypyridin-3-yl]-1,4-diazepane

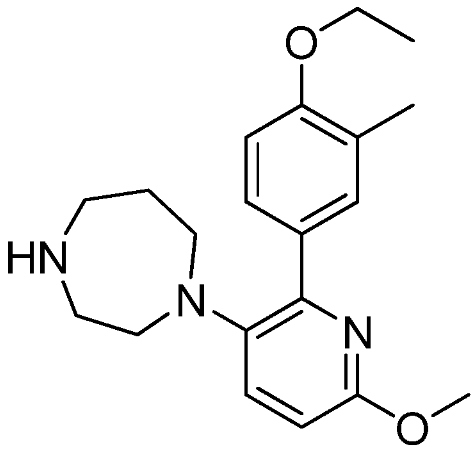

The title compound was obtained in the same way as in Reference Example 38 using 1-[2-(4-ethoxy-3-methylphenyl)-6-methoxypyridin-3-yl]-1,4-diazepan-5-one (600 mg, 1.69 mmol) synthesized in Reference Example 67. Yield: 89%.
$^1$H-NMR ($CDCl_3$): δ 1.44 (3H, t, J = 7.0 Hz), 1.62 (1H, brs), 1.63-1.74 (2H, m), 2.28 (3H, s), 2.77-2.84 (2H, m), 2.90-2.98 (2H, m), 2.99-3.10 (4H, m), 3.93 (3H, s), 4.08 (2H, q, J = 6.8 Hz), 6.59 (1H, d, J = 8.7 Hz), 6.85 (1H, d, J = 8.7 Hz), 7.43 (1H, d, J = 8.7 Hz), 7.74-7.84 (2H, m).

Reference Example 69

1-(6'-ethoxy-6-methoxy-2,3'-bipyridin-3-yl)-1,4-diazepan-5-one

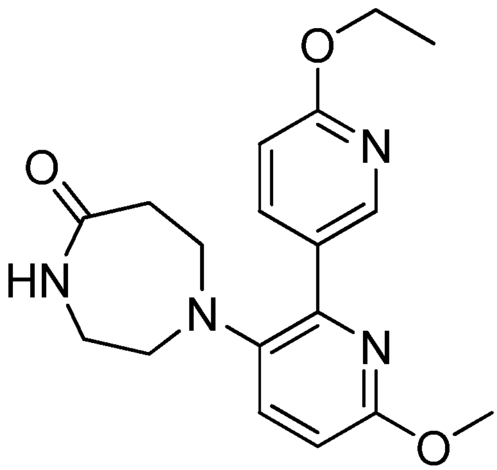

The title compound was obtained in the same way as in Reference Example 2 using 1-(2-bromo-6-methoxypyridin-3-yl)-1,4-diazepan-5-one (300 mg, 1.00 mmol) synthesized in Reference Example 34 and(6-ethoxypyridin-3-yl)boronic acid (167 mg, 1.00 mmol). Yield: 91%.
$^1$H-NMR ($CDCl_3$): δ 1.43 (3H, t, J = 7.2 Hz), 2.60-2.72 (2H, m), 2.93-3.00 (2H, m), 3.00-3.08 (2H, m), 3.26-3.38 (2H, m), 3.94 (3H, s), 4.42 (2H, q, J = 7.2 Hz), 5.92 (1H, t, J = 5.3 Hz), 6.67 (1H, d, J = 8.7 Hz), 6.78 (1H, d, J = 8.7 Hz), 7.43 (1H, d, J = 8.7 Hz), 8.29 (1H, dd, J = 8.5, 2.4 Hz), 8.90 (1H, d, J = 2.6 Hz).

Reference Example 70

2-bromo-6-methoxy-4-methylpyridin-3-amine

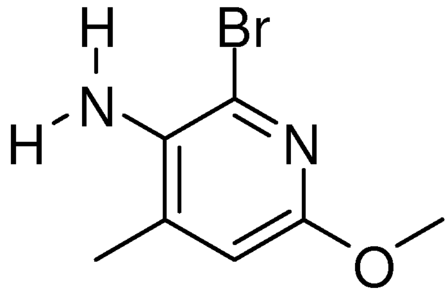

The title compound was obtained in the same way as in Reference Example 1 using 6-methoxy-4-methylpyridin-3-amine (7.56 g, 54.7 mmol). Yield: 54%.
$^1$H-NMR ($CDCl_3$): δ 2.20 (3H, s), 3.54-3.80 (2H, m), 3.84 (3H, s), 6.47 (1H, s).

Reference Example 71

1-(2-bromo-6-methoxy-4-methylpyridin-3-yl)-4-(4-methoxyphenyl)piperazine

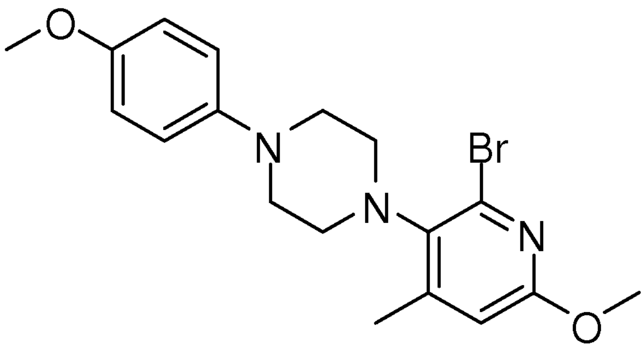

The title compound was obtained in the same way as in Reference Example 22 using 2-bromo-6-methoxy-4-methylpyridin-3-amine (3.45 g, 15.9 mmol) synthesized in Reference Example 70. Yield: 9%.
$^1$H-NMR ($CDCl_3$): δ 2.34 (3H, s), 2.95-3.13 (4H, m), 3.23-3.38 (2H, m), 3.48-3.65 (2H, m), 3.78 (3H, s), 3.89 (3H, s), 6.52 (1H, s), 6.81-7.03 (4H, m).

Reference Example 72

3-[(tert-butoxycarbonyl)(4-methoxyphenyl)amino]propanoic acid

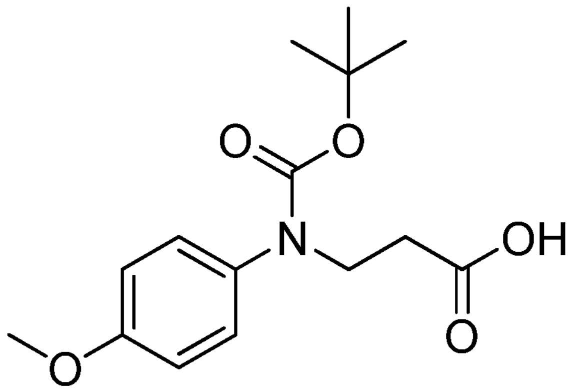

To a THF (6 mL)-water (6 mL) mixed solution of 3-[(4-methoxyphenyl)amino]propanoic acid (800 mg, 4.10 mmol), a 1 N aqueous sodium hydroxide solution (5 mL) was added to adjust the pH of the solution to 9. To the reaction solution, di-tert-butyl dicarbonate (0.942 mL, 4.10 mmol) was added, and the mixture was then stirred at room temperature for 4 hours with its pH kept between 8.5 and 9 using a 1 N aqueous sodium hydroxide solution. The reaction solution was washed with diethyl ether, and the aqueous phase was rendered acidic using 1 N hydrochloric acid, followed by extraction with ethyl acetate. The obtained organic phase was dried over anhydrous magnesium sulfate, and the solvent was distilled off under reduced pressure to obtain 921 mg of the title compound (yield: 76%).
$^1$H-NMR ($CDCl_3$): δ 1.41 (9H, brs), 2.61 (2H, t, J = 7.2 Hz), 3.80 (3H, s), 3.89 (2H, t, J = 7.2 Hz), 6.80-6.91 (2H, m), 7.08 (2H, d, J = 8.3 Hz).

Reference Example 73

Tert-butyl (3-{[2-(4-ethoxyphenyl)-6-methoxypyridin-3-yl]amino}-3-oxopropyl)(4-methoxyphenyl)carbamate

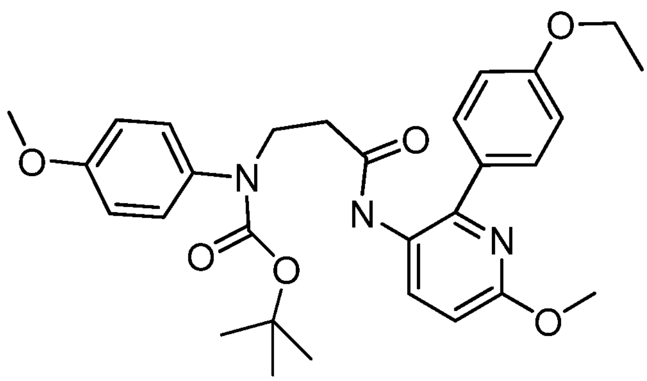

To a DMF (8 mL) solution of 2-(4-ethoxyphenyl)-6-methoxypyridin-3-amine (762 mg, 3.12 mmol) synthesized in Reference Example 8, 3-[(tert-butoxycarbonyl)(4-methoxyphenyl)amino]propanoic acid (921 mg, 3.12 mmol) synthesized in Reference Example 72, and triethylamine (0.651 mL, 4.68 mmol), WSC (717 mg, 3.74 mmol) and HOBt (505 mg, 3.74 mmol) were added, and the mixture was stirred at room temperature for 14 hours. To the reaction solution, a saturated aqueous solution of sodium bicarbonate was added, followed by extraction with ethyl acetate. The extracts were mixed, washed with water, and then dried over anhydrous magnesium sulfate. The solvent was distilled off under reduced pressure, and the obtained residue was purified by silica gel column chromatography (hexane-ethyl acetate 100:0-75: 25) to obtain 1.20 g of the title compound (yield: 74%).
1H-NMR ($CDCl_3$): δ 1.36 (9H, s), 1.46 (3H, t, J = 7.0 Hz), 2.52 (2H, t, J = 7.2 Hz), 3.79 (3H, s), 3.88-3.98 (5H, m), 4.10 (2H, q, J = 6.9 Hz), 6.68 (1H, d, J = 9.0 Hz), 6.78-6.87 (2H, m), 6.95-7.08 (4H, m), 7.34 (1H, brs), 7.54 (2H, d, J = 8.7 Hz), 8.17 (1H, d, J = 8.7 Hz).

Reference Example 74

3-[(2-bromopropanoyl)(4-methoxyphenyl)amino]-N-[2-(4-ethoxyphenyl)-6-methoxypyridin-3-yl]propanamide

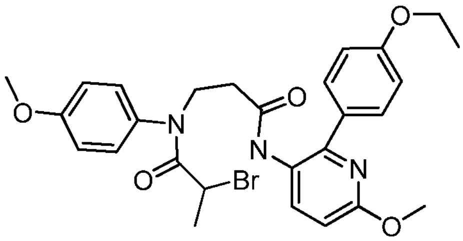

To an ethyl acetate (3 mL) solution of tert-butyl (3-{[2-(4-ethoxyphenyl)-6-methoxypyridin-3-yl]amino}-3-oxopropyl)(4-methoxyphenyl)carbamate (400 mg, 0.77 mmol) synthesized in Reference Example 73, 4 N hydrochloric acid/ethyl acetate (1 mL) was added, and the mixture was stirred at room temperature for 1 hour. The solvent was distilled off under reduced pressure, and ethyl acetate (4 mL) and a saturated aqueous solution of sodium bicarbonate (3 mL) were added to the obtained residue. To the reaction mixture, 2-bromopropionyl chloride (0.159 mL, 1.54 mmol) was added dropwise, and the mixture was stirred at room temperature for 17 hours. To the reaction solution, water was added, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 100:0-60:40) to obtain 350 mg of the title compound (yield: 82%).
$^1$H-NMR ($CDCl_3$): δ 1.46 (3H, t, J = 7.0 Hz), 1.68 (3H, d, J = 6.8 Hz), 2.58 (2H, t, J = 7.2 Hz), 3.83 (3H, s), 3.93 (3H, s), 3.94-4.05 (2H, m), 4.05-4.16 (2H, m), 4.20 (1H, q, J = 6.8 Hz), 6.68 (1H, d, J = 8.7 Hz), 6.92 (2H, d, J = 9.0 Hz), 7.00 (2H, d, J = 9.0 Hz), 7.16 (2H, brs), 7.42 (1H, s), 7.55 (2H, d, J = 9.0 Hz), 8.13 (1H, d, J = 9.0 Hz).

Example 1

4-{3-[2-(3,5-dimethoxyphenyl)morpholin-4-yl]-6-methoxypyridin-2-yl}-N,N-dimethylaniline

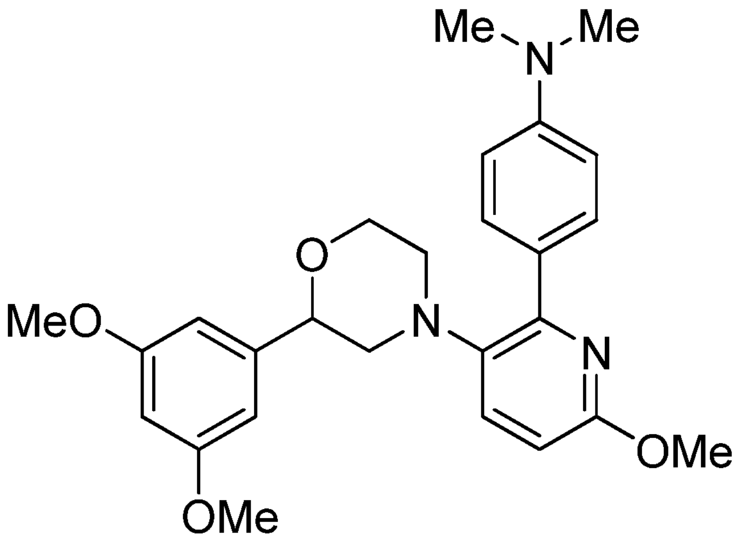

A mixture of 4-(3-bromo-6-methoxypyridin-2-yl)-N,N-dimethylaniline (370 mg, 1.20 mmol) synthesized in Reference Example 3, 2-(3,5-dimethoxyphenyl)morpholine (270 mg, 1.21 mmol) synthesized in Reference Example 41, tris(dibenzylideneacetone)dipalladium (0) (110 mg, 0.12 mmol), Xantphos (139 mg, 0.24 mmol), sodium tert-butoxide (346 mg, 3.60 mmol), and toluene (9.0 mL) was stirred at 160°C for 1 hour under microwave irradiation. After cooling to room temperature, the reaction solution was partitioned with water and ethyl acetate, and the organic phase was washed with

saturated brine and then dried over anhydrous magnesium sulfate. The solvent was distilled off under reduced pressure, and the obtained residue was purified by basic silica gel column chromatography (hexane-ethyl acetate 49:1-87:13) to obtain 260 mg of the title compound (yield: 48%) as an oil.

$^1$H-NMR ($CDCl_3$) : δ 2.58 (1H, dd, J = 9.9, 11.4 Hz), 2.74-2.88 (1H, m), 2.91-3.06 (7H, m), 3.07-3.17 (1H, m), 3.75 (6H, s), 3.82-3.97 (4H, m), 3.98-4.07 (1H, m), 4.58 (1H, dd, J = 2.4, 9.9 Hz), 6.35 (1H, t, J = 2.1 Hz), 6.41 (2H, d, J = 2.1 Hz), 6.55 (1H, d, J = 8.7 Hz), 6.76-6.84 (2H, m), 7.29 (1H, J = 8.7 Hz, d), 8.10-8.17 (2H, m).

Example 2

4-(6-methoxy-3-{2-[3-(trifluoromethyl)phenyl]morpholin-4-yl}pyridin-2-yl)-N,N-dimethylaniline

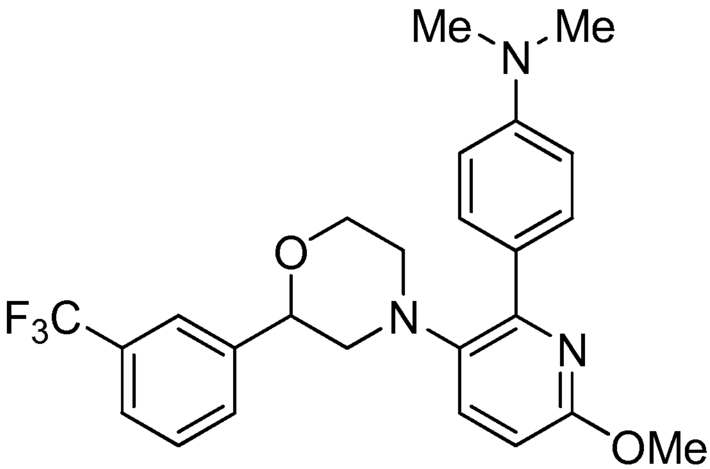

The title compound was obtained in the same way as in Example 1 using 4-(3-bromo-6-methoxypyridin-2-yl)-N,N-dimethylaniline (287 mg, 0.934 mmol) synthesized in Reference Example 3 and 2-[3-(trifluoromethyl)phenyl]morpholine (250 mg, 0.934 mmol). Yield: 35%. Melting point: 114-115°C (ethyl acetate-hexane).

$^1$H-NMR ($CDCl_3$) : δ 2.56 (1H, dd, J = 10.2, 11.4 Hz), 2.79-2.91 (1H, m), 2.94-3.07 (7H, m), 3.08-3.17 (1H, m), 3.89 (1H, dd, J = 2.7, 11.4 Hz), 3.95 (3H, s), 4.01-4.09 (1H, m), 4.68 (1H, dd, J = 2.7, 10.2 Hz), 6.56 (1H, d, J = 8.4 Hz), 6.77-6.86 (2H, m), 7.30 (1H, d, J = 8.4 Hz), 7.39-7.46 (2H, m), 7.47-7.56 (2H, m), 8.08-8.17 (2H, m).

Example 3

4-{6-methoxy-3-[2-(4-methoxyphenyl)morpholin-4-yl]pyridin-2-yl}-N,N-dimethylaniline

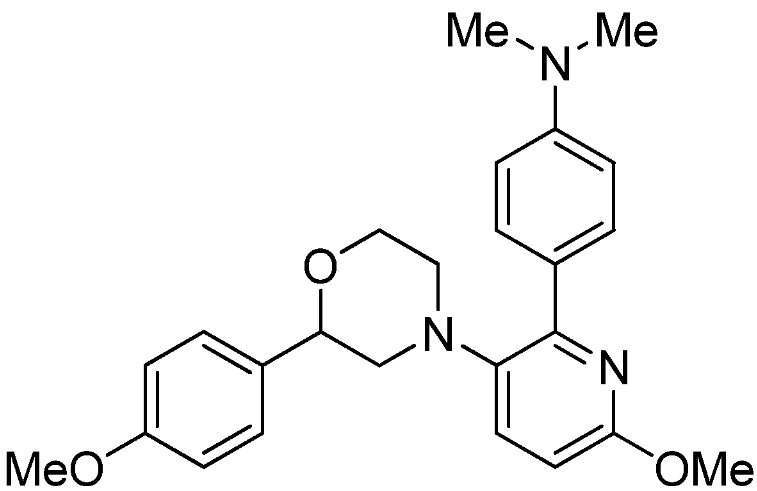

The title compound was obtained in the same way as in Example 1 using 4-(3-bromo-6-methoxypyridin-2-yl)-N,N-dimethylaniline (500 mg, 1.63 mmol) synthesized in Reference Example 3 and 2-(4-methoxyphenyl)morpholine (472 mg, 2.44 mmol). Yield: 9%, oil.

$^1$H-NMR ($CDCl_3$) : δ 2.66 (1H, dd, J = 10.2, 11.1 Hz), 2.72-2.84 (1H, m), 2.90-3.12 (8H, m), 3.78 (3H, s), 3.81-4.03 (5H, m), 4.56-4.64 (1H, m), 6.55 (1H, d, J = 8.7 Hz), 6.76-6.89 (4H, m), 7.18-7.27 (2H, m), 7.31 (1H, d, J = 8.7 Hz), 8.13-8.21 (2H, m).

Example 4

4-{3-[2-(4-fluorophenyl)morpholin-4-yl]-6-methoxypyridin-2-yl}-N,N-dimethylaniline

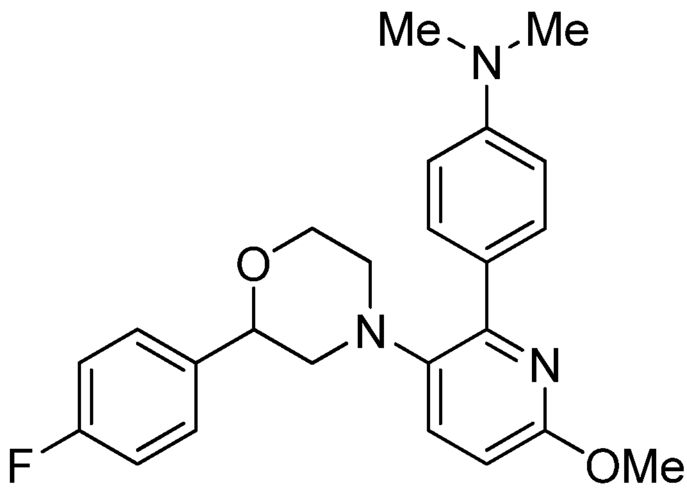

The title compound was obtained in the same way as in Example 1 using 4-(3-bromo-6-methoxypyridin-2-yl)-N,N-dimethylaniline (500 mg, 1.63 mmol) synthesized in Reference Example 3 and 2-(4-fluorophenyl)morpholine oxalate (664 mg, 2.45 mmol). Yield: 27%, oil.

$^1$H-NMR ($CDCl_3$) : δ 2.60 (1H, dd, J = 10.2, 11.7 Hz), 2.74-2.86 (1H, m), 2.92-3.01 (1H, m), 3.03 (6H, s), 3.04-3.12 (1H, m), 3.82-3.94 (1H, m), 3.95 (3H, s), 3.96-4.05 (1H, m), 4.62 (1H, dd, J = 2.4, 10.2 Hz), 6.55 (1H, d, J = 8.7 Hz), 6.75-6.85 (2H, m), 6.94-7.05 (2H, m), 7.20-7.29 (2H, m), 7.31 (1H, d, J = 8.7 Hz), 8.11-8.20 (2H, m).

Example 5

4-(6-methoxy-3-{2-[4-(trifluoromethyl)phenyl]morpholin-4-yl}pyridin-2-yl)-N,N-dimethylaniline

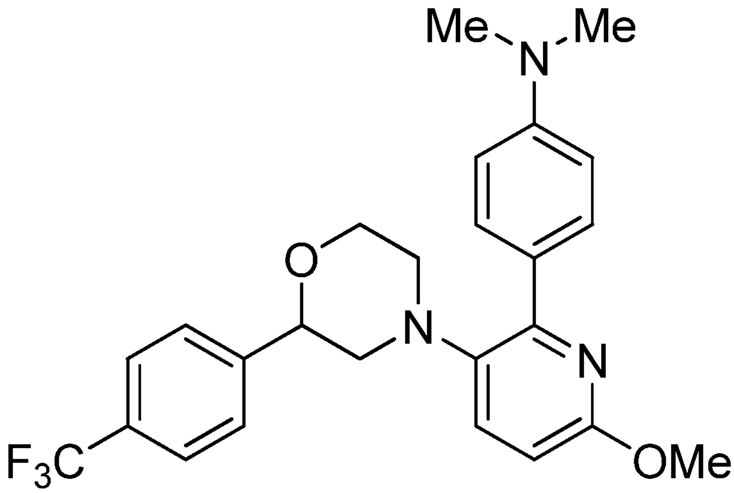

The title compound was obtained in the same way as in Example 1 using 4-(3-bromo-6-methoxypyridin-2-yl)-N,N-dimethylaniline (500 mg, 1.63 mmol) synthesized in Reference Example 3 and 2-(4-trifluoromethylphenyl)morpholine oxalate (664 mg, 2.45 mmol). Specifically, a mixture of 4-(3-bromo-6-methoxypyridin-2-yl)-N,N-dimethylaniline (500 mg, 1.63 mmol), 2-(4-trifluoromethylphenyl)morpholine oxalate (787 mg, 2.45 mmol), tris(dibenzylideneacetone)dipalladium (0) (149 mg, 0.163 mmol), Xantphos (189 mg, 0.326 mmol), sodium tert-butoxide (470 mg, 4.89 mmol), and toluene (10.0 mL) was stirred at 160°C for 1 hour under microwave irradiation. After cooling to room temperature, the reaction solution was partitioned with water and ethyl acetate, and the organic phase was washed with saturated brine and then dried over anhydrous magnesium sulfate. The solvent was distilled off under reduced pressure, and the obtained residue was purified by basic silica gel chromatography (hexane-ethyl acetate 97:3-85:15) to obtain 140 mg of the title compound (yield: 19%) as an amorphous solid.

$^1$H-NMR ($CDCl_3$): δ 2.56 (1H, dd, J = 9.9, 11.4 Hz), 2.77-2.91 (1H, m), 2.94-3.06 (7H, m), 3.08-3.16 (1H, m), 3.84-3.97 (4H, m), 4.00-4.08 (1H, m), 4.69 (1H, dd, J = 1.8, 9.9 Hz), 6.55 (1H, d, J = 8.7 Hz), 6.77-6.85 (2H, m), 7.30 (1H, d, J = 8.7 Hz), 7.33-7.41 (2H, m), 7.52-7.60 (2H, m), 8.10-8.18 (2H, m).

Example 6

4-(6-methoxy-3-{2-[4-(trifluoromethyl)phenyl]morpholin-4-yl}pyridin-2-yl)-N,N-dimethylaniline (retention time: earlier)

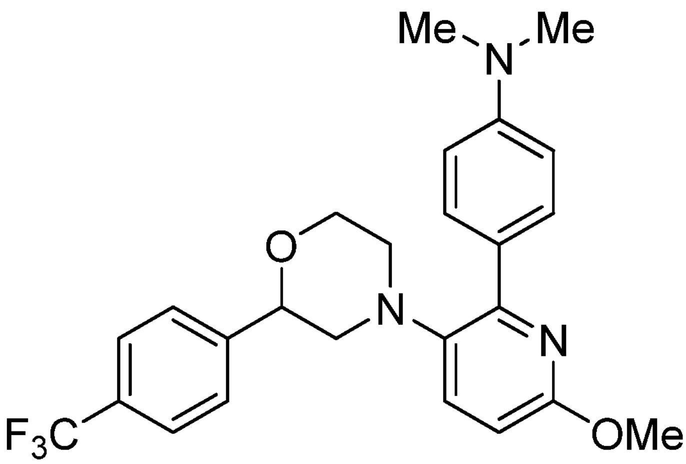

4-(6-methoxy-3-{2-[4-(trifluoromethyl)phenyl]morpholin-4-yl}pyridin-2-yl)-N,N-dimethylaniline (630 mg) obtained in Example 5 was fractionated using high-performance liquid chromatography (column: CHIRALCEL OD manufactured by Daicel Corp., mobile phase: hexane). A solution of a fraction containing an optically active form having an earlier retention time was concentrated and crystallized from ethyl acetate-hexane to obtain the title compound (190 mg). Melting point: 160-161°C.

Example 7

4-(6-methoxy-3-{2-[4-(trifluoromethyl)phenyl]morpholin-4-yl}pyridin-2-yl)-N,N-dimethylaniline (retention time: later)

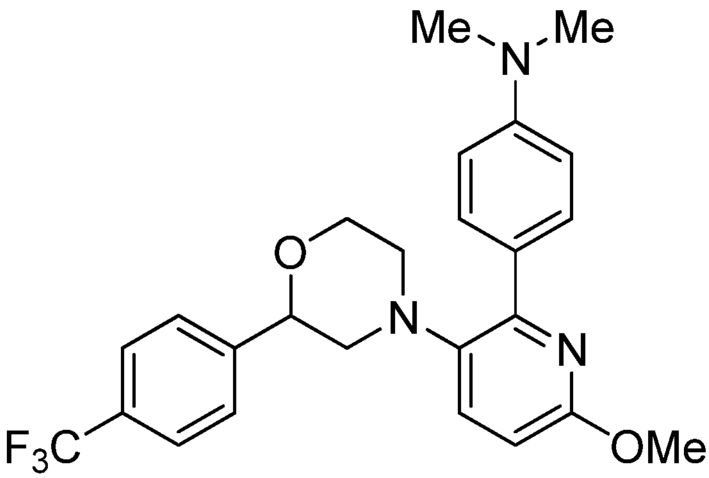

A solution of a fraction containing an optically active form having a later retention time in Example 6 was concentrated and crystallized from ethyl acetate-hexane to obtain the title compound (170 mg). Melting point: 160-161°C.

Example 8

4-{6-methoxy-3-[2-(2-methyl-1,3-thiazol-4-yl)morpholin-4-yl]pyridin-2-yl}-N,N-dimethylaniline

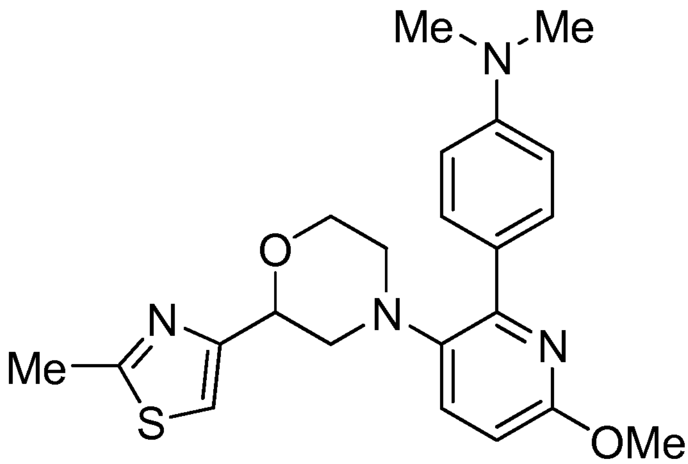

The title compound was obtained in the same way as in Example 1 using 4-(3-bromo-6-methoxypyridin-2-yl)-N,N-dimethylaniline (120 mg, 0.391 mmol) synthesized in Reference Example 3 and 2-(2-methyl-1,3-thiazol-4-yl)morpholine (60 mg, 0.326 mmol) synthesized in Reference Example 44. Yield: 41%, oil.
$^1$H-NMR ($CDCl_3$) : δ 2.43 (3H, d, J = 0.6 Hz), 2.68-2.80 (1H, m), 2.85-3.00 (2H, m), 3.01 (6H, s), 3.42-3.51 (1H, m), 3.80-3.91 (1H, m), 3.92-4.00 (4H, m), 4.99 (1H, dd, J = 2.4, 9.9 Hz), 6.56 (1H, d, J = 8.4 Hz), 6.72-6.80 (2H, m), 6.83-6.87 (1H, m), 7.37 (1H, d, J = 8.4 Hz), 8.07-8.16 (2H, m).

Example 9

4-(6-methoxy-3-{2-[3-(trifluoromethyl)benzyl]morpholin-4-yl}pyridin-2-yl)-N,N-dimethylaniline

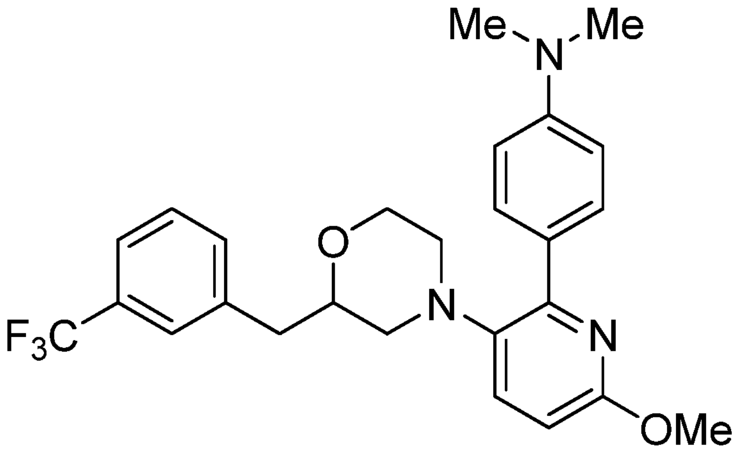

The title compound was obtained in the same way as in Example 1 using 4-(3-bromo-6-methoxypyridin-2-yl)-N,N-dimethylaniline (300 mg, 0.977 mmol) synthesized in Reference Example 3 and 2-(3-trifluoromethylbenzyl)morpholine (287 mg, 1.17 mmol). Yield: 35%, oil.
$^1$H-NMR ($CDCl_3$) : δ 2.47 (1H, dd, J = 9.9, 11.1 Hz), 2.62-2.78 (2H, m), 2.81-2.99 (3H, m), 3.00 (6H, s), 3.64-3.90 (3H, m), 3.93 (3H, s), 6.56 (1H, d, J = 8.7 Hz), 6.66-6.75 (2H, m), 7.27-7.49 (5H, m), 8.01-8.10 (2H, m).

Example 10

4-{6-methoxy-2-[4-(1-methylethyl)phenyl]pyridin-3-yl}-2-(4-methoxyphenyl)morpholine

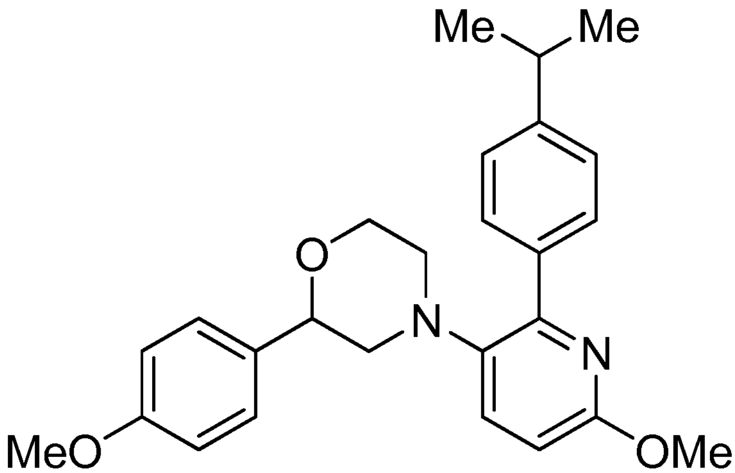

The title compound was obtained in the same way as in Example 1 using 3-bromo-6-methoxy-2-[4-(1-methylethyl)phenyl]pyridine (250 mg, 0.816 mmol) synthesized in Reference Example 11 and 2-(4-methoxyphenyl)morpholine (189 mg, 0.980 mmol). Yield: 18%, oil.
$^1$H-NMR ($CDCl_3$): δ 1.31 (6H, d, J = 6.9 Hz), 2.54 (1H, dd, J = 10.2, 11.4 Hz), 2.79-3.06 (4H, m), 3.77 (3H, s), 3.78-3.89 (1H, m), 3.94 (3H, s), 3.96-4.05 (1H, m), 4.48 (1H, dd, J = 2.4, 10.2 Hz), 6.63 (1H, d, J = 8.7 Hz), 6.77-6.86 (2H, m), 7.07-7.16 (2H, m), 7.28-7.38 (3H, m), 8.00-8.09 (2H, m).

Example 11

2-(3,5-dimethoxyphenyl)-4-{6-methoxy-2-[4-(1-methylethyl)phenyl]pyridin-3-yl}morpholine

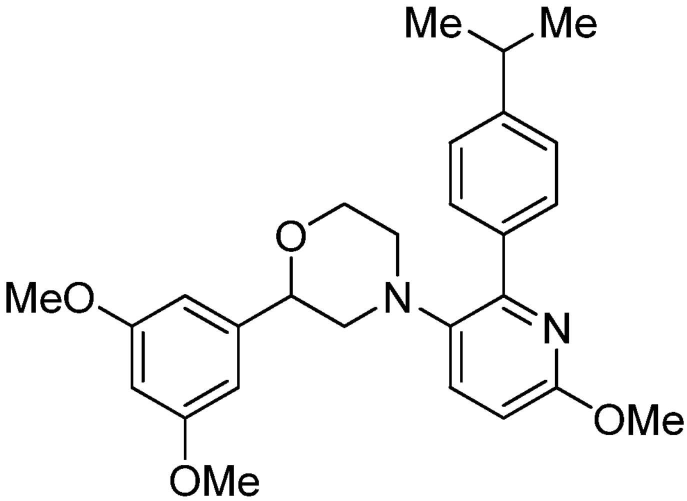

The title compound was obtained in the same way as in Example 1 using 3-bromo-6-methoxy-2-[4-(1-methylethyl)phenyl] pyridine (300 mg, 0.980 mmol) synthesized in Reference Example 11 and 2-(3,5-dimethoxyphenyl)morpholine (270 mg, 1.21 mmol). Yield: 22%, oil.
$^1$H-NMR ($CDCl_3$): δ 1.31 (6H, d, J = 6.9 Hz), 2.56 (1H, dd, J = 9.9, 11.7 Hz), 2.79-3.08 (4H, m), 3.74 (6H, s), 3.77-3.88 (1H, m), 3.94 (3H, s), 3.97-4.05 (1H, m), 4.48 (1H, dd, J = 2.1, 9.9 Hz), 6.31-6.39 (3H, m), 6.63 (1H, d, J = 8.7 Hz), 7.28-7.36 (3H, m), 8.02-8.10 (2H, m).

Example 12

4-{3-[4-(4-fluorophenyl)piperazin-1-yl]-6-methoxypyridin-2-yl}-N,N-dimethylaniline

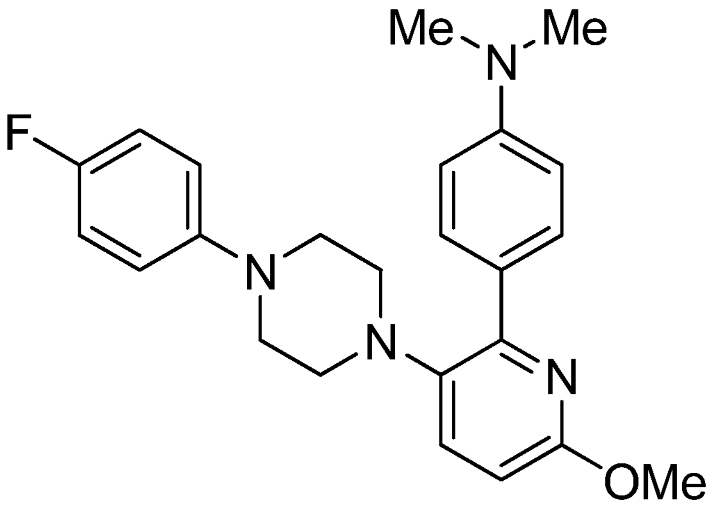

The title compound was obtained in the same way as in Example 1 using 4-(3-bromo-6-methoxypyridin-2-yl)-N,N-dimethylaniline (1.00 g, 3.26 mmol) synthesized in Reference Example 3 and 1-(4-fluorophenyl)piperazine (1.18 g, 6.52 mmol). Specifically, a mixture of 4-(3-bromo-6-methoxypyridin-2-yl)-N,N-dimethylaniline (1.00 g, 3.26 mmol), 1-(4-fluorophenyl)piperazine (1.18 g, 6.52 mmol), tris(dibenzylideneacetone)dipalladium (0) (299 mg, 0.326 mmol), Xantphos (377 mg, 0.652 mmol), sodium tert-butoxide (940 mg, 9.78 mmol), and toluene (16 mL) was stirred at 160°C for 1.5

hours under microwave irradiation. After cooling to room temperature, the reaction solution was partitioned with water and ethyl acetate, and the organic phase was washed with saturated brine and then dried over anhydrous magnesium sulfate. The solvent was distilled off under reduced pressure, and the obtained residue was purified by silica gel chromatography (hexane-ethyl acetate 98:2-88:12) and crystallized from ethyl acetate and hexane to obtain 630 mg of the title compound (yield: 48%). Melting point: 168-169°C (ethyl acetate-hexane).
$^1$H-NMR ($CDCl_3$) : δ 2.96-3.04 (10H, m), 3.12-3.21 (4H, m), 3.96 (3H, s), 6.58 (1H, d, J = 8.7 Hz), 6.71-6.79 (2H, m), 6.84-7.02 (4H, m), 7.39 (1H, d, J = 8.7 Hz), 8.13-8.21 (2H, m).

Example 13

Ethyl 4-{2-[4-(dimethylamino)phenyl]-6-methoxypyridin-3-yl}-2-(4-methoxyphenyl)piperazine-1-carboxylate

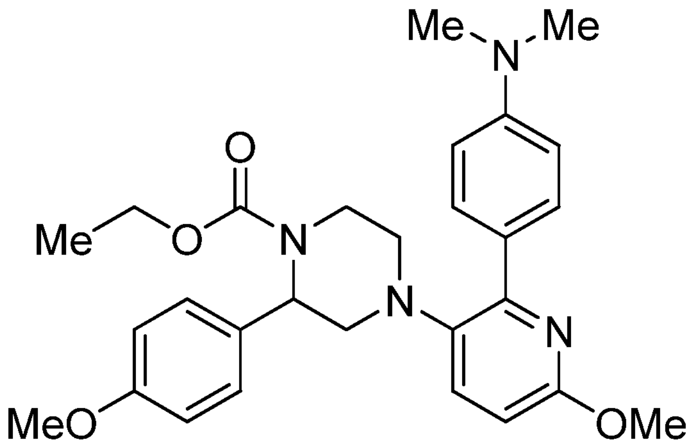

The title compound was obtained in the same way as in Example 1 using 4-(3-bromo-6-methoxypyridin-2-yl)-N,N-dimethylaniline (260 mg, 0.846 mmol) synthesized in Reference Example 3 and ethyl 2-(4-methoxyphenyl)piperazine-1-carboxylate (333 mg, 1.26 mmol) synthesized in Reference Example 47. Yield: 12%, oil.
$^1$H-NMR ($CDCl_3$) : δ 1.26 (3H, t, J = 7.2 Hz), 2.45-2.58 (1H, m), 2.86-3.13 (8H, m), 3.20-3.30 (1H, m), 3.52-3.62 (1H, m), 3.80 (3H, s), 3.81-3.91 (1H, m), 3.93 (3H, s), 4.19 (2H, q, J = 7.2 Hz), 5.40-5.47 (1H, m), 6.45-6.54 (2H, m), 6.59 (1H, d, J = 8.7 Hz), 6.80-6.89 (2H, m), 7.19-7.28 (2H, m), 7.39 (1H, d, J = 8.7 Hz), 7.69-7.78 (2H, m).

Example 14

4-{6-methoxy-3-[3-(4-methoxyphenyl)piperazin-1-yl]pyridin-2-yl}-N,N-dimethylaniline

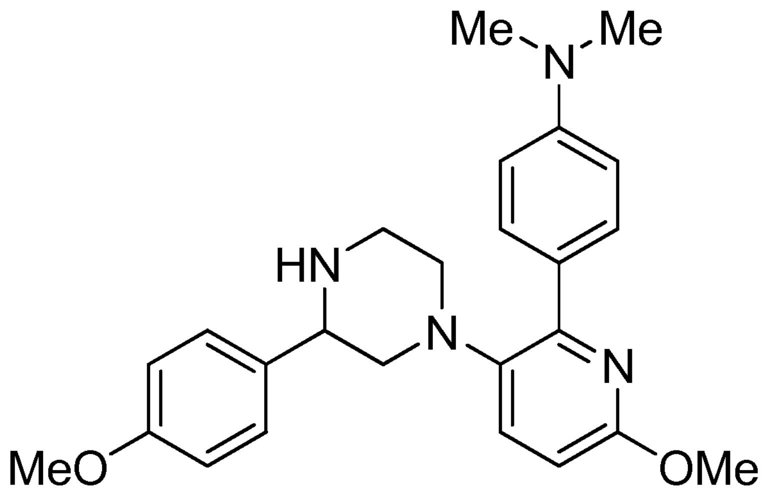

A mixture of ethyl 4-{2-[4-(dimethylamino)phenyl]-6-methoxypyridin-3-yl}-2-(4-methoxyphenyl)piperazine-1-carboxylate (40 mg, 0.0815 mmol) obtained in Example 13, an 8 N aqueous potassium hydroxide solution (0.5 mL), and propanol (2.0 mL) was stirred at 140°C for 1 hour under microwave irradiation. After cooling to room temperature, the reaction solution was partitioned with water and ethyl acetate, and the organic phase was washed with saturated brine and then dried over anhydrous magnesium sulfate. The solvent was distilled off under reduced pressure, and the obtained residue was purified by silica gel column chromatography (hexane-ethyl acetate 90:10-0:100) to obtain 8 mg of the title compound (yield: 23%) as an oil.

$^1$H-NMR ($CDCl_3$) : δ 2.52-2.75 (2H, m), 2.96-3.17 (10H, m), 3.78 (3H, s), 3.87-3.97 (4H, m), 6.53 (1H, d, J = 8.7 Hz), 6.75-6.88 (4H, m), 7.19-7.29 (2H, m), 7.34 (1H, d, J = 8.7 Hz), 8.14-8.24 (2H, m).

Example 15

Ethyl 4-[6-methoxy-2-(4-methylphenyl)pyridin-3-yl]-2-(4-methoxyphenyl)piperazine-1-carboxylate

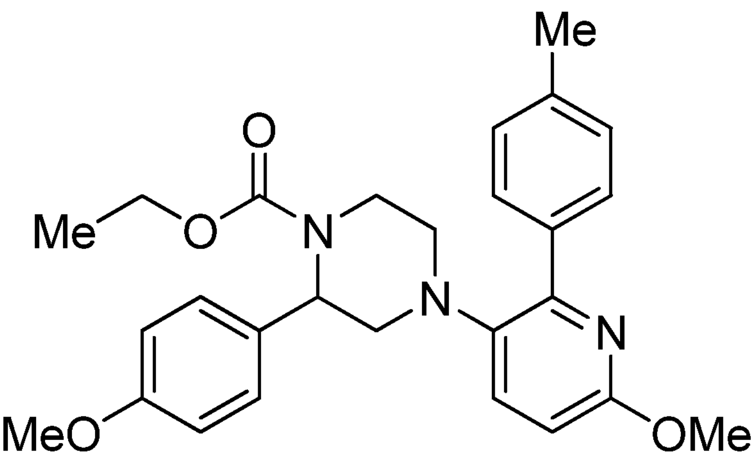

The title compound was obtained in the same way as in Example 1 using 3-bromo-6-methoxy-2-(4-methylphenyl)pyridine (250 mg, 0.899 mmol) synthesized in Reference Example 7 and ethyl 2-(4-methoxyphenyl)piperazine-1-carboxylate (350 mg, 1.32 mmol) synthesized in Reference Example 47. Yield: 29%, oil.

$^1$H-NMR ($CDCl_3$) : δ 1.25 (3H, t, J = 7.2 Hz), 2.33 (3H, s), 2.47-2.60 (1H, m), 2.79-2.89 (1H, m), 2.92-3.05 (1H, m), 3.23 (1H, dd, J = 3.9, 11.7 Hz), 3.51-3.60 (1H, m), 3.77-3.88 (4H, m), 3.93 (3H, s), 4.18 (2H, q, J = 7.2 Hz), 5.38-5.43 (1H, m), 6.67 (1H, d, J = 8.7 Hz), 6.79-6.88 (2H, m), 6.94-7.01 (2H, m), 7.14-7.23 (2H, m), 7.43 (1H, d, J = 8.7 Hz), 7.58-7.65 (2H, m).

Example 16

1-[6-methoxy-2-(4-methylphenyl)pyridin-3-yl]-3-(4-methoxyphenyl)piperazine

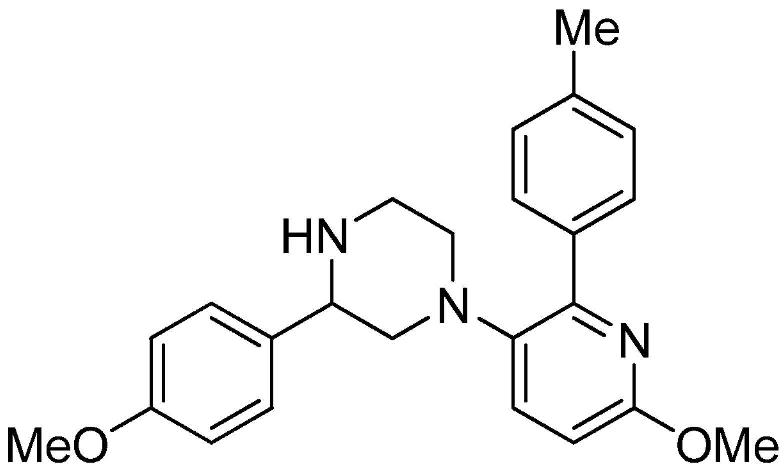

A mixture of ethyl 4-[6-methoxy-2-(4-methylphenyl)pyridin-3-yl]-2-(4-methoxyphenyl)piperazine-1-carboxylate (100 mg, 0.217 mmol) obtained in Example 15, an 8 N aqueous sodium hydroxide solution (0.5 mL), and ethanol (2.0 mL) was stirred for 24 hours under heating to reflux. After cooling to room temperature, the reaction solution was partitioned with water and ethyl acetate, and the organic phase was washed with saturated brine and then dried over anhydrous magnesium sulfate. The solvent was distilled off under reduced pressure, and the residue was then dissolved in propanol (2.0 mL). To the solution, an 8 N aqueous potassium hydroxide solution (0.5 mL) was added, and the mixture was stirred for 24 hours under heating to reflux. After cooling to room temperature, the reaction solution was partitioned with water and ethyl acetate, and the organic phase was washed with saturated brine and then dried over anhydrous magnesium sulfate. The solvent was distilled off under reduced pressure, and the obtained residue was purified by silica gel column chromatography (hexane-ethyl acetate 90:10-0:100) to obtain 20 mg of the title compound (yield: 24%) as an oil.
$^1$H-NMR ($CDCl_3$) : δ 2.42 (3H, s), 2.50-2.62 (1H, m), 2.66-2.79 (1H, m), 2.92-3.11 (4H, m), 3.78 (3H, s), 3.79-3.88 (1H, m), 3.93 (3H, s), 6.61 (1H, d, J = 8.7 Hz), 6.79-6.88 (2H, m), 7.15-7.31 (4H, m), 7.36 (1H, d, J = 8.7 Hz), 8.01-8.12 (2H, m).

Example 17

1-{6-methoxy-2-[4-(1-methylethyl)phenyl]pyridin-3-yl}-3-(4-methoxyphenyl)piperazine

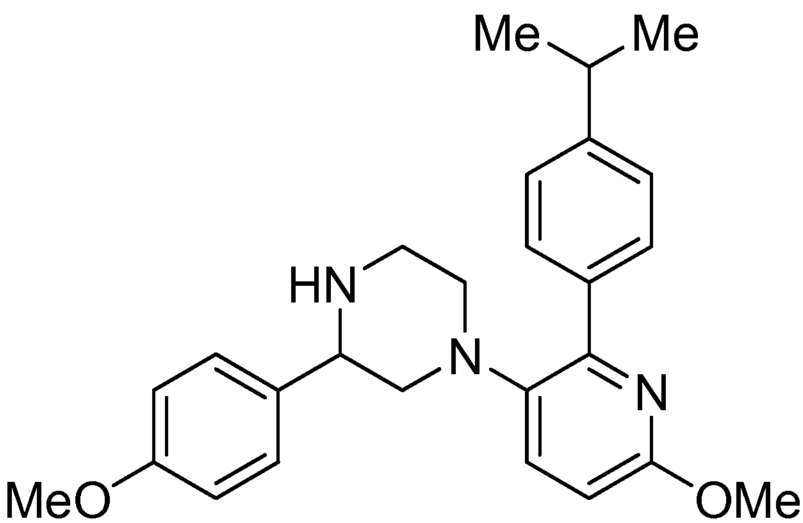

The title compound was obtained in the same way as in Example 1 using 3-bromo-6-methoxy-2-[4-(1-methylethyl)phenyl] pyridine (500 mg, 1.63 mmol) synthesized in Reference Example 11 and 2-(4-methoxyphenyl)piperazine (450 mg, 1.70 mmol). Yield: 22%, oil.

$^1$H-NMR ($CDCl_3$) : δ 1.32 (6H, d, J = 6.6 Hz), 2.46 (1H, dd, J = 10.2, 10.8 Hz), 2.71-2.84 (1H, m), 2.90-3.13 (5H, m), 3.73-3.84 (4H, m), 3.93 (3H, s), 6.61 (1H, d, J = 8.7 Hz), 6.75-6.83 (2H, m), 7.08-7.17 (2H, m), 7.27-7.38 (3H, m), 8.01-8.10 (2H, m).

Example 18

1-acetyl-4-{6-methoxy-2-[4-(1-methylethyl)phenyl]pyridin-3-yl}-2-(4-methoxyphenyl)piperazine

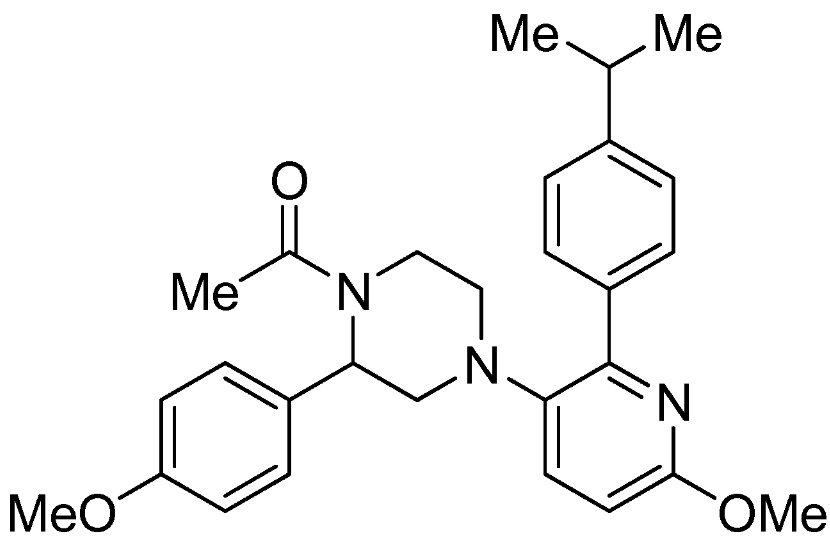

To a THF (2.0 mL) solution of 1-{6-methoxy-2-[4-(1-methylethyl)phenyl]pyridin-3-yl}-3-(4-methoxyphenyl)piperazine synthesized in Example 17, triethylamine (62 mg, 0.610 mmol) and acetyl chloride (48 mg, 0.610 mmol) were added under ice cooling, and the mixture was then heated to room temperature and stirred for 1 hour. The reaction solution was partitioned with water and ethyl acetate, and the organic phase was washed with saturated brine and then dried over anhydrous magnesium sulfate. The solvent was distilled off under reduced pressure, and the obtained residue was purified by silica gel column chromatography (hexane-ethyl acetate 90:10-65:35) to obtain 75 mg of the title compound (yield: 80%) as an oil.

$^1$H-NMR ($CDCl_3$) : δ 1.23 (6H, d, J = 6.9 Hz), 2.15 (3H, s), 2.42-2.57 (1H, m), 2.73-3.00 (2H, m), 3.08-3.49 (2H, m), 3.57-3.70 (1H, m), 3.83 (3H, s), 3.94 (3H, s), 4.20-5.20 (1H, m), 5.70-7.90 (11H, m).

Example 19

4-{6-methoxy-3-[4-(4-methoxyphenyl)piperazin-1-yl]pyridin-2-yl}-N,N-dimethylaniline

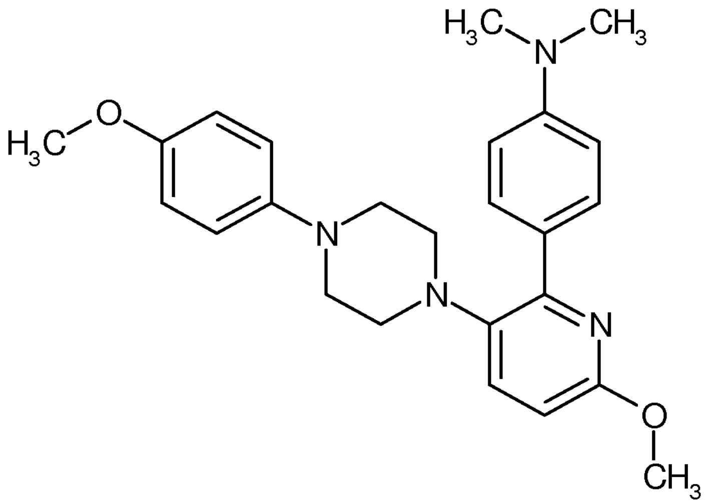

The title compound was obtained in the same way as in Reference Example 31 using 4-(6-methoxy-3-piperazin-1-ylpyridin-2-yl)-N,N-dimethylaniline (200 mg, 0.640 mmol) synthesized in Reference Example 5 and 1-bromo-4-methoxybenzene (240 mg, 1.28 mmol). Specifically, a mixture of 4-(6-methoxy-3-piperazin-1-ylpyridin-2-yl)-N,N-dimethylaniline (200 mg, 0.640 mmol), 1-bromo-4-methoxybenzene (240 mg, 1.28 mmol), palladium acetate (7.19 mg, 0.0320 mmol), BINAP (59.8 mg, 0.0900 mmol), sodium tert-butoxide (185 mg, 1.92 mmol), and toluene (7.0 mL) was stirred at 120°C for 12 hours in an argon atmosphere. After cooling to room temperature, the reaction solution was partitioned with water and ethyl acetate, and the organic phase was dried over anhydrous magnesium sulfate. The solvent was distilled off under reduced pressure, and the obtained residue was purified by silica gel chromatography (hexane-ethyl acetate 100:0-80:20) and crystallized from hexane to obtain 129 mg of the title compound (yield: 48%). Melting point: 163-165°C (hexane).
$^1$H-NMR ($CDCl_3$) : δ 2.95-3.06 (10H, m), 3.10-3.18 (4H, m), 3.77 (3H, s), 3.96 (3H, s), 6.58 (1H, d, J = 8.7 Hz), 6.75 (2H, d, J = 9.0 Hz), 6.86 (2H, d, J = 9.3 Hz), 6.91 (2H, d, J = 9.3 Hz), 7.39 (1H, d, J = 8.7 Hz), 8.18 (2H, d, J = 9.0 Hz).

Example 20

4-{3-[4-(2-fluoro-4-methoxyphenyl)piperazin-1-yl]-6-methoxypyridin-2-yl}-N,N-dimethylaniline

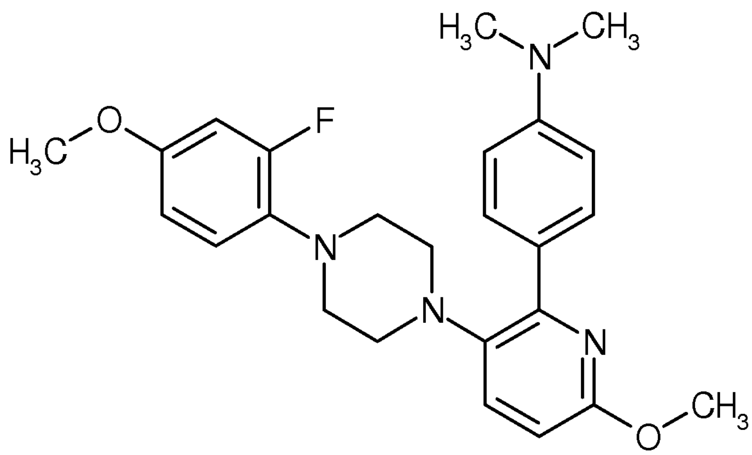

The title compound was obtained in the same way as in Reference Example 4 using 4-(6-methoxy-3-piperazin-1-ylpyridin-2-yl)-N,N-dimethylaniline (250 mg, 0.800 mmol) synthesized in Reference Example 5 and 1-bromo-2-fluoro-4-methoxybenzene (328 mg, 1.60 mmol). Yield: 52%. Melting point: 160-162°C (ethyl acetate-hexane).
$^1$H-NMR ($CDCl_3$) : δ 2.97-3.11 (14H, m), 3.76 (3H, s), 3.96 (3H, s) 6.56-6.70 (3H, m), 6.77 (2H, d, J = 8.7 Hz), 6.93 (1H, t, J = 9.5 Hz), 7.41 (1H, d, J = 8.7 Hz), 8.19 (2H, d, J = 9.1 Hz).

Example 21

4-{3-[4-(3-fluoro-4-methylphenyl)piperazin-1-yl]-6-methoxypyridin-2-yl}-N,N-dimethylaniline

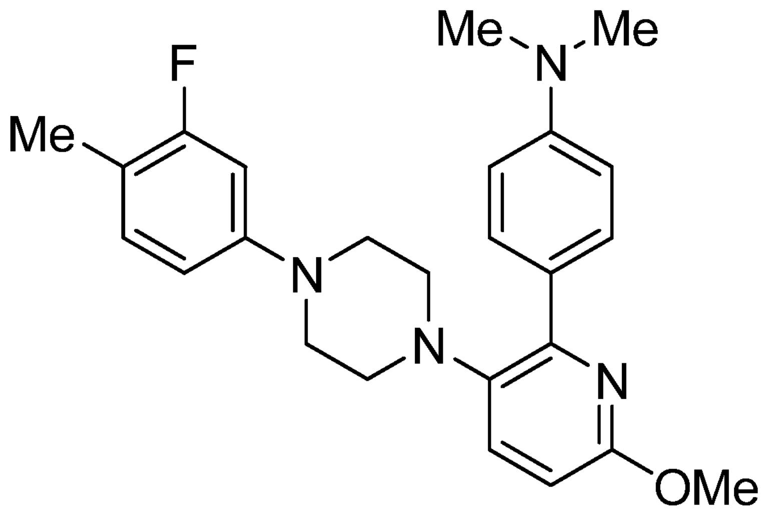

The title compound was obtained in the same way as in Reference Example 31 using 4-(6-methoxy-3-piperazin-1-ylpyridin-2-yl)-N,N-dimethylaniline (100 mg, 0.320 mmol) synthesized in Reference Example 5 and 4-bromo-2-fluoro-1-methylbenzene (90.7 mg, 0.480 mmol). Yield: 52%. Melting point: 183-186°C (ethyl acetate-hexane).
$^1$H-NMR ($CDCl_3$) : δ 2.28 (3H, s), 2.94-3.07 (10H, m), 3.06-3.18 (4H, m), 3.95 (3H, s), 6.58 (1H, d, J = 8.7 Hz), 6.76 (2H, d, J = 9.1 Hz), 6.81-6.94 (3H, m), 7.41 (1H, d, J = 8.3 Hz), 8.19 (2H, d, J = 9.1 Hz).

Example 22

4-{6-methoxy-3-[4-(5-methylpyridin-2-yl)piperazin-1-yl]pyridin-2-yl}-N,N-dimethylaniline

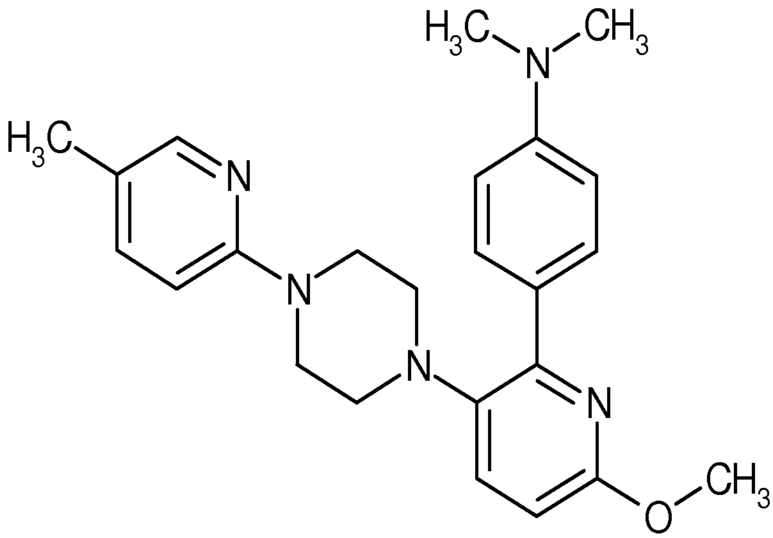

The title compound was obtained in the same way as in Reference Example 31 using 4-(6-methoxy-3-piperazin-1-ylpyridin-2-yl)-N,N-dimethylaniline (100 mg, 0.320 mmol) synthesized in Reference Example 5 and 2-bromo-5-methylpyridine (82.6 mg, 0.480 mmol). Yield: 48%. Melting point: 153-155°C (ethyl acetate-hexane).

$^1$H-NMR ($CDCl_3$) : δ 2.20 (3H, s), 2.90-3.03 (10H, m), 3.46-3.62 (4H, m), 3.95 (3H, s), 6.53-6.63 (2H, m), 6.75 (2H, d, J = 9.1 Hz), 7.32 (1H, dd, J = 8.5, 2.5 Hz), 7.37 (1H, d, J = 8.7 Hz), 8.03 (1H, d, J = 2.7 Hz), 8.19 (2H, d, J = 9.1 Hz).

Example 23

2-(4-{2-[4-(dimethylamino)phenyl]-6-methoxypyridin-3-yl}piperazin-1-yl)-5-methylbenzonitrile

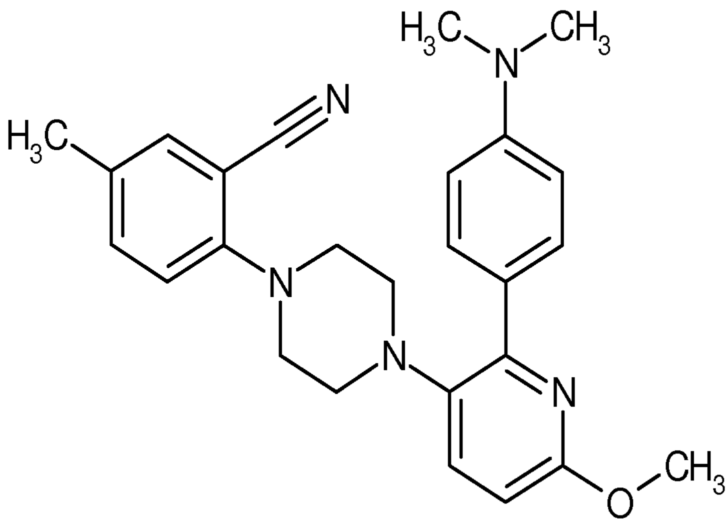

The title compound was obtained in the same way as in Reference Example 31 using 4-(6-methoxy-3-piperazin-1-ylpyridin-2-yl)-N,N-dimethylaniline (100 mg, 0.320 mmol) synthesized in Reference Example 5 and 2-bromo-5-methylbenzonitrile (94.1 mg, 0.480 mmol). Yield: 48%. Melting point: 158-162°C (ethyl acetate-hexane).
$^1$H-NMR ($CDCl_3$):δ 2.30 (3H, s), 2.98-3.11 (10H, m), 3.15-3.34 (4H, m), 3.96 (3H, s), 6.59 (1H, d, J = 8.7 Hz), 6.77 (2H, d, J = 9.0 Hz), 6.93 (1H, d, J = 8.3 Hz), 7.27-7.32 (1H, m), 7.37 (1H, d, J = 1.9 Hz), 7.42 (1H, d, J = 8.7 Hz), 8.16 (2H, d, J = 9.0 Hz).

Example 24

4-{3-[4-(4-chlorophenyl)piperazin-1-yl]-6-methoxypyridin-2-yl}-N,N-dimethylaniline

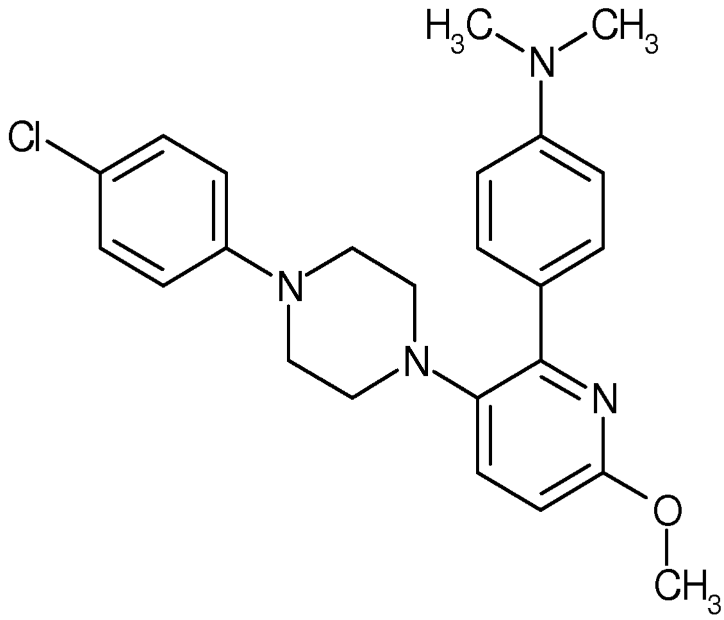

A mixture of 4-(3-bromo-6-methoxypyridin-2-yl)-N,N-dimethylaniline (300 mg, 0.977 mmol) synthesized in Reference Example 3, 1-(4-chlorophenyl)piperazine (288 mg, 1.47 mmol), tris(dibenzylideneacetone)dipalladium (0) (27.0 mg, 0.0295 mmol), Xantphos (52.0 mg, 0.0899 mmol), sodium tert-butoxide (192 mg, 1.95 mmol), and toluene (2.0 mL) was stirred at 120°C for 2 days. After cooling to room temperature, the reaction solution was partitioned with water and dichloromethane, and the organic phase was washed with saturated brine and then dried over anhydrous magnesium sulfate. The solvent was distilled off under reduced pressure, and the obtained residue was purified by silica gel column chromatography (hexane-ethyl acetate 15:1) to obtain 145 mg of the title compound (yield: 35%) as an amorphous solid. $^1$H-NMR ($CDCl_3$) : δ 2.98-3.01 (4H, m), 3.01 (6H, s), 3.20 (4H, t, J = 4.4 Hz), 3.96 (3H, s), 6.58 (1H, d, J = 8.4 Hz), 6.75 (2H, d, J = 8.4 Hz), 6.86 (2H, d, J = 8.8 Hz), 7.21 (2H, d, J = 8.8 Hz), 7.38 (1H, d, J = 8.4 Hz), 8.17 (2H, d, J = 8.8 Hz).

Example 25

4-{6-methoxy-3-[4-(4-methylphenyl)piperazin-1-yl]pyridin-2-yl}-N,N-dimethylaniline

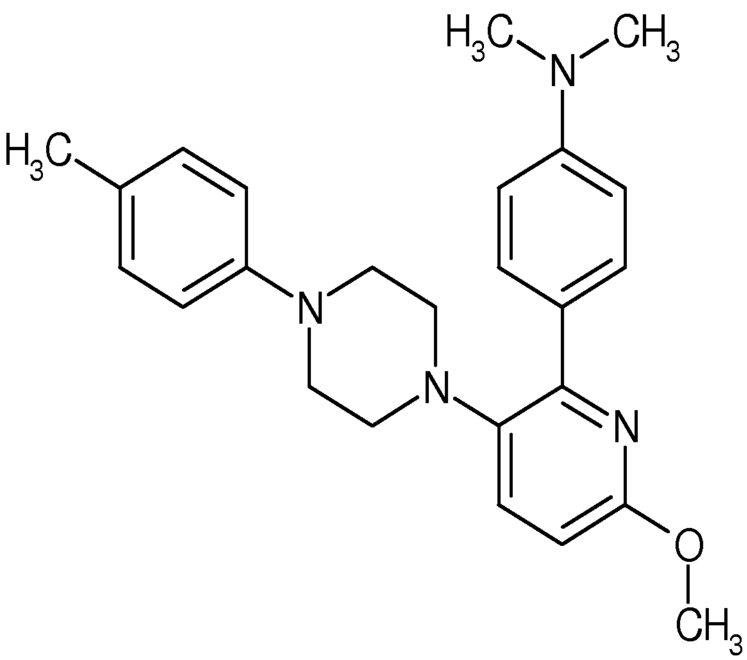

The title compound was obtained in the same way as in Reference Example 31 using 4-(3-bromo-6-methoxypyridin-2-yl)-N,N-dimethylaniline (307 mg, 1.00 mmol) synthesized in Reference Example 3 and 1-(4-methylphenyl)piperazine (353 mg, 2.00 mmol). Specifically, a mixture of 4-(3-bromo-6-methoxypyridin-2-yl)-N,N-dimethylaniline (307 mg, 1.00 mmol), 1-(4-methylphenyl)piperazine (353 mg, 2.00 mmol), palladium acetate (11.2 mg, 0.0500 mmol), BINAP (93.4 mg, 0.150 mmol), sodium tert-butoxide (288 mg, 3.00 mmol), and toluene (7.0 mL) was stirred at 120°C for 12 hours in an argon atmosphere. After cooling to room temperature, the reaction solution was partitioned with water and ethyl acetate, and the organic phase was dried over anhydrous magnesium sulfate. The solvent was distilled off under reduced pressure, and the obtained residue was purified by silica gel chromatography (hexane-ethyl acetate 100:0-95:5) and crystallized from hexane to obtain 150 mg of the title compound (yield: 37%). Melting point: 170-173°C (hexane).
$^1$H-NMR ($CDCl_3$) : δ 2.27 (3H, s), 2.94-3.05 (10H, m), 3.13-3.25 (4H, m) 3.96 (3H, s), 6.58 (1H, d, J = 8.7 Hz), 6.74 (2H, d, J = 9.4 Hz), 6.86 (2H, d, J = 8.7 Hz), 7.08 (2H, d, J = 8.7 Hz), 7.39 (1H, d, J = 8.7 Hz), 8.18 (2H, d, J = 9.4 Hz).

Example 26

4-{3-[4-(3,4-dimethoxyphenyl)piperazin-1-yl]-6-methoxypyridin-2-yl}-N,N-dimethylaniline

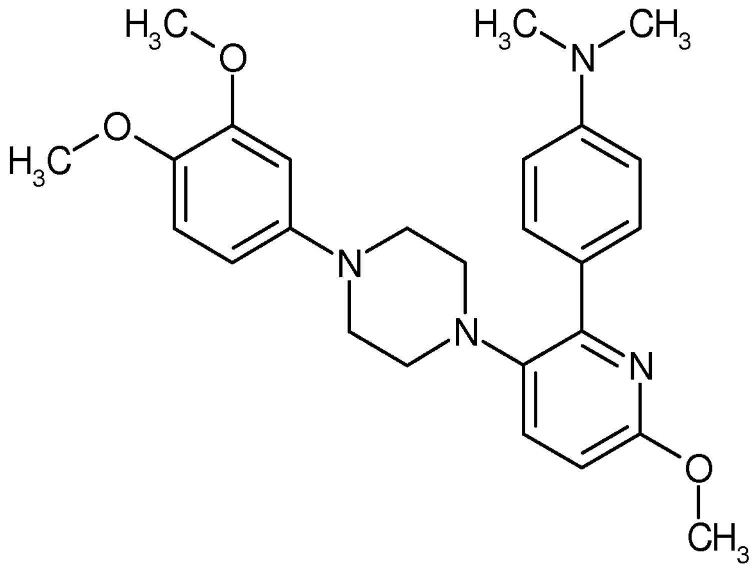

The title compound was obtained in the same way as in Example 24 using 4-(3-bromo-6-methoxypyridin-2-yl)-N,N-dimethylaniline (500 mg, 1.63 mmol) synthesized in Reference Example 3 and 1-(3,4-dimethoxyphenyl)piperazine (632 mg, 2.44 mmol). Yield: 25%, amorphous solid.
$^1$H-NMR ($CDCl_3$) : δ 3.01 (6H, s), 3.01 (4H, brs), 3.16 (4H, brs), 3.85 (3H, s), 3.87 (3H, s), 3.96 (3H, s), 6.48 (1H, d, J = 8.8 Hz), 6.59 (2H, d, J = 8.4 Hz), 6.75 (2H, d, J = 8.8 Hz), 6.81 (1H, d, J = 8.4 Hz), 7.40 (1H, d, J = 8.8 Hz), 8.19 (2H, d, J = 8.8 Hz).

Example 27

4-[6-methoxy-2-(4-methylphenyl)pyridin-3-yl]-2-(4-methoxyphenyl)-1-methylpiperazine

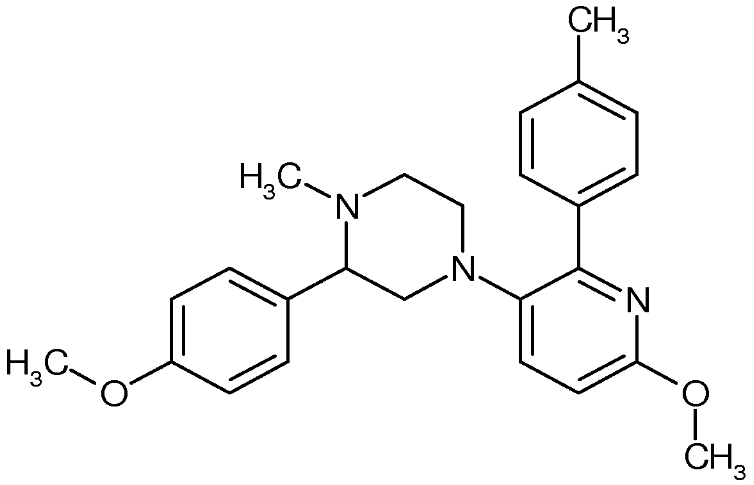

A mixture of 3-bromo-6-methoxy-2-(4-methylphenyl)pyridine (500 mg, 1.79 mmol) synthesized in Reference Example 7, 2-(4-methoxyphenyl)-1-methylpiperazine (464 mg, 2.25 mmol), tris(dibenzylideneacetone)dipalladium (0) (82.0 mg, 0.0895 mmol), BINAP (112 mg, 0.180 mmol), sodium tert-butoxide (518 mg, 5.39 mmol), and toluene (6.0 mL) was stirred at 140°C for 5 hour under microwave irradiation. After cooling to room temperature, the reaction solution was partitioned with water and ethyl acetate, and the organic phase was dried over anhydrous magnesium sulfate. The solvent was distilled off under reduced pressure, and the obtained residue was purified by reverse-phase high-performance liquid chromatography (C18, mobile phase: water-acetonitrile 35:65-5:95) to obtain 150 mg of the title compound (yield: 21%) as an oil.
$^{1}$H-NMR ($CDCl_3$): δ 2.04 (3H, s), 2.35-2.41 (1H, m), 2.43 (3H, s), 2.64 (1H, t, J = 11.0 Hz), 2.81-2.92 (3H, m), 2.99-3.03 (2H, m), 3.77 (3H, s), 3.92 (3H, s), 6.59 (1H, d, J = 8.8 Hz), 6.83 (2H, d, J = 8.8 Hz), 7.17 (2H, d, J = 7.6 Hz), 7.25 (2H, d, J = 7.6 Hz), 7.34 (1H, d, J = 8.8 Hz), 8.03 (2H, d, J = 8.4 Hz).

Example 28

4-{6-methoxy-3-[3-(4-methoxyphenyl)-4-methylpiperazin-1-yl]pyridin-2-yl}-N,N-dimethylaniline

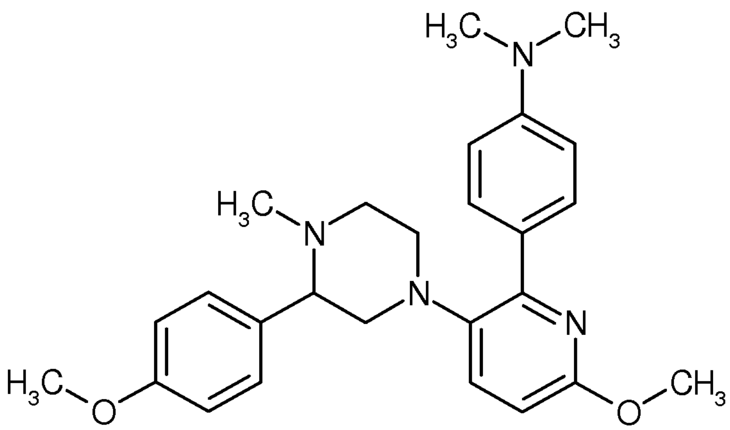

The title compound was obtained in the same way as in Example 1 using 4-(3-bromo-6-methoxypyridin-2-yl)-N,N-dimethylaniline (1.00 g, 3.26 mmol) synthesized in Reference Example 3 and 2-(4-methoxyphenyl)-1-methylpiperazine (1.01 g, 4.88 mmol). Yield: 29%. Melting point: 178-181°C (ethyl acetate-hexane).
$^{1}$H-NMR ($CDCl_3$): δ 2.11 (3H, s), 2.38-2.61 (1H, m), 3.04 (12H, s), 3.79 (3H, s), 3.93 (3H, s), 6.52 (1H, d, J = 8.3 Hz), 6.76-6.89 (4H, m), 7.16-7.28 (2H, m), 7.32 (1H, d, J = 8.7 Hz), 8.16 (2H, d, J = 8.7 Hz).

Example 29

1-[6-methoxy-2-(4-methylphenyl)pyridin-3-yl]-4-(4-methylphenyl)piperazine

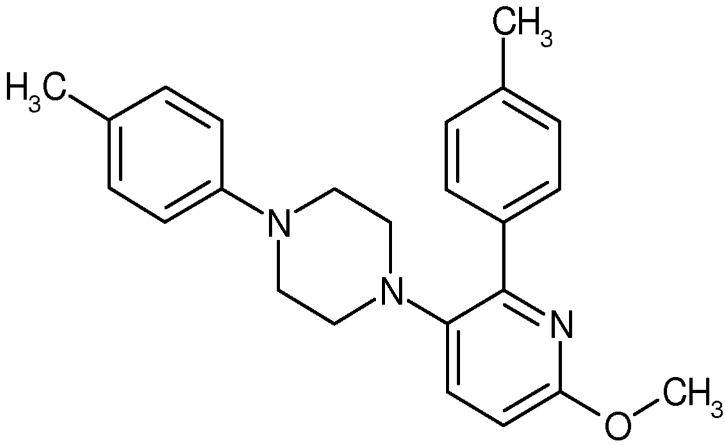

The title compound was obtained in the same way as in Reference Example 31 using 3-bromo-6-methoxy-2-(4-methylphenyl)pyridine (4.00 g, 14.4 mmol) synthesized in Reference Example 7 and 1-(4-methylphenyl)piperazine (4.67 g, 28.8 mmol). Yield: 65%. Melting point: 145-148°C (ethyl acetate-hexane).
$^1$H-NMR ($CDCl_3$) : δ 2.27 (3H, s), 2.38 (3H, s), 2.90-3.05 (4H, m), 3.09-3.21 (4H, m), 3.95 (3H, s), 6.66 (1H, d, J = 8.7 Hz), 6.84 (2H, d, J = 8.7 Hz), 7.08 (2H, d, J = 7.9 Hz), 7.20 (2H, d, J = 7.9 Hz), 7.42 (1H, d, J = 8.7 Hz), 8.05 (2H, d, J = 7.9 Hz).

Example 30

4-{3-[4-(4-ethoxyphenyl)piperazin-1-yl]-6-methoxypyridin-2-yl}-N,N-dimethylaniline

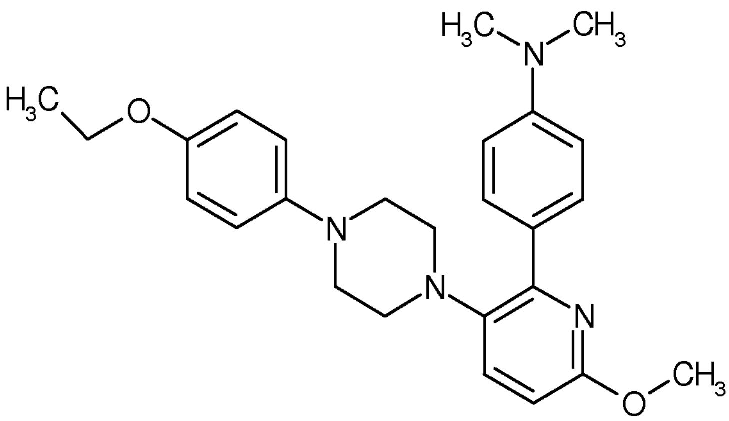

The title compound was obtained in the same way as in Reference Example 31 using 4-(3-bromo-6-methoxypyridin-2-yl)-N,N-dimethylaniline (307 mg, 1.00 mmol) synthesized in Reference Example 3 and 1-(4-ethoxyphenyl)piperazine (413 mg, 2.00 mmol). Yield: 30%. Melting point: 150-155°C (ethyl acetate-heptane).
$^1$H-NMR ($CDCl_3$) : δ 1.39 (3H, t, J = 7.0 Hz), 2.96-3.05 (10H, m), 3.09-3.18 (4H, m), 3.91-4.05 (5H, m), 6.58 (1H, d, J = 8.7 Hz), 6.75 (2H, d, J = 9.0 Hz), 6.80-6.86 (2H, m), 6.88-6.94 (2H, m), 7.40 (1H, d, J = 8.7 Hz), 8.18 (2H, d, J = 9.0 Hz).

Example 31

4-{3-[4-(3,4-difluorophenyl)piperazin-1-yl]-6-methoxypyridin-2-yl}-N,N-dimethylaniline

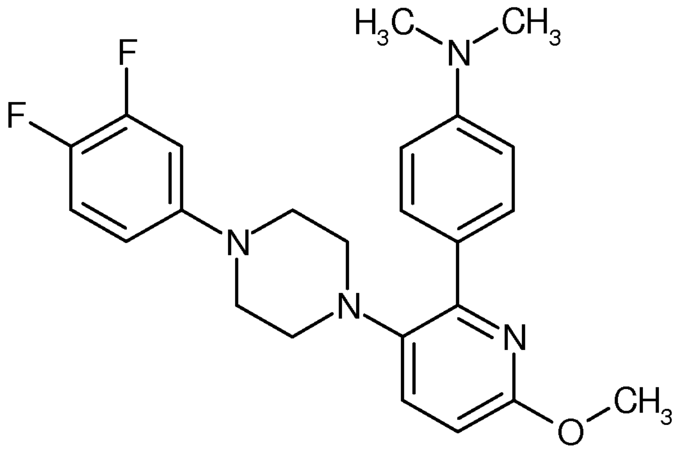

The title compound was obtained in the same way as in Reference Example 31 using 4-(3-bromo-6-methoxypyridin-2-yl)-N,N-dimethylaniline (307 mg, 1.00 mmol) synthesized in Reference Example 3 and 1-(3,4-difluorophenyl)piperazine (396 mg, 2.00 mmol). Yield: 9.6%. Melting point: 155-157°C (ethyl acetate-heptane).
$^1$H-NMR ($CDCl_3$) : δ 2.92-3.04 (10H, m), 3.11-3.20 (4H, m), 3.96 (3H, s), 6.55-6.64 (2H, m), 6.66-6.79 (3H, m), 6.97-7.10 (1H, m), 7.38 (1H, d, J = 8.3 Hz), 8.15 (2H, d, J = 9.0 Hz).

Example 32

1-[2-(4-ethoxyphenyl)-6-methoxypyridin-3-yl]-4-(4-fluorophenyl)piperazine

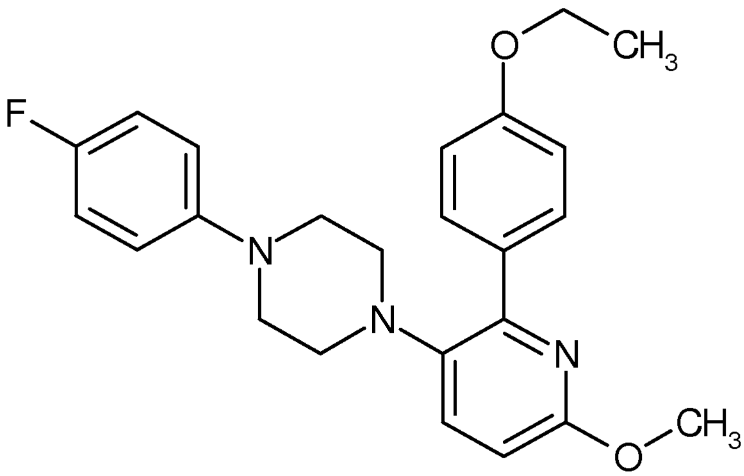

The title compound was obtained in the same way as in Reference Example 31 using 3-bromo-2-(4-ethoxyphenyl)-6-methoxypyridine (308 mg, 1.00 mmol) synthesized in Reference Example 9 and 1-(4-fluorophenyl)piperazine (360 mg, 2.00 mmol). Yield: 8.8%. Melting point: 134-137°C (ethyl acetate-heptane).
$^1$H-NMR ($CDCl_3$) : δ 1.44 (3H, t, J = 7.0 Hz), 2.94-3.02 (4H, m), 3.08-3.17 (4H, m), 3.96 (3H, s), 4.08 (2H, q, J = 7.2 Hz), 6.64 (1H, d, J = 8.7 Hz), 6.82-7.05 (6H, m), 7.42 (1H, d, J = 8.7 Hz), 8.13 (2H, d, J = 9.0 Hz).

Example 33

1-(3,4-difluorophenyl)-4-[2-(4-ethoxyphenyl)-6-methoxypyridin-3-yl]piperazine

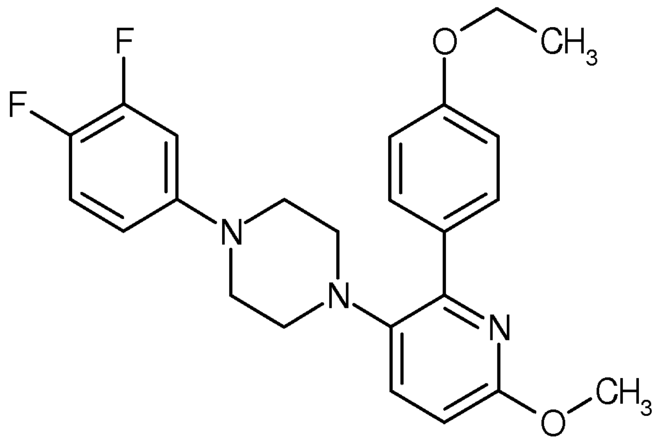

The title compound was obtained in the same way as in Reference Example 31 using 3-bromo-2-(4-ethoxyphenyl)-6-methoxypyridine (308 mg, 1.00 mmol) synthesized in Reference Example 9 and 1-(3,4-difluorophenyl)piperazine (396 mg, 2.00 mmol). Yield: 7.1%. Melting point: 101-104°C (ethyl acetate-heptane).
$^1$H-NMR ($CDCl_3$) : δ 1.44 (3H, t, J = 7.0 Hz), 2.90-3.01 (4H, m), 3.07-3.17 (4H, m), 3.95 (3H, s), 4.08 (2H, q, J = 7.2 Hz), 6.54-6.77 (3H, m), 6.93 (2H, d, J = 8.7 Hz), 6.97-7.11 (1H, m), 7.40 (1H, d, J = 8.7 Hz), 8.11 (2H, d, J = 9.0 Hz).

Example 34

4-{6-methoxy-3-[3-(4-methoxybenzyl)-4-methylpiperazin-1-yl]pyridin-2-yl}-N,N-dimethylaniline

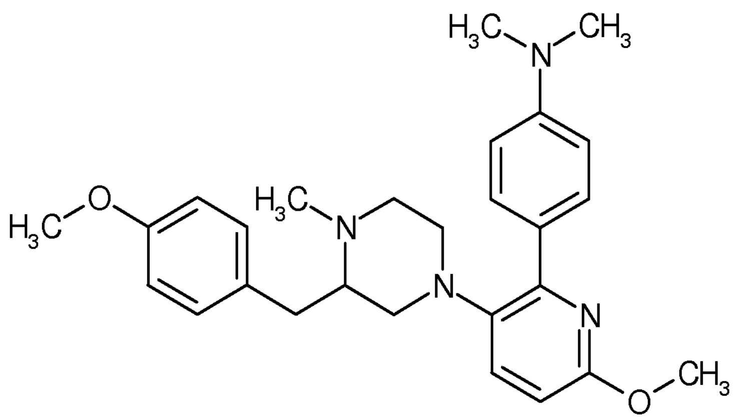

The title compound was obtained in the same way as in Example 1 using 4-(3-bromo-6-methoxypyridin-2-yl)-N,N-dimethylaniline (260 mg, 0.846 mmol) synthesized in Reference Example 3 and 2-(4-methoxybenzyl)-1-methylpiperazine (330 mg, 1.50 mmol) synthesized in Reference Example 30. Oil.
$^1$H-NMR ($CDCl_3$) : δ 2.24-2.54 (7H, m), 2.71-3.13 (11H, m), 3.76 (3H, s), 3.91 (3H, s), 6.51 (1H, d, J = 8.7 Hz), 6.69 (2H, d, J = 1.9 Hz), 6.72 (2H, d, J = 2.3 Hz), 6.89 (2H, d, J = 8.7 Hz), 7.22-7.30 (1H, m), 8.01 (2H, d, J = 9.0 Hz).

Example 35

1-(4-methoxyphenyl)-4-[2-(4-methylphenyl)pyridin-3-yl]piperazine

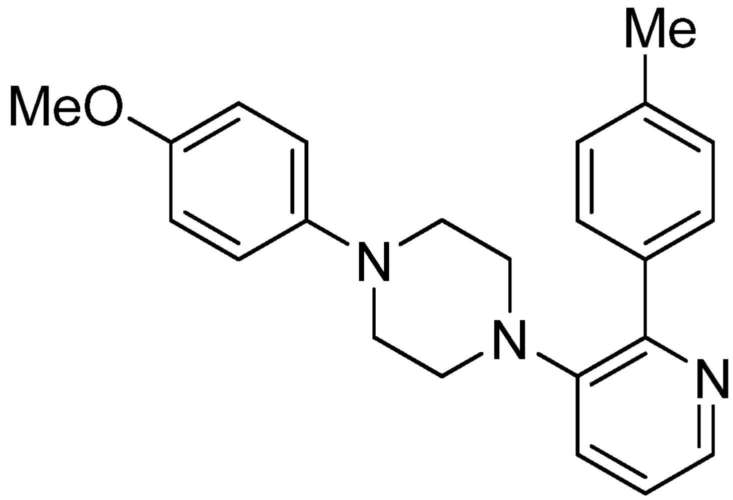

The title compound was obtained in the same way as in Example 1 using 3-bromo-2-(4-methylphenyl)pyridine (1.00 g, 4.03 mmol) synthesized in Reference Example 24 and 1-(4-methoxyphenyl)piperazine (1.16 g, 6.05 mmol). Yield: 48%. Amorphous solid.

$^1$H-NMR ($CDCl_3$) : δ 2.38 (3H, s), 2.98-3.14 (8H, m), 3.77 (3H, s), 6.82-6.91 (4H, m), 7.16-7.72 (3H, m), 7.34-7.40 (1H, m), 7.88 (2H, d, J = 8.4 Hz), 8.32-8.40 (1H, m).

Example 36

1-[6-bromo-2-(4-methylphenyl)pyridin-3-yl]-4-(4-methoxyphenyl)piperazine

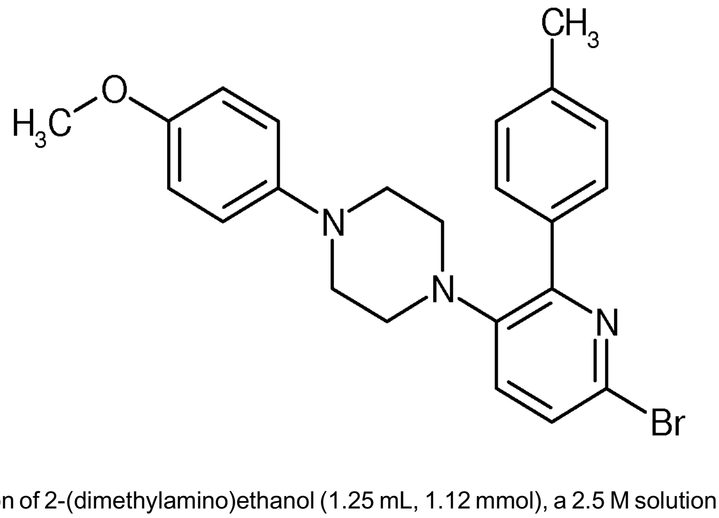

To a hexane (5 mL) solution of 2-(dimethylamino)ethanol (1.25 mL, 1.12 mmol), a 2.5 M solution of butyllithium in hexane (10.0 mL, 25.0 mmol) was slowly added dropwise at 0°C, and the mixture was stirred at 0°C for 30 minutes. A hexane (5 mL) solution of 1-(4-methoxyphenyl)-4-[2-(4-methylphenyl)pyridin-3-yl]piperazine (5.18 g, 14.4 mmol) synthesized in Example 35 was added dropwise thereto, and the mixture was stirred at 0°C for 1 hour. After cooling to -78°C, a hexane (5 mL) solution of tetrabromomethane (4.84 g, 14.6 mmol) was added dropwise thereto, and the mixture was stirred at -78°C for 1 hour and then raised the temperature to room temperature. The reaction was stopped by addition of water, followed by extraction with ethyl acetate. The obtained organic phase was dried over anhydrous magnesium sulfate.

The solvent was distilled off under reduced pressure, and the obtained residue was purified by silica gel column chromatography (hexane-ethyl acetate 6:1) to obtain 1.05 g of the title compound (yield: 57%). Amorphous solid.
$^1$H-NMR ($CDCl_3$): δ 2.37 (3H, s), 2.99-3.08 (8H, m), 3.77 (3H, s), 6.83-6.90 (4H, m), 7.22 (3H, t, J = 8.8 Hz), 7.33 (1H, t, J = 8.4 Hz), 7.89 (2H, d, J = 8.0 Hz).

Example 37

N-benzyl-5-[4-(4-methoxyphenyl)piperazin-1-yl]-6-(4-methylphenyl)pyridin-2-amine

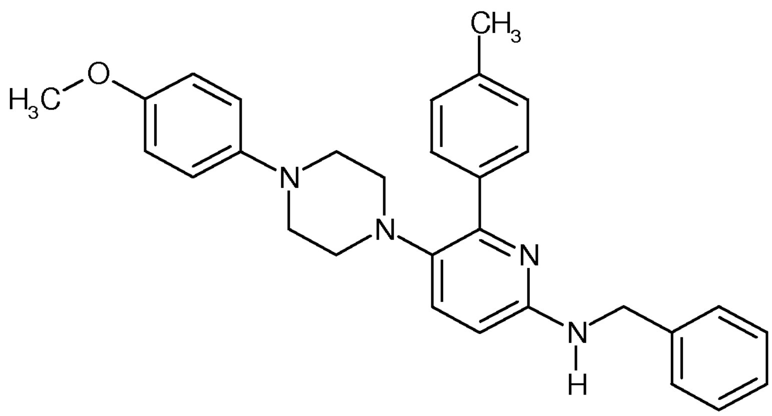

The title compound was obtained in the same way as in Reference Example 31 using 1-[6-bromo-2-(4-methylphenyl) pyridin-3-yl]-4-(4-methoxyphenyl)piperazine (1.14 g, 2.60 mmol) synthesized in Example 36 and benzylamine (557 mg, 5.20 mmol). Yield: 79%. Melting point: 145-151°C (ethyl acetate-heptane).
$^1$H-NMR ($CDCl_3$) : δ 2.36 (3H, s), 2.88-2.98 (4H, m), 3.00-3.08 (4H, m), 3.77 (3H, s), 4.50 (2H, d, J = 5.7 Hz), 4.83 (1H, brs), 6.32 (1H, d, J = 8.7 Hz), 6.80-6.86 (2H, m), 6.86-6.93 (2H, m), 7.17 (2H, d, J = 8.0 Hz), 7.21-7.43 (6H, m), 7.92 (2H, d, J = 8.0 Hz).

Example 38

5-[4-(4-methoxyphenyl)piperazin-1-yl]-6-(4-methylphenyl)pyridin-2-amine

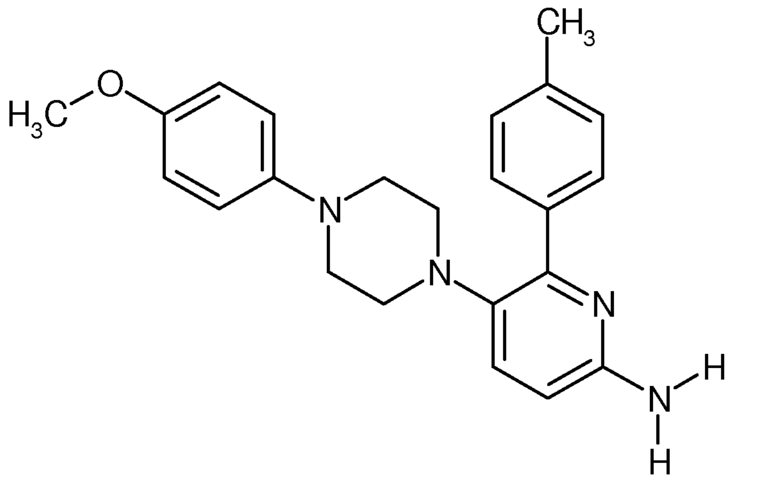

To N-benzyl-5-[4-(4-methoxyphenyl)piperazin-1-yl]-6-(4-methylphenyl)pyridin-2-amine (200 mg, 0.430 mmol) synthesized in Example 37, concentrated sulfuric acid (1 mL) was added, and the mixture was stirred at room temperature for

12 hours. The reaction was stopped by addition of a saturated aqueous solution of sodium bicarbonate, followed by extraction with ethyl acetate. The obtained organic phase was dried over anhydrous magnesium sulfate. The solvent was distilled off under reduced pressure, and the residue was crystallized from an ethyl acetate-heptane solvent system to obtain 91.5 mg of the title compound (yield: 57%). Melting point: 193-200°C.
$^1$H-NMR ($CDCl_3$) : δ 2.36 (3H, s), 2.88-2.97 (4H, m), 2.99-3.08 (4H, m), 3.77 (3H, s), 4.39 (2H, brs), 6.47 (1H, d, J = 8.7 Hz), 6.80-6.86 (2H, m), 6.86-6.91 (2H, m), 7.18 (2H, d, J = 7.9 Hz), 7.33 (1H, d, J = 8.3 Hz), 7.85 (2H, d, J = 7.9 Hz).

Example 39

1-(4-methylphenyl)-4-[2-(4-methylphenyl)pyridin-3-yl]piperazine

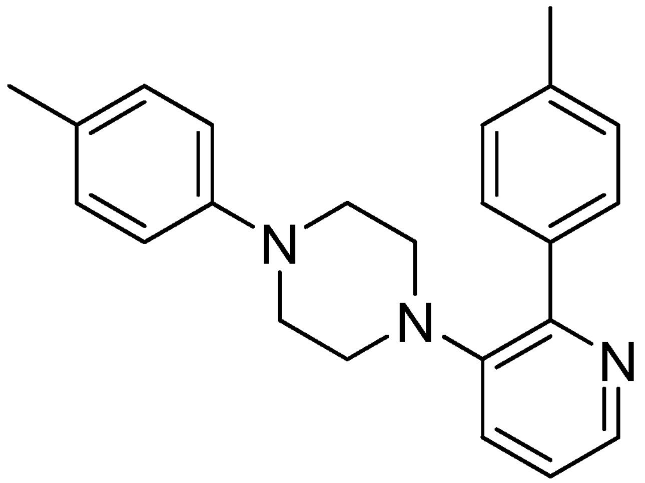

The title compound was obtained in the same way as in Example 1 using 3-bromo-2-(4-methylphenyl)pyridine (1.00 g, 4.03 mmol) synthesized in Reference Example 24 and 1-(4-methylphenyl)piperazine (1.07 g, 6.05 mmol). Yield: 48%. Amorphous solid.
$^1$H-NMR ($CDCl_3$) : δ 2.26 (3H, s), 2.36 (3H, s), 2.96-3.04 (4H, m), 3.07-3.14 (4H, m), 6.82 (2H, d, J = 8.4 Hz), 7.06 (2H, d, J = 8.4 Hz), 7.15 (1H, dd, J = 8.2, 4.6 Hz), 7.21 (2H, d, J = 8.4 Hz), 7.33 (1H, dd, J = 8.2, 1.4 Hz), 7.88 (2H, d, J = 8.0 Hz), 8.34 (1H, dd, J = 4.6, 1.4 Hz).

Example 40

1-[6-bromo-2-(4-methylphenyl)pyridin-3-yl]-4-(4-methylphenyl)piperazine

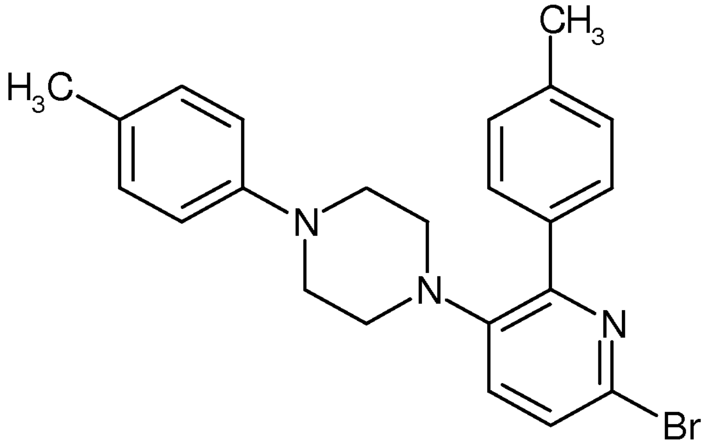

The title compound was obtained in the same way as in Example 36 using 1-(4-methylphenyl)-4-[2-(4-methylphenyl)pyridin-3-yl]piperazine (1.50 g, 4.37 mmol) synthesized in Example 39. Yield: 57%. Amorphous solid.
$^{1}$H-NMR ($CDCl_3$) : δ 2.27 (3H, s), 2.37 (3H, s), 2.96-3.02 (4H, m), 3.08-3.16 (4H, m), 6.82 (2H, d, J = 8.4 Hz), 7.07 (2H, d, J = 8.8 Hz), 7.21 (3H, t, J = 8.8 Hz), 7.32 (1H, d, J = 8.4 Hz), 7.89 (2H, d, J = 8.0 Hz).

Example 41

N-benzyl-6-(4-methylphenyl)-5-[4-(4-methylphenyl)piperazin-1-yl]pyridin-2-amine

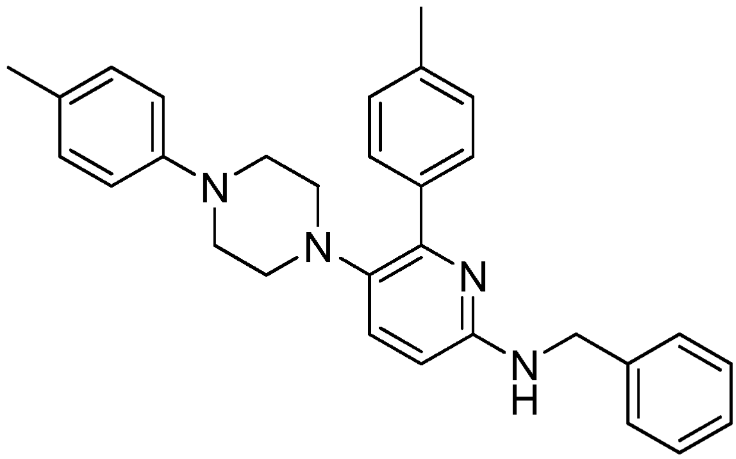

The title compound was obtained in the same way as in Reference Example 31 using 1-[6-bromo-2-(4-methylphenyl)pyridin-3-yl]-4-(4-methylphenyl)piperazine (1.00 g, 2.37 mmol) synthesized in Example 40 and benzylamine (317 mg, 2.96 mmol). Yield: 40%, amorphous solid.
$^{1}$H-NMR ($CDCl_3$) : δ 2.26 (3H, s), 2.36 (3H, s), 2.88-2.96 (4H,m), 3.06-3.16 (4H, m), 4.49 (2H, d, J = 5.6 Hz), 6.31 (1H, d, J = 8.4 Hz), 6.83 (2H, dd, J = 6.4, 2.0 Hz), 7.06 (2H, d, J = 8.4 Hz), 7.17 (2H, d, J = 8.0 Hz), 7.29-7.41 (6H, m), 7.91 (2H, dd, J = 6.4, 1.6 Hz).

Example 42

6-(4-methylphenyl)-5-[4-(4-methylphenyl)piperazin-1-yl]pyridin-2-amine

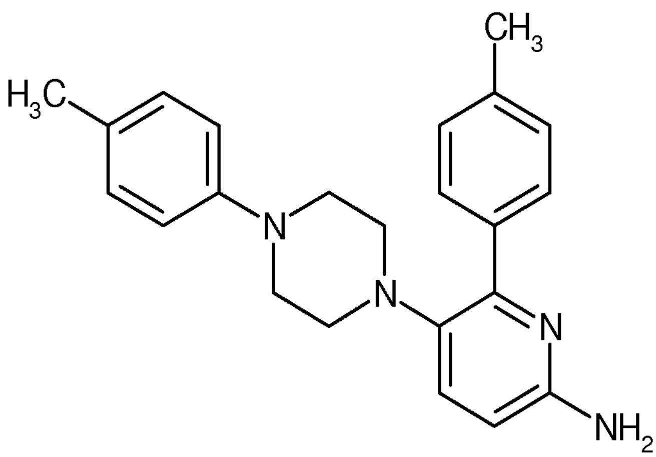

The title compound was obtained in the same way as in Example 38 using N-benzyl-6-(4-methylphenyl)-5-[4-(4-methylphenyl)piperazin-1-yllpyridin-2-amine (100 mg, 0.233 mmol) synthesized in Example 41. Yield: 88%. Amorphous solid.

$^1$H-NMR ($CDCl_3$) : δ 2.26 (3H, s), 2.36 (3H, s), 2.86-2.98 (4H, m), 3.04-3.18 (4H, m), 4.27 (2H, s), 6.46 (1H, d, J = 8.8 Hz), 6.82 (2H, d, J = 8.4 Hz), 7.06 (2H, d, J = 8.0 Hz), 7.17 (2H, d, J = 8.0 Hz), 7.30 (1H, d, J = 8.8 Hz), 7.85 (2H, d, J = 8.0 Hz).

Example 43

1-[6-methyl-2-(4-methylphenyl)pyridin-3-yl]-4-(4-methylphenyl)piperazine

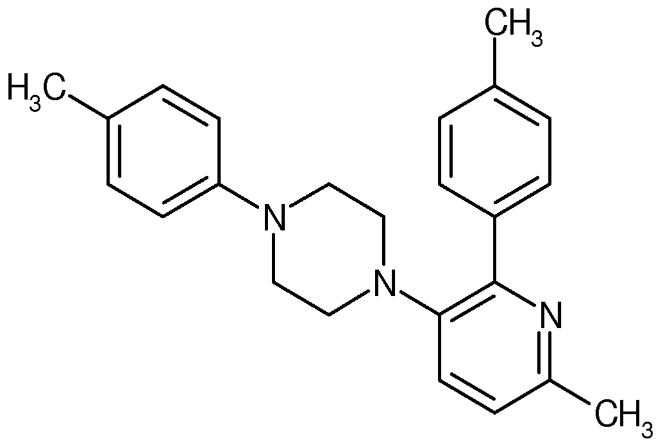

To a 1,4-dioxane (50 mL) solution of 1-[6-bromo-2-(4-methylphenyl)pyridin-3-yl]-4-(4-methylphenyl)piperazine (1.40 g, 3.31 mmol) synthesized in Example 40, a 50% solution of trimethylboroxine in THF (1.41 mL, 4.97 mmol), tetrakis (triphenylphosphine)palladium (192 mg, 0.166 mmol), and potassium carbonate (916 mg, 6.63 mmol) were added, and the mixture was stirred at 100°C for 16 hours in a nitrogen atmosphere. After cooling to room temperature, water was added to the mixed solution, followed by extraction with dichloromethane. The extracts were dried over anhydrous magnesium sulfate.
The solvent was distilled off under reduced pressure, and the obtained residue was purified by silica gel column chromatography (hexane-ethyl acetate 6:1) to obtain 530 mg of the title compound (yield: 45%). Melting point: 131-139°C (ethyl acetate-heptane).
$^1$H-NMR ($CDCl_3$): δ 2.26 (3H, s), 2.36 (3H, s), 2.53 (3H, s), 2.94-3.02 (4H, m), 3.06-3.14 (4H, m), 6.83 (2H, d, J = 8.8 Hz), 7.03 (1H, d, J = 8.0 Hz), 7.07 (2H, d, J = 8.8 Hz), 7.19 (2H, d, J = 8.4 Hz), 7.27 (1H, d, J = 8.0 Hz), 7.85 (2H, d, J = 8.0 Hz).

Example 44

1-(4-methoxyphenyl)-4-[2-(4-methoxyphenyl)pyridin-3-yl]piperazine

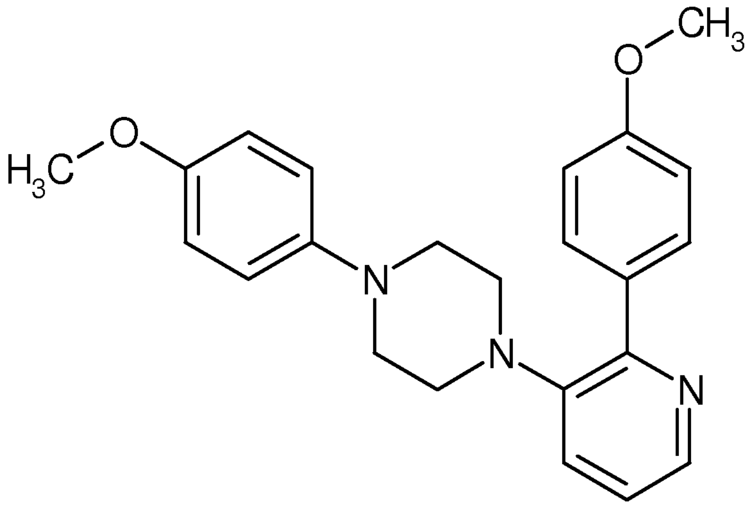

The title compound was obtained in the same way as in Example 1 using 3-bromo-2-(4-methoxyphenyl)pyridine (1.00 g, 3.79 mmol) synthesized in Reference Example 26 and 1-(4-methoxyphenyl)piperazine (1.09 g, 5.68 mmol). Yield: 48%. Amorphous solid.

$^1$H-NMR ($CDCl_3$) : δ 2.98-3.12 (8H, m), 3.77 (3H, s), 3.85 (3H, s), 6.83-6.86 (2H, m), 6.87-6.92 (2H, m), 6.93-6.96 (2H, m), 7.16 (1H, dd, J = 8.2, 4.6 Hz), 7.35 (1H, dd, J = 8.2, 1.4 Hz), 7.94-8.02 (2H, m), 8.32-8.38 (1H, m).

Example 45

N,N-dimethyl-4-{3-[4-(4-methylphenyl)piperazin-1-yl]pyridin-2-yl}aniline

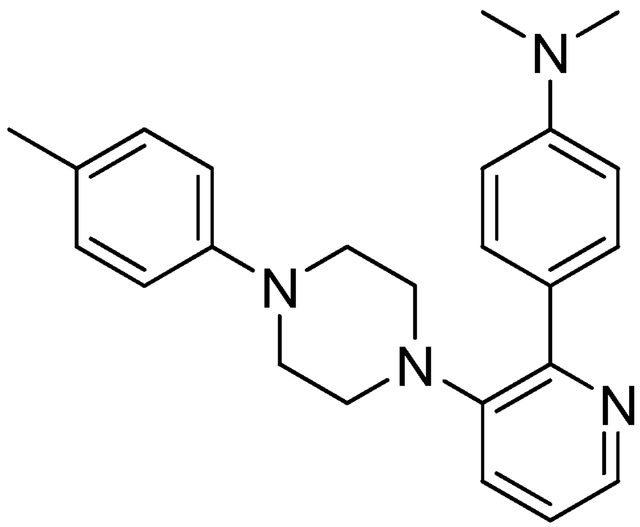

The title compound was obtained in the same way as in Example 1 using 4-(3-bromopyridin-2-yl)-N,N-dimethylaniline (1.00 g, 3.79 mmol) synthesized in Reference Example 28 and 1-(4-methylphenyl)piperazine (1.09 g, 5.68 mmol). Yield: 49%, amorphous solid.

$^1$H-NMR ($CDCl_3$) : δ 2.04 (3H, s), 2.98 (6H, s), 3.00-3.09 (4H, m), 3.10-3.20 (4H, m), 6.74 (2H, dd, J = 6.8, 2.0 Hz), 6.84 (2H, dd, J = 6.6, 2.2 Hz), 7.06-7.15 (3H, m), 7.31 (1H, dd, J = 8.2, 1.4 Hz), 7.98 (2H, dd, J = 6.8, 2.4 Hz), 8.32 (1H, dd, J = 4.4, 1.6 Hz).

Example 46

4-{6-bromo-3-[4-(4-methylphenyl)piperazin-1-yl]pyridin-2-yl}-N,N-dimethylaniline

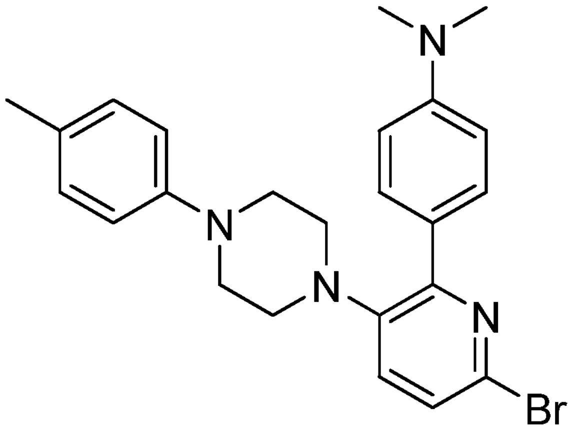

The title compound was obtained in the same way as in Example 36 using N,N-dimethyl-4-{3-[4-(4-methylphenyl)piperazin-1-yllpyridin-2-yllaniline (1.00 g, 2.68 mmol) synthesized in Example 45. Yield: 42%. Amorphous solid.
$^1$H-NMR ($CDCl_3$) : δ 2.27 (3H, s), 2.99 (6H, s), 3.01-3.06 (4H, m), 3.12-3.20 (4H, m), 6.71 (2H, d, J = 9.2 Hz), 6.84 (2H, d, J = 8.8 Hz), 7.08 (2H, d, J = 8.8 Hz), 7.17 (1H, d, J = 8.4 Hz), 7.23 (1H, d, J = 8.4 Hz), 8.01 (2H, d, J = 8.4 Hz).

Example 47

N,N-dimethyl-4-{6-methyl-3-[4-(4-methylphenyl)piperazin-1-yl]pyridin-2-yl}aniline

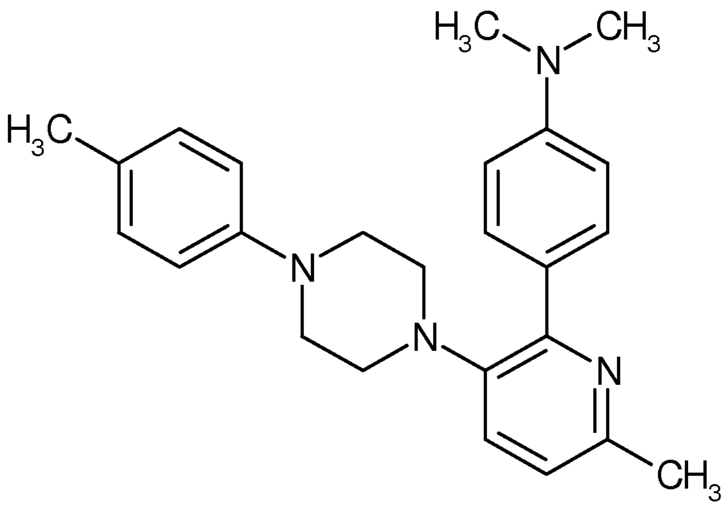

The title compound was obtained in the same way as in Example 43 using 4-{6-bromo-3-[4-(4-methylphenyl)piperazin-1-yllpyridin-2-yll-N,N-dimethylaniline (200 mg, 0.443 mmol) synthesized in Example 46 and a 50% solution of trimethylboroxine in THF (0.187 mL, 0.665 mmol). Yield: 35%. Melting point: 160-168°C (ethyl acetate-heptane).
$^1$H-NMR ($CDCl_3$): δ 2.27 (3H, s), 2.52 (3H, s), 2.97 (6H, s), 2.98-3.04 (4H, m), 3.10-3.18 (4H, m), 6.74 (2H, d, J = 9.2 Hz), 6.84 (2H, d, J = 8.4 Hz), 6.96 (1H, d, J = 8.4 Hz), 7.07 (2H, d, J = 8.4 Hz), 7.24 (1H, d, J = 8.0 Hz), 7.96 (2H, d, J = 8.8 Hz).

Example 48

1-[6-methoxy-2-(4-methylphenyl)pyridin-3-yl]-4-(4-methoxyphenyl)piperazine

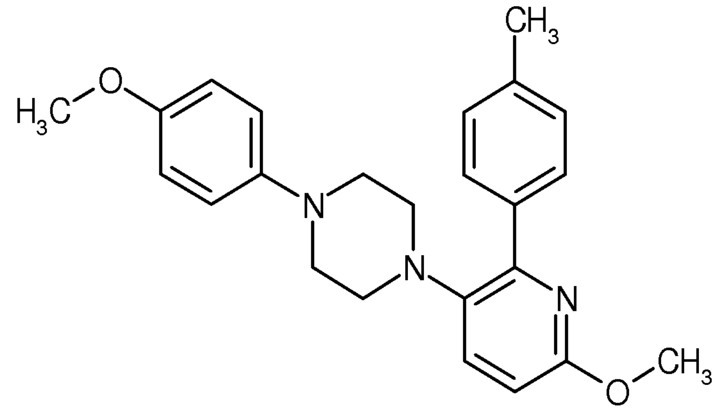

To a toluene (5 mL) solution of 1-(2-bromo-6-methoxypyridin-3-yl)-4-(4-methoxyphenyl)piperazine (204 mg, 0.540 mmol) synthesized in Reference Example 21, (4-methylphenyl)boronic acid (147 mg, 1.08 mmol), tetrakis(triphenylphosphine) palladium (62.0 mg, 0.0540 mmol), sodium carbonate (114 mg, 1.08 mmol), water (1.5 mL), and ethanol (2.5 mL) were added, and the mixture was stirred at 100°C for 12 hours in an argon atmosphere. After cooling to room temperature, water was added to the mixed solution, followed by extraction with ethyl acetate. The organic phase was dried over anhydrous magnesium sulfate. The solvent was distilled off under reduced pressure, and the obtained residue was purified by silica gel column chromatography (hexane-ethyl acetate 100:0-80:20) to obtain 174 mg of the title compound (yield: 83%). Melting point: 143-146°C (hexane).
$^1$H-NMR ($CDCl_3$): δ 2.38 (3H, s), 2.92-3.03 (4H, m), 3.03-3.14 (4H, m), 3.77 (3H, s), 3.95 (3H, s), 6.66 (1H, d, J = 8.7 Hz), 6.78-6.97 (4H, m), 7.20 (2H, d, J = 8.3 Hz), 7.43 (1H, d, J = 8.7 Hz), 8.05 (2H, d, J = 8.3 Hz).

Example 49

1-(4-methoxyphenyl)-4-{6-methoxy-2-[4-(trifluoromethoxy)phenyl]pyridin-3-yl}piperazine

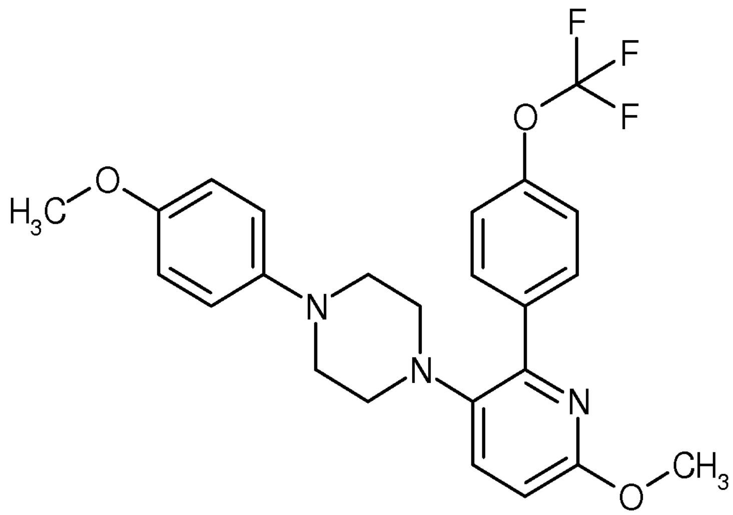

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and [4-(trifluoromethoxy)phenyl]boronic acid (165 mg, 0.800 mmol). Yield: 71%. Melting point: 110-111°C (hexane-methanol).
$^1$H-NMR ($CDCl_3$) : δ 2.94-3.02 (4H, m), 3.03-3.15 (4H, m), 3.77 (3H, s), 3.96 (3H, s), 6.71 (1H, d, J = 8.7 Hz), 6.81-6.96 (4H, m), 7.26 (2H, d, J = 9.0 Hz), 7.48 (1H, d, J = 8.7 Hz), 8.21 (2H, d, J = 9.0 Hz).

Example 50

1-[6-methoxy-2-(4-methoxyphenyl)pyridin-3-yl]-4-(4-methoxyphenyl)piperazine

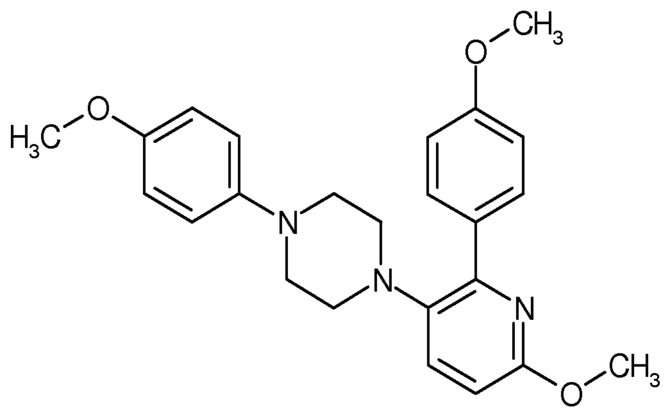

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (4-methoxyphenyl)boronic acid (122 mg, 0.800 mmol). Yield: 81%. Melting point: 128-130°C (ethyl acetate-hexane).
$^1$H-NMR ($CDCl_3$) : δ 2.93-3.03 (4H, m), 3.05-3.16 (4H, m), 3.77 (3H, s), 3.85 (3H, s), 3.95 (3H, s), 6.64 (1H, d, J = 8.7 Hz), 6.80-6.98 (6H, m), 7.43 (1H, d, J = 8.7 Hz), 8.16 (2H, d, J = 8.7 Hz).

Example 51

1-{6-methoxy-2-[4-(1-methylethyl)phenyl]pyridin-3-yl}-4-(4-methoxyphenyl)piperazine

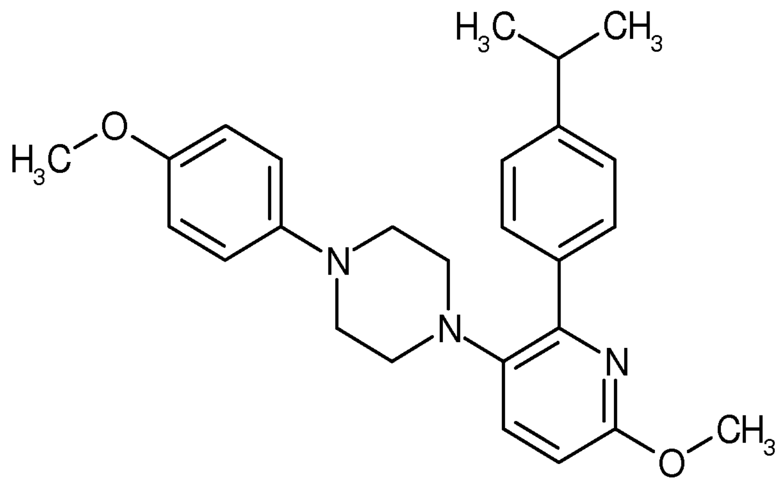

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and [4-(1-methylethyl)phenyl] boronic acid (147 mg, 0.800 mmol). Yield: 66%. Melting point: 128-130°C (ethyl acetate-hexane).
$^1$H-NMR ($CDCl_3$) : δ 1.28 (6H, d, J = 6.8 Hz), 2.85-3.03 (5H, m), 3.04-3.13 (4H, m), 3.77 (3H, s), 3.96 (3H, s), 6.66 (1H, d, J = 8.7 Hz), 6.80-6.98 (4H, m), 7.20-7.30 (2H, m), 7.44 (1H, d, J = 8.7 Hz), 8.09 (2H, d, J = 8.3 Hz).

Example 52

1-{6-methoxy-2-[4-(1-methylethoxy)phenyl]pyridin-3-yl}-4-(4-methoxyphenyl)piperazine

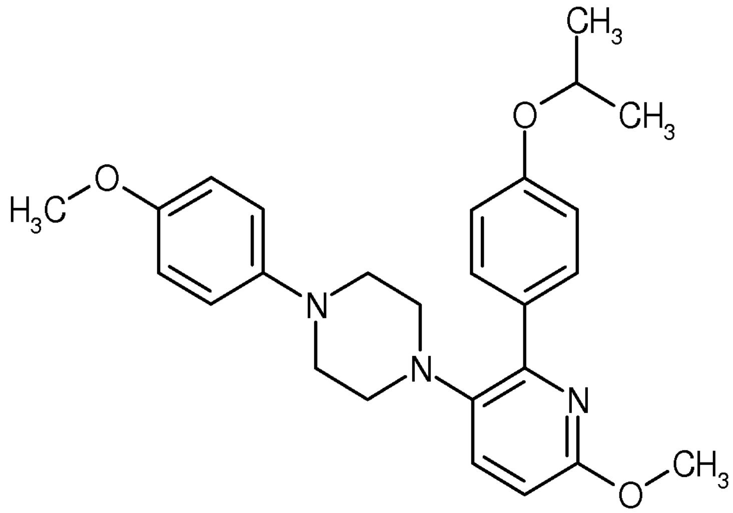

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and [4-(1-methylethoxy)phenyl] boronic acid (144 mg, 0.800 mmol). Yield: 82%. Melting point: 132-135°C (ethyl acetate-hexane).
$^1$H-NMR ($CDCl_3$) : δ 1.36 (6H, d, J = 6.1 Hz), 2.92-3.06 (4H, m), 3.06-3.15 (4H, m), 3.77 (3H, s), 3.96 (3H, s), 4.53-4.68 (1H, m), 6.63 (1H, d, J = 8.7 Hz), 6.80-6.99 (6H, m), 7.42 (1H, d, J = 8.3 Hz), 8.14 (2H, d, J = 9.1 Hz).

Example 53

1-[2-(4-ethylphenyl)-6-methoxypyridin-3-yl]-4-(4-methoxyphenyl)piperazine

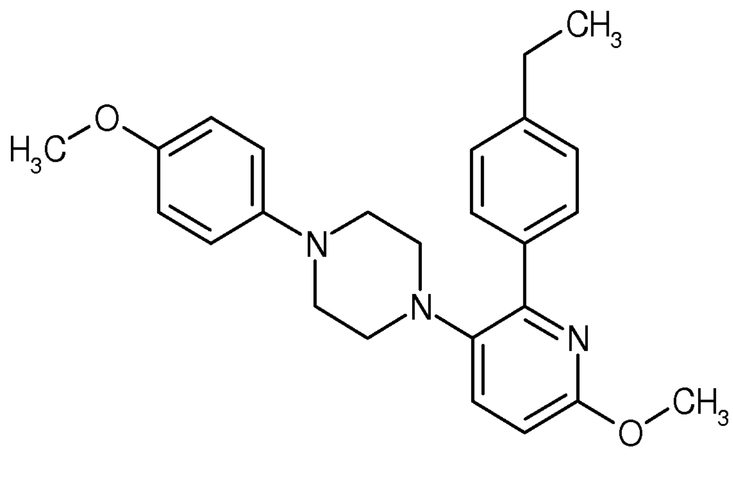

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (4-ethylphenyl)boronic acid (120 mg, 0.800 mmol). Yield: 72%. Melting point: 120-122°C (ethyl acetate-hexane).
$^1$H-NMR ($CDCl_3$) : δ 1.27 (3H, t, J = 7.6 Hz), 2.68 (2H, q, J = 7.6 Hz), 2.94-3.04 (4H, m), 3.04-3.16 (4H, m), 3.77 (3H, s), 3.95 (3H, s), 6.66 (1H, d, J = 8.7 Hz), 6.80-6.87 (2H, m), 6.88-6.96 (2H, m), 7.23 (2H, d, J = 8.3 Hz), 7.43 (1H, d, J = 8.7 Hz), 8.08 (2H, d, J = 8.3 Hz).

Example 54

1-[2-(4-chlorophenyl)-6-methoxypyridin-3-yl]-4-(4-methoxyphenyl)piperazine

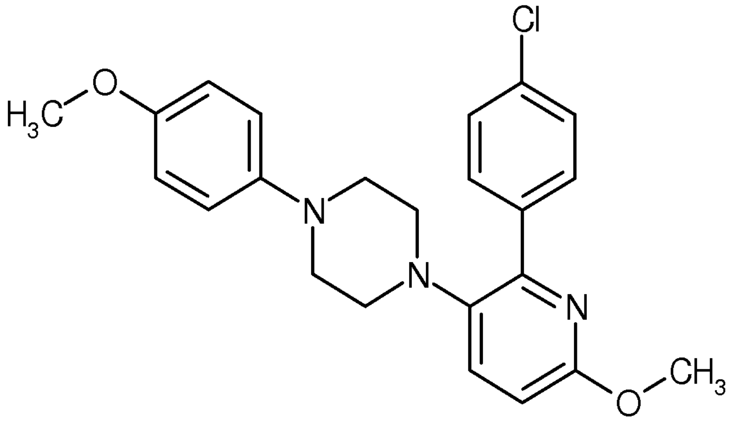

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (4-chlorophenyl)boronic acid (125 mg, 0.800 mmol). Yield: 73%. Melting point: 140-142°C (ethyl acetate-hexane).
$^1$H-NMR ($CDCl_3$) : δ 2.92-3.02 (4H, m), 3.04-3.20 (4H, m), 3.78 (3H, s), 3.95 (3H, s), 6.70 (1H, d, J = 8.7 Hz), 6.84 (2H, d, J = 9.3 Hz), 6.91 (2H, d, J = 9.3 Hz), 7.37 (2H, d, J = 9.0 Hz), 7.46 (1H, d, J = 8.7 Hz), 8.12 (2H, d, J = 9.0 Hz).

Example 55

1-[2-(4-ethoxyphenyl)-6-methoxypyridin-3-yl]-4-(4-methoxyphenyl)piperazine

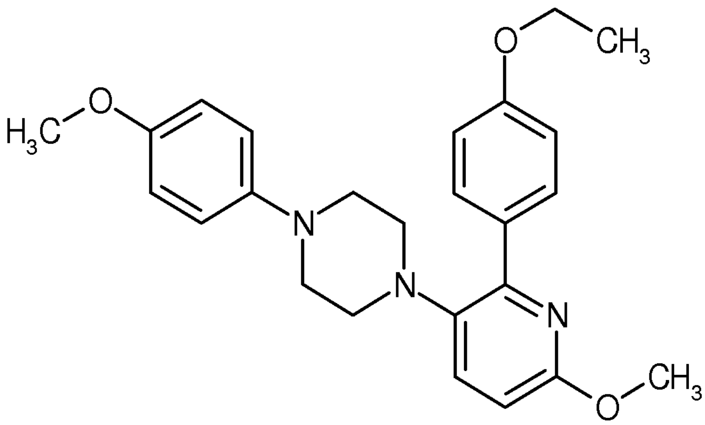

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (4-ethoxyphenyl)boronic acid (133 mg, 0.800 mmol). Specifically, to a toluene (4.0 mL) solution of 1-(2-bromo-6-methoxypyridin-3-yl)-4-(4-methoxyphenyl)piperazine (150 mg, 0.400 mmol), (4-ethoxyphenyl)boronic acid (133 mg, 0.800 mmol), tetrakis(triphenylphosphine)palladium (46.2 mg, 0.0400 mmol), sodium carbonate (84.8 mg, 0.800 mmol), water (1.0 mL), and ethanol (2.0 mL) were added, and the mixture was stirred at 100°C for 12 hours in an argon atmosphere. After cooling to room temperature, water was added to the mixed solution, followed by extraction with ethyl acetate. The organic phase was dried over anhydrous magnesium sulfate. The solvent was distilled off under reduced pressure, and the obtained residue was purified by silica gel column chromatography (hexane-ethyl acetate 100:0-85:15) and crystallized from hexane to obtain 132 mg of the title compound (yield: 79%). Melting point: 125-128°C (ethyl acetate-hexane).

$^{1}$H-NMR ($CDCl_3$) : δ 1.44 (3H, t, J = 7.0 Hz), 2.93-3.03 (4H, m), 3.04-3.15 (4H, m), 3.77 (3H, s), 3.95 (3H, s), 4.08 (2H, q, J = 6.9 Hz), 6.63 (1H, d, J = 8.7 Hz), 6.80-6.98 (6H, m), 7.42 (1H, d, J = 8.7 Hz), 8.14 (2H, d, J = 9.0 Hz).

Example 56

5-methoxy-2-{6-methoxy-3-[4-(4-methoxyphenyl)piperazin-1-yl]pyridin-2-yl}benzaldehyde

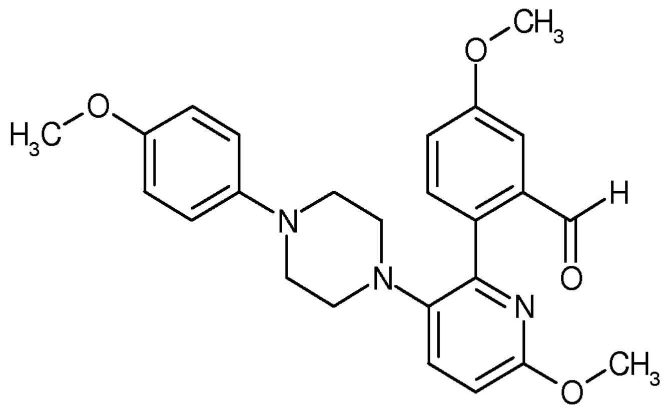

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (2-formyl-4-methoxyphenyl) boronic acid (144 mg, 0.800 mmol). Yield: 73%. Melting point: 120-127°C (ethyl acetate-hexane).
$^{1}$H-NMR ($CDCl_3$) : δ 2.80-2.95 (8H, m), 3.76 (3H, s), 3.90 (3H, s), 3.95 (3H, s), 6.76 (1H, d, J = 8.7 Hz), 6.79-6.87 (4H, m), 7.23 (1H, dd, J = 8.5, 2.8 Hz), 7.42-7.49 (2H, m), 7.65 (1H, d, J = 8.3 Hz), 9.79 (1H, s).

Example 57

1-(4-methoxyphenyl)-4-{6-methoxy-2-[4-(trifluoromethyl)phenyl]pyridin-3-yl}piperazine

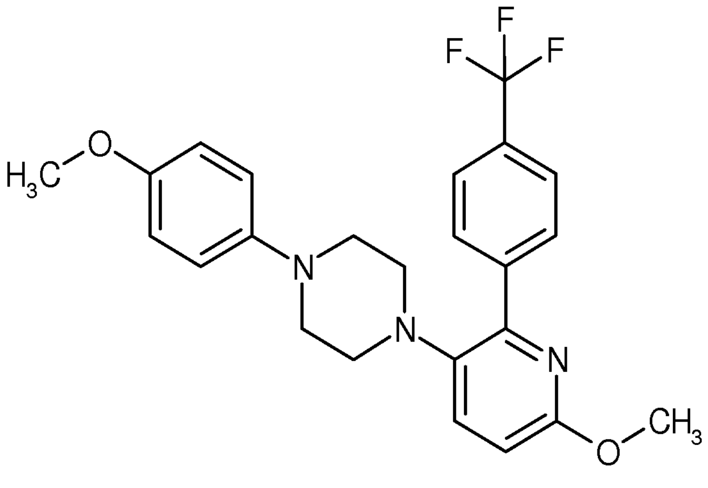

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and [4-(trifluoromethyl)phenyl] boronic acid (152 mg, 0.800 mmol). Yield: 72%. Melting point: 129-133°C (ethyl acetate-heptane).
$^{1}$H-NMR ($CDCl_3$): δ 2.94-3.02 (4H, m), 3.03-3.13 (4H, m), 3.77 (3H, s), 3.96 (3H, s), 6.74 (1H, d, J = 8.7 Hz), 6.81-6.96 (4H, m), 7.50 (1H, d, J = 8.7 Hz), 7.66 (2H, d, J = 8.0 Hz), 8.27 (2H, d, J = 8.3 Hz).

Example 58

1-[6-methoxy-2-(4-methoxy-3-methylphenyl)pyridin-3-yl]-4-(4-methoxyphenyl)piperazine

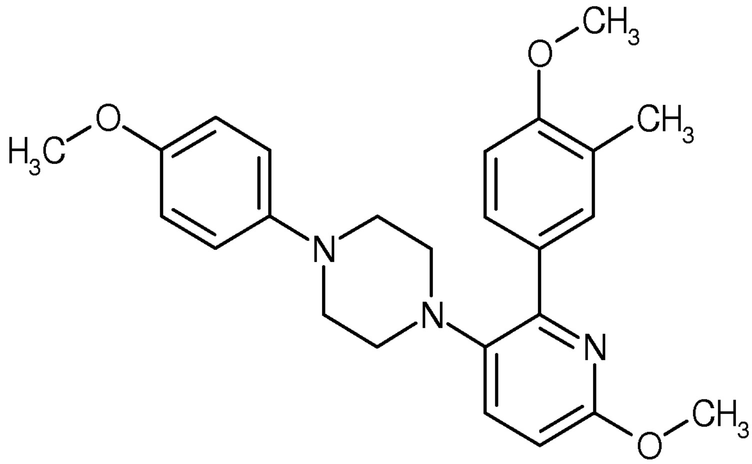

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (4-methoxy-3-methylphenyl) boronic acid (133 mg, 0.800 mmol). Yield: 76%. Melting point: 113-117°C (ethyl acetate-heptane).
$^1$H-NMR ($CDCl_3$) : δ 2.26 (3H, s), 2.94-3.04 (4H, m), 3.05-3.16 (4H, m), 3.77 (3H, s), 3.87 (3H, s), 3.96 (3H, s), 6.63 (1H, d, J = 8.7 Hz), 6.80-6.95 (5H, m), 7.41 (1H, d, J = 8.7 Hz), 7.96-8.08 (2H, m).

Example 59

1-[2-(3-fluoro-4-methoxyphenyl)-6-methoxypyridin-3-yl]-4-(4-methoxyphenyl)piperazine

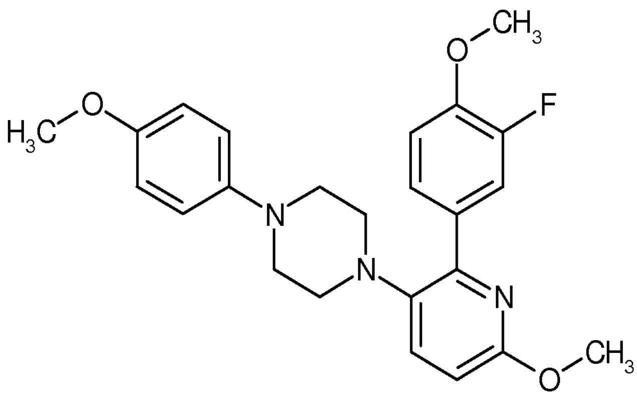

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (3-fluoro-4-methoxyphenyl) boronic acid (136 mg, 0.800 mmol). Yield: 83%. Melting point: 121-125°C (ethyl acetate-heptane).
$^1$H-NMR ($CDCl_3$) : δ 2.96-3.04 (4H, m), 3.08-3.18 (4H, m), 3.78 (3H, s), 3.93 (3H, s), 3.96 (3H, s), 6.67 (1H, d, J = 8.7 Hz), 6.81-7.04 (5H, m), 7.45 (1H, d, J = 8.7 Hz) 7.97-8.13 (2H, m).

Example 60

1-[2-(2,2-dimethyl-2,3-dihydro-1-benzofuran-5-yl)-6-methoxypyridin-3-yl]-4-(4-methoxyphenyl)piperazine

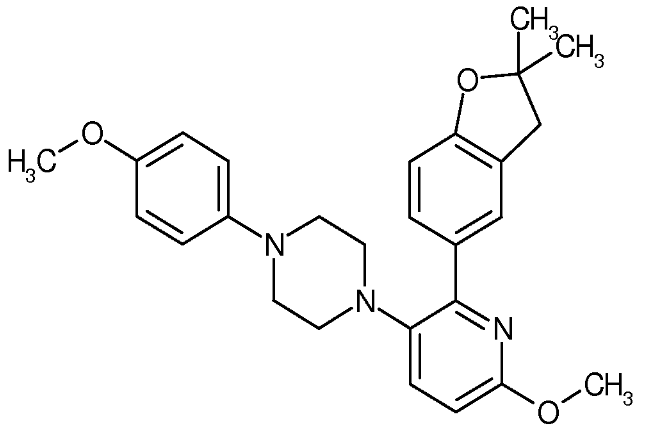

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (2,2-dimethyl-2,3-dihydro-1-benzofuran-5-yl)boronic acid (154 mg, 0.800 mmol). Yield: 77%. Melting point: 146-151°C (ethyl acetate-heptane). $^1$H-NMR ($CDCl_3$): δ 1.50 (6H, s), 2.93-3.18 (10H, m), 3.78 (3H, s), 3.96 (3H, s), 6.62 (1H, d, J = 8.3 Hz), 6.74 (1H, d, J = 8.3 Hz), 6.79-6.97 (4H, m) 7.41 (1H, d, J = 8.7 Hz), 7.94-7.99 (1H, m), 7.99-8.07 (1H, m).

Example 61

1-[2-(3,4-dimethoxyphenyl)-6-methoxypyridin-3-yl]-4-(4-methoxyphenyl)piperazine

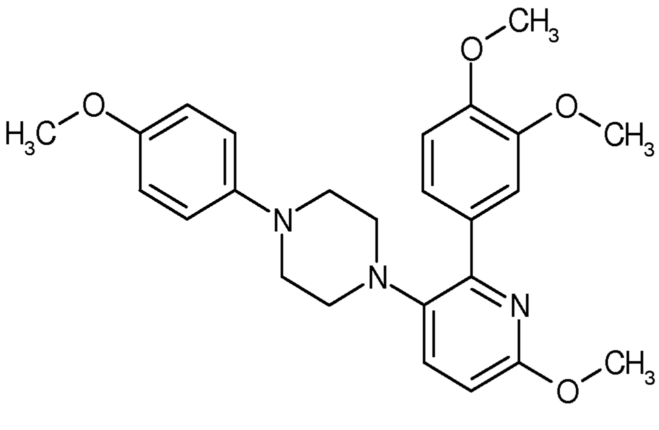

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (3,4-dimethoxyphenyl) boronic acid (146 mg, 0.800 mmol). Yield: 84%. Melting point: 120-128°C (ethyl acetate-heptane). $^1$H-NMR ($CDCl_3$): δ 2.93-3.06 (4H, m), 3.06-3.17 (4H, m), 3.77 (3H, s), 3.93 (6H, s), 3.96 (3H, s), 6.66 (1H, d, J = 8.7 Hz), 6.78-6.97 (5H, m), 7.44 (1H, d, J = 8.7 Hz), 7.79 (1H, dd, J = 8.5, 2.1 Hz), 7.86 (1H, d, J = 2.3 Hz).

Example 62

1-[2-(4-ethoxy-3-fluorophenyl)-6-methoxypyridin-3-yl]-4-(4-methoxyphenyl)piperazine

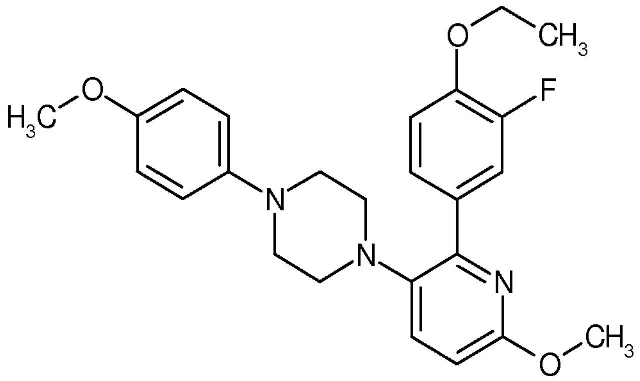

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (4-ethoxy-3-fluorophenyl) boronic acid (147 mg, 0.800 mmol). Yield: 92%. Melting point: 128-132°C (ethyl acetate-heptane).
$^{1}$H-NMR ($CDCl_3$) : δ 1.48 (3H, t, J = 7.0 Hz), 2.93-3.06 (4H, m), 3.07-3.18 (4H, m), 3.78 (3H, s), 3.96 (3H, s), 4.16 (2H, q, J = 7.2 Hz), 6.66 (1H, d, J = 8.7 Hz), 6.81-7.02 (5H, m), 7.45 (1H, d, J = 8.7 Hz), 7.95-8.01 (1H, m), 8.05 (1H, dd, J = 13.4, 2.1 Hz).

Example 63

1-[2-(4-ethoxy-2-methylphenyl)-6-methoxypyridin-3-yl]-4-(4-methoxyphenyl)piperazine

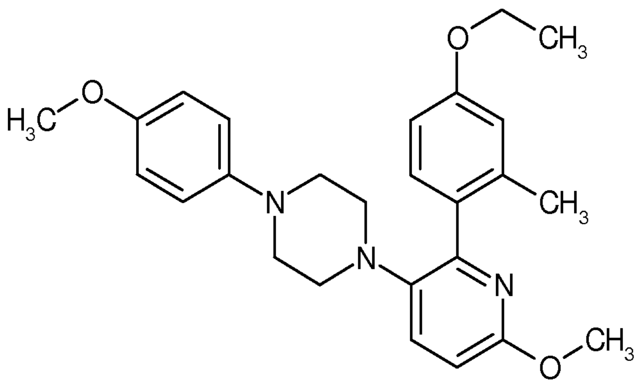

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (4-ethoxy-2-methylphenyl) boronic acid (144 mg, 0.800 mmol). Yield: 89%. Melting point: 75-80°C (ethyl acetate-heptane).
$^{1}$H-NMR ($CDCl_3$) : δ 1.42 (3H, t, J = 7.0 Hz), 2.26 (3H, s), 2.84-2.96 (8H, m), 3.76 (3H, s), 3.89 (3H, s), 4.05 (2H, q, J = 7.2 Hz), 6.68 (1H, d, J = 8.7 Hz), 6.72-6.89 (6H, m), 7.32-7.43 (2H, m).

Example 64

1-[2-(4-ethoxy-3-methylphenyl)-6-methoxypyridin-3-yl]-4-(4-methoxyphenyl)piperazine

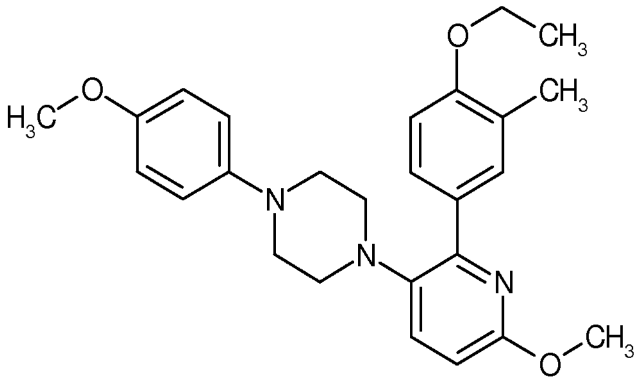

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (4-ethoxy-3-methylphenyl) boronic acid (144 mg, 0.800 mmol). Yield: 77%. Melting point: 117-120°C (ethyl acetate-heptane).
$^1$H-NMR ($CDCl_3$) : δ 1.44 (3H, t, J = 7.0 Hz), 2.26 (3H, s), 2.93-3.03 (4H, m), 3.04-3.16 (4H, m), 3.77 (3H, s), 3.96 (3H, s), 4.08 (2H, q, J = 6.8 Hz), 6.62 (1H, d, J = 8.7 Hz), 6.80-6.96 (5H, m), 7.41 (1H, d, J = 8.7 Hz), 7.96-8.04 (2H, m).

Example 65

1-[2-(3-ethoxyphenyl)-6-methoxypyridin-3-yl]-4-(4-methoxyphenyl)piperazine

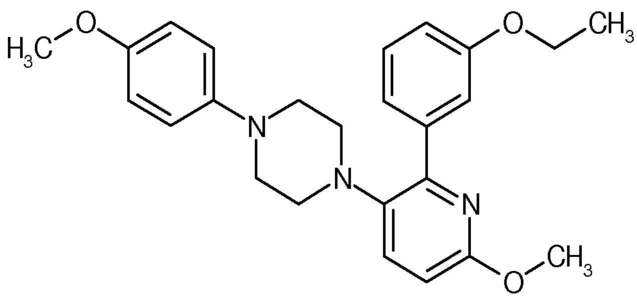

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (3-ethoxyphenyl)boronic acid (133 mg, 0.800 mmol). Yield: 85%. Melting point: 146-148°C (ethyl acetate-heptane).
$^1$H-NMR ($CDCl_3$) : δ 1.42 (3H, t, J = 7.0 Hz), 2.93-3.03 (4H, m), 3.03-3.14 (4H, m), 3.77 (3H, s), 3.96 (3H, s), 4.09 (2H, q, J = 6.8 Hz), 6.68 (1H, d, J = 8.7 Hz), 6.78-6.96 (5H, m), 7.29-7.35 (1H, m), 7.44 (1H, d, J = 8.7 Hz), 7.65-7.69 (1H, m), 7.70-7.77 (1H, m).

Example 66

1-[2-(2-fluoro-4-methoxyphenyl)-6-methoxypyridin-3-yl]-4-(4-methoxyphenyl)piperazine

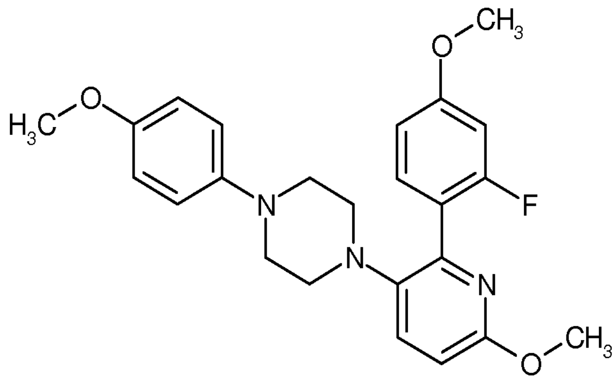

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (2-fluoro-4-methoxyphenyl) boronic acid (136 mg, 0.800 mmol). Yield: 86%. Melting point: 132-135°C (ethyl acetate-heptane).
$^1$H-NMR ($CDCl_3$) : δ 2.86-3.03 (8H, m), 3.76 (3H, s), 3.83 (3H, s), 3.92 (3H, s), 6.61-6.92 (7H, m), 7.44-7.54 (2H, m).

Example 67

1-[2-(2-ethoxyphenyl)-6-methoxypyridin-3-yl]-4-(4-methoxyphenyl)piperazine

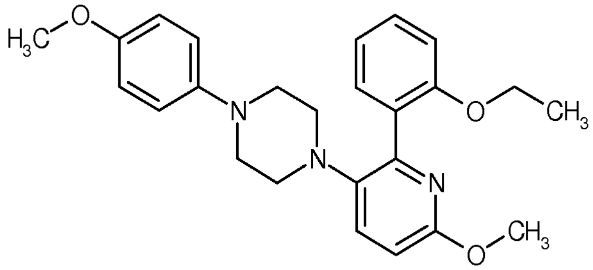

The title compound was obtained in the same way as in Example 48 using 1-(2-bromo-6-methoxypyridin-3-yl)-4-(4-methoxyphenyl)piperazine (80.0 mg, 0.213 mmol) synthesized in Reference Example 21 and (2-ethoxyphenyl)boronic acid (70.6 mg, 0.425 mmol). Yield: 88%, amorphous solid.
$^1$H-NMR ($CDCl_3$) : δ 1.26 (3H, t, J = 7.0 Hz), 2.82-2.96 (8H, m), 3.75 (3H, s), 3.91 (3H, s), 4.03 (2H, q, J = 7.2 Hz), 6.65-6.74 (1H, m), 6.76-6.87 (4H, m), 6.89-6.95 (1H, m), 6.95-7.04 (1H, m), 7.27-7.33 (1H, m), 7. 36 (1H, dd, J = 7.4, 1.7 Hz), 7.43 (1H, d, J = 8.7 Hz).

Example 68

4-{3-[4-(4-methoxyphenyl)piperazin-1-yl]pyridin-2-yl}-N,N-dimethylaniline

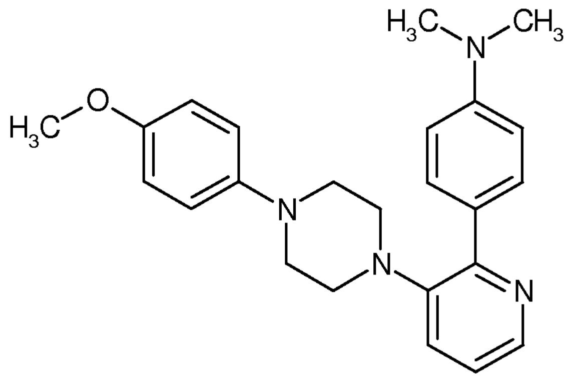

The title compound was obtained in the same way as in Example 48 using 1-(2-bromopyridin-3-yl)-4-(4-methoxyphenyl) piperazine (137 mg, 0.393 mmol) synthesized in Reference Example 22 and [4-(dimethylamino)phenyl]boronic acid (130 mg, 0.786 mmol). Yield: 62%. Melting point: 160-163°C (ethyl acetate-hexane).
$^1$H-NMR ($CDCl_3$): δ 3.00 (6H, s), 3.03-3.17 (8H, m), 3.77 (3H, s), 6.75 (2H, d, J = 8.7 Hz), 6.81-6.88 (2H, m), 6.88-6.97 (2H, m), 7.11 (1H, dd, J = 8.1, 4.7 Hz), 7.29-7.37 (1H, m), 7.98 (2H, d, J = 9.1 Hz), 8.29-8.36 (1H, m).

Example 69

1-(4-methoxyphenyl)-4-{2-[4-(1-methylethyl)phenyl]pyridin-3-yl}piperazine

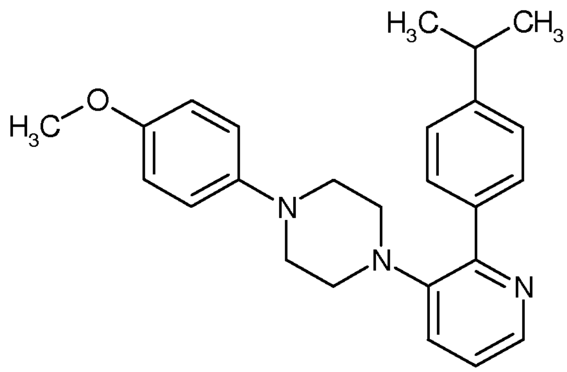

The title compound was obtained in the same way as in Example 48 using 1-(2-bromopyridin-3-yl)-4-(4-methoxyphenyl) piperazine (157 mg, 0.451 mmol) synthesized in Reference Example 22 and [4-(1-methylethyl)phenyl]boronic acid (148 mg, 0.902 mmol). Yield: 73%. Melting point: 130-133°C (ethyl acetate-hexane).
$^1$H-NMR ($CDCl_3$) : δ 1.27 (6H, d, J = 6.8 Hz), 2.85-3.00 (1H, m), 3.01-3.13 (8H, m), 3.77 (3H, s), 6.81-6.87 (2H, m), 6.87-6.93 (2H, m), 7.18 (1H, dd, J = 8.1, 4.7 Hz), 7.23-7.30 (2H, m), 7.36 (1H, dd, J = 7.9, 1.5 Hz) 7.90 (2H, d, J = 8.7 Hz), 8.35 (1H, dd, J = 4.5, 1.5 Hz).

Example 70

1-(4-methoxyphenyl)-4-{2-[4-(trifluoromethoxy)phenyl]pyridin-3-yl}piperazine

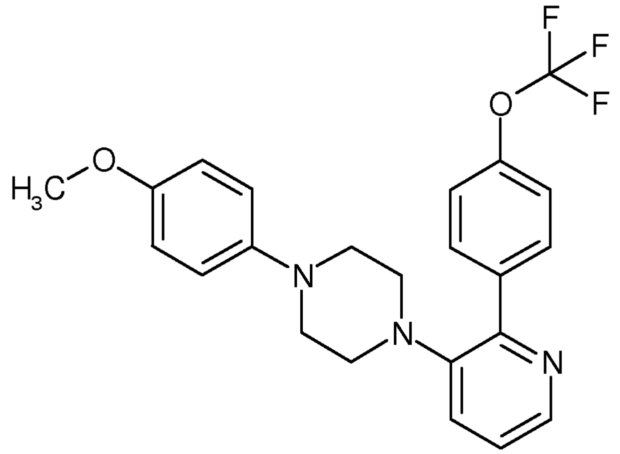

The title compound was obtained in the same way as in Example 48 using 1-(2-bromopyridin-3-yl)-4-(4-methoxyphenyl) piperazine (137 mg, 0.393 mmol) synthesized in Reference Example 22 and [4-(trifluoromethoxy)phenyl]boronic acid (162 mg, 0.786 mmol). Yield: 53%. Melting point: 108-110°C (ethyl acetate-hexane).
$^{1}$H-NMR ($CDCl_3$) : δ 2.96-3.12 (8H, m), 3.77 (3H, s), 6.82-6.87 (2H, m), 6.87-6.92 (2H, m), 7.19-7.33 (3H, m), 7.41 (1H, dd, J = 8.4, 1.5 Hz), 8.05 (2H, d, J = 8.7 Hz), 8.37 (1H, dd, J = 4.5, 1.5 Hz).

Example 71

(5-methoxy-2-{6-methoxy-3-[4-(4-methoxyphenyl)piperazin-1-yl]pyridin-2-yl}phenyl)methanol

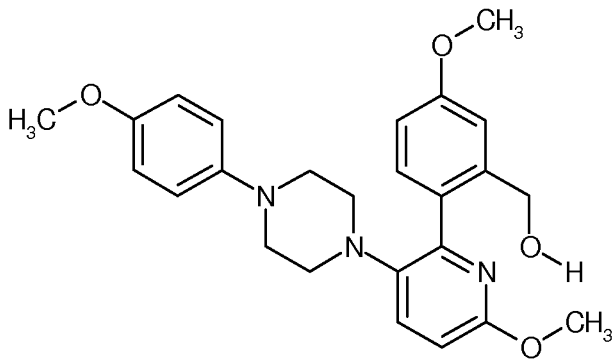

To a THF (5 mL)-methanol (1 mL) mixed solution of 5-methoxy-2-{6-methoxy-3-[4-(4-methoxyphenyl)piperazin-1-yl] pyridin-2-yl}benzaldehyde (96.0 mg, 0.221 mmol) synthesized in Example 56, sodium borohydride (42.0 mg, 11.1 mmol) was added at room temperature, and the mixture was stirred at room temperature for 12 hours. To the reaction solution, water was added, followed by extraction with ethyl acetate. The organic phase was dried over anhydrous magnesium sulfate. The solvent was distilled off under reduced pressure, and the obtained residue was purified by silica gel column chromatography (hexane-ethyl acetate 100:0-70:30) to obtain 67.9 mg of the title compound (yield: 71%). Melting point: 163-165°C (hexane-acetone).
$^{1}$H-NMR ($CDCl_3$) : δ 2.88-3.01 (8H, m), 3.75 (3H, s), 3.86 (3H, s), 3.89 (3H, s), 4.37 (2H, d, J = 6.4 Hz), 5.09 (1H, t, J = 6.6 Hz), 6.75 (1H, d, J = 9.0 Hz), 6.77-6.87 (4H, m), 6.89 (1H, dd, J = 8.5, 2.8 Hz), 7.03 (1H, d, J = 3.0 Hz), 7.48 (1H, d, J = 8.7 Hz), 7.54 (1H, d, J = 8.3 Hz).

Example 72

1-(4-{6-methoxy-3-[4-(4-methoxyphenyl)piperazin-1-yl]pyridin-2-yl}phenyl)ethanone

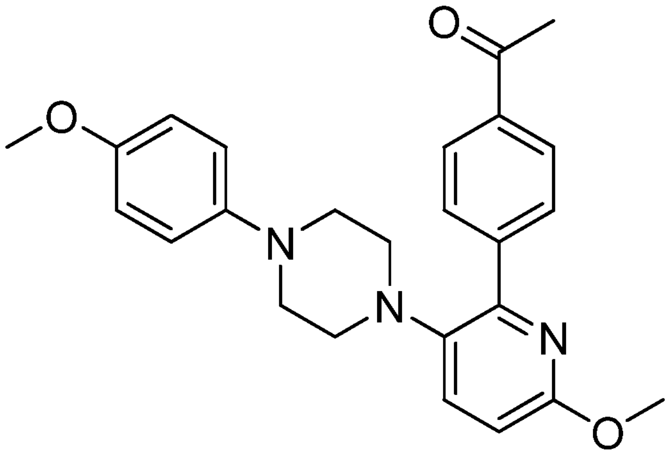

The title compound was obtained in the same way as in Example 48 using 1-(2-bromo-6-methoxypyridin-3-yl)-4-(4-methoxyphenyl)piperazine (750 mg, 1.99 mmol) synthesized in Reference Example 21 and (4-acetylphenyl)boronic acid (654 mg, 3.99 mmol). Yield: 85%, amorphous solid.
$^1$H-NMR ($CDCl_3$) : δ 2.63 (3H, s), 2.95-3.04 (4H, m), 3.04-3.13 (4H, m), 3.77 (3H, s), 3.96 (3H, s), 6.74 (1H, d, J = 8.7 Hz), 6.81-6.93 (4H, m), 7.49 (1H, d, J = 8.7 Hz), 8.00 (2H, d, J = 8.7 Hz), 8.26 (2H, d, J = 8.7 Hz).

Example 73

1-(4-{6-methoxy-3-[4-(4-methoxyphenyl)piperazin-1-yl]pyridin-2-yl}phenyl)ethanol

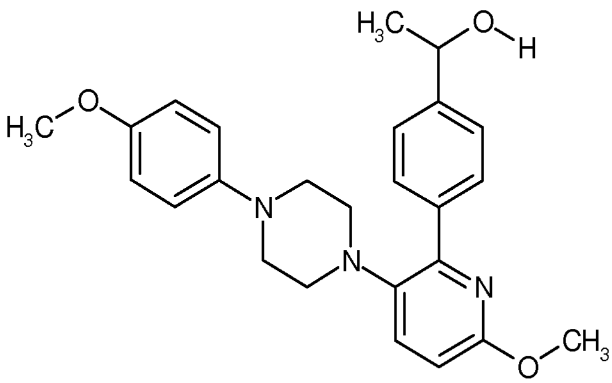

The title compound was obtained in the same way as in Example 71 using 1-(4-{6-methoxy-3-[4-(4-methoxyphenyl) piperazin-1-yl]pyridin-2-yl}phenyl)ethanone (271 mg, 0.650 mmol) synthesized in Example 72. Yield: 74%. Melting point: 104-110°C (ethyl acetate-hexane). $^1$H-NMR ($CDCl_3$) : δ 1.49-1.57 (3H, m), 1.76-1.82 (1H, m), 2.94-3.03 (4H, m), 3.04-3.13 (4H, m), 3.77 (3H, s), 3.96 (3H, s), 4.88-5.01 (1H, m), 6.68 (1H, d, J = 8.7 Hz), 6.80-6.95 (4H, m), 7.41 (2H, d, J = 8.3 Hz), 7.46 (1H, d, J = 8.7 Hz), 8.15 (2H, d, J = 8.3 Hz).

Example 74

2-(4-{6-methoxy-3-[4-(4-methoxyphenyl)piperazin-1-yl]pyridin-2-yl}phenyl)propan-2-ol

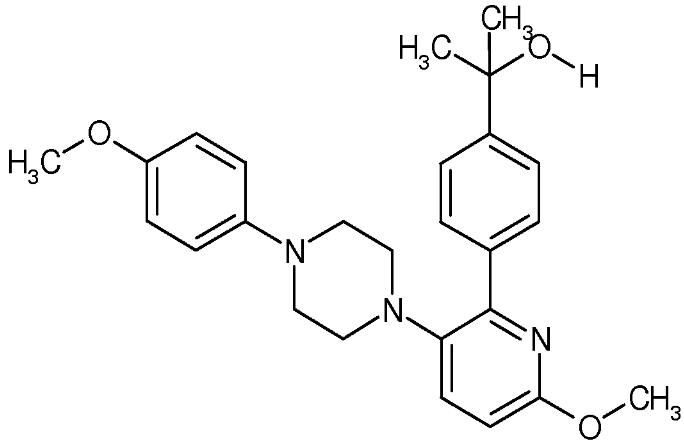

To a THF (10 mL) solution of 1-(4-{6-methoxy-3-[4-(4-methoxyphenyl)piperazin-1-yl]pyridin-2-yl}phenyl)ethanone (180 mg, 0.430 mmol) synthesized in Example 72, a 1 M solution of methyl magnesium bromide in THF (2.15 mL, 2.15 mmol) was added at room temperature, and the mixture was stirred at room temperature for 12 hours. To the reaction solution, water was added, followed by extraction with ethyl acetate. The organic phase was dried over anhydrous magnesium sulfate. The solvent was distilled off under reduced pressure, and the obtained residue was purified by silica gel column chromatography (hexane-ethyl acetate 100:0-60:40) to obtain 22.0 mg of the title compound (yield: 12%). Melting point: 138-144°C (ethyl acetate-hexane).
$^1$H-NMR ($CDCl_3$): δ 1.62 (6H, s), 1.74 (1H, s), 2.95-3.04 (4H, m), 3.06-3.14 (4H, m), 3.77 (3H, s), 3.96 (3H, s), 6.68 (1H, d, J = 8.7 Hz), 6.82-6.87 (2H, m), 6.88-6.94 (2H, m), 7.46 (1H, d, J = 8.7 Hz), 7.52 (2H, d, J = 8.7 Hz), 8.15 (2H, d, J = 8.7 Hz).

Example 75

4-(4-methoxyphenyl)-1-{6-methoxy-2-[4-(trifluoromethoxy)phenyl]pyridin-3-yl}piperidin-4-ol

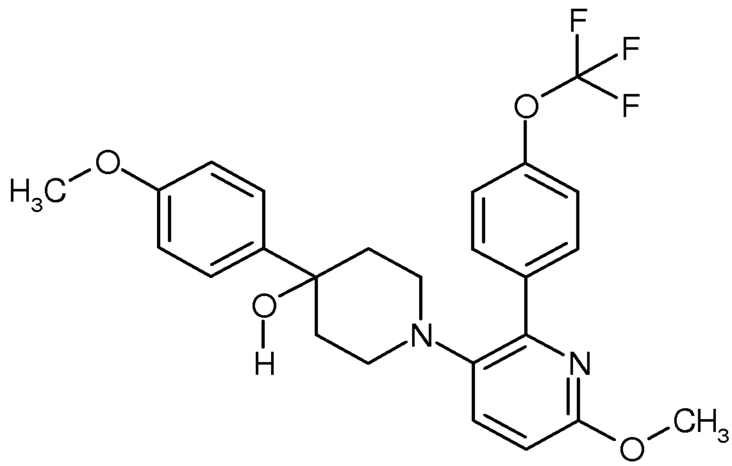

To a THF (10 mL) solution of 1-bromo-4-methoxybenzene (306 mg, 1.64 mmol), a 1.6 M solution of butyllithium in hexane (0.850 mL, 1.37 mmol) was added dropwise at -10°C, and the mixture was stirred at 0°C for 30 minutes. This solution was added to a THF (10 mL) solution of 1-{6-methoxy-2-[4-(trifluoromethoxy)phenyl]pyridin-3-yl}piperidin-4-one (200 mg, 0.546 mmol) synthesized in Reference Example 48, and the mixture was stirred at room temperature for 12 hours. To the reaction solution, a saturated aqueous solution of ammonium chloride was added, followed by extraction with ethyl acetate. The organic phase was dried over anhydrous magnesium sulfate, and the solvent was distilled off under reduced pressure. The obtained residue was purified by silica gel column chromatography (hexane-ethyl acetate 100: 0-80:20) to obtain 44.6 mg of the title compound (yield: 6.0%). Melting point: 100-112°C (hexane-acetone).
$^1$H-NMR ($CDCl_3$) : δ 1.43 (1H, s), 1.73-1.89 (2H, m), 2.01-2.16 (2H, m), 2.82-2.96 (2H, m), 3.00-3.16 (2H, m), 3.82 (3H, s), 3.95 (3H, s), 6.70 (1H, d, J = 8.7 Hz), 6.92 (2H, d, J = 8.7 Hz), 7.20-7.32 (2H, m), 7.42 (2H, d, J = 9.0 Hz), 7.53 (1H,

d, J = 8.7 Hz), 8.23 (2H, d, J = 9.0 Hz).

Example 76

1-{6-methoxy-2-[4-(1-methylethyl)phenyl]pyridin-3-yl}-3-(4-methoxyphenyl)piperidin-4-one

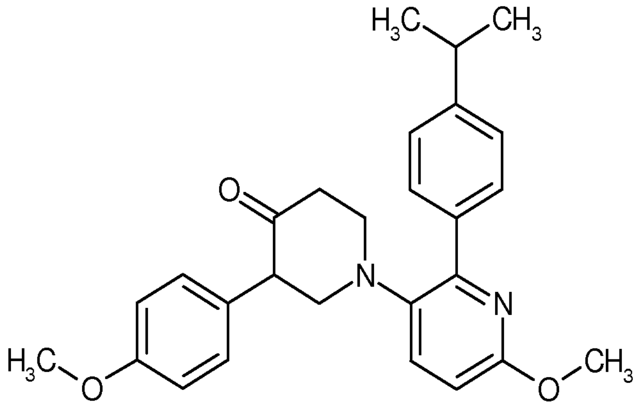

To a THF (10 mL) solution of 1-{6-methoxy-2-[4-(1-methylethyl)phenyl]pyridin-3-yl}piperidin-4-one (300 mg, 0.925 mmol) synthesized in Reference Example 49, 1-bromo-4-methoxybenzene (191 mg, 1.02 mmol), bis(tri-tert-butylphosphine) palladium (23.6 mg, 0.0463 mmol), and sodium tert-butoxide (267 mg, 2.78 mmol) were added, and the mixture was stirred at 70°C for 12 hours in an argon atmosphere. After cooling to room temperature, water was added to the mixed solution, followed by extraction with ethyl acetate. The organic phase was dried over anhydrous magnesium sulfate, and the solvent was then distilled off under reduced pressure. The obtained residue was purified by silica gel column chromatography (hexane-ethyl acetate 100:0-20:80) to obtain 93.8 mg of the title compound (yield: 24%) as an amorphous solid.
$^1$H-NMR ($CDCl_3$) : δ 1.30 (8H, d, J = 6.8 Hz), 2.58-2.70 (2H, m), 2.90-3.06 (1H, m), 3.65-3.81 (5H, m), 3.99 (3H, s), 6.77 (3H, dd, J = 13.0, 8.9 Hz), 7.03 (2H, d, J = 8.7 Hz), 7.15 (1H, s), 7.35 (2H, d, J = 8.3 Hz), 7.50 (1H, d, J = 8.7 Hz), 7.66 (2H, d, J = 8.3 Hz).

Example 77

4-{5-methoxy-3-[4-(4-methylphenyl)piperazin-1-yl]pyridin-2-yl}-N,N-dimethylaniline

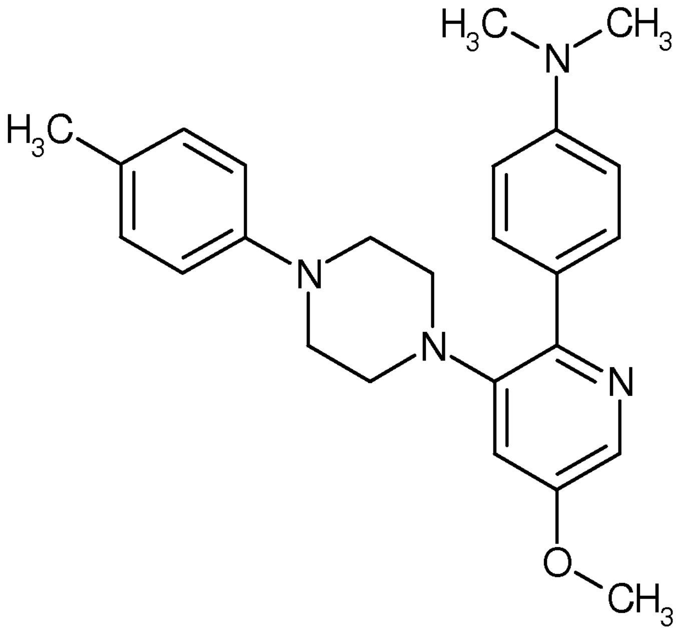

The title compound was obtained in the same way as in Reference Example 2 using 1-(2-chloro-5-methoxypyridin-3-yl)-4-(4-methylphenyl)piperazine (192 mg, 0.672 mmol) synthesized in Reference Example 31 and [4-(dimethylamino) phenyl]boronic acid (221 mg, 1.34 mmol). Yield: 48%. Melting point: 190-193°C (ethyl acetate-hexane).
$^1$H-NMR ($CDCl_3$) : δ 2.27 (3H, s), 2.98 (6H, s), 3.01-3.08 (4H, m), 3.11-3.22 (4H, m), 3.89 (3H, s), 6.74 (2H, d, J = 9.1 Hz), 6.84 (2H, d, J = 8.7 Hz), 6.89 (1H, d, J = 2.7 Hz), 7.08 (2H, d, J = 8.3 Hz), 7.88 (2H, d, J = 9.1 Hz), 8.04 (1H, d, J = 2.3 Hz).

Example 78

1-{2-[4-(dimethylamino)phenyl]-6-methoxypyridin-3-yl}-4-(4-methoxyphenyl)piperazine-2,5-dione

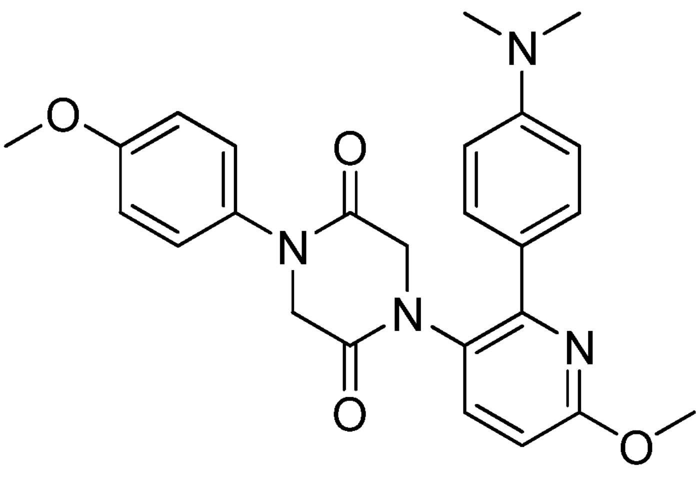

A mixture of 2-[N-(bromoacetyl)-N-(4-methoxyphenyl)amino]-N-{2-[4-(dimethylamino)phenyl]-6-methoxypyridin-3-yl} acetamide (241 mg, 0.46 mmol) synthesized in Reference Example 13, cesium carbonate (150 mg, 0.46 mmol), and DMF (2.5 mL) was stirred at 100°C for 2 hours. After cooling to room temperature, water was added to the reaction solution, followed by extraction with ethyl acetate. The extracts were mixed, washed with water, and then dried over anhydrous magnesium sulfate. The solvent was distilled off under reduced pressure, and the obtained residue was purified by silica gel column chromatography (hexane-ethyl acetate 90:10-40:60) and recrystallized from hexane-ethyl acetate to obtain 126 mg of the title compound (yield: 61%). Melting point: 138-139°C.
$^1$H-NMR ($CDCl_3$) : δ 3.02 (6H, s), 3.82 (3H, s), 3.99 (3H, s), 4.01 (2H, s), 4.42 (2H, s), 6.71 (1H, d, J = 8.7 Hz), 6.75 (2H,

d, J = 9.0 Hz), 6.94 (2H, d, J = 9.0 Hz), 7.20 (2H, d, J = 9.0 Hz), 7.51 (3H, dd, J = 8.5, 4.3 Hz).

Example 79

4-{2-[4-(dimethylamino)phenyl]-6-methoxypyridin-3-yl}-1-(4-methoxyphenyl)-3-methylpiperazine-2,5-dione

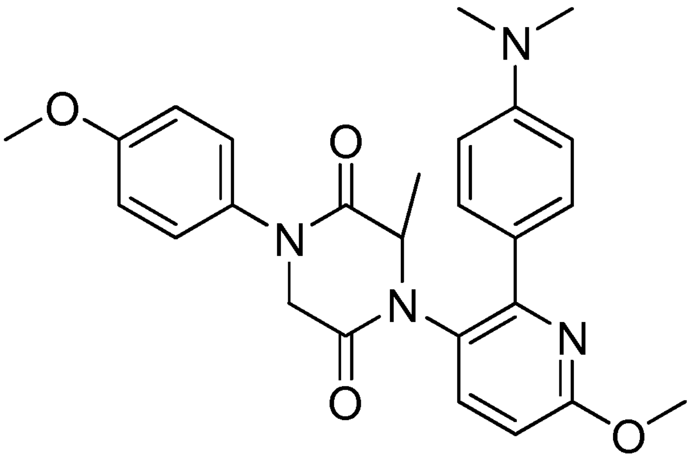

The title compound was obtained in the same way as in Example 78 using 2-bromo-N-[2-({2-[4-(dimethylamino)phenyl]-6-methoxypyridin-3-yl}amino)-2-oxoethyl]-N-(4-methoxyphenyl)propanamide (270 mg, 0.50 mmol) synthesized in Reference Example 14. Yield: 48%.
Melting point: 207-209°C (THF-hexane).
$^1$H-NMR ($CDCl_3$) : δ 1.43 (3H, d, J = 7.2 Hz), 3.01 (6H, s), 3.82 (3H, s), 3.85-3.93 (1H, m), 3.99 (3H, s), 4.25-4.42 (1H, m), 4.52-4.67 (1H, m), 6.63-6.79 (3H, m), 6.93 (2H, d, J = 8.7 Hz), 7.17 (2H, d, J = 8.7 Hz), 7.47 (1H, d, J = 8.7 Hz), 7.54 (2H, d, J = 8.7 Hz).

Example 80

4-{6-methoxy-3-[4-(4-methoxyphenyl)-2-methylpiperazin-1-yl]pyridin-2-yl}-N,N-dimethylaniline

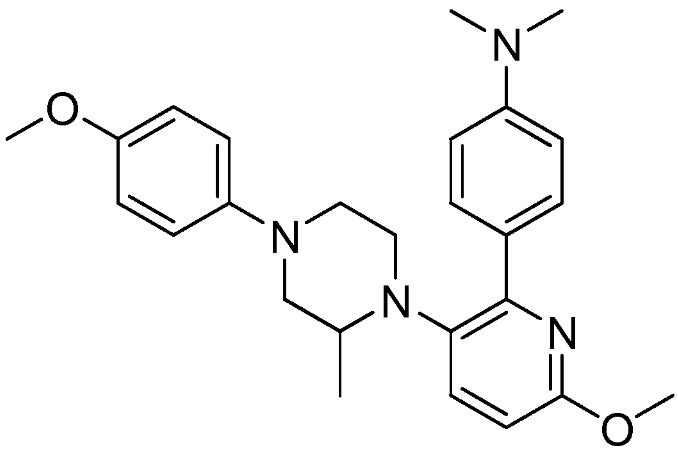

To a THF (2 mL) suspension of lithium aluminum hydride (38.0 mg, 1.00 mmol), a THF (2 mL) suspension of 4-{2-[4-(dimethylamino)phenyl]-6-methoxypyridin-3-yl}-1-(4-methoxyphenyl)-3-methylpiperazine-2,5-dione (115 mg, 0.25 mmol) synthesized in Example 79 was added dropwise under ice cooling, and the mixture was then refluxed for 14 hours. After standing to cool to room temperature, water (0.040 mL), a 15% aqueous sodium hydroxide solution (0.040

mL), and water (0.120 mL) were added dropwise in this order to the reaction solution, and the obtained suspension was filtered through celite. The filtrate was concentrated under reduced pressure, and the obtained residue was purified by silica gel column chromatography (hexane-ethyl acetate 100:0-85:15) and then recrystallized from hexane-ethyl acetate to obtain 33 mg of the title compound (yield: 31%). Melting point: 140-148°C.
$^1$H-NMR ($CDCl_3$): δ 0.94 (3H, d, J = 6.0 Hz), 2.67 (1H, dd, J = 11.1, 8.9 Hz), 2.86-2.96 (2H, m), 3.00 (6H, s), 3.03-3.10 (1H, m), 3.19-3.35 (2H, m), 3.40 (1H, d, J = 11.3 Hz), 3.78 (3H, s), 3.98 (3H, s), 6.61 (1H, d, J = 8.7 Hz), 6.73 (2H, d, J = 9.0 Hz), 6.81-6.89 (2H, m), 6.90-7.00 (2H, m), 7.49 (1H, d, J = 8.7 Hz), 8.26 (2H, d, J = 9.0 Hz).

Example 81

1-{2-[4-(dimethylamino)phenyl]-6-methoxypyridin-3-yl}-4-(4-methoxyphenyl)-3-methylpiperazine-2,5-dione

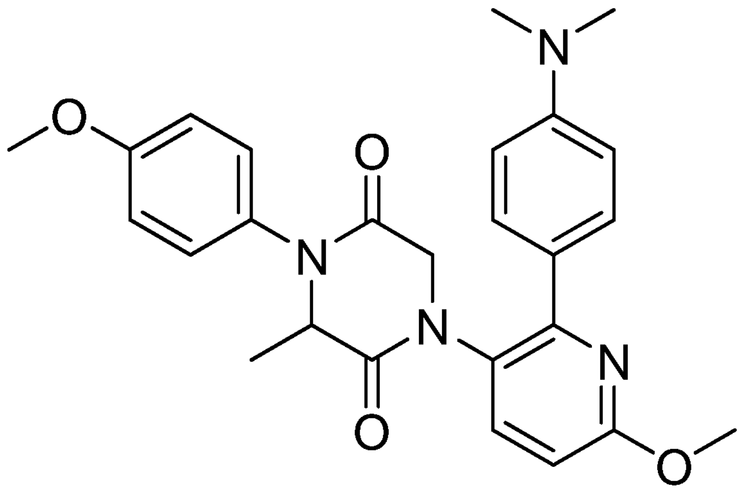

The title compound was obtained in the same way as in Example 78 using 2-[N-(bromoacetyl)-N-(4-methoxyphenyl) amino]-N-{2-[4-(dimethylamino)phenyl]-6-methoxypyridin-3-yl}propanamide (240 mg, 0.44 mmol) synthesized in Reference Example 18. Yield: quantitative, oil.
$^1$H-NMR ($CDCl_3$) : δ 1.53 (3H, d, J = 7.2 Hz), 2.94-3.07 (6H, m), 3.71-3.87 (4H, m), 3.92-4.08 (4H, m), 4.30-4.46 (1H, m), 6.64-6.81 (3H, m), 6.90-7.01 (2H, m), 7.16 (2H, d, J = 8.7 Hz), 7.50 (3H, dd, J = 18.5, 8.7 Hz).

Example 82

4-{6-methoxy-3-[4-(4-methoxyphenyl)-3-methylpiperazin-1-yl]pyridin-2-yl}-N,N-dimethylaniline

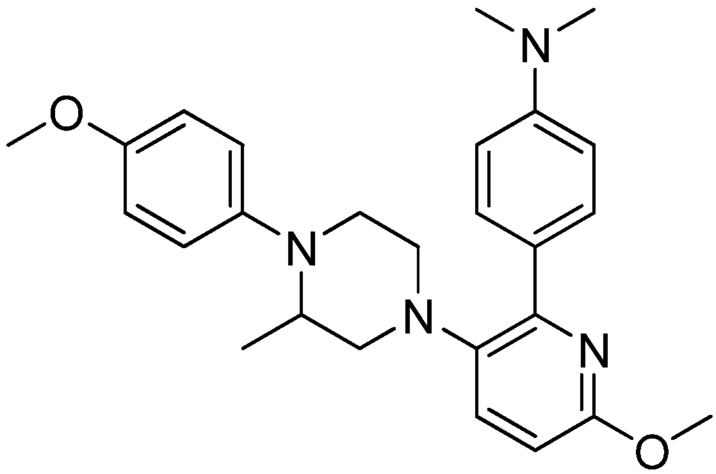

The title compound was obtained in the same way as in Example 80 using 1-{2-[4-(dimethylamino)phenyl]-6-methoxypyridin-3-yl}-4-(4-methoxyphenyl)-3-methylpiperazine-2,5-dione (203 mg, 0.44 mmol) synthesized in Example 81. Yield: 32%. Melting point: 139-140°C (ethyl acetate-hexane).
$^1$H-NMR ($CDCl_3$) : δ 0.98 (3H, d, J = 6.4 Hz), 2.73-2.88 (2H, m), 2.93-3.08 (9H, m), 3.13 (1H, dd, J = 10.6, 3.0 Hz), 3.60 (1H, dd, J = 8.9, 5.9 Hz), 3.78 (3H, s), 3.95 (3H, s), 6.58 (1H, d, J = 8.7 Hz), 6.77 (2H, d, J = 9.1 Hz), 6.81-6.90 (2H, m), 6.91-7.02 (2H, m), 7.42 (1H, d, J = 8.7 Hz), 8.13 (2H, d, J = 8.7 Hz).

Example 83

4-{2-[4-(dimethylamino)phenyl]-6-methoxypyridin-3-yl}-1-(4-fluorophenyl)piperazin-2-one

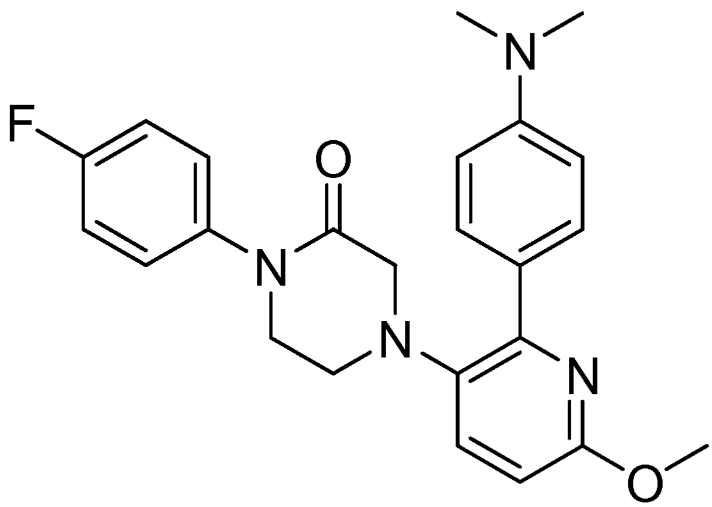

A mixture of 4-(3-bromo-6-methoxypyridin-2-yl)-N,N-dimethylaniline (200 mg, 0.65 mmol) synthesized in Reference Example 3, 1-(4-fluorophenyl)piperazin-2-one hydrochloride (180 mg, 0.78 mmol), palladium acetate (7.41 mg, 0.033 mmol), BINAP (61.0 mg, 0.098 mmol), sodium tert-butoxide (187 mg, 1.95 mmol), and toluene (2 mL) was stirred at 150°C for 1 hour under microwave irradiation. After cooling to room temperature, water was added to the reaction solution, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 100:0-50:50) and basic silica gel column chromatography (hexane-ethyl acetate 100: 0-70:30) and recrystallized from hexane-ethyl acetate to obtain 15 mg of the title compound (yield: 5.5%). Melting point: 158-164°C.
$^1$H-NMR ($CDCl_3$) : δ 3.02 (6H, s), 3.13-3.24 (2H, m), 3.53-3.63 (2H, m), 3.78 (2H, s), 3.96 (3H, s), 6.61 (1H, d, J = 8.7

Hz), 6.76 (2H, d, J = 9.0 Hz), 7.04-7.15 (2H, m), 7.27-7.32 (2H, m), 7.37 (1H, d, J = 8.7 Hz), 7.97-8.11 (2H, m).

Example 84

4-{2-[4-(dimethylamino)phenyl]-6-methoxypyridin-3-yl}-1-[4-(trifluoromethoxy)phenyl]piperazin-2-one

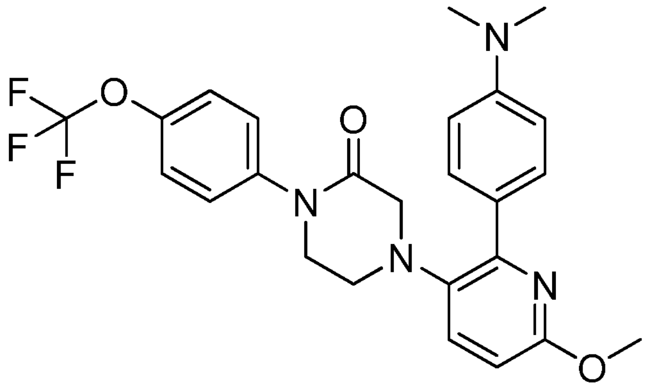

A mixture of 4-(3-bromo-6-methoxypyridin-2-yl)-N,N-dimethylaniline (100 mg, 0.33 mmol) synthesized in Reference Example 3, 1-[4-(trifluoromethoxy)phenyl]piperazin-2-one hydrochloride (119 mg, 0.40 mmol), tris(dibenzylideneacetone)dipalladium (0) (15.6 mg, 0.017 mmol), Xantphos (14.5 mg, 0.025 mmol), cesium carbonate (323 mg, 0.99 mmol), and toluene (1 mL) was stirred at 150°C for 1 hour under microwave irradiation. After cooling to room temperature, water was added to the reaction solution, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 100:0-75:25) and basic silica gel column chromatography (hexane-ethyl acetate 100:0-80:20) to obtain 12 mg of the title compound (yield: 7.5%). Melting point: 116-120°C (ethyl acetate).
$^1$H-NMR ($CDCl_3$) : δ 3.02 (6H, s), 3.19 (2H, t, J = 5.3 Hz), 3.57-3.67 (2H, m), 3.79 (2H, s), 3.96 (3H, s), 6.61 (1H, d, J = 8.3 Hz), 6.76 (2H, d, J = 9.0 Hz), 7.20-7.30 (2H, m), 7.33-7.42 (3H, m), 8.03 (2H, d, J = 9.0 Hz).

Example 85

4-{6-methoxy-3-[4-(4-methoxyphenyl)-1,4-diazepan-1-yl]pyridin-2-yl}-N,N-dimethylaniline

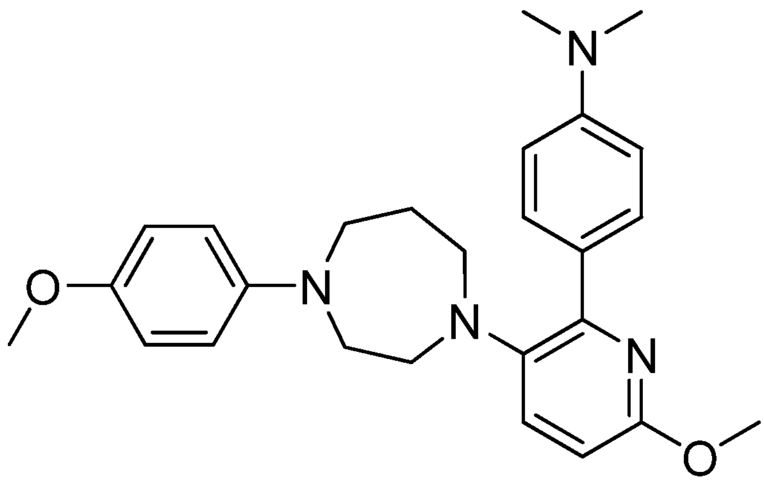

The title compound was obtained in the same way as in Example 83 using 4-(3-bromo-6-methoxypyridin-2-yl)-N,N-dimethylaniline (500 mg, 1.63 mmol) synthesized in Reference Example 3 and 1-(4-methoxyphenyl)-1,4-diazepane (505 mg, 2.45 mmol). Specifically, a mixture of 4-(3-bromo-6-methoxypyridin-2-yl)-N,N-dimethylaniline (500 mg, 1.63 mmol), 1-(4-methoxyphenyl)-1,4-diazepane (505 mg, 2.45 mmol), palladium acetate (74.1 mg, 0.33 mmol), BINAP (305 mg, 0.49 mmol), sodium tert-butoxide (470 mg, 4.89 mmol), and toluene (5 mL) was stirred at 150°C for 4 hours under microwave irradiation. After cooling to room temperature, water was added to the reaction solution, followed by extraction with ethyl acetate. The extracts were mixed and dried over anhydrous magnesium sulfate. The solvent was distilled off under reduced pressure, and the obtained residue was fractionated by silica gel column chromatography (hexane-ethyl acetate 100:0-80:20). A fraction containing the compound of interest was concentrated under reduced pressure, and the obtained residue was separated by reverse-phase high-performance liquid chromatography (C18, mobile phase: water/acetonitrile (system containing 0.1% TFA)). To the obtained fraction, a saturated aqueous solution of sodium bicarbonate was added, followed by extraction with ethyl acetate. The organic phase was dried over anhydrous magnesium sulfate and then concentrated under reduced pressure to obtain 11 mg of the title compound (yield: 1.6%) as an oil. Oil.

$^1$H-NMR ($CDCl_3$) : δ 1.86-1.94 (2H, m), 2.93-3.03 (8H, m), 3.08-3.16 (2H, m), 3.43-3.56 (4H, m), 3.75 (3H, s), 3.93 (3H, s), 6.52 (1H, d, J = 8.7 Hz), 6.65 (2H, d, J = 9.4 Hz), 6.70 (2H, d, J = 9.0 Hz), 6.77-6.86 (2H, m), 7.35 (1H, d, J = 8.7 Hz), 8.01 (2H, d, J = 9.0 Hz).

Example 86

4-{6-methoxy-3-[4-(4-methylphenyl)-1,4-diazepan-1-yl]pyridin-2-yl}-N,N-dimethylaniline

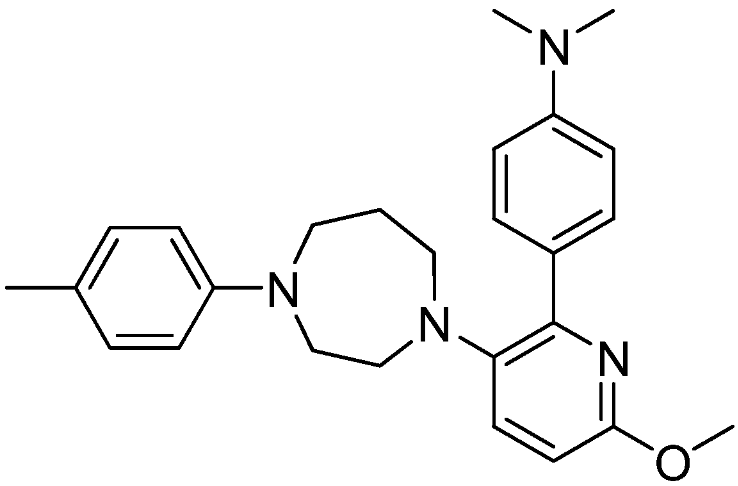

The title compound was obtained in the same way as in Example 83 using 4-[3-(1,4-diazepan-1-yl)-6-methoxypyridin-2-yl]-N,N-dimethylaniline (200 mg, 0.61 mmol) synthesized in Reference Example 38 and 1-iodo-4-methylbenzene (201 mg, 0.92 mmol). Yield: 10%, amorphous solid.

$^1$H-NMR ($CDCl_3$) : δ 1.85-1.93 (2H, m), 2.24 (3H, s), 2.92-3.03 (8H, m), 3.06-3.16 (2H, m), 3.47-3.60 (4H, m), 3.93 (3H, s), 6.51 (1H, d, J = 8.3 Hz), 6.61 (2H, d, J = 8.7 Hz), 6.70 (2H, d, J = 9.1 Hz), 7.02 (2H, d, J = 8.3 Hz), 7.34 (1H, d, J = 8.7 Hz), 8.01 (2H, d, J = 8.7 Hz).

Example 87

4-{3-[4-(2-fluoro-4-methoxyphenyl)-1,4-diazepan-1-yl]-6-methoxypyridin-2-yl}-N,N-dimethylaniline

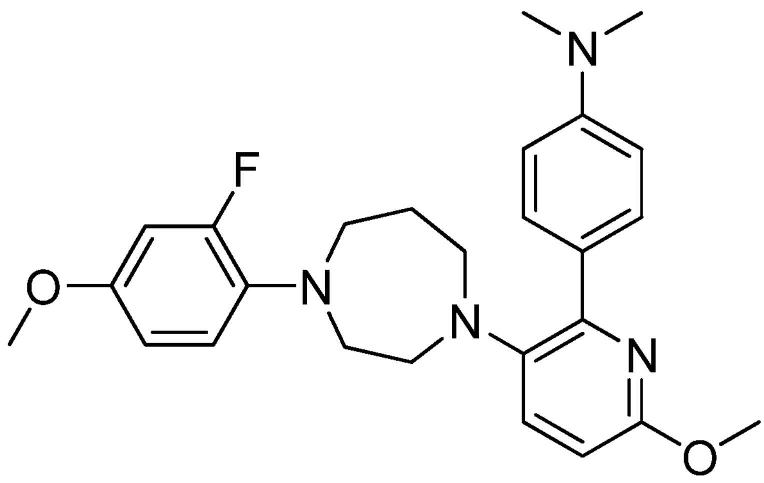

The title compound was obtained in the same way as in Example 83 using 4-[3-(1,4-diazepan-1-yl)-6-methoxypyridin-2-yl]-N,N-dimethylaniline (200 mg, 0.61 mmol) synthesized in Reference Example 38 and 1-bromo-2-fluoro-4-methoxybenzene (189 mg, 0.92 mmol). Yield: 22%, oil.
$^1$H-NMR ($CDCl_3$) : δ 1.89-1.97 (2H, m), 2.99 (6H, s), 3.09-3.16 (2H, m), 3.16-3.23 (2H, m), 3.27 (2H, d, J = 5.7 Hz), 3.33 (2H, t, J = 6.1 Hz), 3.75 (3H, s), 3.94 (3H, s), 6.52-6.67 (3H, m), 6.74 (2H, d, J = 8.7 Hz), 6.80-6.90 (1H, m), 7.45 (1H, d, J = 8.7 Hz), 8.01-8.11 (2H, m).

Example 88

4-{3-[4-(3-fluoro-4-methylphenyl)-1,4-diazepan-1-yl]-6-methoxypyridin-2-yl}-N,N-dimethylaniline

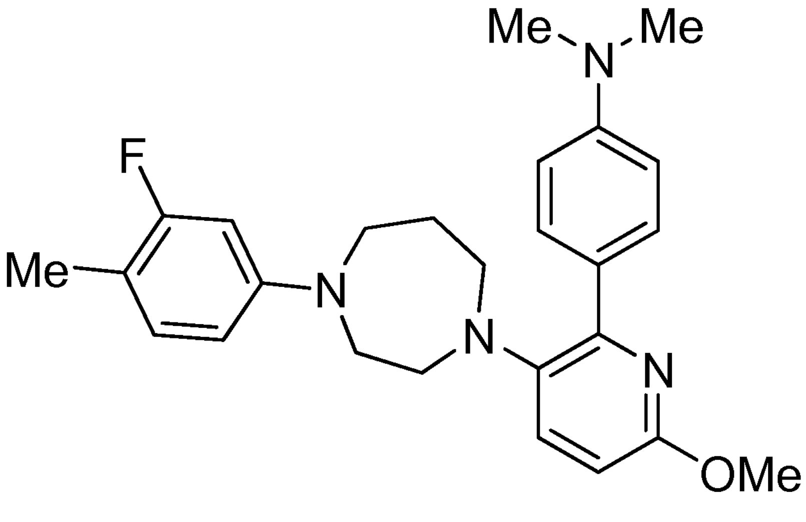

The title compound was obtained in the same way as in Example 83 using 4-[3-(1,4-diazepan-1-yl)-6-methoxypyridin-2-yl]-N,N-dimethylaniline (200 mg, 0.61 mmol) synthesized in Reference Example 38 and 4-bromo-2-fluoro-1-methylbenzene (0.116 mL, 0.92 mmol). Yield: 48%, oil.
$^1$H-NMR ($CDCl_3$): δ 1.83-1.91 (2H, m), 2.15 (3H, s), 2.93-3.01 (8H, m), 3.06-3.14 (2H, m), 3.43-3.56 (4H, m), 3.93 (3H, s), 6.29-6.40 (2H, m), 6.52 (1H, d, J = 8.7 Hz), 6.69 (2H, d, J = 9.0 Hz), 6.97 (1H, t, J = 9.0 Hz), 7.34 (1H, d, J = 8.7 Hz), 7.98 (2H, d, J = 9.0 Hz).

Example 89

4-{3-[4-(3,4-dimethoxyphenyl)-1,4-diazepan-1-yl]-6-methoxypyridin-2-yl}-N,N-dimethylaniline

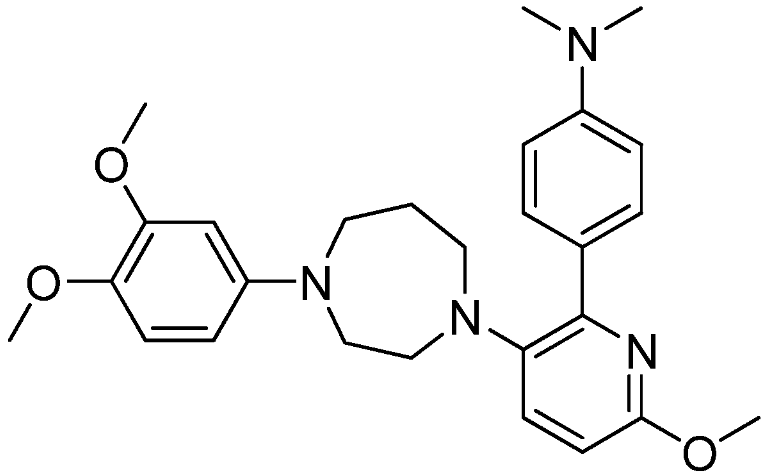

The title compound was obtained in the same way as in Example 83 using 4-[3-(1,4-diazepan-1-yl)-6-methoxypyridin-2-yl]-N,N-dimethylaniline (200 mg, 0.61 mmol) synthesized in Reference Example 38 and 4-bromo-1,2-dimethoxybenzene (0.132 mL, 0.92 mmol). Yield: 32%, oil.
$^1$H-NMR ($CDCl_3$) : δ 1.87-1.95 (2H, m), 2.97-3.04 (8H, m), 3.09-3.17 (2H, m), 3.45-3.57 (4H, m), 3.81 (3H, s), 3.84 (3H, s), 3.93 (3H, s), 6.20 (1H, dd, J = 8.7, 2.7 Hz), 6.30 (1H, d, J = 3.0 Hz), 6.52 (1H, d, J = 8.7 Hz), 6.70 (2H, d, J = 9.1 Hz), 6.79 (1H, d, J = 8.7 Hz), 7.36 (1H, d, J = 8.7 Hz), 7.98-8.06 (2H, m).

Example 90

4-{6-methoxy-3-[4-(5-methylpyridin-2-yl)-1,4-diazepan-1-yl]pyridin-2-yl}-N,N-dimethylaniline

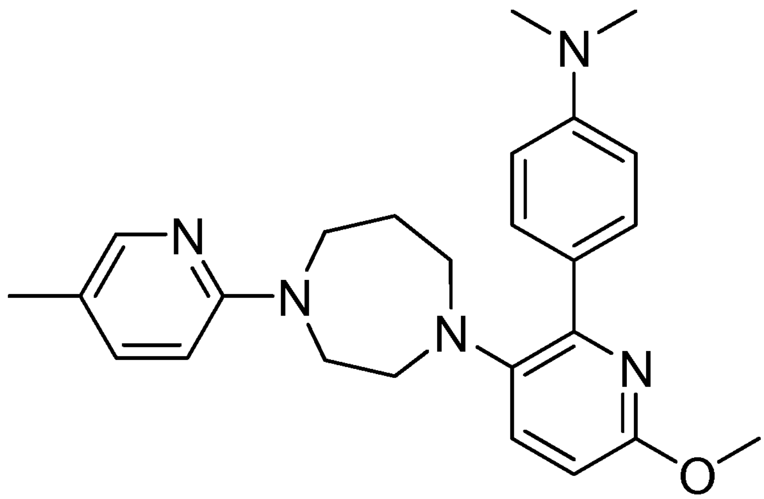

The title compound was obtained in the same way as in Example 83 using 4-[3-(1,4-diazepan-1-yl)-6-methoxypyridin-2-yl]-N,N-dimethylaniline (200 mg, 0.61 mmol) synthesized in Reference Example 38 and 2-bromo-5-methylpyridine (160 mg, 0.92 mmol). Yield: 41%, amorphous solid.
$^1$H-NMR ($CDCl_3$) : δ 1.83-1.91 (2H, m), 2.17 (3H, s), 2.92-3.03 (8H, m), 3.09-3.17 (2H, m), 3.68 (2H, t, J = 6.2 Hz), 3.72-3.78 (2H, m), 3.93 (3H, s), 6.39 (1H, d, J = 8.7 Hz), 6.51 (1H, d, J = 8.3 Hz), 6.69 (2H, d, J = 9.0 Hz), 7.26 (1H, dd,

J = 8.5, 2.4 Hz), 7.34 (1H, d, J = 8.3 Hz), 7.96-8.05 (3H, m).

Example 91

1-{6-methoxy-2-[4-(1-methylethyl)phenyl]pyridin-3-yl}-4-(4-methoxyphenyl)-1,4-diazepane

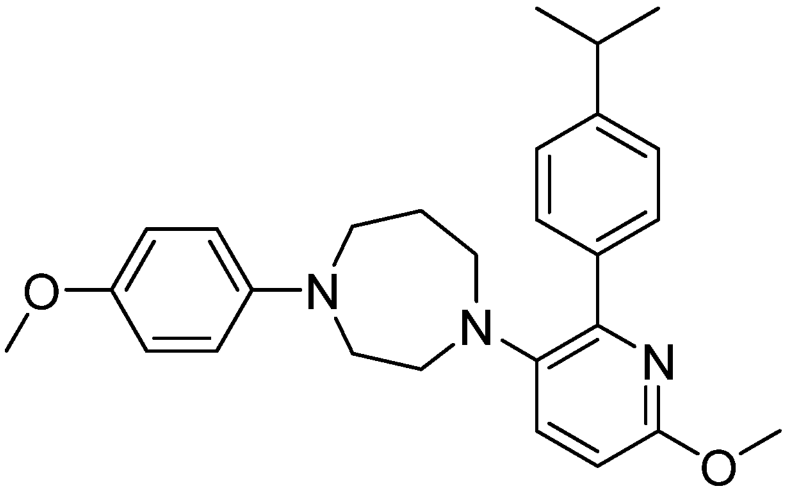

The title compound was obtained in the same way as in Example 83 using 1-{6-methoxy-2-[4-(1-methylethyl)phenyl] pyridin-3-yl}-1,4-diazepane (200 mg, 0.61 mmol) synthesized in Reference Example 39 and 1-bromo-4-methoxybenzene (0.136 mL, 0.92 mmol). Yield: 40%, oil.
$^1$H-NMR ($CDCl_3$) : δ 1.28 (6H, d, J = 6.8 Hz), 1.74-1.82 (2H, m), 2.87-3.01 (3H, m), 3.05-3.15 (2H, m), 3.38-3.45 (2H, m), 3.48 (2H, t, J = 6.2 Hz), 3.75 (3H, s), 3.93 (3H, s), 6.54-6.67 (3H, m), 6.81 (2H, d, J = 9.0 Hz), 7.22 (2H, d, J = 8.3 Hz), 7.38 (1H, d, J = 8.7 Hz), 7.88 (2H, d, J = 8.3 Hz).

Example 92

1-{2-[4-(dimethylamino)phenyl]-6-methoxypyridin-3-yl}-4-(4-methoxyphenyl)-1,4-diazepan-5-one

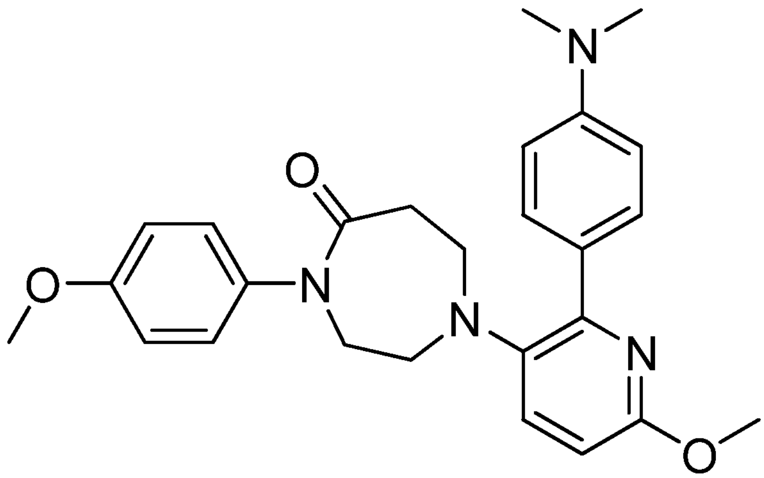

A mixture of 1-{2-[4-(dimethylamino)phenyl]-6-methoxypyridin-3-yl}-1,4-diazepan-5-one (200 mg, 0.59 mmol) synthesized in Reference Example 35, 1-iodo-4-methoxybenzene (166 mg, 0.71 mmol), copper iodide (5.71 mg, 0.030 mmol), potassium carbonate (163 mg, 1.18 mmol), N,N'-dimethylethylenediamine (0.00635 mL, 0.059 mmol), and toluene (3

mL) was stirred at 150°C for 1 hour under microwave irradiation. After cooling to room temperature, water was added to the reaction solution, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 100:0-50:50) and recrystallized from hexane-ethyl acetate to obtain 17 mg of the title compound (yield: 6%). Melting point: 192-193°C.
$^1$H-NMR ($CDCl_3$) : δ 2.86-2.94 (2H, m), 3.02 (6H, s), 3.06-3.13 (2H, m), 3.13-3.20 (2H, m), 3.76-3.84 (5H, m), 3.95 (3H, s), 6.58 (1H, d, J = 8.3 Hz), 6.77 (2H, d, J = 9.1 Hz), 6.83-6.91 (2H, m), 7.06-7.13 (2H, m), 7.40 (1H, d, J = 8.7 Hz), 8.08 (2H, d, J = 9.1 Hz).

Example 93

1-{2-[4-(dimethylamino)phenyl]-6-methoxypyridin-3-yl}-4-(4-methylphenyl)-1,4-diazepan-5-one

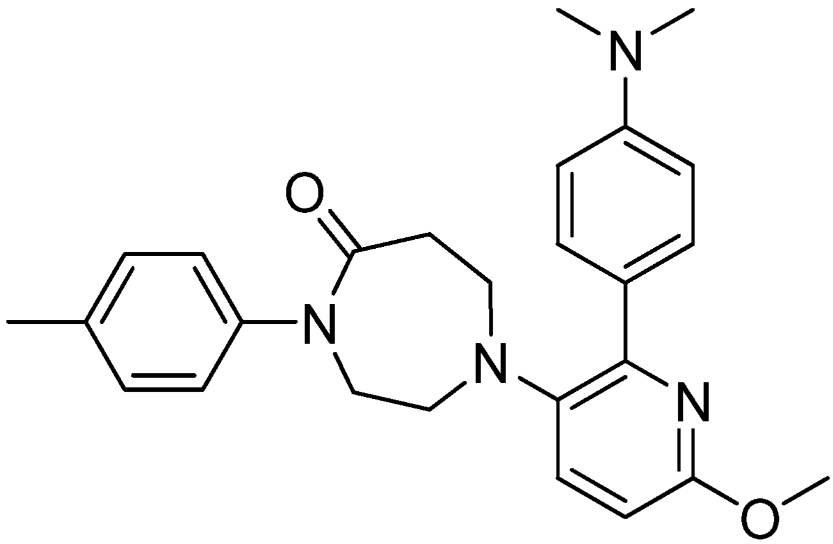

A mixture of 1-{2-[4-(dimethylamino)phenyl]-6-methoxypyridin-3-yl}-1,4-diazepan-5-one (200 mg, 0.59 mmol) synthesized in Reference Example 35, 1-iodo-4-methylbenzene (155 mg, 0.71 mmol), copper iodide (22.9 mg, 0.12 mmol), potassium carbonate (163 mg, 1.18 mmol), N,N'-dimethylethylenediamine (0.0258 mL, 0.24 mmol), and toluene (3 mL) was stirred at 150°C for 1 hour under microwave irradiation. After cooling to room temperature, water was added to the reaction solution, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 90:10-50:50) and recrystallized from hexane-ethyl acetate to obtain 28.7 mg of the title compound (yield: 11%). Melting point: 174-175°C.
$^1$H-NMR ($CDCl_3$) : δ 2.32 (3H, s), 2.86-2.94 (2H, m), 3.02 (6H, s), 3.07-3.13 (2H, m), 3.13-3.20 (2H, m), 3.78-3.86 (2H, m), 3.95 (3H, s), 6.58 (1H, d, J = 8.7 Hz), 6.77 (2H, d, J = 9.1 Hz), 7.03-7.10 (2H, m), 7.12-7.19 (2H, m), 7.39 (1H, d, J = 8.7 Hz), 8.01-8.13 (2H, m).

Example 94

1-{2-[4-(dimethylamino)phenyl]-6-methoxypyridin-3-yl}-4-(4-fluorophenyl)-1,4-diazepan-5-one

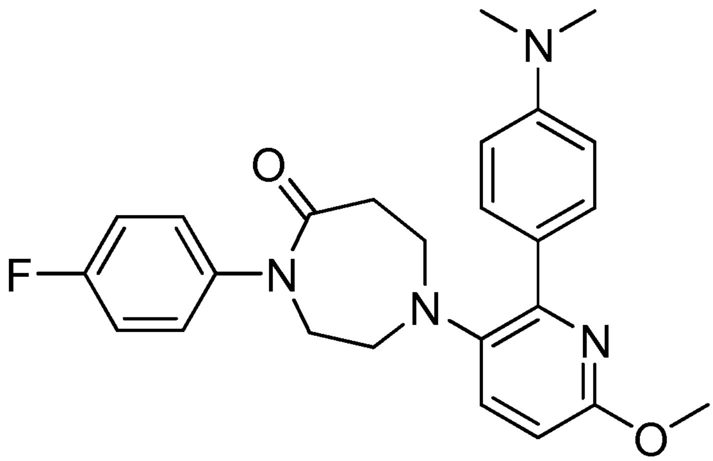

A mixture of 1-{2-[4-(dimethylamino)phenyl]-6-methoxypyridin-3-yl}-1,4-diazepan-5-one (200 mg, 0.59 mmol) synthesized in Reference Example 35, 1-bromo-4-fluorobenzene (0.078 mL, 0.71 mmol), copper iodide (57.1 mg, 0.30 mmol), potassium carbonate (163 mg, 1.18 mmol), N,N'-dimethylethylenediamine (0.0635 mL, 0.59 mmol), and toluene (3 mL) was stirred at 150°C for 1 hour under microwave irradiation. After cooling to room temperature, water was added to the reaction solution, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 100:0-50:50) and recrystallized from hexane-ethyl acetate to obtain 20.7 mg of the title compound (yield: 8%). Melting point: 130-139°C.

$^1$H-NMR ($CDCl_3$) : δ 2.87-2.95 (2H, m), 3.02 (6H, s), 3.07-3.14 (2H, m), 3.14-3.22 (2H, m), 3.77-3.87 (2H, m), 3.95 (3H, s), 6.59 (1H, d, J = 8.7 Hz), 6.77 (2H, d, J = 9.0 Hz), 7.00-7.09 (2H, m), 7.10-7.20 (2H, m), 7.39 (1H, d, J = 8.7 Hz), 8.02-8.11 (2H, m).

Example 95

1-[2-(4-ethoxyphenyl)-6-methoxypyridin-3-yl]-4-(4-methoxyphenyl)-1,4-diazepan-5-one

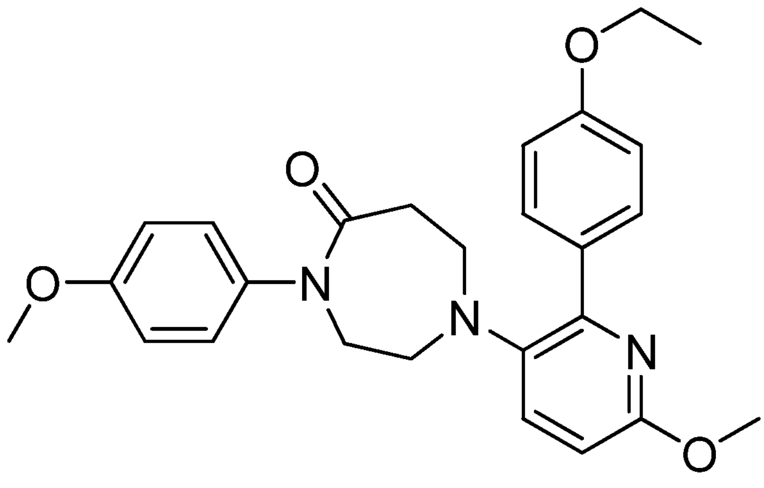

The title compound was obtained in the same way as in Example 94 using 1-[2-(4-ethoxyphenyl)-6-methoxypyridin-3-yl]-1,4-diazepan-5-one (200 mg, 0.59 mmol) synthesized in Reference Example 37 and 1-iodo-4-methoxybenzene (166 mg, 0.71 mmol). Yield: 16%. Melting point: 146-151°C (ethyl acetate-hexane).

$^1$H-NMR ($CDCl_3$) : δ 1.45 (3H, t, J = 7.2 Hz), 2.82-2.90 (2H, m), 3.03-3.11 (2H, m), 3.11-3.19 (2H, m), 3.73-3.78 (2H, m), 3.79 (3H, s), 3.95 (3H, s), 4.09 (2H, q, J = 6.8 Hz), 6.64 (1H, d, J = 8.3 Hz), 6.83-6.91 (2H, m), 6.91-6.99 (2H, m), 7.04-7.12 (2H, m), 7.42 (1H, d, J = 8.7 Hz), 7.98-8.07 (2H, m).

Example 96

1-[2-(4-ethoxyphenyl)-6-methoxypyridin-3-yl]-4-(4-methylphenyl)-1,4-diazepan-5-one

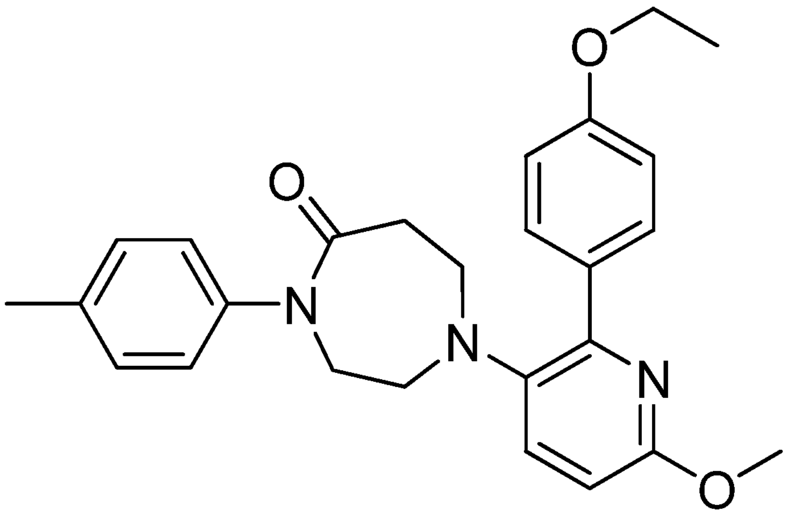

The title compound was obtained in the same way as in Example 94 using 1-[2-(4-ethoxyphenyl)-6-methoxypyridin-3-yl]-1,4-diazepan-5-one (200 mg, 0.59 mmol) synthesized in Reference Example 37 and 1-iodo-4-methylbenzene (155 mg, 0.71 mmol). Yield: 17%. Melting point: 167-168°C (ethyl acetate-hexane).
$^1$H-NMR ($CDCl_3$) : δ 1.45 (3H, t, J = 7.0 Hz), 2.32 (3H, s), 2.83-2.91 (2H, m), 3.04-3.11 (2H, m), 3.11-3.19 (2H, m), 3.75-3.82 (2H, m), 3.95 (3H, s), 4.09 (2H, q, J = 6.8 Hz), 6.64 (1H, d, J = 8.3 Hz), 6.91-6.98 (2H, m), 7.02-7.08 (2H, m), 7.12-7.20 (2H, m), 7.42 (1H, d, J = 8.7 Hz), 7.99-8.06 (2H, m).

Example 97

1-[2-(4-ethoxyphenyl)-6-methoxypyridin-3-yl]-4-(4-fluorophenyl)-1,4-diazepan-5-one

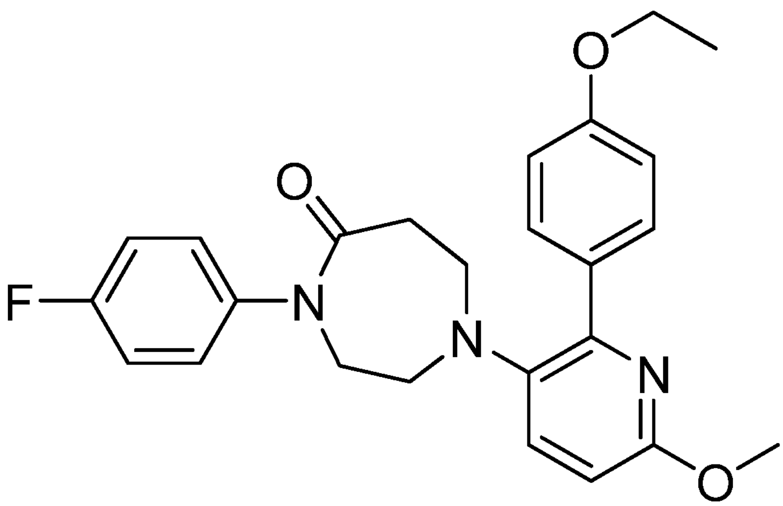

The title compound was obtained in the same way as in Example 94 using 1-[2-(4-ethoxyphenyl)-6-methoxypyridin-3-yl]-1,4-diazepan-5-one (200 mg, 0.59 mmol) synthesized in Reference Example 37 and 1-bromo-4-fluorobenzene (0.078 mL, 0.71 mmol). Yield: 1%. Melting point: 136-137°C (ethyl acetate-hexane).
$^1$H-NMR ($CDCl_3$) : δ 1.45 (3H, t, J = 6.8 Hz), 2.82-2.93 (2H, m), 3.01-3.11 (2H, m), 3.11-3.22 (2H, m), 3.73-3.83 (2H,

m), 3.95 (3H, s), 4.09 (2H, q, J = 7.2 Hz), 6.64 (1H, d, J = 8.3 Hz), 6.95 (2H, d, J = 8.7 Hz), 6.98-7.08 (2H, m), 7.09-7.22 (2H, m), 7.42 (1H, d, J = 8.7 Hz), 8.01 (2H, d, J = 9.1 Hz).

Example 98

4-{6-methoxy-3-[5-(4-methoxyphenyl)-4-methyl-1,4-diazepan-1-yl]pyridin-2-yl}-N,N-dimethylaniline

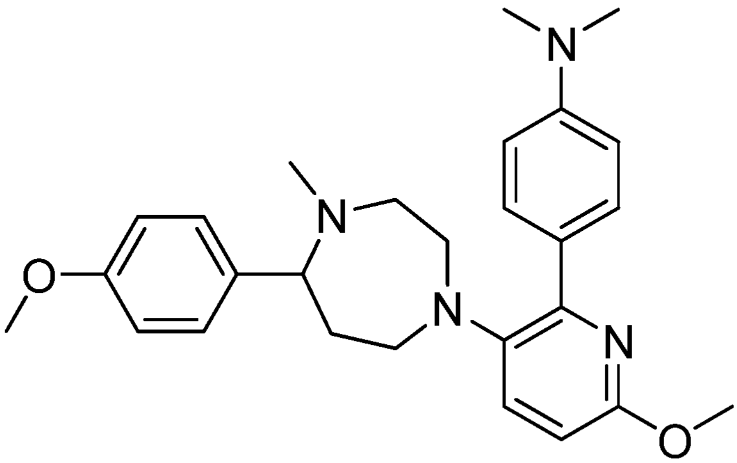

To a THF (3 mL) solution of a Grignard reagent prepared from 1-bromo-4-methoxybenzene (0.244 mL, 1.95 mmol) and magnesium (47.4 mg, 1.95 mmol), a THF (2 mL) solution of 1-{2-[4-(dimethylamino)phenyl]-6-methoxypyridin-3-yl}-4-methyl-1,4-diazepan-5-one (230 mg, 0.65 mmol) synthesized in Reference Example 50 was added, and the mixture was stirred at 60°C for 5 hours. After standing to cool to room temperature, sodium cyanotrihydroborate (163 mg, 2.60 mmol) and acetic acid (3 mL) were added thereto, and the mixture was stirred at room temperature for 4 hours. The reaction solution was neutralized by addition of an aqueous sodium hydroxide solution, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 80:20-0:100) and basic silica gel column chromatography (hexane-ethyl acetate 100:0-90:10) to obtain 51 mg of the title compound (yield: 18%). Oil.
$^1$H-NMR ($CDCl_3$) : δ 1.91-2.10 (2H, m), 2.18 (3H, s), 2.86-2.96 (1H, m), 3.00 (6H, s), 3.02-3.32 (5H, m), 3.37 (1H, dd, J = 7.8, 4.0 Hz), 3.79 (3H, s), 3.93 (3H, s), 6.54 (1H, d, J = 8.3 Hz), 6.77 (2H, d, J = 8.7 Hz), 6.84 (2H, d, J = 8.7 Hz), 7.27 (2H, d, J = 8.7 Hz), 7.41 (1H, d, J = 8.7 Hz), 8.07 (2H, d, J = 8.7 Hz).

Example 99

1-[2-(4-ethoxyphenyl)-6-methoxypyridin-3-yl]-5-(4-methoxyphenyl)-4-methyl-1,4-diazepane

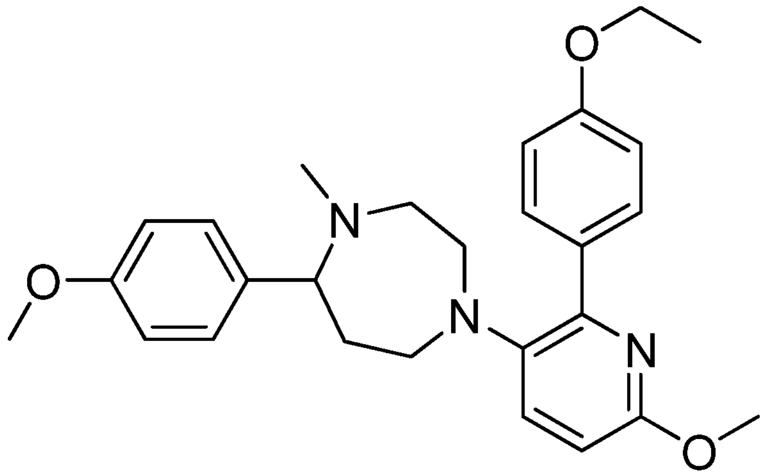

To a THF (2 mL) suspension of 1-[2-(4-ethoxyphenyl)-6-methoxypyridin-3-yl]-4-methyl-1,4-diazepan-5-one (300 mg, 0.84 mmol) synthesized in Reference Example 51, a 0.50 M solution of bromo(4-methoxyphenyl)magnesium in THF (5.04 mL, 2.52 mmol) was added dropwise, and the mixture was stirred at 60°C for 5 hours. After standing to cool to room temperature, sodium cyanotrihydroborate (211 mg, 3.36 mmol) and acetic acid (3 mL) were added thereto, and the mixture was stirred at room temperature for 16 hours. The reaction solution was neutralized by addition of an aqueous sodium hydroxide solution, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 80:20-0:100) and basic silica gel column chromatography (hexane-ethyl acetate 100:0-90:10) to obtain 55 mg of the title compound (yield: 14%). Melting point: 103-107°C (diethyl ether).
$^1$H-NMR ($CDCl_3$) : δ 1.44 (3H, t, J = 7.0 Hz), 1.79-2.10 (2H, m), 2.17 (3H, s), 2.80-3.31 (6H, m), 3.36 (1H, dd, J = 7.2, 4.2 Hz), 3.79 (3H, s), 3.93 (3H, s), 4.09 (2H, q, J = 7.2 Hz), 6.59 (1H, d, J = 8.7 Hz), 6.80-6.89 (2H, m), 6.90-7.00 (2H, m), 7.19-7.34 (2H, m), 7.43 (1H, d, J = 8.7 Hz), 7.97-8.08 (2H, m).

Example 100

1-[2-(4-ethoxyphenyl)-6-methoxypyridin-3-yl]-4-methyl-5-(4-methylphenyl)-1,4-diazepane

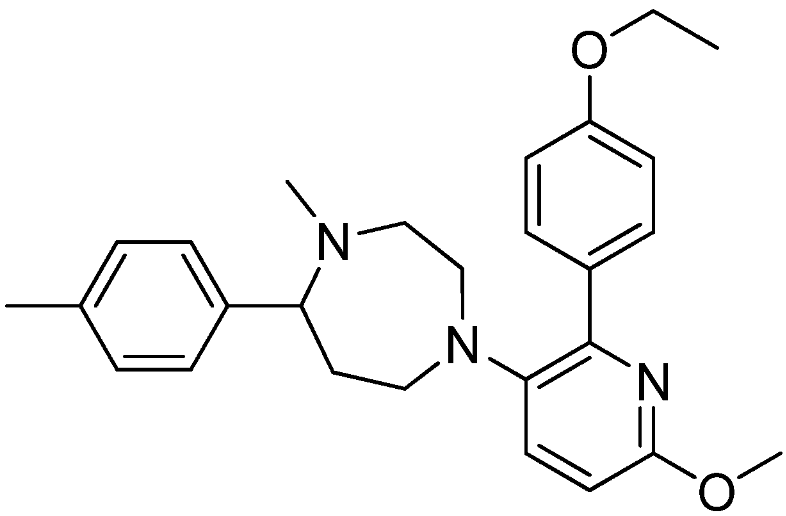

The title compound was obtained in the same way as in Example 99 using 1-[2-(4-ethoxyphenyl)-6-methoxypyridin-3-yl]-4-methyl-1,4-diazepan-5-one (300 mg, 0.84 mmol) synthesized in Reference Example 51 and a 1.0 M solution of bromo(4-methylphenyl)magnesium in THF (2.52 mL, 2.52 mmol). Yield: 6%, oil.
$^1$H-NMR ($CDCl_3$) : δ 1.44 (3H, t, J = 7.0 Hz), 1.85-2.10 (2H, m), 2.18 (3H, s), 2.32 (3H, s), 2.82-3.30 (6H, m), 3.32-3.43

(1H, m), 3.93 (3H, s), 4.09 (2H, q, J = 6.8 Hz), 6.59 (1H, d, J = 8.7 Hz), 6.93 (2H, d, J = 8.7 Hz), 7.06-7.15 (2H, m), 7.23 (2H, d, J = 8.0 Hz), 7.43 (1H, d, J = 8.7 Hz), 8.03 (2H, d, J = 9.1 Hz).

Example 101

1-[2-(4-ethoxyphenyl)-6-methoxypyridin-3-yl]-5-(4-fluorophenyl)-4-methyl-1,4-diazepane

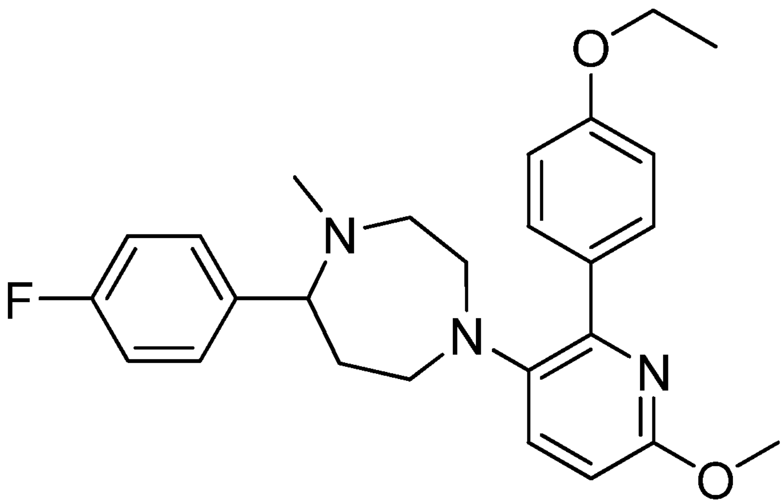

The title compound was obtained in the same way as in Example 99 using 1-[2-(4-ethoxyphenyl)-6-methoxypyridin-3-yl]-4-methyl-1,4-diazepan-5-one (300 mg, 0.84 mmol) synthesized in Reference Example 51 and a 1.0 M solution of bromo(4-fluorophenyl)magnesium in THF (2.52 mL, 2.52 mmol). Yield: 7%, oil.
$^1$H-NMR ($CDCl_3$) : δ 1.45 (3H, t, J = 7.0 Hz), 1.88-2.05 (2H, m), 2.17 (3H, s), 2.84-3.29 (6H, m), 3.41 (1H, brs), 3.93 (3H, s), 4.09 (2H, q, J = 7.2 Hz), 6.60 (1H, d, J = 8.3 Hz), 6.89-7.03 (4H, m), 7.31 (2H, dd, J = 8.7, 5.7 Hz), 7.43 (1H, d, J = 8.7 Hz), 8.02 (2H, d, J = 9.0 Hz).

Example 102

4-{6-methoxy-3-[4-methyl-5-(4-methylphenyl)-1,4-diazepan-1-yl]pyridin-2-yl}-N,N-dimethylaniline

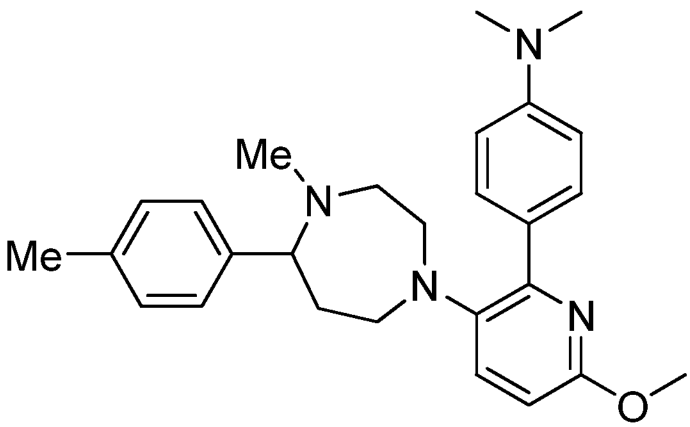

The title compound was obtained in the same way as in Example 99 using 1-{2-[4-(dimethylamino)phenyl]-6-methoxypyridin-3-yl}-4-methyl-1,4-diazepan-5-one (300 mg, 0.846 mmol) synthesized in Reference Example 50 and a 1.0 M solution of bromo(4-methylphenyl)magnesium in THF (2.5 mL, 2.5 mmol). Yield: 5%, oil.

$^1$H-NMR ($CDCl_3$): δ 1.89-2.11 (2H, m), 2.19 (3H, s), 2.32 (3H, s), 2.86-2.97 (1H, m), 2.98-3.32 (11H, m), 3.39 (1H, dd, J = 3.6, 7.5 Hz), 3.93 (3H, s), 6.54 (1H, d, J = 8.7 Hz), 6.73-6.81 (2H, m), 7.08-7.15 (2H, m), 7.20-7.28 (2H, m), 7.41 (1H, d, J = 8.7 Hz), 8.03-8.11 (2H, m).

Example 103

4-{3-[5-(4-fluorophenyl)-4-methyl-1,4-diazepan-1-yl]-6-methoxypyridin-2-yl}-N,N-dimethylaniline

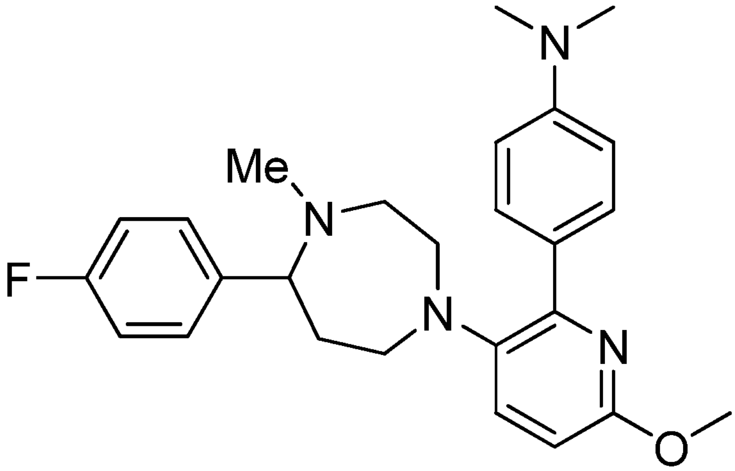

The title compound was obtained in the same way as in Example 99 using 1-{2-[4-(dimethylamino)phenyl]-6-methoxypyridin-3-yl}-4-methyl-1,4-diazepan-5-one (300 mg, 0.846 mmol) synthesized in Reference Example 50 and a 1.0 M solution of bromo(4-fluorophenyl)magnesium in THF (4.2 mL, 4.2 mmol). Yield: 5%, oil.
$^1$H-NMR ($CDCl_3$): δ 1.89-2.09 (2H, m), 2.18 (3H, s), 2.89-3.29 (12H, m), 3.43 (1H, dd, J = 4.2, 7.2 Hz), 3.94 (3H, s), 6.54 (1H, d, J = 8.7 Hz), 6.73-6.81 (2H, m), 6.93-7.03 (2H, m), 7.28-7.37 (2H, m), 7.41 (1H, d, J = 8.7 Hz), 8.03-8.11 (2H, m).

Example 104

4-{3-[4-ethyl-5-(4-methylphenyl)-1,4-diazepan-1-yl]-6-methoxypyridin-2-yl}-N,N-dimethylaniline

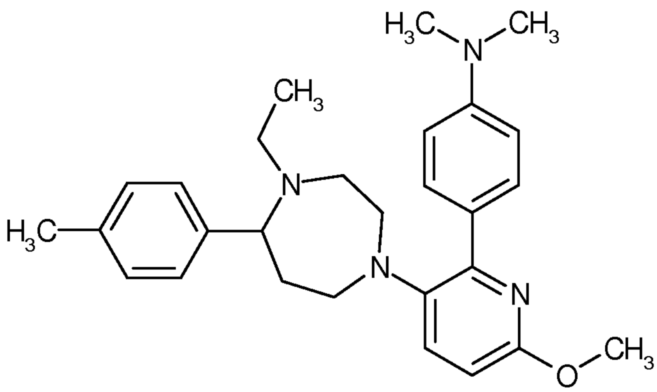

The title compound was obtained in the same way as in Example 99 using 1-{2-[4-(dimethylamino)phenyl]-6-methoxypyridin-3-yl}-4-ethyl-1,4-diazepan-5-one (330 mg, 0.896 mmol) synthesized in Reference Example 52 and a 1.0 M solution of bromo(4-methylphenyl)magnesium in THF (2.69 mL, 2.69 mmol). Yield: 31%, oil.
$^1$H-NMR ($CDCl_3$) : δ 0.91 (3H, t, J = 7.0 Hz), 1.90-2.16 (2H, m), 2.28-2.57 (5H, m), 2.88-3.25 (12H, m), 3.70-3.79 (1H, m), 3.93 (3H, s), 6.53 (1H, d, J = 8.3 Hz), 6.76 (2H, d, J = 9.1 Hz), 7.11 (2H, d, J = 8.0 Hz), 7.28 (2H, d, J = 8.0 Hz), 7.41

(1H, d, J = 8.7 Hz), 8.10 (2H, d, J = 9.1 Hz).

Example 105

4-{3-[4-ethyl-5-(4-fluorophenyl)-1,4-diazepan-1-yl]-6-methoxypyridin-2-yl}-N,N-dimethylaniline

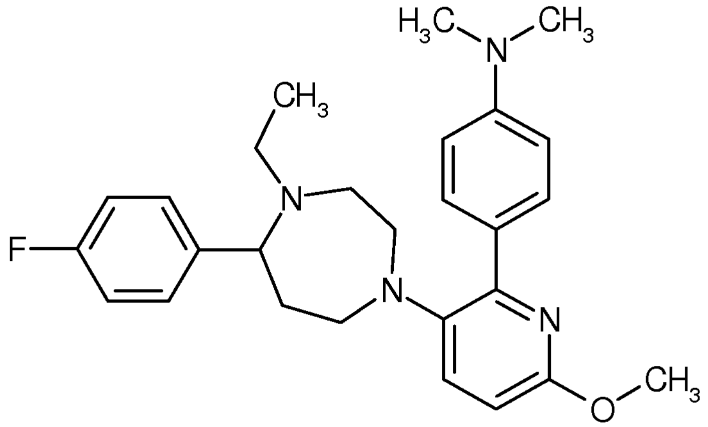

The title compound was obtained in the same way as in Example 99 using 1-{2-[4-(dimethylamino)phenyl]-6-methoxypyridin-3-yl}-4-ethyl-1,4-diazepan-5-one (490 mg, 1.32 mmol) synthesized in Reference Example 52 and a 1.0 M solution of bromo(4-fluorophenyl)magnesium in THF (6.60 mL, 6.60 mmol). Yield: 21%, oil.
$^1$H-NMR ($CDCl_3$) : δ 0.91 (3H, t, J = 7.0 Hz), 1.83-2.00 (1H, m), 2.03-2.15 (1H, m), 2.30-2.54 (2H, m), 2.87-3.21 (12H, m), 3.73-3.83 (1H, m), 3.93 (3H, s), 6.54 (1H, d, J = 8.7 Hz), 6.76 (2H, d, J = 9.1 Hz), 6.97 (2H, t, J = 8.7 Hz), 7.30-7.44 (3H, m), 8.10 (2H, d, J = 9.1 Hz).

Example 106

4-{3-[4-ethyl-5-(4-methoxyphenyl)-1,4-diazepan-1-yl]-6-methoxypyridin-2-yl}-N,N-dimethylaniline

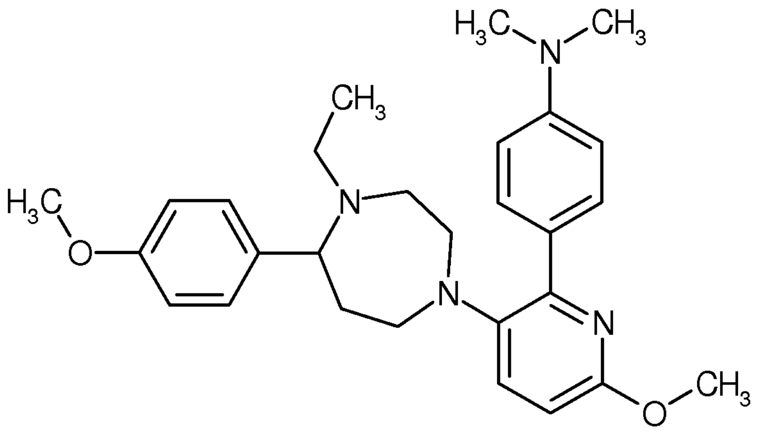

The title compound was obtained in the same way as in Example 99 using 1-{2-[4-(dimethylamino)phenyl]-6-methoxypyridin-3-yl}-4-ethyl-1,4-diazepan-5-one (490 mg, 1.32 mmol) synthesized in Reference Example 52 and a 0.50 M solution of bromo(4-methoxyphenyl)magnesium in THF (13.2 mL, 6.60 mmol). Yield: 40%, oil.
$^1$H-NMR ($CDCl_3$) : δ 0.91 (3H, t, J = 7.0 Hz), 1.90-2.15 (2H, m), 2.29-2.57 (2H, m), 2.86-3.24 (12H, m), 3.68-3.76 (1H, m), 3.79 (3H, s), 3.93 (3H, s), 6.53 (1H, d, J = 8.7 Hz), 6.76 (2H, d, J = 8.7 Hz), 6.84 (2H, d, J = 8.7 Hz), 7.31 (2H, d, J = 8.7 Hz), 7.42 (1H, d, J = 8.3 Hz), 8.10 (2H, d, J = 9.1 Hz).

Example 107

4-{6-methoxy-3-[2-(4-methylphenyl)morpholin-4-yl]pyridin-2-yl}-N,N-dimethylaniline

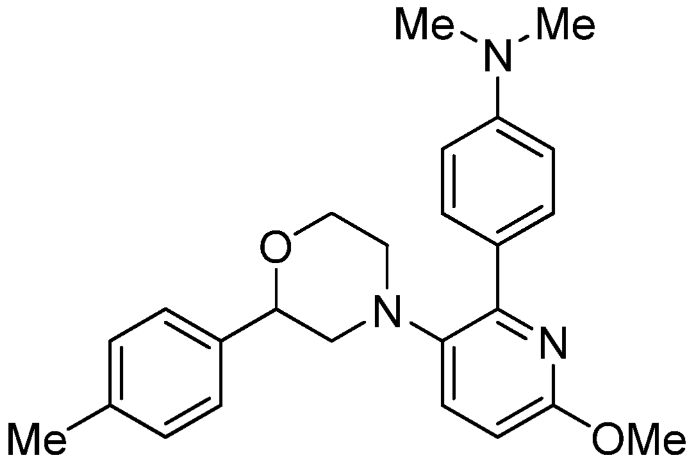

The title compound was obtained in the same way as in Example 1 using 4-(3-bromo-6-methoxypyridin-2-yl)-N,N-dimethylaniline (500 mg, 1.63 mmol) synthesized in Reference Example 3 and 2-(4-methylphenyl)morpholine (350 mg, 1.96 mmol). Yield: 46%, amorphous solid.
$^1$H-NMR ($CDCl_3$) : δ 2.32 (3H, s), 2.65 (1H, dd, J = 9.5, 10.2 Hz), 2.72-2.84 (1H, m), 2.91-3.01 (1H, m), 3.03 (6H, s), 3.05-3.13 (1H, m), 3.82-3.93 (1H, m), 3.94 (3H, s), 3.95-4.04 (1H, m), 4.61 (1H, dd, J = 2.1, 10.2 Hz), 6.54 (1H, d, J = 8.4 Hz), 6.76-6.84 (2H, m), 7.08-7.22 (4H, m), 7.30 (1H, d, J = 8.4 Hz), 8.13-8.20 (2H, m).

Example 108

1-[2-(4-ethoxyphenyl)-6-methoxypyridin-3-yl]-4-ethyl-5-(4-methoxyphenyl)-1,4-diazepane

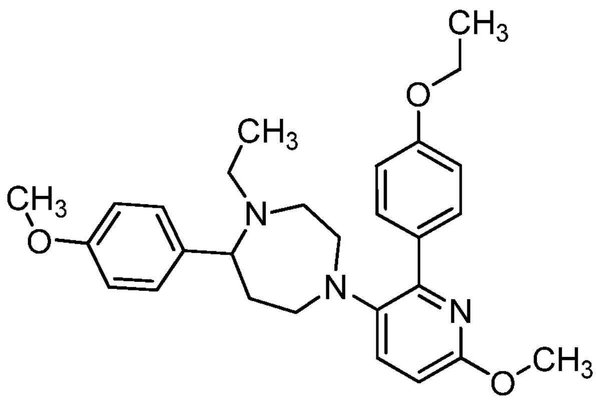

The title compound was obtained in the same way as in Example 99 using 1-[2-(4-ethoxyphenyl)-6-methoxypyridin-3-yl]-4-ethyl-1,4-diazepan-5-one (300 mg, 0.81 mmol) synthesized in Reference Example 53 and a 0.5 M solution of bromo (4-methoxyphenyl)magnesium in THF (4.86 mL, 2.43 mmol). Yield: 30%, oil.
$^1$H-NMR ($CDCl_3$) : δ 0.91 (3H, t, J = 7.0 Hz), 1.44 (3H, t, J = 7.0 Hz), 1.83-2.15 (2H, m), 2.25-2.58 (2H, m), 2.83-3.03 (3H, m), 3.03-3.18 (3H, m), 3.72 (1H, t, J = 5.5 Hz), 3.79 (3H, s), 3.93 (3H, s), 4.09 (2H, q, J = 7.2 Hz), 6.59 (1H, d, J = 8.7 Hz), 6.84 (2H, d, J = 8.3 Hz), 6.93 (2H, d, J = 8.7 Hz), 7.30 (2H, d, J = 8.3 Hz), 7.43 (1H, d, J = 8.7 Hz), 8.05 (2H, d, J = 8.7 Hz).

Example 109

1-[2-(4-ethoxyphenyl)-6-methoxypyridin-3-yl]-4-ethyl-5-(4-methylphenyl)-1,4-diazepane

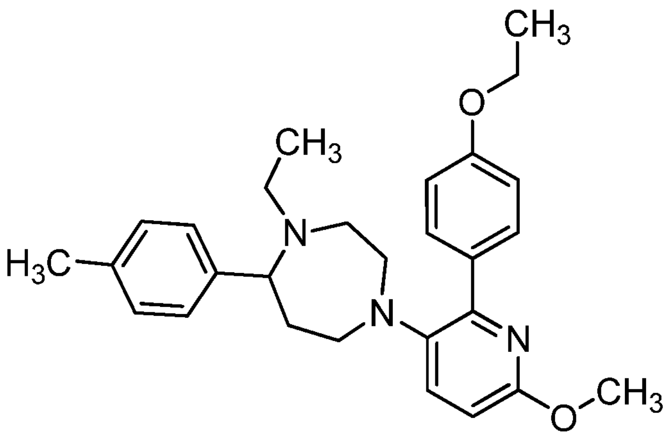

The title compound was obtained in the same way as in Example 99 using 1-[2-(4-ethoxyphenyl)-6-methoxypyridin-3-yl]-4-ethyl-1,4-diazepan-5-one (300 mg, 0.81 mmol) synthesized in Reference Example 53 and a 1.0 M solution of bromo (4-methylphenyl)magnesium in THF (2.43 mL, 2.43 mmol). Yield: 30%, oil.
$^1$H-NMR ($CDCl_3$) : δ 0.91 (3H, t, J = 7.2 Hz), 1.44 (3H, t, J = 7.0 Hz), 1.86-2.14 (2H, m), 2.32 (3H, s), 2.34-2.56 (2H, m), 2.85-3.03 (3H, m), 3.03-3.19 (3H, m), 3.73 (1H, t, J = 5.7 Hz), 3.93 (3H, s), 4.09 (2H, q, J = 6.8 Hz), 6.58 (1H, d, J = 8.7 Hz), 6.92 (2H, d, J = 9.0 Hz), 7.11 (2H, d, J = 7.9 Hz), 7.23-7.32 (2H, m), 7.43 (1H, d, J = 8.7 Hz), 8.06 (2H, d, J = 8.7 Hz).

Example 110

1-[2-(4-ethoxyphenyl)-6-methoxypyridin-3-yl]-4-ethyl-5-(4-fluorophenyl)-1,4-diazepane

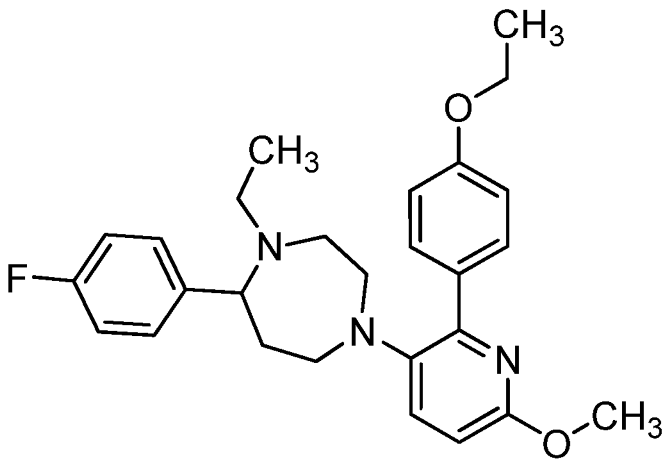

The title compound was obtained in the same way as in Example 99 using 1-[2-(4-ethoxyphenyl)-6-methoxypyridin-3-yl]-4-ethyl-1,4-diazepan-5-one (300 mg, 0.81 mmol) synthesized in Reference Example 53 and a 1.0 M solution of bromo (4-fluorophenyl)magnesium in THF (2.43 mL, 2.43 mmol). Yield: 28%, oil.
$^1$H-NMR ($CDCl_3$) : δ 0.91 (3H, t, J = 7.2 Hz), 1.44 (3H, t, J = 7.0 Hz), 1.82-1.96 (1H, m), 1.99-2.14 (1H, m), 2.27-2.52 (2H, m), 2.85-3.02 (3H, m), 3.02-3.20 (3H, m), 3.76 (1H, t, J = 5.7 Hz), 3.93 (3H, s), 4.09 (2H, q, J = 6.8 Hz), 6.59 (1H, d, J = 8.7 Hz), 6.88-7.04 (4H, m), 7.34 (2H, dd, J = 8.5, 5.5 Hz), 7.43 (1H, d, J = 8.7 Hz), 8.05 (2H, d, J = 9.0 Hz).

Example 111

4-{6-methoxy-3-[5-(3-methoxyphenyl)-4-methyl-1,4-diazepan-1-yl]pyridin-2-yl}-N,N-dimethylaniline

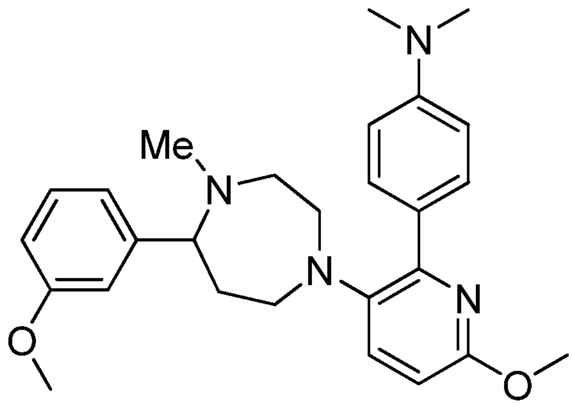

The title compound was obtained in the same way as in Example 99 using 1-{2-[4-(dimethylamino)phenyl]-6-methoxypyridin-3-yl}-4-methyl-1,4-diazepan-5-one (320 mg, 0.903 mmol) synthesized in Reference Example 50 and a 1.0 M solution of bromo(3-methoxyphenyl)magnesium in THF (4.5 mL, 4.5 mmol). Yield: 10%, oil.
$^1$H-NMR ($CDCl_3$): δ 1.90-2.14 (2H, m), 2.21 (3H, s), 2.86-3.31 (12H, m), 3.41 (1H, dd, J = 4.2, 7.2 Hz), 3.81 (3H, s), 3.93 (3H, s), 6.54 (1H, d, J = 8.4 Hz), 6.71-6.82 (3H, m), 6.89-7.00 (2H, m), 7.21 (1H, t, J = 7.5 Hz), 7.41 (1H, d, J = 8.4 Hz), 8.03-8.11 (2H, m).

Example 112

4-{3-[5-(2,4-dimethoxyphenyl)-4-ethyl-1,4-diazepan-1-yl]-6-methoxypyridin-2-yl}-N,N-dimethylaniline

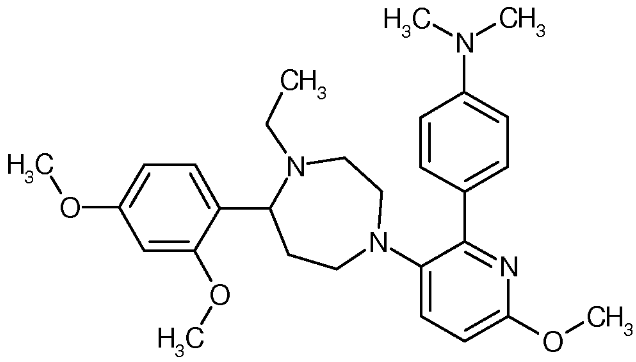

The title compound was obtained in the same way as in Example 99 using 1-{2-[4-(dimethylamino)phenyl]-6-methoxypyridin-3-yl}-4-ethyl-1,4-diazepan-5-one (283 mg, 0.768 mmol) synthesized in Reference Example 52 and a 0.5 M solution of bromo(2,4-dimethoxyphenyl)magnesium in THF (7.68 mL, 3.84 mmol). Yield: 25%, amorphous solid.
$^1$H-NMR ($CDCl_3$) : δ 0.92 (3H, t, J = 7.2 Hz), 1.85-1.99 (1H, m), 2.04-2.16 (1H, m), 2.27-2.54 (2H, m), 2.84-3.22 (12H, m), 3.77 (3H, s), 3.80 (3H, s), 3.93 (3H, s), 4.07-4.16 (1H, m), 6.38-6.58 (3H, m), 6.76 (2H, d, J = 9.1 Hz), 7.41 (1H, d, J = 8.7 Hz), 7.61 (1H, d, J = 8.3 Hz), 8.14 (2H, d, J = 9.1 Hz).

Example 113

1-[2-(4-ethoxy-2-fluorophenyl)-6-methoxypyridin-3-yl]-4-(4-methoxyphenyl)piperazine

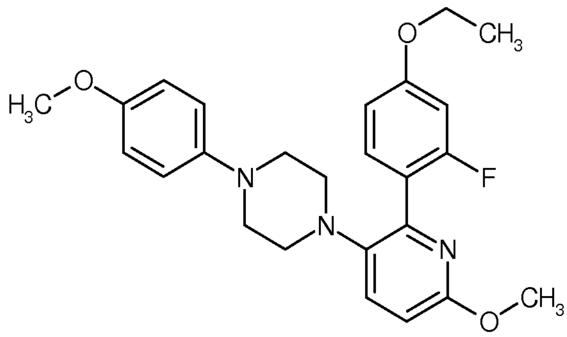

The title compound was obtained in the same way as in Example 48 using 1-(2-bromo-6-methoxypyridin-3-yl)-4-(4-methoxyphenyl)piperazine (190 mg, 0.505 mmol) synthesized in Reference Example 21 and 2-(4-ethoxy-2-fluorophenyl)-4,4,5,5-tetramethyl-1,3,2-dioxaborolane (269 mg, 1.01 mmol) synthesized in Reference Example 54. Yield: 81%. Melting point: 148-149°C (heptane-ethyl acetate).
$^{1}$H-NMR ($CDCl_3$) : δ 1.43 (3H, t, J = 7.0 Hz), 2.87-3.02 (8H, m), 3.76 (3H, s), 3.92 (3H, s), 4.05 (2H, q, J = 7.2 Hz), 6.61-6.69 (1H, m), 6.70-6.78 (2H, m), 6.79-6.90 (4H, m), 7.44-7.54 (2H, m).

Example 114

1-(4-fluorophenyl)-4-{6-methoxy-2-[4-(1-methylethyl)phenyl]pyridin-3-yl}piperazine

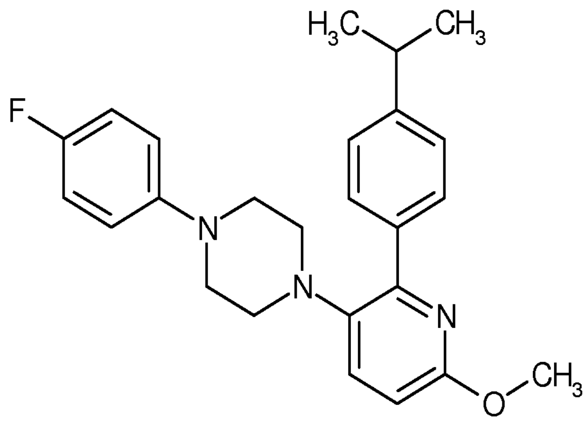

The title compound was obtained in the same way as in Example 1 using 3-bromo-6-methoxy-2-[4-(1-methylethyl)phenyl] pyridine (459 mg, 1.50 mmol) synthesized in Reference Example 11 and 1-(4-fluorophenyl)piperazine (541 mg, 3.00 mmol). Yield: 24%. Melting point: 108-110°C (heptane).
$^{1}$H-NMR ($CDCl_3$) : δ 1.28 (6H, d, J = 6.8 Hz), 2.88-3.02 (5H, m), 3.08-3.17 (4H, m), 3.96 (3H, s), 6.66 (1H, d, J = 8.7 Hz), 6.83-7.01 (4H, m), 7.22-7.30 (2H, m), 7.43 (1H, d, J = 8.7 Hz), 8.08 (2H, d, J = 8.3 Hz).

Example 115

1-(3,4-difluorophenyl)-4-{6-methoxy-2-[4-(1-methylethyl)phenyl]pyridin-3-yl}piperazine

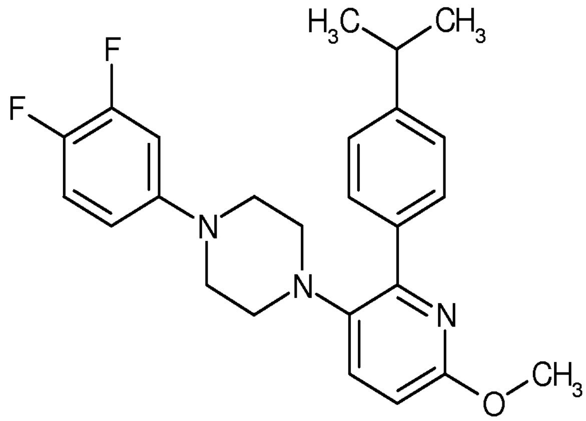

The title compound was obtained in the same way as in Example 1 using 3-bromo-6-methoxy-2-[4-(1-methylethyl)phenyl] pyridine (459 mg, 1.50 mmol) synthesized in Reference Example 11 and 1-(3,4-difluorophenyl)piperazine (595 mg, 3.00 mmol). Yield: 25%. Melting point: 93-95°C (heptane).
$^1$H-NMR ($CDCl_3$) : δ 1.28 (6H, d, J = 6.8 Hz), 2.85-3.04 (5H, m), 3.06-3.19 (4H, m), 3.96 (3H, s), 6.51-6.79 (3H, m), 6.96-7.11 (1H, m), 7.20-7.34 (2H, m), 7.42 (1H, d, J = 8.7 Hz), 8.07 (2H, d, J = 8.3 Hz).

Example 116

5-(4-methoxyphenyl)-1-{6-methoxy-2-[4-(trifluoromethoxy)phenyl]pyridin-3-yl}-4-methyl-1,4-diazepane

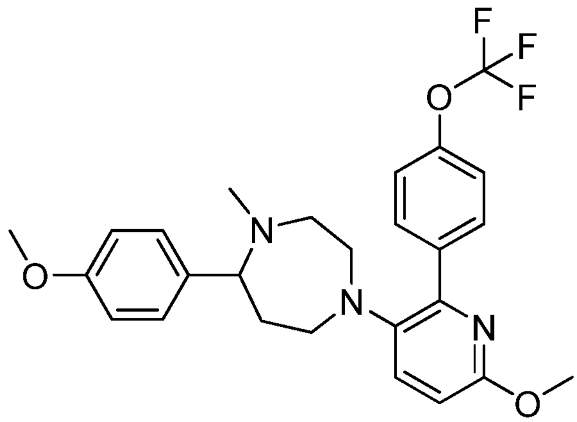

The title compound was obtained in the same way as in Example 99 using 1-{6-methoxy-2-[4-(trifluoromethoxy)phenyl] pyridin-3-yl}-4-methyl-1,4-diazepan-5-one (300 mg, 0.76 mmol) synthesized in Reference Example 56 and a 0.5 M solution of bromo(4-methoxyphenyl)magnesium in THF (4.56 mL, 2.28 mmol). Yield: 17%, oil.
$^1$H-NMR ($CDCl_3$) : δ 1.84-2.04 (2H, m), 2.16 (3H, s), 2.80-3.02 (2H, m), 3.03-3.18 (3H, m), 3.20-3.31 (1H, m), 3.34 (1H, dd, J = 7.3, 4.3 Hz), 3. 79 (3H, s), 3. 93 (3H, s), 6.67 (1H, d, J = 8.7 Hz), 6.84 (2H, d, J = 8.7 Hz), 7.25 (4H, d, J = 6.4 Hz), 7.48 (1H, d, J = 8.7 Hz), 8.09 (2H, d, J = 8.7 Hz).

Example 117

1-{2-[4-(dimethylamino)phenyl]-6-methoxypyridin-3-yl}-4-(4-methoxyphenyl)-6,6-dimethyl-1,4-diazepan-5-one

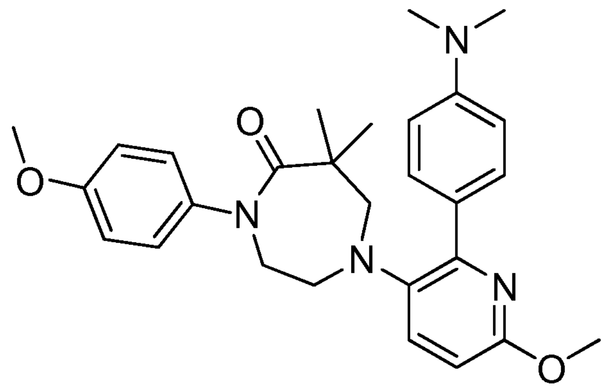

To a THF (2 mL) solution of diisopropylamine (0.191 mL, 1.35 mmol), a 1.6 M solution of butyllithium in hexane (0.844 mL, 1.35 mmol) was added dropwise at -78°C, and the mixture was stirred for 30 minutes. To the reaction solution, a THF (6 mL) solution of 1-{2-[4-(dimethylamino)phenyl]-6-methoxypyridin-3-yl}-4-(4-methoxyphenyl)-1,4-diazepan-5-one (200 mg, 0.45 mmol) synthesized in Example 92 was added at -78°C, and the mixture was stirred for 1 hour. To the reaction solution, iodomethane (0.084 mL, 1.35 mmol) was added at -78°C, and the mixture was then stirred at room temperature for 1 hour. To the reaction solution, a saturated aqueous solution of ammonium chloride was added, followed by extraction with ethyl acetate. The obtained organic phase was washed with saturated brine and dried over anhydrous magnesium sulfate. The solvent was distilled off under reduced pressure, and the obtained residue was dissolved again in THF (6 mL). The solution was added at -78°C to a mixture prepared from diisopropylamine (0.191 mL, 1.35 mmol), a 1.6 M solution of butyllithium in hexane (0.844 mL, 1.35 mmol), and THF (2 mL), and the mixture was stirred for 1 hour. To the reaction solution, iodomethane (0.084 mL, 1.35 mmol) was added at -78°C, and the mixture was then stirred at room temperature for 1 hour. To the reaction solution, a saturated aqueous solution of ammonium chloride was added, followed by extraction with ethyl acetate. The obtained organic phase was washed with saturated brine and dried over anhydrous magnesium sulfate. The solvent was distilled off under reduced pressure, and the obtained residue was fractionated by silica gel column chromatography (hexane-ethyl acetate 100:0-50:50). A solution of a fraction containing the principal product was concentrated to obtain 109 mg of the title compound (yield: 51%) as an amorphous solid.
$^1$H-NMR ($CDCl_3$) : δ 1.35 (6H, s), 2.95-3.02 (8H, m), 3.03 (2H, s), 3.65-3.75 (2H, m), 3.77 (3H, s), 3.95 (3H, s), 6.61 (1H, d, J = 8.7 Hz), 6.76 (2H, d, J = 9.0 Hz), 6.80-6.87 (2H, m), 6.94-7.02 (2H, m), 7.44 (1H, d, J = 8.7 Hz), 7.87-7.96 (2H, m).

Example 118

1-{2-[4-(dimethylamino)phenyl]-6-methoxypyridin-3-yl}-4-(4-methoxyphenyl)-6-methyl-1,4-diazepan-5-one

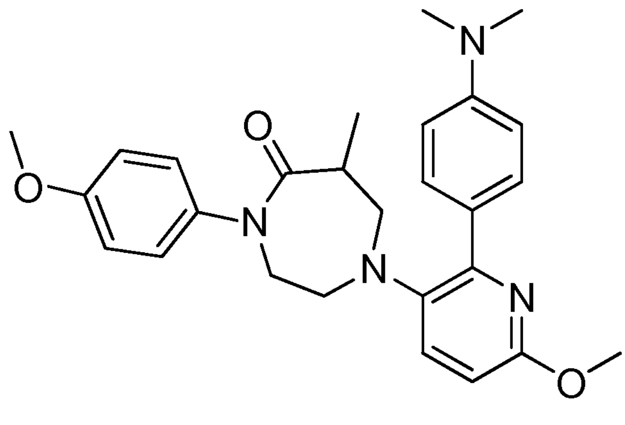

A solution of a fraction containing side products in Example 117 was concentrated to obtain 24.8 mg of the title compound (yield: 12%) as an amorphous solid.
$^1$H-NMR ($CDCl_3$) : δ 1.05 (3H, d, J = 6.8 Hz), 2.80-3.26 (11H, m), 3.53 (1H, dd, J = 15.3, 5.1 Hz), 3.79 (3H, s), 3.95 (3H, s), 4.14 (1H, dd, J = 15.4, 10.2 Hz), 6.58 (1H, d, J = 8.7 Hz), 6.78 (2H, d, J = 9.0 Hz), 6.83-6.93 (2H, m), 7.04-7.14 (2H, m), 7.35 (1H, d, J = 8.7 Hz), 8.05 (2H, d, J = 9.0 Hz).

Example 119

4-{6-methoxy-3-[4-(4-methoxyphenyl)-6,6-dimethyl-1,4-diazepan-1-yl]pyridin-2-yl}-N,N-dimethylaniline

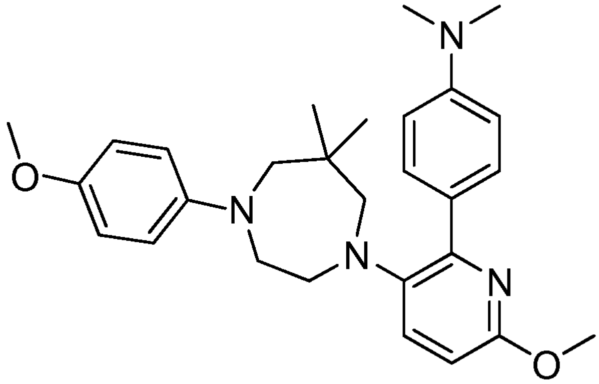

To a THF (1.5 mL) solution of 1-{2-[4-(dimethylamino)phenyl]-6-methoxypyridin-3-yl}-4-(4-methoxyphenyl)-6,6-dimethyl-1,4-diazepan-5-one (80 mg, 0.17 mmol) synthesized in Example 117, lithium aluminum hydride (12.9 mg, 0.34 mmol) was added under ice cooling, and the mixture was then refluxed for 5 hours. After standing to cool to room temperature, water (0.0129 mL), a 15% aqueous sodium hydroxide solution (0.0129 mL), and water (0.0387 mL) were added dropwise in this order to the reaction solution, and the obtained suspension was filtered through celite. The filtrate was concentrated under reduced pressure, and the obtained residue was purified by silica gel column chromatography (hexane-ethyl acetate 100:0-90:10) to obtain 70 mg of the title compound (yield: 89%) as an amorphous solid.
$^1$H-NMR ($CDCl_3$) : δ 0.93 (6H, s), 2.80 (2H, s), 2.96 (6H, s), 3.06-3.13 (2H, m), 3.13-3.21 (2H, m), 3.25 (2H, s), 3.76 (3H, s), 3.94 (3H, s), 6.56 (1H, d, J = 8.7 Hz), 6.68 (4H, dd, J = 9.1, 2.3 Hz), 6.75-6.84 (2H, m), 7.42 (1H, d, J = 8.7 Hz), 7.90-7.97 (2H, m).

Example 120

1-{2-[4-(1-methylethyl)phenyl]pyridin-3-yl}-4-(4-methylphenyl)piperazine

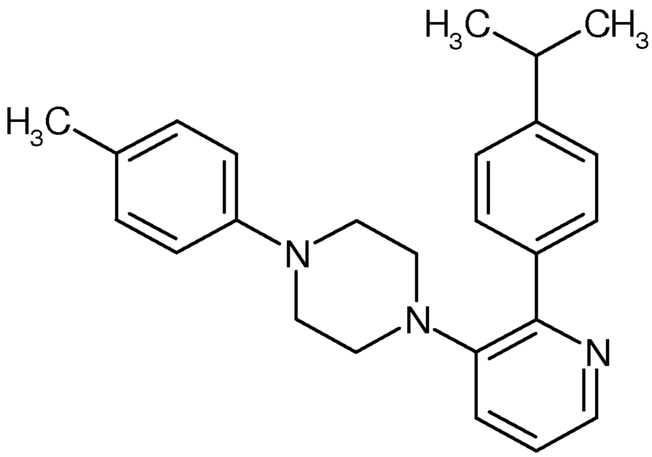

The title compound was obtained in the same way as in Example 1 using 3-bromo-2-[4-(1-methylethyl)phenyl]pyridine (1.00 g, 3.62 mmol) synthesized in Reference Example 58 and 1-(4-methylphenyl)piperazine (770 mg, 4.35 mmol). Yield: 45%, amorphous solid.
$^1$H-NMR ($CDCl_3$): δ 1.27 (6H, d, J = 6.8 Hz), 2.27 (3H, s), 2.93 (1H, septet, 6.8 Hz), 3.02-3.13 (8H, m), 6.83-6.85 (2H, m), 7.07-7.09 (2H, m), 7.17 (1H, dd, J = 8.0, 4.4 Hz), 7.25-7.27 (2H, m), 7.36 (1H, dd, J = 8.0, 1.2 Hz), 7.89-7.92 (2H,

m), 8.35 (1H, dd, J = 4.4, 1.2 Hz).

Example 121

1-{6-bromo-2-[4-(1-methylethyl)phenyl]pyridin-3-yl}-4-(4-methylphenyl)piperazine

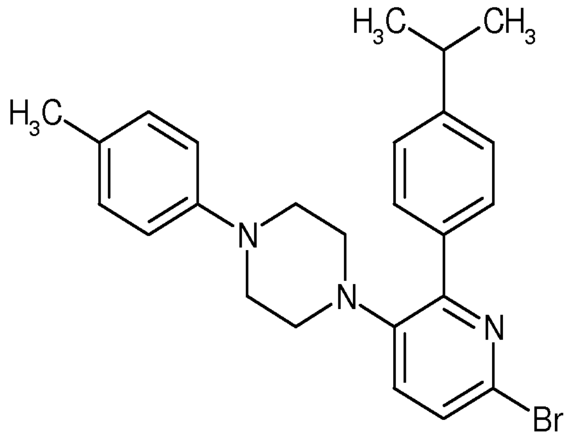

The title compound was obtained in the same way as in Example 36 using 1-{2-[4-(1-methylethyl)phenyl]pyridin-3-yl}-4-(4-methylphenyl)piperazine (5.00 g, 13.5 mmol) synthesized in Example 120. Yield: 40%, amorphous solid.
$^1$H-NMR ($CDCl_3$): δ 1.26 (6H, d, J = 6.8 Hz), 2.27 (3H, s), 2.94 (1H, septet, J = 6.8 Hz), 2.98-3.13 (8H, m), 6.82-6.84 (2H, m), 7.07-7.09 (2H, m), 7.22-7.26 (4H, m), 7.33 (1H, d, J = 8.4 Hz), 7.92 (1H, d, J = 8.4 Hz).

Example 122

4-{3-[4-(3-fluoro-4-methylphenyl)-1,4-diazepan-1-yl]-6-methoxypyridin-2-yl}-N,N-dimethylaniline dihydrochloride

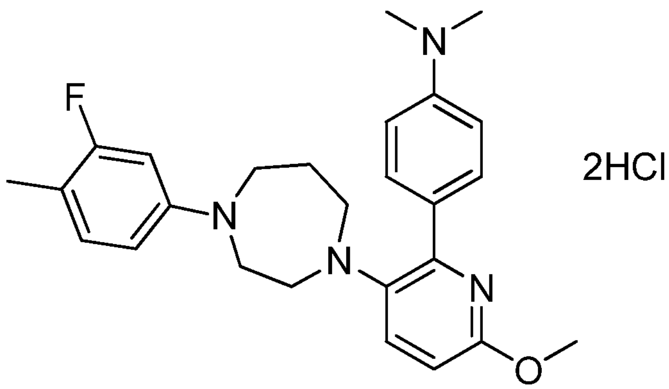

A mixture of 4-[3-(1,4-diazepan-1-yl)-6-methoxypyridin-2-yl]-N,N-dimethylaniline (200 mg, 0.61 mmol) synthesized in Reference Example 38, 4-bromo-2-fluoro-1-methylbenzene (0.116 mL, 0.92 mmol), palladium acetate (6.96 mg, 0.031 mmol), BINAP (57.3 mg, 0.092 mmol), sodium tert-butoxide (176 mg, 1.83 mmol), and toluene (2 mL) was stirred at 150°C for 1 hour under microwave irradiation. After cooling to room temperature, water was added to the reaction solution, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 100:0-90:10). The obtained compound was converted to hydrochloride using 4 N hydrochloric acid/ethyl acetate to obtain 88.5 mg of the title compound (yield: 29%).
$^1$H-NMR (DMSO-$d_6$) : δ 1.60-1.78 (2H, m), 2.09 (3H, s), 2.87 (2H, t, J = 5.5 Hz), 3.08 (8H, s), 3.39-3.54 (4H, m), 3.83 (3H, s), 6.40-6.58 (2H, m), 6.72 (1H, d, J = 8.7 Hz), 7.04 (1H, t, J = 9.3 Hz), 7.38 (2H, brs), 7.63 (1H, d, J = 8.7 Hz), 7.96

(2H, d, J = 8.7 Hz).

Example 123

1-{6-methoxy-2-[4-(1-methylethyl)phenyl]pyridin-3-yl}-5-(4-methoxyphenyl)-4-methyl-1,4-diazepane

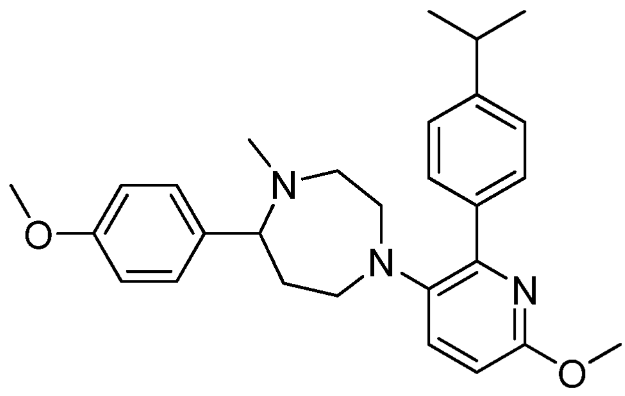

The title compound was obtained in the same way as in Example 99 using 1-{6-methoxy-2-[4-(1-methylethyl)phenyl] pyridin-3-yl}-4-methyl-1,4-diazepan-5-one (300 mg, 0.85 mmol) synthesized in Reference Example 59 and a 0.5 M solution of bromo(4-methoxyphenyl)magnesium in THF (5.10 mL, 2.55 mmol). Yield: 16%, oil.
$^{1}$H-NMR ($CDCl_3$) : δ 1.29 (6H, d, J = 6.8 Hz), 1.84-1.96 (1H, m), 2.04 (1H, brs), 2.16 (3H, s), 2.80-3.20 (6H, m), 3.25 (1H, d, J = 9.4 Hz), 3.32-3.42 (1H, m), 3.79 (3H, s), 3.93 (3H, s), 6.62 (1H, d, J = 8.7 Hz), 6.79-6.89 (2H, m), 7.20-7.32 (4H, m), 7.45 (1H, d, J = 8.7 Hz), 7.96 (2H, d, J = 8.3 Hz).

Example 124

1-[6-methoxy-2-(4-methoxyphenyl)pyridin-3-yl]-5-(4-methoxyphenyl)-4-methyl-1,4-diazepane

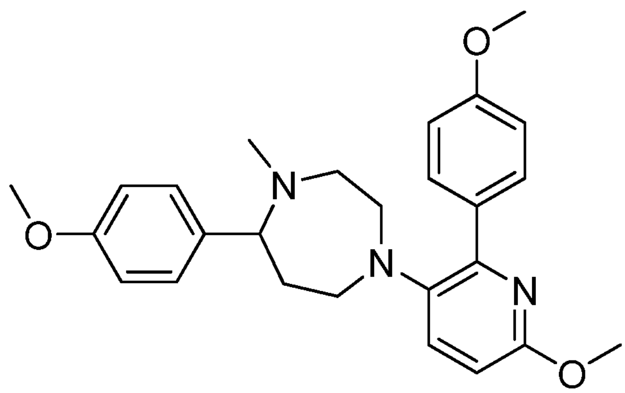

The title compound was obtained in the same way as in Example 99 using 1-[6-methoxy-2-(4-methoxyphenyl)pyridin-3-yl]-4-methyl-1,4-diazepan-5-one (300 mg, 0.88 mmol) synthesized in Reference Example 61 and a 0.5 M solution of bromo(4-methoxyphenyl)magnesium in THF (5.28 mL, 2.64 mmol). Yield: 22%, oil.
$^{1}$H-NMR ($CDCl_3$): δ 1.85-2.11 (2H, m), 2.17 (3H, s), 2.82-3.19 (5H, m), 3.19-3.31 (1H, m), 3.36 (1H, dd, J = 7.5, 4.1 Hz), 3.79 (3H, s), 3.86 (3H, s), 3.93 (3H, s), 6.60 (1H, d, J = 8.7 Hz), 6.80-6.89 (2H, m), 6.90-7.00 (2H, m), 7.21-7.31 (2H, m), 7.44 (1H, d, J = 8.7 Hz), 8.00-8.08 (2H, m).

Example 125

4-{3-[4-(2-fluoro-4-methylphenyl)-1,4-diazepan-1-yl]-6-methoxypyridin-2-yl}-N,N-dimethylaniline

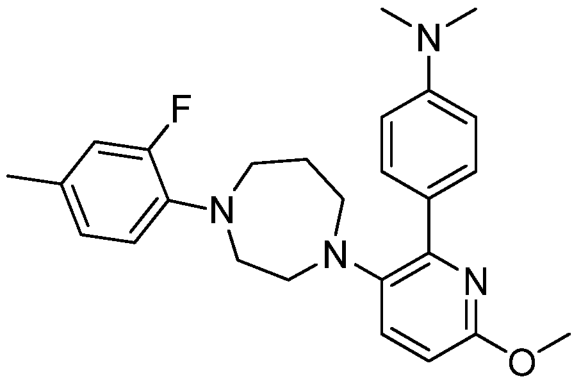

The title compound was obtained in the same way as in Example 83 using 4-[3-(1,4-diazepan-1-yl)-6-methoxypyridin-2-yl]-N,N-dimethylaniline (200 mg, 0.61 mmol) synthesized in Reference Example 38 and 1-bromo-2-fluoro-4-methylbenzene (0.116 mL, 0.92 mmol). Yield: 42%, oil.
$^1$H-NMR ($CDCl_3$): δ 1.87-2.00 (2H, m), 2.25 (3H, s), 2.99 (6H, s), 3.06-3.14 (2H, m), 3.15-3.23 (2H, m), 3.31-3.37 (2H, m), 3.39 (2H, t, J = 6.2 Hz), 3.94 (3H, s), 6.54 (1H, d, J = 8.7 Hz), 6.69-6.88 (5H, m), 7.43 (1H, d, J = 8.7 Hz), 8.06 (2H, d, J = 9.1 Hz).

Example 126

1-[2-(4-ethoxyphenyl)-6-methoxypyridin-3-yl]-4-(4-methoxyphenyl)-1,4-diazepane

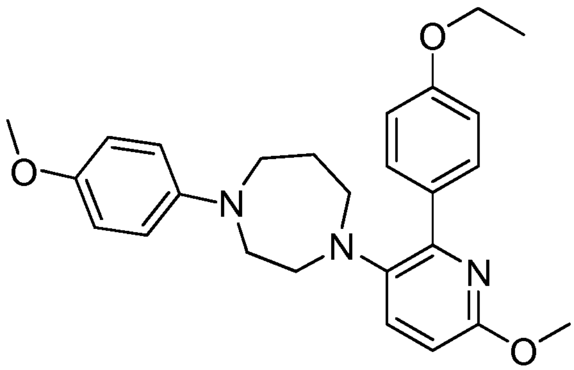

The title compound was obtained in the same way as in Example 83 using 1-[2-(4-ethoxyphenyl)-6-methoxypyridin-3-yl]-1,4-diazepane (200 mg, 0.61 mmol) synthesized in Reference Example 62 and 1-bromo-4-methoxybenzene (0.115 mL, 0.92 mmol). Yield: 20%, amorphous solid.
$^1$H-NMR ($CDCl_3$) : δ 1.44 (3H, t, J = 7.0 Hz), 1.77-1.90 (2H, m), 2.92-2.99 (2H, m), 3.05-3.15 (2H, m), 3.42-3.54 (4H, m), 3.75 (3H, s), 3.93 (3H, s), 4.07 (2H, q, J = 6.8 Hz), 6.57 (1H, d, J = 8.7 Hz), 6.60-6.69 (2H, m), 6.77-6.84 (2H, m), 6.84-6.92 (2H, m), 7.37 (1H, d, J = 8.7 Hz), 7.90-8.00 (2H, m).

Example 127

1-{6-methoxy-2-[4-(1-methylethyl)phenyl]pyridin-3-yl}-4-(4-methylphenyl)piperazine

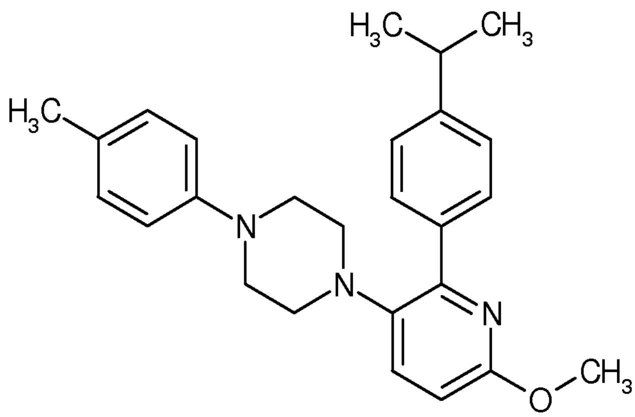

The title compound was obtained in the same way as in Example 1 using 3-bromo-6-methoxy-2-[4-(1-methylethyl)phenyl] pyridine (459 mg, 1.50 mmol) synthesized in Reference Example 11 and 1-(4-methylphenyl)piperazine (487 mg, 3.00 mmol). Yield: 52%. Melting point: 128-129°C (heptane).
$^1$H-NMR ($CDCl_3$): δ 1.27 (6H, d, J = 6.8 Hz), 2.27 (3H, s), 2.87-3.03 (5H, m), 3.10-3.20 (4H, m), 3.96 (3H, s), 6.65 (1H, d, J = 8.3 Hz), 6.80-6.89 (2H, m), 7.04-7.13 (2H, m), 7.21-7.29 (2H, m), 7.43 (1H, d, J = 8.7 Hz), 8.06-8.13 (2H, m).

Example 128

1-(4-methoxyphenyl)-4-[6-methoxy-2-(1,3,5-trimethyl-1H-pyrazol-4-yl)pyridin-3-yl]piperazine

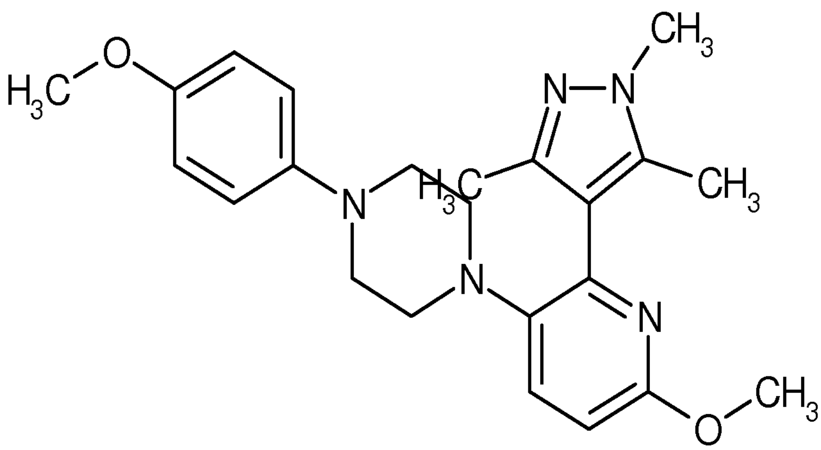

The title compound was obtained in the same way as in Example 48 using 1-(2-bromo-6-methoxypyridin-3-yl)-4-(4-methoxyphenyl)piperazine (200 mg, 0.529 mmol) synthesized in Reference Example 21 and 1,3,5-trimethyl-4-(4,4,5,5-tetramethyl-1,3,2-dioxaborolan-2-yl)-1H-pyrazole (250 mg, 1.06 mmol). Yield: 63%. Melting point: 125-128°C (heptane-ethyl acetate).
$^1$H-NMR ($CDCl_3$) : δ 2.22-2.26 (3H, m), 2.26-2.31 (3H, m), 2.87-2.97 (4H, m), 2.97-3.07 (4H, m), 3.76 (3H, s), 3.77 (3H, s), 3.91 (3H, s), 6.66 (1H, d, J = 8.7 Hz), 6.78-6.92 (4H, m), 7.42 (1H, d, J = 8.7 Hz).

Example 129

4-{6-ethoxy-3-[4-(4-methoxyphenyl)piperazin-1-yl]pyridin-2-yl}-N,N-dimethylaniline

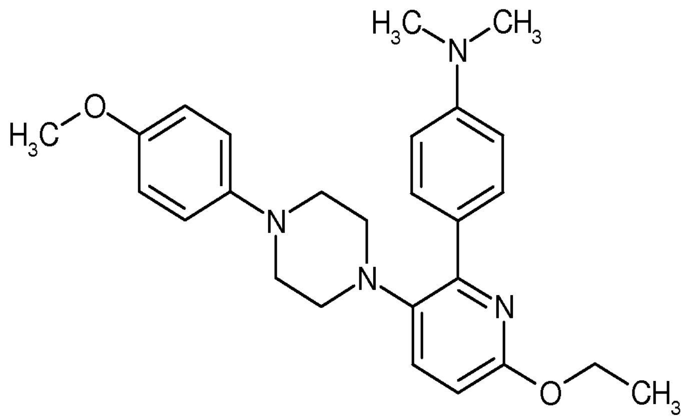

The title compound was obtained in the same way as in Example 48 using 1-(2-bromo-6-ethoxypyridin-3-yl)-4-(4-methoxyphenyl)piperazine (156 mg, 0.400 mmol) synthesized in Reference Example 64 and [4-(dimethylamino)phenyl]boronic acid (132 mg, 0.800 mmol). Yield: 87%. Melting point: 160-163°C (heptane-ethyl acetate).
$^1$H-NMR ($CDCl_3$): δ 1.40 (3H, t, J = 7.2 Hz), 2.95-3.04 (10H, m), 3.10-3.18 (4H, m), 3.77 (3H, s), 4.40 (2H, q, J = 6.9 Hz), 6.56 (1H, d, J = 8.7 Hz), 6.74 (2H, d, J = 9.0 Hz), 6.82-6.95 (4H, m), 7.39 (1H, d, J = 8.7 Hz), 8.16 (2H, d, J = 9.0 Hz).

Example 130

1-[6-ethoxy-2-(4-ethoxyphenyl)pyridin-3-yl]-4-(4-methoxyphenyl)piperazine

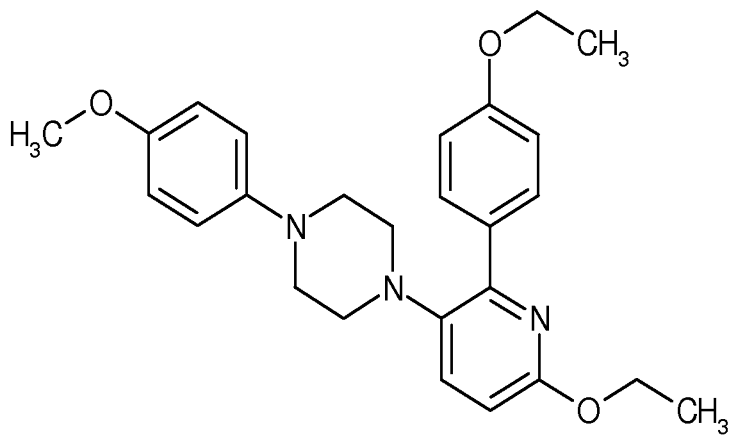

The title compound was obtained in the same way as in Example 48 using 1-(2-bromo-6-ethoxypyridin-3-yl)-4-(4-methoxyphenyl)piperazine (156 mg, 0.400 mmol) synthesized in Reference Example 64 and (4-ethoxyphenyl)boronic acid (133 mg, 0.800 mmol). Yield: 90%. Melting point: 131-134°C (heptane-ethyl acetate).
$^1$H-NMR ($CDCl_3$) : δ 1.33-1.49 (6H, m), 2.93-3.04 (4H, m), 3.05-3.15 (4H, m), 3.77 (3H, s), 4.08 (2H, q, J = 7.2 Hz), 4.39 (2H, q, J = 7.2 Hz), 6.61 (1H, d, J = 8.7 Hz), 6.80-6.96 (6H, m), 7.41 (1H, d, J = 8.3 Hz), 8.12 (2H, d, J = 8.7 Hz).

Example 131

4-{6-methoxy-3-[4-(4-methoxyphenyl)piperazin-1-yl]pyridin-2-yl}phenol

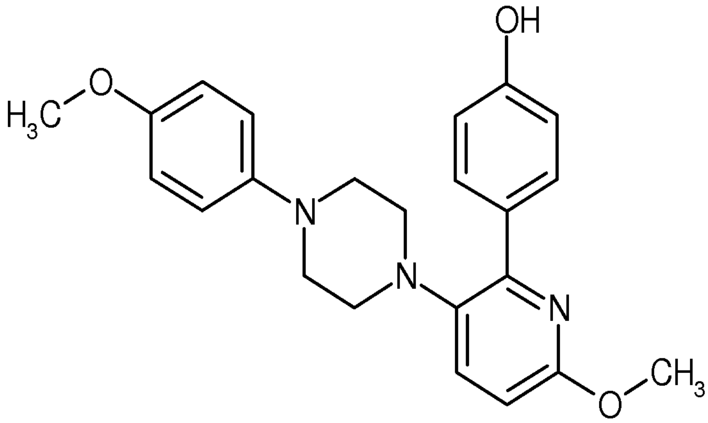

The title compound was obtained in the same way as in Example 48 using 1-(2-bromo-6-methoxypyridin-3-yl)-4-(4-methoxyphenyl)piperazine (1.00 g, 2.64 mmol) synthesized in Reference Example 21 and (4-hydroxyphenyl)boronic acid (729 mg, 5.29 mmol). Yield: 100%. Melting point: 211-213°C (heptane-ethyl acetate).
$^1$H-NMR ($CDCl_3$) : δ 2.93-3.04 (4H, m), 3.06-3.16 (4H, m), 3.77 (3H, s), 3.95 (3H, s), 4.84 (1H, s), 6.64 (1H, d, J = 8.7 Hz), 6.80-6.98 (6H, m), 7.43 (1H, d, J = 8.7 Hz), 8.10 (2H, d, J = 8.7 Hz).

Example 132

4-{6-methoxy-3-[4-(4-methoxyphenyl)piperazin-1-yl]pyridin-2-yl}benzonitrile

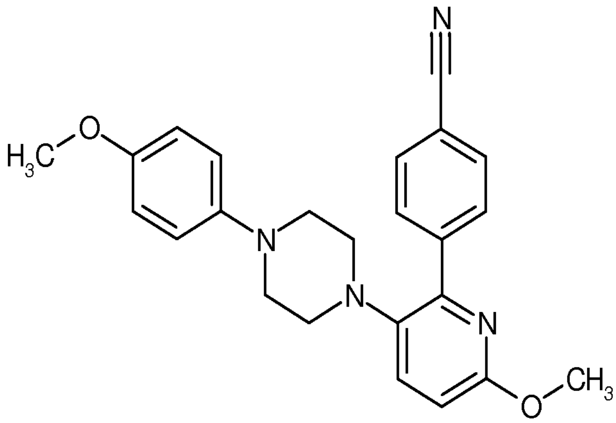

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (4-cyanophenyl)boronic acid (120 mg, 0.800 mmol). Yield: 86%. Melting point: 159-161°C (heptane-ethyl acetate).
$^1$H-NMR ($CDCl_3$) : δ 2.90-3.03 (4H, m), 3.04-3.16 (4H, m), 3.78 (3H, s), 3.95 (3H, s), 6.76 (1H, d, J = 8.7 Hz), 6.81-6.96 (4H, m), 7.51 (1H, d, J = 8.7 Hz), 7.69 (2H, d, J = 8.7 Hz), 8.29 (2H, d, J = 8.7 Hz).

Example 133

1-[2-(3,5-dimethylisoxazol-4-yl)-6-methoxypyridin-3-yl]-4-(4-methoxyphenyl)piperazine

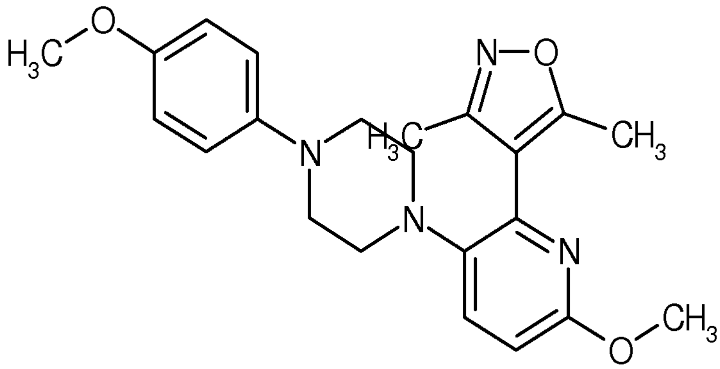

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and 3,5-dimethyl-4-(4,4,5,5-tetramethyl-1,3,2-dioxaborolan-2-yl)isoxazole (179 mg, 0.800 mmol). Yield: 71%. Melting point: 179-181°C (heptane-ethyl acetate).
$^1$H-NMR ($CDCl_3$) : δ 2.34 (3H, s), 2.44 (3H, s), 2.89-2.98 (4H, m), 3.02-3.10 (4H, m), 3.77 (3H, s), 3.90 (3H, s), 6.73 (1H, d, J = 8.7 Hz), 6.81-6.93 (4H, m), 7.49 (1H, d, J = 8.7 Hz).

Example 134

4-{6-methoxy-3-[4-(4-methoxyphenyl)-5-methyl-1,4-diazepan-1-yl]pyridin-2-yl}-N,N-dimethylaniline

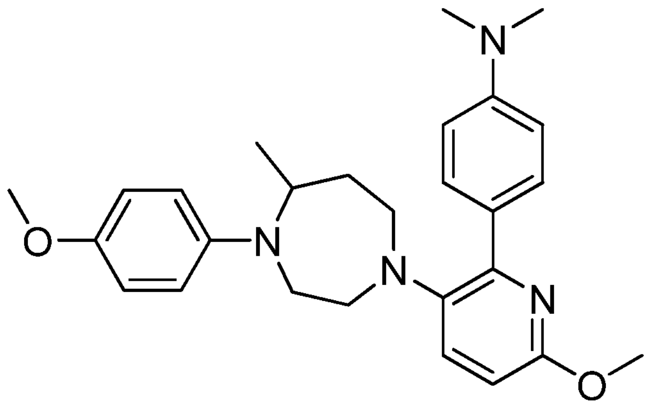

The title compound was obtained in the same way as in Example 99 using 1-{2-[4-(dimethylamino)phenyl]-6-methoxypyridin-3-yl}-4-(4-methoxyphenyl)-1,4-diazepan-5-one (200 mg, 0.45 mmol) synthesized in Example 92 and a 1 M solution of methyl magnesium bromide in THF (1.35 mL, 1.35 mmol). Yield: 18%, amorphous solid.
$^1$H-NMR ($CDCl_3$) : δ 1.17 (3H, d, J = 6.4 Hz), 1.85-2.20 (2H, m), 2.77-3.15 (4H, m), 3.02 (6H, s), 3.34-3.60 (2H, m), 3.69-3.82 (4H, m), 3.94 (3H, s), 6.51 (1H, d, J = 8.7 Hz), 6.56-6.64 (2H, m), 6.75-6.80 (2H, m), 6.80-6.86 (2H, m), 7.34 (1H, d, J = 8.7 Hz), 8.11-8.20 (2H, m).

Example 135

4-{3-[4-(3-fluoro-4-methoxyphenyl)-1,4-diazepan-1-yl]-6-methoxypyridin-2-yl}-N,N-dimethylaniline

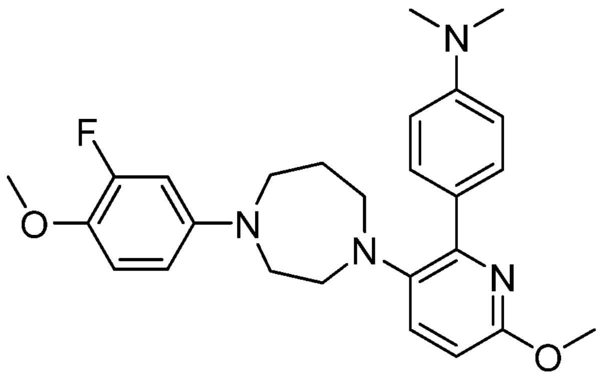

The title compound was obtained in the same way as in Example 83 using 4-[3-(1,4-diazepan-1-yl)-6-methoxypyridin-2-yl]-N,N-dimethylaniline (200 mg, 0.61 mmol) synthesized in Reference Example 38 and 4-bromo-2-fluoro-1-methoxybenzene (0.122 mL, 0.92 mmol). Yield: 23%. Melting point: 130-138°C (ethyl acetate-hexane). $^1$H-NMR ($CDCl_3$) : δ 1.81-1.93 (2H, m), 2.93-3.02 (8H, m), 3.06-3.15 (2H, m), 3.41-3.55 (4H, m), 3.82 (3H, s), 3.93 (3H, s), 6.29-6.37 (1H, m), 6.45 (1H, dd, J = 14.8, 3.0 Hz), 6.52 (1H, d, J = 8.3 Hz), 6.69 (2H, d, J = 9.1 Hz), 6.86 (1H, t, J = 9.3 Hz), 7.35 (1H, d, J = 8.7 Hz), 7.91-8.03 (2H, m).

Example 136

1-[2-(4-ethoxyphenyl)-6-methoxypyridin-3-yl]-4-(3-fluoro-4-methylphenyl)-1,4-diazepane

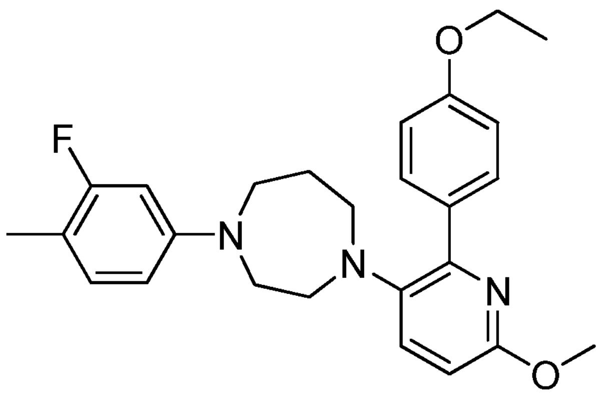

The title compound was obtained in the same way as in Example 83 using 1-[2-(4-ethoxyphenyl)-6-methoxypyridin-3-yl]-1,4-diazepane (200 mg, 0.61 mmol) synthesized in Reference Example 62 and 4-bromo-2-fluoro-1-methylbenzene (0.116 mL, 0.92 mmol). Yield: 48%, oil.
$^1$H-NMR ($CDCl_3$) : δ 1.44 (3H, t, J = 7.0 Hz), 1.75-1.88 (2H, m), 2.15 (3H, s), 2.89-2.99 (2H, m), 3.05-3.13 (2H, m), 3.41-3.46 (2H, m), 3.49 (2H, t, J = 6.4 Hz), 3.92 (3H, s), 4.07 (2H, q, J = 7.2 Hz), 6.31 (1H, dd, J = 7.7, 2.1 Hz), 6.33-6.37 (1H, m), 6.57 (1H, d, J = 8.7 Hz), 6.83-6.89 (2H, m), 6.92-7.02 (1H, m), 7.36 (1H, d, J = 8.7 Hz), 7.88-7.95 (2H, m).

Example 137

1-[2-(4-ethoxy-3-fluorophenyl)-6-methoxypyridin-3-yl]-4-(4-methoxyphenyl)-1,4-diazepane

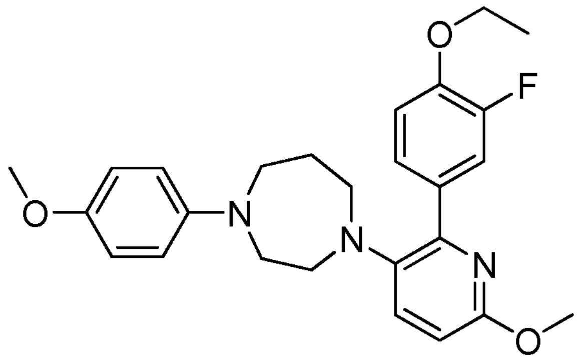

The title compound was obtained in the same way as in Example 83 using 1-[2-(4-ethoxy-3-fluorophenyl)-6-methoxypyridin-3-yl]-1,4-diazepane (200 mg, 0.58 mmol) synthesized in Reference Example 66 and 1-bromo-4-methoxybenzene (0.109 mL, 0.87 mmol). Yield: 13%. Melting point: 101-102°C (ethyl acetate-hexane).
$^1$H-NMR ($CDCl_3$) : δ 1.48 (3H, t, J = 7.0 Hz), 1.82-1.95 (2H, m), 2.93-3.02 (2H, m), 3.06-3.16 (2H, m), 3.45-3.55 (4H, m), 3.76 (3H, s), 3.93 (3H, s), 4.14 (2H, q, J = 6.8 Hz), 6.59 (1H, d, J = 8.7 Hz), 6.62-6.68 (2H, m), 6.78-6.85 (2H, m), 6.88 (1H, t, J = 8.7 Hz), 7.39 (1H, d, J = 8.7 Hz), 7.78-7.84 (1H, m), 7.89 (1H, dd, J = 13.2, 1.9 Hz).

Example 138

1-[2-(4-ethoxy-3-methylphenyl)-6-methoxypyridin-3-yl]-4-(4-methoxyphenyl)-1,4-diazepane

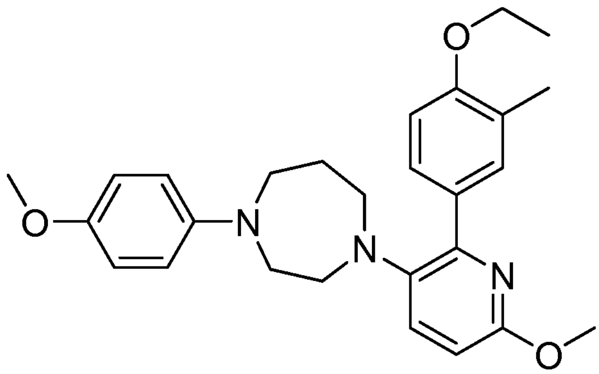

The title compound was obtained in the same way as in Example 83 using 1-[2-(4-ethoxy-3-methylphenyl)-6-methoxypyridin-3-yl]-1,4-diazepane (200 mg, 0.59 mmol) synthesized in Reference Example 68 and 1-bromo-4-methoxybenzene (0.112 mL, 0.89 mmol). Yield: 18%, oil.
$^1$H-NMR ($CDCl_3$) : δ 1.45 (3H, t, J = 7.0 Hz), 1.76-1.88 (2H, m), 2.24 (3H, s), 2.91-2.98 (2H, m), 3.07-3.14 (2H, m), 3.41-3.47 (2H, m), 3.50 (2H, t, J = 6.2 Hz), 3.75 (3H, s), 3.93 (3H, s), 4.07 (2H, q, J = 6.8 Hz), 6.56 (1H, d, J = 8.7 Hz), 6.60-6.66 (2H, m), 6.75-6.84 (3H, m), 7.36 (1H, d, J = 8.7 Hz), 7.77-7.82 (1H, m), 7.83 (1H, s).

Example 139

1-(4-methoxyphenyl)-4-{6-methoxy-2-[4-(2,2,2-trifluoroethoxy)phenyl]pyridin-3-yl}piperazine

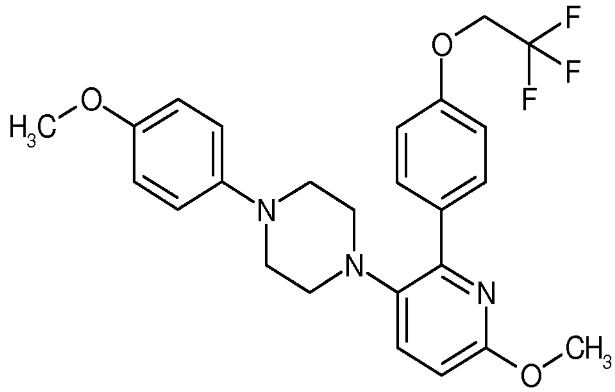

To a mixed solution of 4-{6-methoxy-3-[4-(4-methoxyphenyl)piperazin-1-yl]pyridin-2-yl}phenol (150 mg, 0.383 mmol) synthesized in Example 131 and potassium carbonate (79.4 mg, 0.575 mmol) in DMF (5 mL), 1,1,1-trifluoro-2-iodoethane (161 mg, 0.766 mmol) was added, and the mixture was stirred at 90°C for 12 hours. To the reaction solution, water was added, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 100:0-75:25) to obtain 30.6 mg of the title compound (yield: 17%). Melting point: 104-107°C (heptane-ethyl acetate).
$^1$H-NMR ($CDCl_3$): δ 2.92-3.03 (4H, m), 3.05-3.16 (4H, m), 3.78 (3H, s), 3.95 (3H, s), 4.33-4.46 (2H, m), 6.67 (1H, d, J = 8.7 Hz), 6.81-6.94 (4H, m), 6.97 (2H, d, J = 9.1 Hz), 7.45 (1H, d, J = 8.7 Hz), 8.18 (2H, d, J = 9.1 Hz).

Example 140

1-(4-methoxyphenyl)-4-(6-methoxy-2-phenylpyridin-3-yl)piperazine

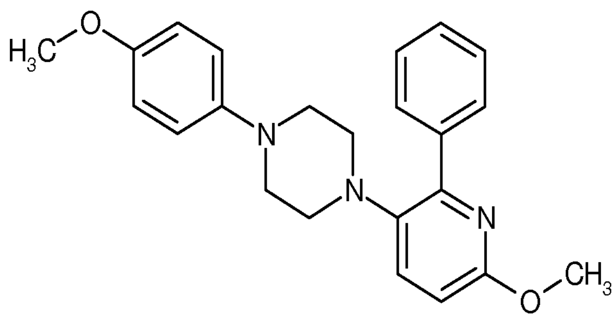

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and phenylboronic acid (97.5 mg, 0.800 mmol). Yield: 77%. Melting point: 141-144°C (heptane-ethyl acetate).
$^1$H-NMR ($CDCl_3$): δ 2.93-3.01 (4H, m), 3.03-3.14 (4H, m), 3.77 (3H, s), 3.96 (3H, s), 6.69 (1H, d, J = 8.7 Hz), 6.80-6.94 (4H, m), 7.29-7.49 (4H, m), 8.08-8.17 (2H, m).

Example 141

1-(4-methoxyphenyl)-4-(6-methoxy-2-thiophen-3-ylpyridin-3-yl)piperazine

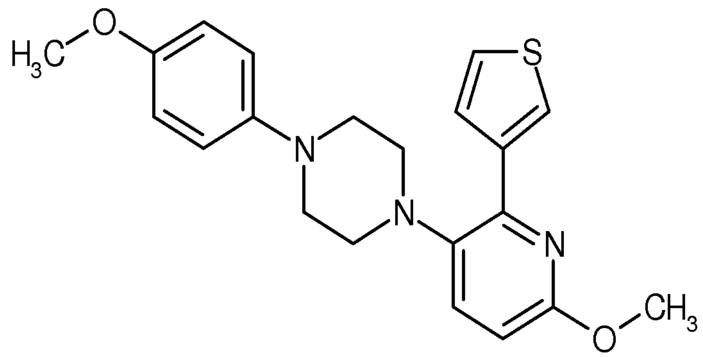

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and thiophen-3-ylboronic acid (102 mg, 0.800 mmol). Yield: 89%. Melting point: 150-152°C (heptane-ethyl acetate).
$^1$H-NMR ($CDCl_3$) : δ 3.01-3.10 (4H, m), 3.16-3.28 (4H, m), 3.79 (3H, s), 3.97 (3H, s), 6.65 (1H, d, J = 8.7 Hz), 6.83-6.91 (2H, m), 6.92-7.00 (2H, m), 7.28-7.34 (1H, m), 7.52 (1H, d, J = 8.7 Hz), 8.05-8.10 (1H, m), 8.40-8.45 (1H, m).

Example 142

1-[6-methoxy-2-(2-methylthiophen-3-yl)pyridin-3-yl]-4-(4-methoxyphenyl)piperazine

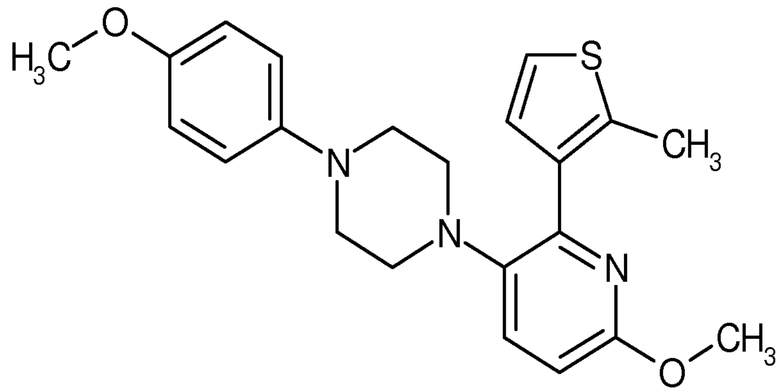

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and 4,4,5,5-tetramethyl-2-(2-methylthiophen-3-yl)-1,3,2-dioxaborolane (179 mg, 0.800 mmol). Yield: 71%. Melting point: 141-144°C (heptane-ethyl acetate).
$^1$H-NMR ($CDCl_3$) : δ 2.56 (3H, s), 2.89-2.99 (4H, m), 3.00-3.10 (4H, m), 3.77 (3H, s), 3.92 (3H, s), 6.67 (1H, d, J = 8.7 Hz), 6.80-6.93 (4H, m), 7.04 (1H, d, J = 5.3 Hz), 7.38- 7.44 (2H, m).

Example 143

1-[2-(2,5-dimethylthiophen-3-yl)-6-methoxypyridin-3-yl]-4-(4-methoxyphenyl)piperazine

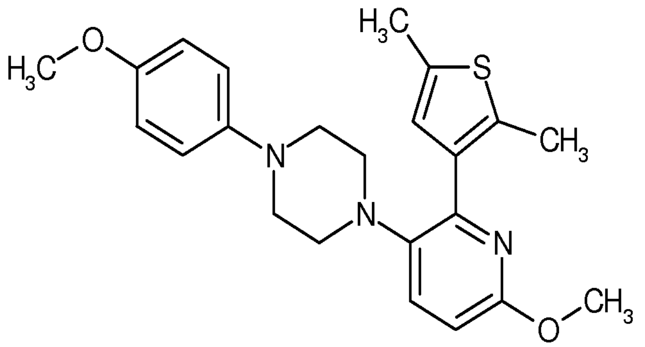

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (2,5-dimethylthiophen-3-yl)boronic acid (125 mg, 0.800 mmol). Yield: 74%. Melting point: 128-131°C (heptane-ethyl acetate).
$^1$H-NMR ($CDCl_3$): δ 2.42 (3H, s), 2.47 (3H, s), 2.88-3.00 (4H, m), 3.02-3.12 (4H, m), 3.77 (3H, s), 3.91 (3H, s), 6.65 (1H, d, J = 8.7 Hz), 6.80-6.95 (4H, m), 7.02-7.07 (1H, m), 7.38 (1H, d, J = 8.7 Hz).

Example 144

6'-ethoxy-6-methoxy-3-[4-(4-methoxyphenyl)-1,4-diazepan-1-yl]-2,3'-bipyridine

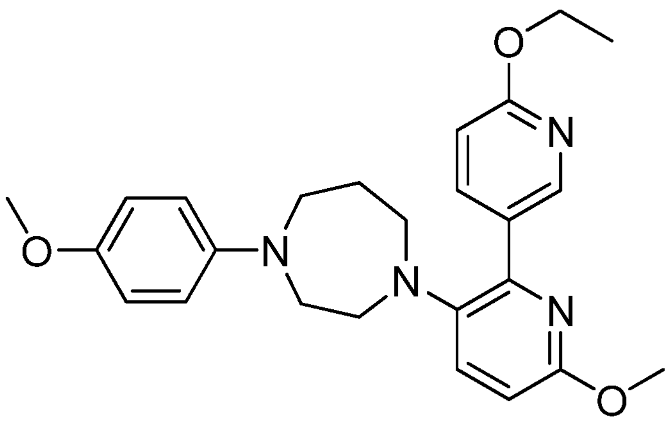

To a THF (6 mL) suspension of 1-(6'-ethoxy-6-methoxy-2,3'-bipyridin-3-yl)-1,4-diazepan-5-one (312 mg, 0.91 mmol) synthesized in Reference Example 69, lithium aluminum hydride (69.1 mg, 1.82 mmol) was carefully added under ice cooling, and the mixture was then refluxed for 14 hours. After cooling to room temperature, water (0.0691 mL), a 15% aqueous sodium hydroxide solution (0.0691 mL), and water (0.207 mL) were added dropwise in this order to the reaction solution, and the obtained suspension was filtered through celite. The filtrate was concentrated under reduced pressure, and the obtained residue was purified by basic silica gel column chromatography (hexane-ethyl acetate 70:30-0:100) to obtain 3-(1,4-diazepan-1-yl)-6'-ethoxy-6-methoxy-2,3'-bipyridine as a crude product. A mixture of the obtained crude product (116 mg), 1-bromo-4-methoxybenzene (0.0663 mL, 0.53 mmol), palladium acetate (4.04 mg, 0.018 mmol), BINAP (33.0 mg, 0.053 mmol), sodium tert-butoxide (101 mg, 1.05 mmol), and toluene (1 mL) was stirred at 150°C for 1 hour under microwave irradiation. After cooling to room temperature, water was added to the reaction solution, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 100:0-90:10) to obtain 18 mg of the title compound (yield: 4.6%). Oil.
$^1$H-NMR ($CDCl_3$) : δ 1.43 (3H, t, J = 7.0 Hz), 1.81-1.95 (2H, m), 2.92-3.01 (2H, m), 3.05-3.16 (2H, m), 3.44-3.56 (4H, m), 3.76 (3H, s), 3.92 (3H, s), 4.40 (2H, q, J = 7.2 Hz), 6.61 (1H, d, J = 7.2 Hz), 6.64 (2H, d, J = 7.6 Hz), 6.70 (1H, d, J = 9.1 Hz), 6.76-6.86 (2H, m), 7.41 (1H, d, J = 8.7 Hz), 8.26 (1H, dd, J = 8.7, 2.3 Hz), 8.83 (1H, d, J = 2.3 Hz).

Example 145

1-[2-(4-ethoxyphenyl)-6-methoxypyridin-3-yl]-4-(4-fluorophenyl)-1,4-diazepane

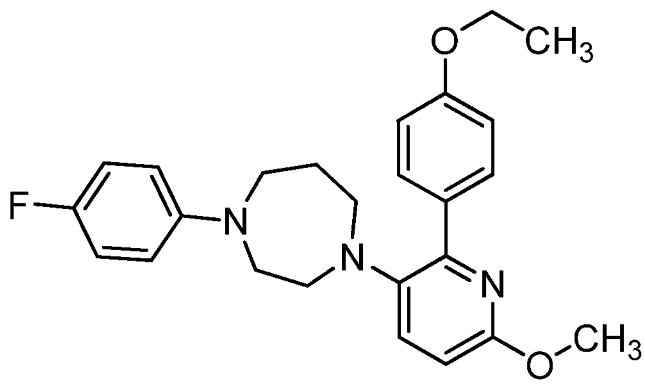

The title compound was obtained in the same way as in Example 83 using 1-[2-(4-ethoxyphenyl)-6-methoxypyridin-3-yl]-1,4-diazepane (200 mg, 0.61 mmol) synthesized in Reference Example 62 and 1-bromo-4-fluorobenzene (0.101 mL, 0.92 mmol). Yield: 24%.
Melting point: 115-130°C (ethyl acetate-hexane).
$^1$H-NMR ($CDCl_3$) : δ 1.44 (3H, t, J = 7.0 Hz), 1.76-1.90 (2H, m), 2.90-3.00 (2H, m), 3.05-3.14 (2H, m), 3.40-3.55 (4H, m), 3.93 (3H, s), 4.07 (2H, q, J = 6.8 Hz), 6.53-6.62 (3H, m), 6.83-6.88 (2H, m), 6.88-6.96 (2H, m), 7.37 (1H, d, J = 8.7 Hz), 7.88-7.98 (2H, m).

Example 146

1-[2-(4-ethoxyphenyl)-6-methoxypyridin-3-yl]-4-(2-fluoro-4-methylphenyl)-1,4-diazepan-5-one

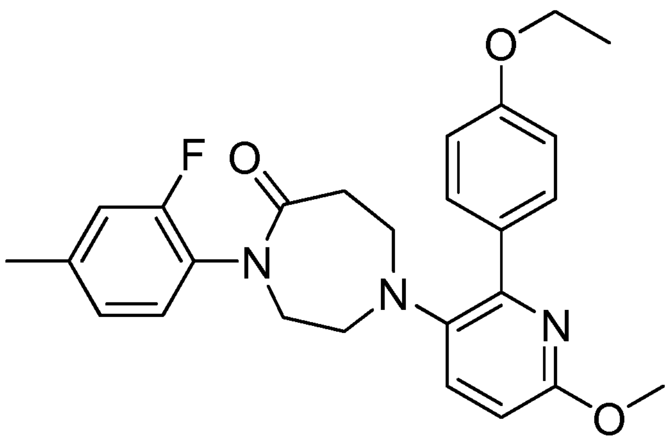

The title compound was obtained in the same way as in Example 94 using 1-[2-(4-ethoxyphenyl)-6-methoxypyridin-3-yl]-1,4-diazepan-5-one (500 mg, 1.46 mmol) synthesized in Reference Example 37 and 1-bromo-2-fluoro-4-methylbenzene (0.277 mL, 2.19 mmol). Yield: 14%. Melting point: 141-142°C (ethyl acetate-hexane).
$^1$H-NMR ($CDCl_3$) : δ 1.45 (3H, t, J = 7.0 Hz), 2.33 (3H, s), 2.82-2.93 (2H, m), 3.06-3.12 (2H, m), 3.12-3.19 (2H, m), 3.64-3.76 (2H, m), 3.95 (3H, s), 4.10 (2H, q, J = 7.2 Hz), 6.64 (1H, d, J = 8.7 Hz), 6.89-7.00 (4H, m), 7.06 (1H, t, J = 7.9 Hz), 7.43 (1H, d, J = 8.7 Hz), 8.03 (2H, d, J = 8.7 Hz).

Example 147

6'-ethoxy-6-methoxy-3-[4-(4-methoxyphenyl)piperazin-1-yl]-2,3'-bipyridine

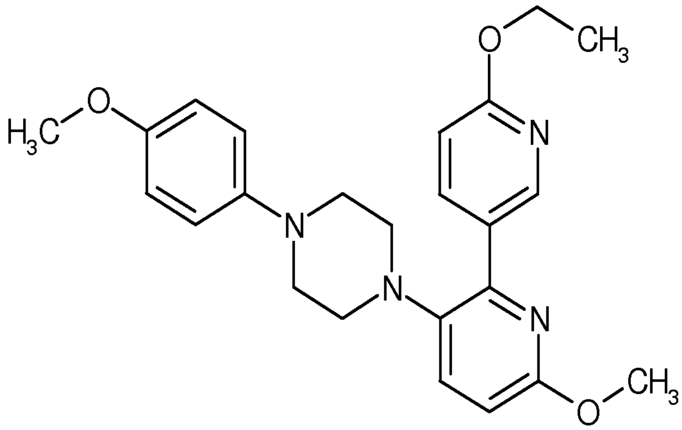

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (6-ethoxypyridin-3-yl)boronic acid (134 mg, 0.800 mmol). Yield: 80%. Melting point: 116-118°C (heptane-ethyl acetate).
$^1$H-NMR ($CDCl_3$) : δ 1.42 (3H, t, J = 7.2 Hz), 2.94-3.04 (4H, m), 3.08-3.20 (4H, m), 3.77 (3H, s), 3.95 (3H, s), 4.41 (2H, q, J = 7.0 Hz), 6.68 (1H, d, J = 8.7 Hz), 6.75 (1H, d, J = 8.7 Hz), 6.80-6.95 (4H, m), 7.48 (1H, d, J = 8.7 Hz), 8.44 (1H, dd, J = 8.7, 2.4 Hz), 8.97 (1H, d, J = 1.9 Hz).

Example 148

6,6'-diethoxy-3-[4-(4-methoxyphenyl)piperazin-1-yl]-2,3'-bipyridine

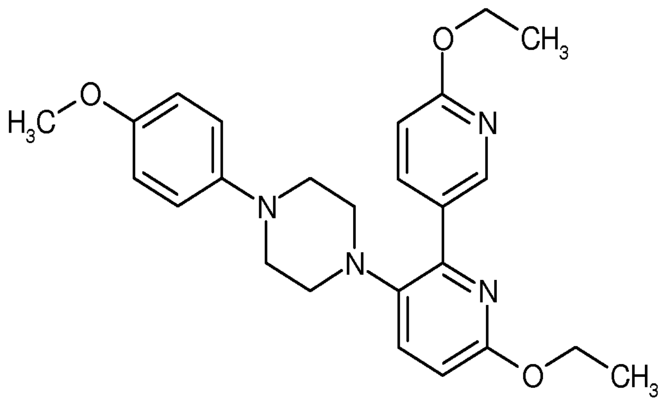

The title compound was obtained in the same way as in Example 48 using 1-(2-bromo-6-ethoxypyridin-3-yl)-4-(4-methoxyphenyl)piperazine (156 mg, 0.400 mmol) synthesized in Reference Example 64 and (6-ethoxypyridin-3-yl)boronic acid (134 mg, 0.800 mmol). Yield: 55%. Melting point: 93-95°C (heptane-ethyl acetate).
$^1$H-NMR ($CDCl_3$) : δ 1.34-1.47 (6H, m), 2.95-3.05 (4H, m), 3.08-3.17 (4H, m), 3.77 (3H, s), 4.32-4.46 (4H, m), 6.66 (1H, d, J = 8.7 Hz), 6.74 (1H, d, J = 8.7 Hz), 6.80-6.94 (4H, m), 7.47 (1H, d, J = 8.7 Hz), 8.42 (1H, dd, J = 8.7, 2.3 Hz), 8.95 (1H, d, J = 1.9 Hz).

Example 149

1-[2-(4-ethoxyphenyl)-6-methoxypyridin-3-yl]-4-(2-fluoro-4-methylphenyl)-1,4-diazepane

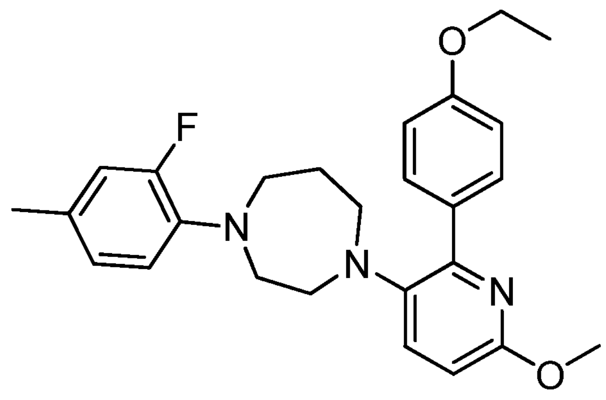

The title compound was obtained in the same way as in Example 83 using 1-[2-(4-ethoxyphenyl)-6-methoxypyridin-3-yl]-1,4-diazepane (200 mg, 0.61 mmol) synthesized in Reference Example 62 and 1-bromo-2-fluoro-4-methylbenzene (0.116 mL, 0.92 mmol). Yield: 13%. Melting point: 76-77°C (ethyl acetate-hexane).
$^1$H-NMR ($CDCl_3$) : δ 1.43 (3H, t, J = 7.0 Hz), 1.83-1.96 (2H, m), 2.25 (3H, s), 3.03-3.12 (2H, m), 3.13-3.21 (2H, m), 3.27-3.33 (2H, m), 3.36 (2H, t, J = 6.2 Hz), 3.93 (3H, s), 4.06 (2H, q, J = 6.9 Hz), 6.59 (1H, d, J = 8.3 Hz), 6.70-6.86 (3H, m), 6.85-6.95 (2H, m), 7.46 (1H, d, J = 8.7 Hz), 7.95-8.06 (2H, m).

Example 150

1-[2-(4-ethoxyphenyl)-6-methoxypyridin-3-yl]-4-(2-fluoro-4-methylphenyl)-5-methyl-1,4-diazepane

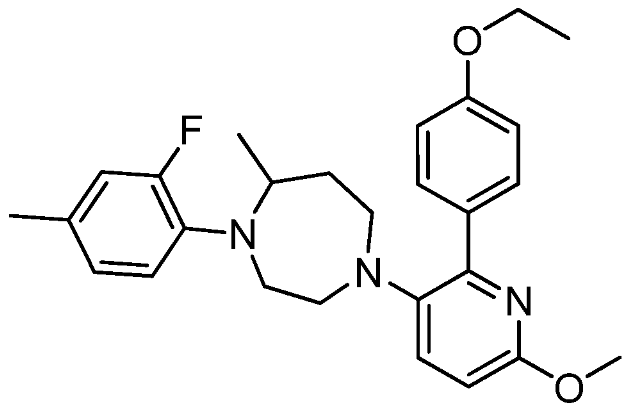

The title compound was obtained in the same way as in Example 99 using 1-[2-(4-ethoxyphenyl)-6-methoxypyridin-3-yl]-4-(2-fluoro-4-methylphenyl)-1,4-diazepan-5-one (254 mg, 0.57 mmol) synthesized in Example 146 and a 1 M solution of methyl magnesium bromide in THF (1.71 mL, 1.71 mmol). Yield: 16%, oil.
$^1$H-NMR ($CDCl_3$): δ 1.04 (3H, d, J = 6.0 Hz), 1.45 (3H, t, J = 7.0 Hz), 1.71-1.87 (1H, m), 1.98-2.15 (1H, m), 2.25 (3H, s), 2.95-3.02 (2H, m), 3.02-3.16 (2H, m), 3.32-3.41 (2H, m), 3.67-3.82 (1H, m), 3.94 (3H, s), 4.10 (2H, q, J = 7.2 Hz), 6.59 (1H, d, J = 8.7 Hz), 6.75-6.88 (3H, m), 6.89-6.98 (2H, m), 7.43 (1H, d, J = 8.7 Hz), 8.03-8.12 (2H, m).

Example 151

1-[2-(4-ethoxy-3-methylphenyl)-6-methoxypyridin-3-yl]-4-(4-methylphenyl)-1,4-diazepane

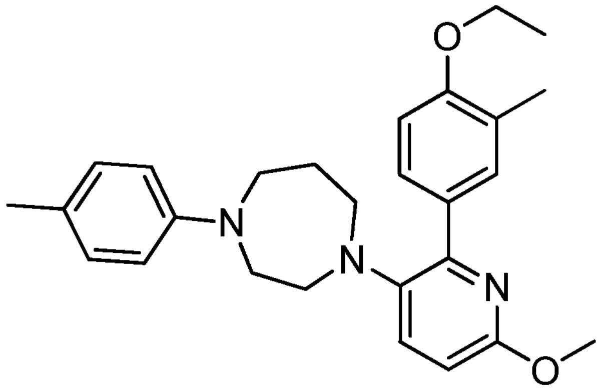

The title compound was obtained in the same way as in Example 83 using 1-[2-(4-ethoxy-3-methylphenyl)-6-methoxypyridin-3-yl]-1,4-diazepane (200 mg, 0.59 mmol) synthesized in Reference Example 68 and 1-bromo-4-methylbenzene (152 mg, 0.89 mmol). Yield: 20%, oil.
$^1$H-NMR ($CDCl_3$) : δ 1.45 (3H, t, J = 6.8 Hz), 1.76-1.89 (2H, m), 2.24 (3H, s), 2.25 (3H, s), 2.87-2.98 (2H, m), 3.04-3.14 (2H, m), 3.43-3.50 (2H, m), 3.52 (2H, t, J = 6.4 Hz), 3.93 (3H, s), 4.07 (2H, q, J = 6.8 Hz), 6.56 (1H, d, J = 8.7 Hz), 6.57-6.62 (2H, m), 6.78 (1H, d, J = 8.3 Hz), 7.01 (2H, d, J = 8.3 Hz), 7.35 (1H, d, J = 8.7 Hz), 7.76-7.82 (1H, m), 7.83 (1H, d, J = 1.5 Hz).

Example 152

1-[2-(4-ethoxy-3-methylphenyl)-6-methoxypyridin-3-yl]-4-(4-methoxyphenyl)-1,4-diazepan-5-one

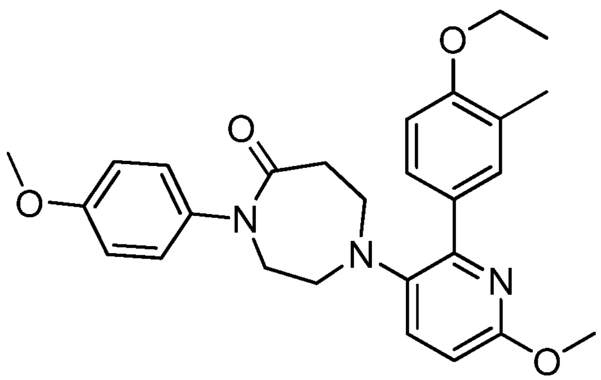

A mixture of 1-[2-(4-ethoxy-3-methylphenyl)-6-methoxypyridin-3-yl]-1,4-diazepan-5-one (500 mg, 1.41 mmol) synthesized in Reference Example 67, 1-iodo-4-methoxybenzene (496 mg, 2.12 mmol), copper iodide (53.3 mg, 0.28 mmol), potassium carbonate (585 mg, 4.23 mmol), 1,10-phenanthroline (50.5 mg, 0.28 mmol), and DMSO (2 mL) was stirred at 130°C for 20 hours in a nitrogen atmosphere. After cooling to room temperature, the reaction solution was diluted with ethyl acetate, washed with water and saturated brine, and then dried over anhydrous magnesium sulfate. The solvent was distilled off under reduced pressure, and the obtained residue was purified by silica gel column chromatography (hexane-ethyl acetate 90:10-50:50) and recrystallized from hexane-ethyl acetate to obtain 120 mg of the title compound (yield: 18%). Melting point: 136-138°C.
$^1$H-NMR ($CDCl_3$): δ 1.46 (3H, t, J = 7.0 Hz), 2.29 (3H, s), 2.81-2.90 (2H, m), 3.04-3.11 (2H, m), 3.11-3.20 (2H, m), 3.72-3.78 (2H, m), 3.79 (3H, s), 3.95 (3H, s), 4.09 (2H, q, J = 6.8 Hz), 6.63 (1H, d, J = 8.7 Hz), 6.82-6.91 (3H, m),

7.05-7.12 (2H, m), 7.40 (1H, d, J = 8.7 Hz), 7.83-7.92 (2H, m).

Example 153

4-[6-methoxy-3-(4-phenylpiperazin-1-yl)pyridin-2-yl]-N,N-dimethylaniline

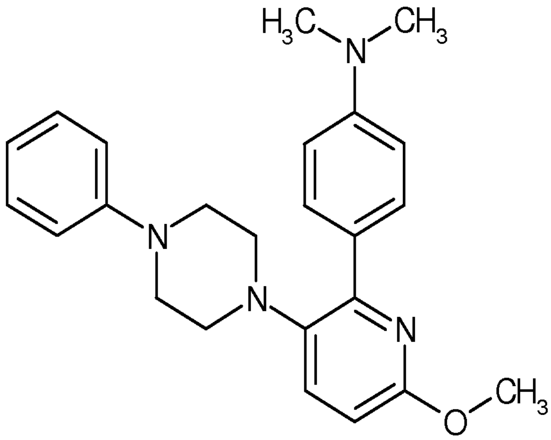

The title compound was obtained in the same way as in Reference Example 31 using 4-(6-methoxy-3-piperazin-1-ylpyridin-2-yl)-N,N-dimethylaniline (150 mg, 0.480 mmol) synthesized in Reference Example 5 and bromobenzene (151 mg, 0.960 mmol). Yield: 74%. Melting point: 145-147°C (heptane-ethyl acetate).
$^1$H-NMR ($CDCl_3$) : δ 2.94-3.06 (10H, m), 3.20-3.31 (4H, m), 3.96 (3H, s), 6.58 (1H, d, J = 8.7 Hz), 6.74 (2H, d, J = 9.1 Hz), 6.83-6.91 (1H, m), 6.91-7.00 (2H, m), 7.24-7.34 (2H, m), 7.39 (1H, d, J = 8.7 Hz), 8.18 (2H, d, J = 9.1 Hz) .

Example 154

2-{6-[4-(1-methylethyl)phenyl]-5-[4-(4-methylphenyl)piperazin-1-yl]pyridin-2-yl}propan-2-ol

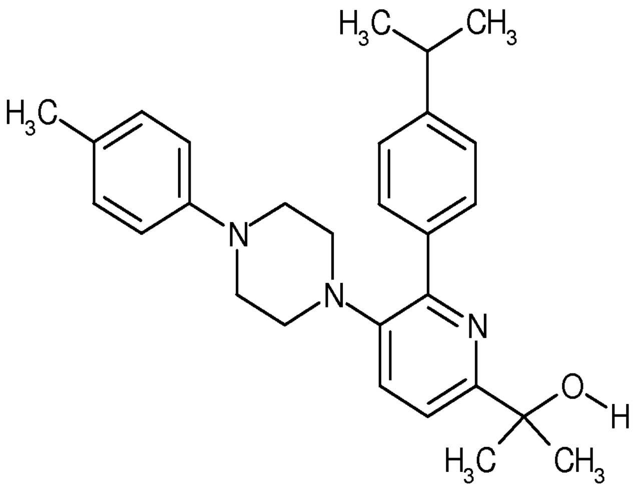

To a THF (10 mL) solution of 1-{6-bromo-2-[4-(1-methylethyl)phenyl]pyridin-3-yl}-4-(4-methylphenyl)piperazine (150 mg, 0.480 mmol) synthesized in Example 121, a 1.6 M solution of butyllithium in hexane (0.500 mL, 0.799 mmol) was added dropwise at -78°C, and the mixture was stirred at -78°C for 30 minutes. To this solution, acetone (193 mg, 3.33 mmol) was added at - 78°C, and the mixture was stirred at room temperature for 12 hours. To the reaction solution, water was added, followed by extraction with ethyl acetate. The organic phase was dried over anhydrous magnesium

sulfate, and the solvent was distilled off under reduced pressure. The obtained residue was purified by silica gel column chromatography (hexane-ethyl acetate 100:0-80:20) to obtain 23.4 mg of the title compound (yield: 8.2%). Melting point: 130-133°C (heptane-ethyl acetate).
$^1$H-NMR ($CDCl_3$): δ 1.28 (6H, d, J = 7.2 Hz), 1.55 (6H, s), 2.27 (3H, s), 2.88-3.09 (5H, m), 3.09-3.20 (4H, m), 5.40 (1H, s), 6.84 (2H, d, J = 8.3 Hz), 7.08 (2H, d, J = 8.3 Hz), 7.22 (1H, d, J = 8.3 Hz), 7.24-7.32 (2H, m), 7.41 (1H, d, J = 8.3 Hz), 7.99 (2H, d, J = 8.3 Hz).

Example 155

4-{3-[4-(3-fluoro-4-methoxyphenyl)piperazin-1-yl]-6-methoxypyridin-2-yl}-N,N-dimethylaniline

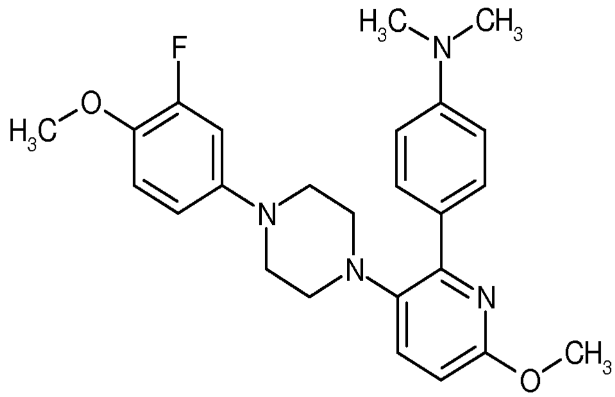

The title compound was obtained in the same way as in Reference Example 31 using 4-(6-methoxy-3-piperazin-1-ylpyridin-2-yl)-N,N-dimethylaniline (150 mg, 0.480 mmol) synthesized in Reference Example 5 and 4-bromo-2-fluoro-1-methoxybenzene (197 mg, 0.960 mmol). Yield: 75%. Melting point: 173-175°C (heptane-ethyl acetate).
$^1$H-NMR ($CDCl_3$): δ 2.95-3.04 (10H, m), 3.10-3.18 (4H, m), 3.85 (3H, s), 3.96 (3H, s), 6.54-6.66 (2H, m), 6.69-6.79 (3H, m), 6.85-6.94 (1H, m), 7.39 (1H, d, J = 8.7 Hz), 8.17 (2H, d, J = 9.1 Hz).

Example 156

1-[2-(4-ethoxyphenyl)-6-methoxypyridin-3-yl]-4-(4-methoxyphenyl)-5-methyl-1,4-diazepane

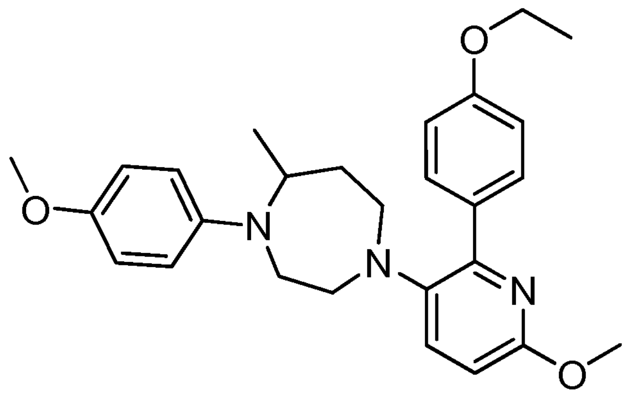

The title compound was obtained in the same way as in Example 99 using 1-[2-(4-ethoxyphenyl)-6-methoxypyridin-3-yl]-4-(4-methoxyphenyl)-1,4-diazepan-5-one (300 mg, 0.67 mmol) synthesized in Example 95 and a 1 M solution of methyl magnesium bromide in THF (2.01 mL, 2.01 mmol). Yield: 10%, amorphous solid.
$^1$H-NMR ($CDCl_3$) : δ 1.15 (3H, d, J = 6.4 Hz), 1.45 (3H, t, J = 7.0 Hz), 1.79-1.96 (1H, m), 2.00-2.15 (1H, m), 2.82 (1H, dd, J = 12.8, 9.4 Hz), 2.91-2.99 (2H, m), 3.04 (1H, dd, J = 12.8, 7.6 Hz), 3.34-3.55 (2H, m), 3.68-3.81 (4H, m), 3.93 (3H,

s), 4.11 (2H, q, J = 6.8 Hz), 6.53-6.64 (3H, m), 6.78-6.86 (2H, m), 6.91-7.00 (2H, m), 7.36 (1H, d, J = 8.7 Hz), 8.04-8.14 (2H, m).

Example 157

1-[2-(4-ethoxy-3-methylphenyl)-6-methoxypyridin-3-yl]-4-(4-methoxyphenyl)-5-methyl-1,4-diazepane

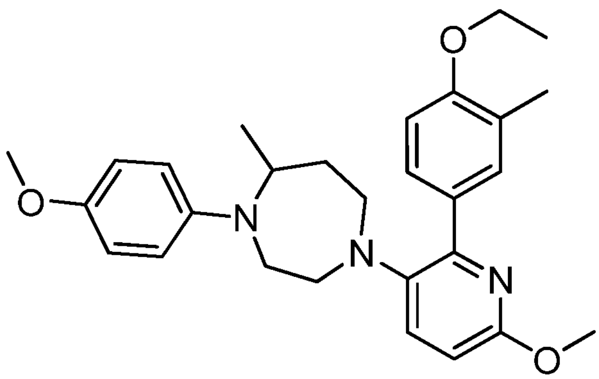

To a THF (3 mL) suspension of 1-[2-(4-ethoxy-3-methylphenyl)-6-methoxypyridin-3-yl]-4-(4-methoxyphenyl)-1,4-diazepan-5-one (300 mg, 0.65 mmol) synthesized in Example 152, a 1 M solution of methyl magnesium bromide in THF (1.95 mL, 1.95 mmol) was added dropwise, and the mixture was stirred at room temperature for 15 hours. To the reaction solution, sodium cyanotrihydroborate (163 mg, 2.60 mmol) and acetic acid (3 mL) were added, and the mixture was stirred at room temperature for 24 hours.
The reaction solution was neutralized by addition of an aqueous sodium hydroxide solution, followed by extraction with ethyl acetate. The extracts were mixed and dried over anhydrous magnesium sulfate. The solvent was distilled off under reduced pressure, and the obtained residue was fractionated by silica gel column chromatography (hexane-ethyl acetate 100:0-92:8). A fraction containing the compound of interest was concentrated under reduced pressure, and the obtained residue was separated by reverse-phase high-performance liquid chromatography (C18, mobile phase: water/acetonitrile (system containing 0.1% TFA)). To the obtained fraction, a saturated aqueous solution of sodium bicarbonate was added, followed by extraction with ethyl acetate. The organic phase was dried over anhydrous magnesium sulfate and then concentrated under reduced pressure to obtain 76 mg of the title compound (yield: 25%) as an amorphous solid.
$^1$H-NMR ($CDCl_3$): δ 1.15 (3H, d, J = 6.0 Hz), 1.46 (3H, t, J = 7.0 Hz), 1.78-1.97 (1H, m), 2.00-2.18 (1H, m), 2.30 (3H, s), 2.82 (1H, dd, J = 13.0, 9.3 Hz), 2.90-3.01 (2H, m), 3.06 (1H, dd, J = 12.8, 7.9 Hz), 3.33-3.57 (2H, m), 3.67-3.82 (4H, m), 3.94 (3H, s), 4.10 (2H, q, J = 6.8 Hz), 6.55 (1H, d, J = 8.7 Hz), 6.57-6.64 (2H, m), 6.78-6.84 (2H, m), 6.86 (1H, d, J = 8.7 Hz), 7.34 (1H, d, J = 8.3 Hz), 7.89-8.00 (2H, m).

Example 158

1-[2-(4-ethoxyphenyl)-6-methoxypyridin-3-yl]-4-(4-methoxyphenyl)-5,5-dimethyl-1,4-diazepane

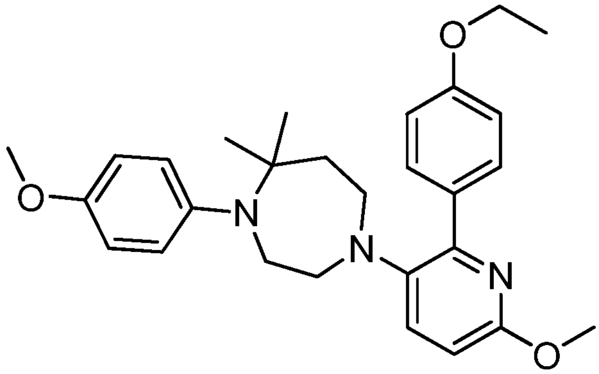

To a THF (2 mL) solution of 1-[2-(4-ethoxyphenyl)-6-methoxypyridin-3-yl]-4-(4-methoxyphenyl)-1,4-diazepan-5-one (200 mg, 0.45 mmol) synthesized in Example 95, titanium tetrachloride (0.0493 mL, 0.45 mmol) was added at -10°C. The reaction solution was stirred at -10°C for 30 minutes. Then, a 1 M solution of methyl magnesium bromide in THF (2.70 mL, 2.70 mmol) was carefully added dropwise thereto, and the mixture was stirred at room temperature for 16 hours. To the reaction solution, an 8 N aqueous sodium hydroxide solution was added, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 100:0-50: 50) to obtain 65 mg of the title compound (yield: 31%) as an amorphous solid.
$^1$H-NMR ($CDCl_3$): δ 0.99 (6H, s), 1.45 (3H, t, J = 7.0 Hz), 1.83-1.94 (2H, m), 2.86-2.96 (2H, m), 3.07-3.16 (2H, m), 3.19-3.28 (2H, m), 3.77 (3H, s), 3.94 (3H, s), 4.11 (2H, q, J = 7.2 Hz), 6.60 (1H, d, J = 8.7 Hz), 6.78 (2H, d, J = 9.1 Hz), 6.94 (2H, d, J = 9.1 Hz), 6.97-7.04 (2H, m), 7.48 (1H, d, J = 8.3 Hz), 8.11 (2H, d, J = 8.7 Hz).

Example 159

4-{6-methoxy-3-[4-(4-methoxyphenyl)piperazin-1-yl]-4-methylpyridin-2-yl}-N,N-dimethylaniline

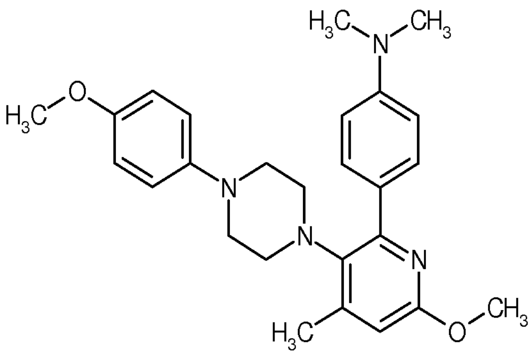

The title compound was obtained in the same way as in Example 48 using 1-(2-bromo-6-methoxy-4-methylpyridin-3-yl)-4-(4-methoxyphenyl)piperazine (211 mg, 0.539 mmol) synthesized in Reference Example 71 and [4-(dimethylamino) phenyl]boronic acid (178 mg, 1.08 mmol). Yield: 88%. Melting point: 185-187°C (heptane-ethyl acetate).
$^1$H-NMR ($CDCl_3$) : δ 2.36 (3H, s), 2.92-3.09 (14H, m), 3.76 (3H, s), 3.90 (3H, s), 6.50 (1H, s), 6.73-6.92 (6H, m), 7.49 (2H, d, J = 9.1 Hz).

Example 160

1-(2-furan-3-yl-6-methoxypyridin-3-yl)-4-(4-methoxyphenyl)piperazine

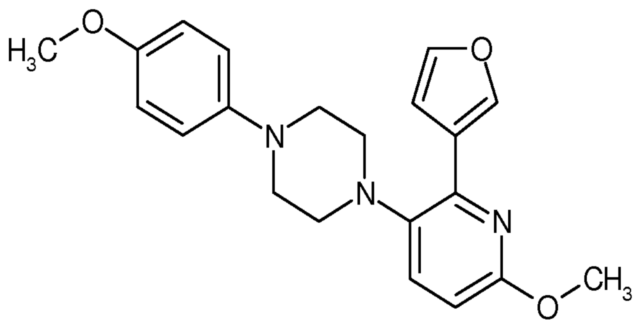

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and furan-3-ylboronic acid (89.5 mg, 0.800 mmol). Yield: 85%. Melting point: 153-155°C (heptane-ethyl acetate).
$^1$H-NMR ($CDCl_3$): δ 3.01-3.13 (4H, m), 3.20-3.31 (4H, m), 3.79 (3H, s), 3.97 (3H, s), 6.63 (1H, d, J = 8.7 Hz), 6.83-6.91 (2H, m), 6.93-7.00 (2H, m), 7.22-7.29 (1H, m), 7.43-7.47 (1H, m), 7.55 (1H, d, J = 8.7 Hz), 8.50-8.55 (1H, m).

Example 161

1-(4-methoxyphenyl)-4-(6-methoxy-2-thiophen-2-ylpyridin-3-yl)piperazine

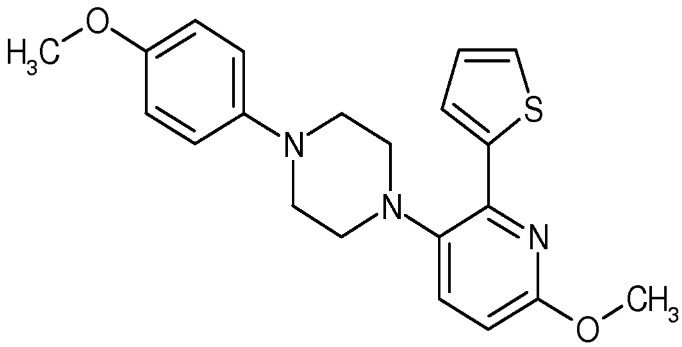

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and thiophen-2-ylboronic acid (102 mg, 0.800 mmol). Yield: 85%. Melting point: 160-162°C (heptane-ethyl acetate).
$^1$H-NMR ($CDCl_3$) : δ 3.04-3.15 (4H, m), 3.26-3.40 (4H, m), 3.79 (3H, s), 4.00 (3H, s), 6.64 (1H, d, J = 8.7 Hz), 6.84-6.92 (2H, m), 6.94-7.03 (2H, m), 7.06-7.14 (1H, m), 7.33-7.37 (1H, m), 7.60 (1H, d, J = 8.7 Hz), 8.18-8.26 (1H, m).

Example 162

4-{3-[4-(4-fluorophenyl)-3,3-dimethylpiperazin-1-yl]-6-methoxypyridin-2-yl}-N,N-dimethylaniline

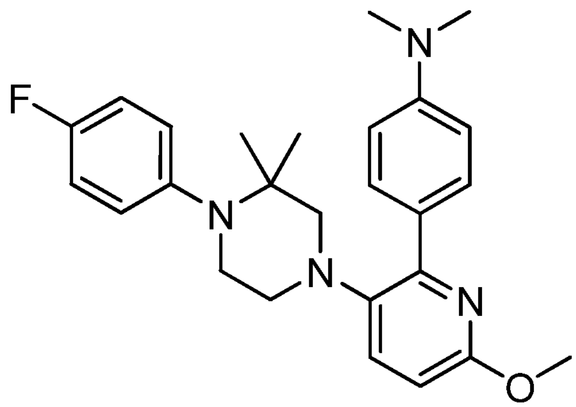

The title compound was obtained in the same way as in Example 158 using 4-{2-[4-(dimethylamino)phenyl]-6-methoxypyridin-3-yl}-1-(4-fluorophenyl)piperazin-2-one (60 mg, 0.14 mmol) synthesized in Example 83 and a 1 M solution of methyl magnesium bromide in THF (0.84 mL, 0.84 mmol). Yield: 6.6%, oil.
$^1$H-NMR ($CDCl_3$): δ 1.06 (6H, s), 2.79 (2H, s), 2.81-2.88 (2H, m), 3.03 (8H, s), 3.95 (3H, s), 6.59 (1H, d, J = 8.7 Hz), 6.80 (2H, d, J = 9.1 Hz), 6.89-7.00 (2H, m), 7.04-7.14 (2H, m), 7.43 (1H, d, J = 8.7 Hz), 8.08 (2H, d, J = 9.1 Hz).

Example 163

4-[2-(4-ethoxyphenyl)-6-methoxypyridin-3-yl]-1-(4-methoxyphenyl)-3-methyl-1,4-diazepane-2,5-dione

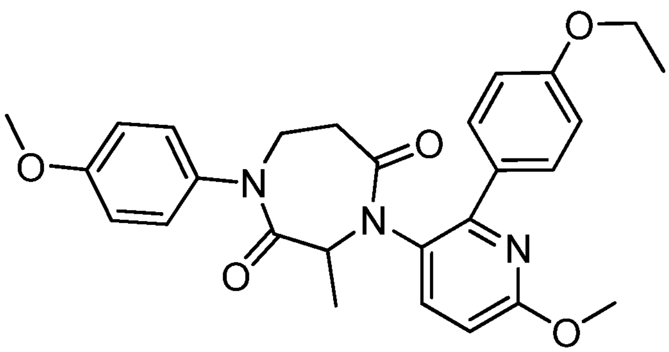

The title compound was obtained in the same way as in Example 78 using 3-[(2-bromopropanoyl)(4-methoxyphenyl)amino]-N-[2-(4-ethoxyphenyl)-6-methoxypyridin-3-yl]propanamide (350 mg, 0.63 mmol) synthesized in Reference Example 74. Yield: 50%. Melting point: 198-200°C (THF-hexane).
$^1$H-NMR ($CDCl_3$): δ 1.27 (3H, d, J = 6.8 Hz), 1.46 (3H, t, J = 7.0 Hz), 3.00-3.12 (1H, m), 3.15-3.30 (1H, m), 3.69 (1H, dt, J = 14.4, 6.1 Hz), 3.81 (3H, s), 3.97 (3H, s), 4.02-4.15 (3H, m), 4.52 (1H, q, J = 6.9 Hz), 6.72 (1H, d, J = 8.3 Hz), 6.87-7.06 (6H, m), 7.33-7.41 (1H, m), 7.52-7.63 (2H, m).

Example 164

1-[2-(4-ethoxyphenyl)-6-methoxypyridin-3-yl]-4-(4-methoxyphenyl)-2-methyl-1,4-diazepane

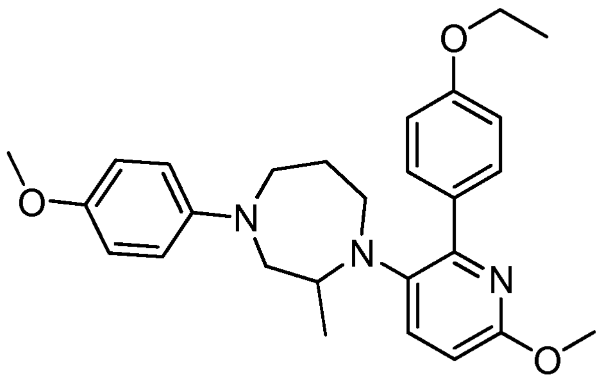

The title compound was obtained in the same way as in Example 80 using 4-[2-(4-ethoxyphenyl)-6-methoxypyridin-3-yl]-1-(4-methoxyphenyl)-3-methyl-1,4-diazepane-2,5-dione (195 mg, 0.41 mmol) synthesized in Example 163. Yield: 11%, amorphous solid.

$^1$H-NMR ($CDCl_3$) : δ 0.87 (3H, d, J = 6.4 Hz), 1.44 (3H, t, J = 7.0 Hz), 1.73-1.89 (1H, m), 1.89-2.08 (1H, m), 2.90-3.02 (1H, m), 3.02-3.14 (1H, m), 3.15-3.32 (2H, m), 3.38-3.55 (2H, m), 3.55-3.64 (1H, m), 3.77 (3H, s), 3.94 (3H, s), 4.07 (2H, q, J = 6.8 Hz), 6.54 (1H, d, J = 8.7 Hz), 6.60-6.70 (2H, m), 6.78-6.93 (4H, m), 7.30 (1H, d, J = 8.7 Hz), 7.97-8.12 (2H, m).

Example 165

1-[2-(3,5-dimethyl-1H-pyrazol-4-yl)-6-methoxypyridin-3-yl]-4-(4-methoxyphenyl)piperazine

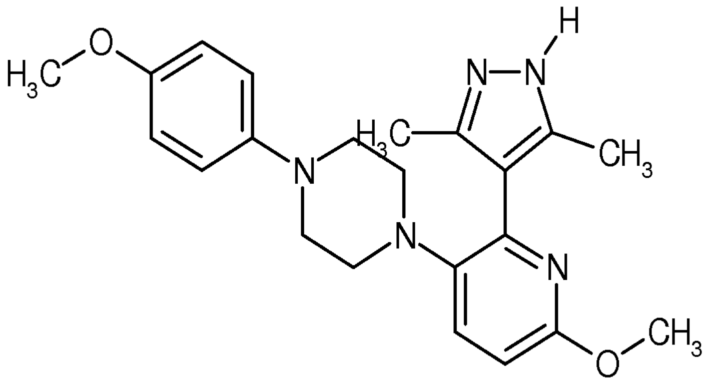

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and 3,5-dimethyl-4-(4,4,5,5-tetramethyl-1,3,2-dioxaborolan-2-yl)-1H-pyrazole (178 mg, 0.800 mmol). Yield: 18%. Melting point: 166-168°C (heptane-ethyl acetate).

$^1$H-NMR ($CDCl_3$) : δ 2.32 (6H, s), 2.86-2.97 (4H, m), 2.98-3.08 (4H, m), 3.77 (3H, s), 3.91 (3H, s), 6.68 (1H, d, J = 8.7 Hz), 6.79-6.93 (4H, m), 7.44 (1H, d, J = 8.7 Hz).

Example 166

1-[2-(5-chlorothiophen-2-yl)-6-methoxypyridin-3-yl]-4-(4-methoxyphenyl)piperazine

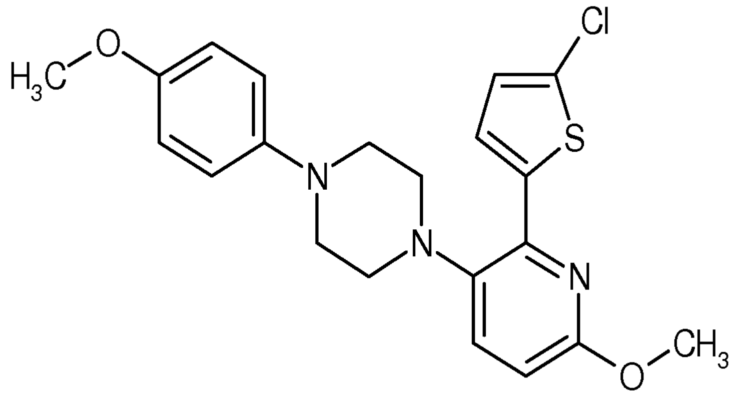

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and 1-[2-(5-chlorothiophen-2-yl)-6-methoxypyridin-3-yl]-4-(4-methoxyphenyl)piperazine (130 mg, 0.800 mmol). Yield: 12%. Melting point: 173-175°C (heptane-ethyl acetate).
$^1$H-NMR ($CDCl_3$) : δ 3.02-3.12 (4H, m), 3.29-3.42 (4H, m), 3.80 (3H, s), 3.98 (3H, s), 6.61-6.69 (1H, m), 6.83-6.95 (3H, m), 6.95-7.04 (2H, m), 7.61 (1H, d, J = 8.7 Hz), 7.95 (1H, d, J = 4.2 Hz).

Example 167

1-[6-methoxy-2-(5-methylthiophen-3-yl)pyridin-3-yl]-4-(4-methoxyphenyl)piperazine

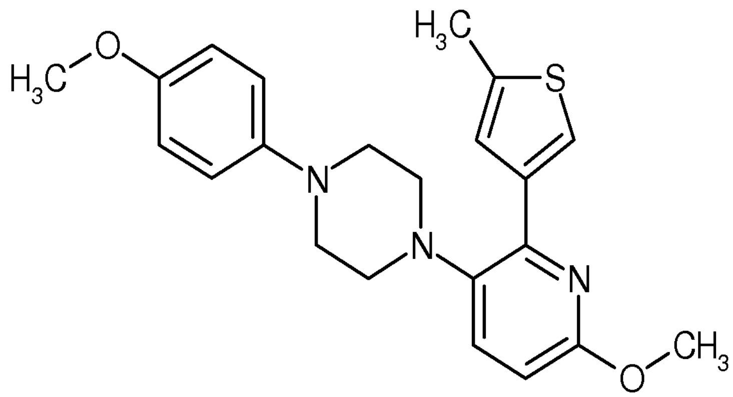

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and 4,4,5,5-tetramethyl-2-(5-methylthiophen-3-yl)-1,3,2-dioxaborolane (179 mg, 0.800 mmol). Yield: 65%. Melting point: 136-138°C (heptane-ethyl acetate).
$^1$H-NMR ($CDCl_3$) : δ 2.48-2.54 (3H, m), 3.01-3.10 (4H, m), 3.17-3.27 (4H, m), 3.79 (3H, s), 3.97 (3H, s), 6.63 (1H, d, J = 8.7 Hz), 6.83-6.91 (2H, m), 6.92-7.01 (2H, m), 7.49 (1H, d, J = 8.7 Hz), 7.67-7.71 (1H, m), 8.17-8.24 (1H, m) .

Example 168

6'-ethoxy-6-methoxy-3-[4-(4-methoxyphenyl)piperazin-l-yl]-4-methyl-2,3'-bipyridine

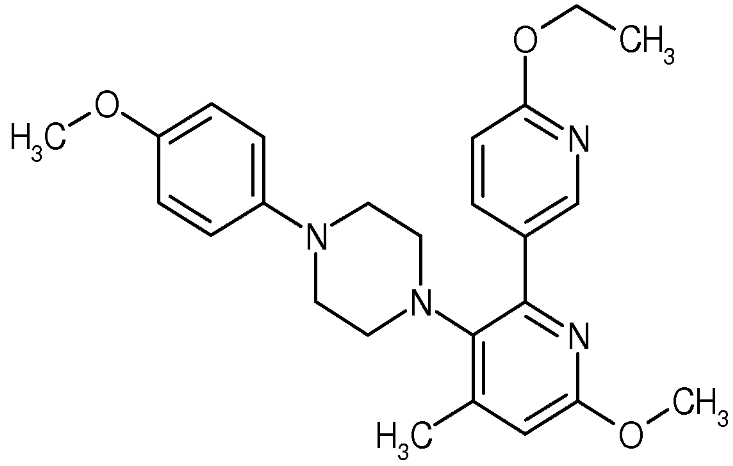

The title compound was obtained in the same way as in Example 48 using 1-(2-bromo-6-methoxy-4-methylpyridin-3-yl)-4-(4-methoxyphenyl)piperazine (140 mg, 0.357 mmol) synthesized in Reference Example 71 and (6-ethoxypyridin-3-yl)boronic acid (119 mg, 0.714 mmol). Yield: 72%. Melting point: 103-105°C (heptane-ethyl acetate).
$^1$H-NMR ($CDCl_3$): δ 1.42 (3H, t, J = 7.0 Hz), 2.39 (3H, s), 2.79-3.31 (8H, m), 3.77 (3H, s), 3.90 (3H, s), 4.40 (2H, q, J = 7.2 Hz), 6.54 (1H, s), 6.76 (1H, d, J = 9.0 Hz), 6.79-6.93 (4H, m), 7.85 (1H, dd, J = 8.5, 2.5 Hz), 8.41 (1H, d, J = 1.9 Hz).

Example 169

1-[6-methoxy-2-(1-methyl-1H-pyrazol-4-yl)pyridin-3-yl]-4-(4-methoxyphenyl)piperazine

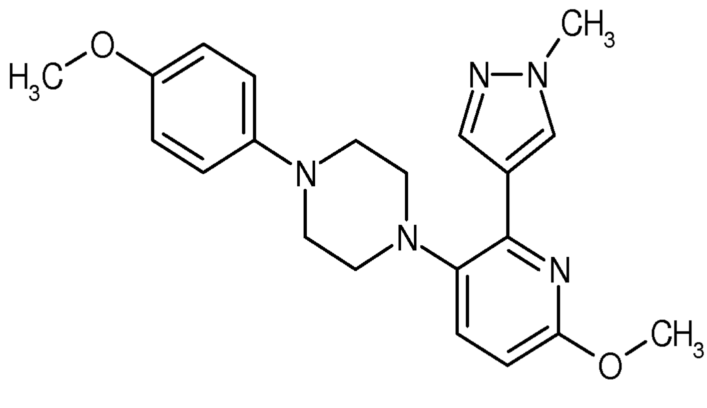

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and 1-methyl-4-(4,4,5,5-tetramethyl-1,3,2-dioxaborolan-2-yl)-1H-pyrazole (167 mg, 0.800 mmol). Yield: 59%. Melting point: 203-205°C (heptane-ethyl acetate).
$^1$H-NMR ($CDCl_3$) : δ 3.00-3.12 (4H, m), 3.19-3.32 (4H, m), 3.79 (3H, s), 3.95 (3H, s), 3.96 (3H, s), 6.59 (1H, d, J = 8.7 Hz), 6.84-6.91 (2H, m), 6.92-7.02 (2H, m), 7.53 (1H, d, J = 8.7 Hz), 8.26 (1H, s), 8.40 (1H, s).

Example 170

1-[2-(4-ethoxy-3-methylphenyl)-6-methoxypyridin-3-yl]-4-(4-fluorophenyl)-1,4-diazepane

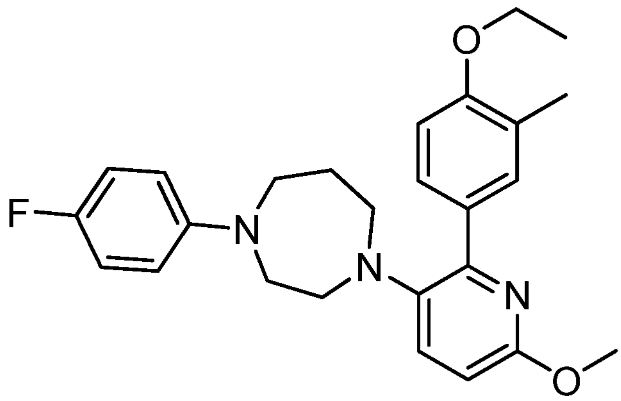

The title compound was obtained in the same way as in Example 83 using 1-[2-(4-ethoxy-3-methylphenyl)-6-methoxypyridin-3-yl]-1,4-diazepane (200 mg, 0.59 mmol) synthesized in Reference Example 68 and 1-bromo-4-fluorobenzene (0.0978 mL, 0.89 mmol). Yield: 43%, oil.

$^1$H-NMR ($CDCl_3$) : δ 1.45 (3H, t, J = 7.0 Hz), 1.75-1.89 (2H, m), 2.23 (3H, s), 2.90-3.01 (2H, m), 3.04-3.15 (2H, m), 3.39-3.46 (2H, m), 3.50 (2H, t, J = 6.2 Hz), 3.93 (3H, s), 4.06 (2H, q, J = 7.2 Hz), 6.51-6.64 (3H, m), 6.76 (1H, d, J = 8.7 Hz), 6.85-6.97 (2H, m), 7.35 (1H, d, J = 8.7 Hz), 7.71-7.85 (2H, m).

Example 171

5-{6-methoxy-3-[4-(4-methoxyphenyl)piperazin-1-yl]pyridin-2-yl}-1-methyl-1H-indazole

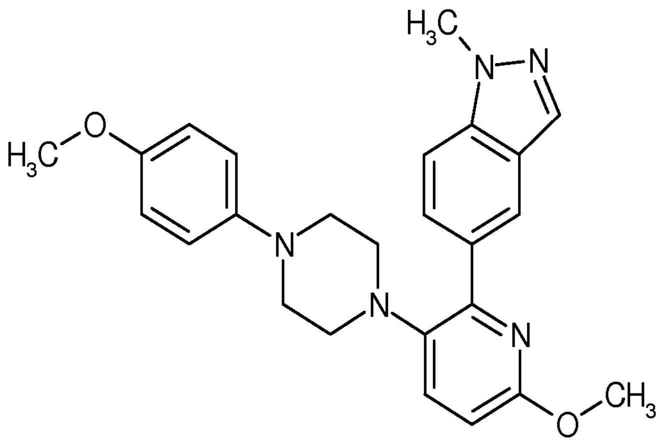

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (1-methyl-1H-indazol-5-yl)boronic acid (141 mg, 0.800 mmol). Yield: 85%. Melting point: 136-139°C (heptane-ethyl acetate).

$^1$H-NMR ($CDCl_3$): δ 2.92-3.13 (8H, m), 3.77 (3H, s), 3.98 (3H, s), 4.10 (3H, s), 6.69 (1H, d, J = 8.7 Hz), 6.79-6.94 (4H, m), 7.36-7.43 (1H, m), 7.47 (1H, d, J = 8.7 Hz), 7.97-8.05 (1H, m), 8.23-8.32 (1H, m), 8.46-8.58 (1H, m).

Example 172

6-{6-methoxy-3-[4-(4-methoxyphenyl)piperazin-1-yl]pyridin-2-yl}-1-methyl-1H-benzimidazole

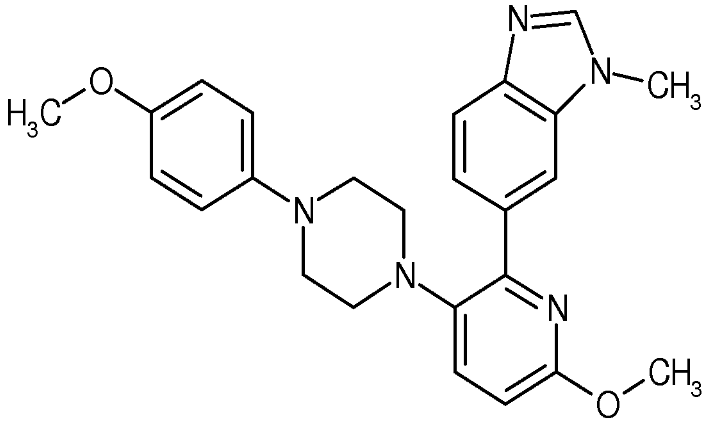

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (1-methyl-1H-benzimidazol-6-yl)boronic acid (141 mg, 0.800 mmol). Yield: 78%. Melting point: 170-174°C (heptane-ethyl acetate).
$^1$H-NMR ($CDCl_3$): δ 2.93-3.17 (8H, m), 3.77 (3H, s), 3.87 (3H, s), 3.99 (3H, s), 6.70 (1H, d, J = 8.7 Hz), 6.77-6.93 (4H, m), 7.48 (1H, d, J = 8.7 Hz), 7.74-7.98 (2H, m), 8.10-8.20 (1H, m), 8.21-8.36 (1H, m).

Example 173

N,N-diethyl-4-{6-methoxy-3-[4-(4-methoxyphenyl)piperazin-1-yl]pyridin-2-yl}aniline

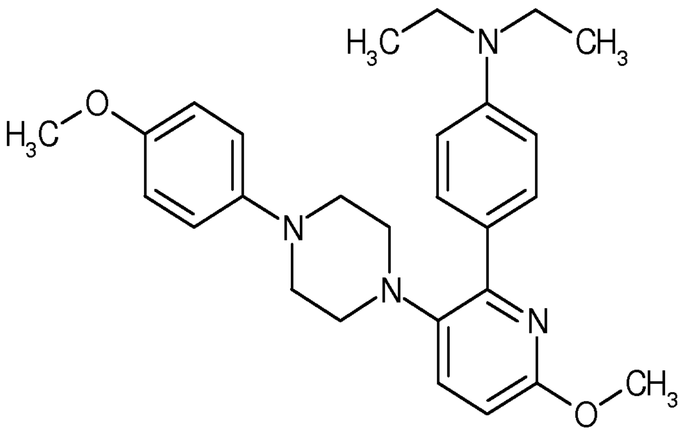

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and N,N-diethyl-4-(4,4,5,5-tetramethyl-1,3,2-dioxaborolan-2-yl)aniline (220 mg, 0.800 mmol). Yield: 84%. Melting point: 153-156°C (heptane-ethyl acetate).
$^1$H-NMR ($CDCl_3$) : δ 1.19 (6H, t, J = 7.2 Hz), 2.98-3.09 (4H, m), 3.10-3.23 (4H, m), 3.40 (4H, q, J = 6.9 Hz), 3.78 (3H, s), 3.96 (3H, s), 6.56 (1H, d, J = 8.7 Hz), 6.69 (2H, d, J = 9.4 Hz), 6.81-6.97 (4H, m), 7.39 (1H, d, J = 8.7 Hz), 8.14-8.26 (2H, m).

Example 174

4-(4-{6-methoxy-3-[4-(4-methoxyphenyl)piperazin-1-yl]pyridin-2-yl}phenyl)morpholine

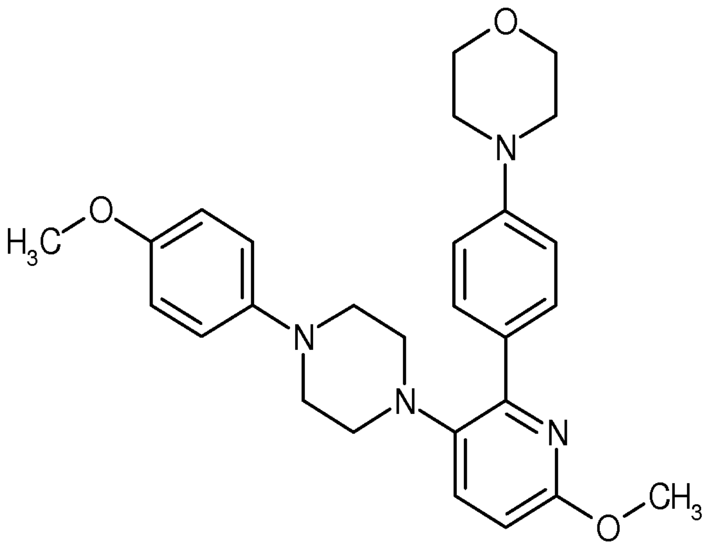

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and (4-morpholin-4-ylphenyl) boronic acid (166 mg, 0.800 mmol). Yield: 92%. Melting point: 193-196°C (heptane-ethyl acetate).
$^1$H-NMR ($CDCl_3$): δ 2.95-3.05 (4H, m), 3.07-3.17 (4H, m), 3.19-3.28 (4H, m), 3.78 (3H, s), 3.83-3.92 (4H, m), 3.95 (3H, s), 6.62 (1H, d, J = 8.3 Hz), 6.75-7.00 (6H, m), 7.42 (1H, d, J = 8.7 Hz), 8.17 (2H, d, J = 9.1 Hz).

Example 175

1-[2-(4-ethoxyphenyl)-6-methoxy-4-methylpyridin-3-yl]-4-(4-methoxyphenyl)piperazine

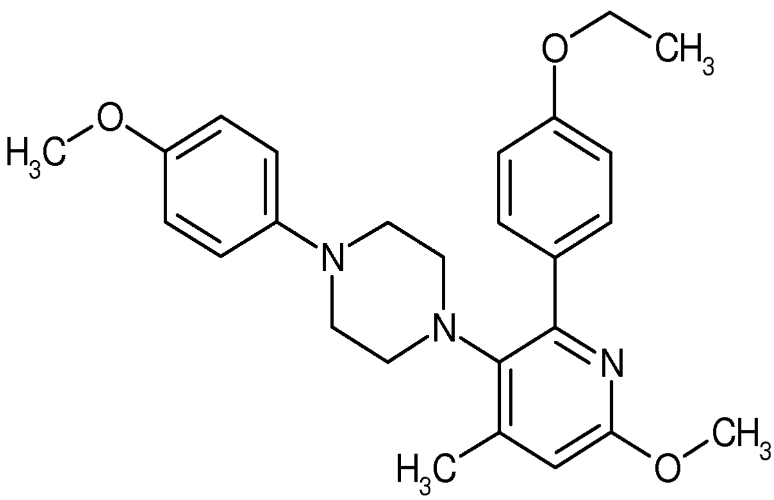

The title compound was obtained in the same way as in Example 48 using 1-(2-bromo-6-methoxy-4-methylpyridin-3-yl)-4-(4-methoxyphenyl)piperazine (229 mg, 0.584 mmol) synthesized in Reference Example 71 and (4-ethoxyphenyl) boronic acid (194 mg, 1.17 mmol). Yield: 89%. Melting point: 128-132°C (heptane-ethyl acetate).
$^1$H-NMR ($CDCl_3$) : δ 1.43 (3H, t, J = 7.0 Hz), 2.37 (3H, s), 2.82-3.10 (8H, m), 3.76 (3H, s), 3.89 (3H, s), 4.07 (2H, q, J = 7.2 Hz), 6.51-6.56 (1H, m), 6.77-6.90 (4H, m), 6.93 (2H, d, J = 8.7 Hz), 7.48 (2H, d, J = 9.1 Hz).

Example 176

1-[2-(4-ethoxyphenyl)-6-methoxypyridin-3-yl]-4-(4-methoxyphenyl)(5,5-D2)-1,4-diazepane

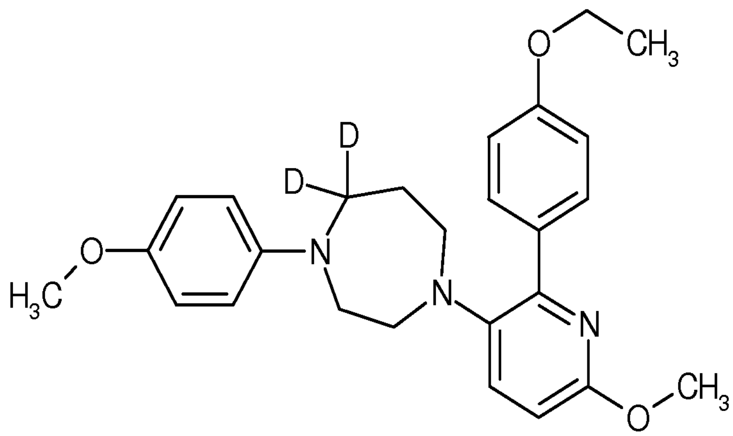

To a THF (15 mL) suspension of lithium aluminum deuteride (317 mg, 7.55 mmol), 1-[2-(4-ethoxyphenyl)-6-methoxypyridin-3-yl]-4-(4-methoxyphenyl)-1,4-diazepan-5-one (657 mg, 1.51 mmol) synthesized in Example 95 was added at 0°C, and the mixture was then refluxed for 12 hours. After cooling to room temperature, water was added to the reaction solution, followed by extraction with ethyl acetate. The extracts were mixed and dried over anhydrous magnesium sulfate. The solvent was distilled off under reduced pressure, and the obtained residue was purified by silica gel column chromatography (hexane-ethyl acetate 100:0-90:10) to obtain 543 mg of the title compound (yield: 83%). Melting point: 88-91°C (heptane-ethyl acetate).
$^1$H-NMR ($CDCl_3$) : δ 1.44 (3H, t, J = 7.0 Hz), 1.75-1.88 (2H, m), 2.88-3.01 (2H, m), 3.05-3.16 (2H, m), 3.40-3.53 (2H, m), 3.76 (3H, s), 3.93 (3H, s), 4.07 (2H, q, J = 6.8 Hz), 6.57 (1H, d, J = 8.3 Hz), 6.62 (2H, d, J = 9.4 Hz), 6.82 (2H, d, J = 9.1 Hz), 6.88 (2H, d, J = 8.7 Hz), 7.37 (1H, d, J = 8.7 Hz), 7.95 (2H, d, J = 9.1 Hz).

Example 177

1-(4-methoxyphenyl)-4-[6-methoxy-2-(4-pyrrolidin-1-ylphenyl)pyridin-3-yl]piperazine

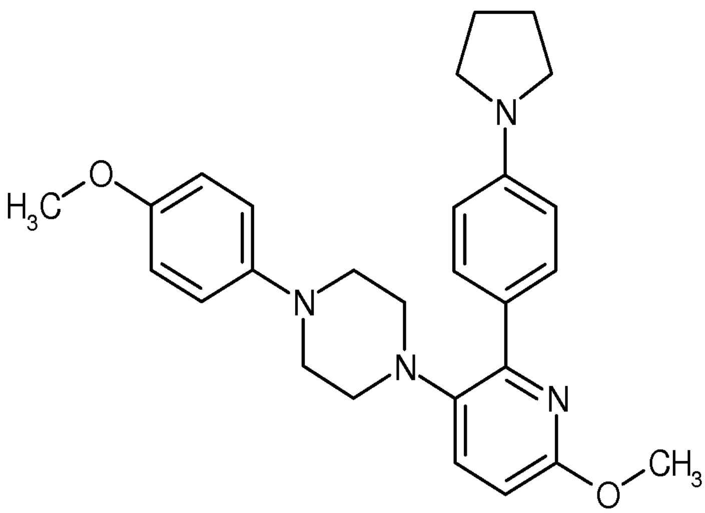

The title compound was obtained in the same way as in Example 48 using 1-(2-bromo-6-methoxypyridin-3-yl)-4-(4-methoxyphenyl)piperazine (150 mg, 0.400 mmol) synthesized in Reference Example 21 and 1-[4-(4,4,5,5-tetramethyl-1,3,2-dioxaborolan-2-yl)phenyl]pyrrolidine (219 mg, 0.800 mmol). Yield: 94%. Melting point: 178-181°C (heptane-ethyl acetate).
$^1$H-NMR ($CDCl_3$) : δ 1.95-2.09 (4H, m), 2.96-3.07 (4H, m), 3.10-3.20 (4H, m), 3.30-3.41 (4H, m), 3.78 (3H, s), 3.96 (3H, s), 6.51-6.66 (3H, m), 6.80-6.98 (4H, m), 7.39 (1H, d, J = 8.7 Hz), 8.19 (2H, d, J = 9.0 Hz).

Example 178

1-(4-bromophenyl)-4-[2-(4-ethoxyphenyl)-6-methoxypyridin-3-yl]-1,4-diazepane

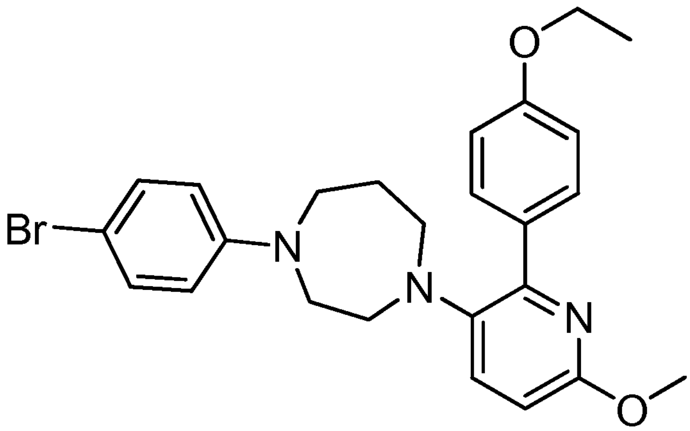

The title compound was obtained in the same way as in Example 83 using 1-[2-(4-ethoxyphenyl)-6-methoxypyridin-3-yl]-1,4-diazepane (1.00 g, 3.05 mmol) synthesized in Reference Example 62 and 1,4-dibromobenzene (1.08 g, 4.58 mmol). Yield: 19%.
$^1$H-NMR ($CDCl_3$) : δ 1.45 (3H, t, J = 7.0 Hz), 1.73-1.88 (2H, m), 2.89-2.99 (2H, m), 3.04-3.14 (2H, m), 3.40-3.47 (2H, m), 3.50 (2H, t, J = 6.0 Hz), 3.92 (3H, s), 4.06 (2H, q, J = 6.8 Hz), 6.48-6.55 (2H, m), 6.58 (1H, d, J = 8.7 Hz), 6.84 (2H, d, J = 9.1 Hz), 7.21-7.31 (2H, m), 7.36 (1H, d, J = 8.7 Hz), 7.86-7.97 (2H, m).

Example 179

1-[2-(4-ethoxyphenyl)-6-methoxypyridin-3-yl]-4-(6-methylpyridin-3-yl)-1,4-diazepane

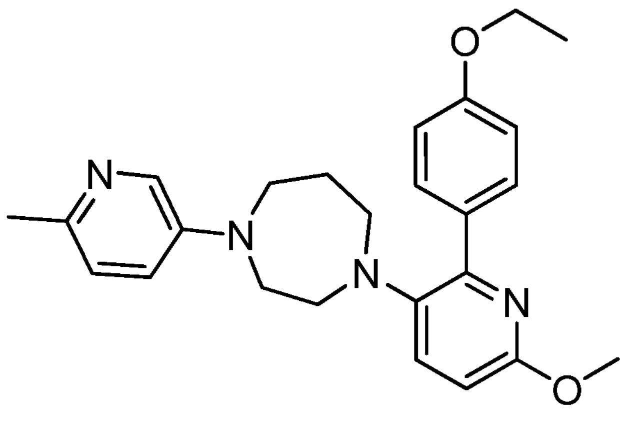

The title compound was obtained in the same way as in Example 83 using 1-[2-(4-ethoxyphenyl)-6-methoxypyridin-3-yl]-1,4-diazepane (200 mg, 0.61 mmol) synthesized in Reference Example 62 and 5-bromo-2-methylpyridine (158 mg, 0.92 mmol). Yield: 35%, oil.
$^1$H-NMR ($CDCl_3$) : δ 1.44 (3H, t, J = 7.0 Hz), 1.76-1.88 (2H, m), 2.43 (3H, s), 2.91-2.99 (2H, m), 3.06-3.14 (2H, m), 3.44-3.50 (2H, m), 3.53 (2H, t, J = 6.2 Hz), 3.92 (3H, s), 4.07 (2H, q, J = 6.8 Hz), 6.58 (1H, d, J = 8.7 Hz), 6.81-6.90 (3H,

m), 6.92-7.00 (1H, m), 7.36 (1H, d, J = 8.7 Hz), 7.85-7.95 (2H, m), 7.99 (1H, d, J = 3.0 Hz).

Experimental Example 1

Neurogenesis promoting effect in rat mixed glial culture

Experimental procedures:

The hippocampus and the cerebral cortex were excised from each of 3-day-old SD rats. A cell suspension was prepared therefrom using a neuron dispersion kit (Sumitomo Bakelite Co., Ltd., MB-X9901) and inoculated at concentration of $10^5$ cells/well to a 96-well plate coated with type I collagen (AGC Techno Glass Co., Ltd., 4860-010). The cells were cultured in a growth medium (D-MEM/F12 containing 10% FBS and PS) at 37°C for 4 days under 5% $CO_2$ conditions. After the culture, the medium was replaced by a differentiation medium (D-MEM/F12 containing PS), and rhIGF-1 (R&D Systems, 291-G1-250, final concentration: 100 ng/ml) and 1 μM compound were added thereto. The cells were further cultured for 3 days. The cultured cells were fixed in 4% paraformaldehyde-PBS (Muto Pure Chemicals Co., Ltd.), subjected to membrane permeation treatment with 0.1% Triton X-100 PBS, and blocked with Block Ace solution (DS Pharma Biomedical Co., Ltd., UK-B80). Anti-neuron-specific class III beta-tubulin Antibody (R&D Systems, MAB1195, clone Tuj-1) was diluted 1000-fold and used as a primary antibody. Anti-Mouse Ig, HRP-Linked F(ab')2 Fragment Sheep (GE Healthcare, NA9310) was diluted 10000-fold and used as a secondary antibody. A plate washer (BIO-TEK INSTRUMENTS ELX405) was used in washing operation. TMB Microwell Peroxidase Substrate System (Kirkegaard & Perry Laboratories, 50-76-00) was used in color reaction, which was performed for 10 minutes and stopped by addition of 1 M phosphoric acid. Absorbance at 450 nm was measured using a plate reader (Labsystems Multiskan BICHROMATIC). The rate of increase in the absorbance of the compound-supplemented group (compound + rhIGF-1) relative to that of a compound-unsupplemented group (control, only rhIGF-1) as 100% was determined in terms of % control. The neuronal differentiation promoting effect of each compound measured by this method is shown in Table 1.

[Table 1-1]

| Example No. | Ratio of absorbance of compound-supplemented group to control (%) |
|---|---|
| 2 | 448 |
| 3 | 303 |
| 5 | 765 |
| 6 | 134 |
| 7 | 1261 |
| 9 | 321 |
| 12 | 345 |
| 13 | 285 |
| 14 | 260 |
| 19 | 263 |
| 20 | 1410 |
| 24 | 462 |
| 25 | 276 |
| 26 | 259 |
| 34 | 783 |
| 49 | 887 |
| 50 | 546 |
| 51 | 856 |
| 52 | 731 |
| 55 | 316 |

(continued)

| Example No. | Ratio of absorbance of compound-supplemented group to control (%) |
|---|---|
| 60 | 218 |
| 62 | 439 |

[Table 1-2]

| | |
|---|---|
| 75 | 249 |
| 82 | 1165 |
| 85 | 385 |
| 87 | 224 |
| 88 | 1048 |
| 89 | 379 |
| 92 | 449 |
| 98 | 397 |
| 108 | 773 |
| 113 | 717 |
| 118 | 528 |
| 119 | 760 |
| 134 | 630 |
| 144 | 1398 |
| 154 | 1187 |
| 157 | 1532 |
| 159 | 611 |
| 160 | 220 |
| 162 | 1090 |
| 168 | 584 |
| 169 | 255 |
| 171 | 312 |
| 174 | 390 |
| 179 | 1346 |

Experimental Example 2

Akt protein degradation inhibitory effect in rat mixed glial culture

Experimental procedures:

The mixed glial cells used in Experimental Example 1 were inoculated at a concentration of 4 X $10^6$ cells/well to a 6-well plate coated with type I collagen (AGC Techno Glass Co., Ltd.). The cells were cultured in a growth medium (D-MEM/F12 containing 10% FBS and PS) at 37°C for 4 days under 5% $CO_2$ conditions. Then, the medium was replaced by a serum-free medium (D-MEM/F12 containing PS), and the cells were cultured at 37°C for 4 days under 5% $CO_2$ conditions to remove serum.
Next, rhIGF-1 (R&D Systems, 291-G1-250, final concentration: 100 ng/ml) and 1 $\mu$M compound were added thereto and reacted for 10 minutes in a temperature-controlled water bath set to 37°C. The culture supernatant was then

aspirated, and the reaction was stopped by addition of 150 μL of RIPA buffer (50 mM Tris-HCl pH 7.5, 5 mM EDTA, 100 mM NaCl, 30 mM NaF, 5 mM sodium diphosphate, 137 mg/l pepstatin A, 2.5 KIU/l aprotinin, 1% NP-40, 6 mM sodium deoxycholate, 1 μM microcystin-LR, 1 μM Z-Leu-Leu-Nva-H (aldehyde), 48 μM leupeptin, 96 μM 4-(2-aminoethyl) benzenesulfonyl fluoride-HCl, 1 mM sodium orthovanadate). After the stop of the reaction, the cell lysate was collected using a cell scraper on ice. Finally, the cell lysate was centrifuged at 15000 rpm for 30 minutes, and the supernatant was used as cell extracts.

Proteins were collected from the cell extracts using trichloroacetic acid and quantified according to the Lowry method. A calibration curve was prepared using bovine serum albumin. The cell extracts were diluted with RIPA so that the protein levels were constant among the test solutions on the basis of the measured values (10 μg/lane). SDS-PAGE was performed under reductive conditions on 10% acrylamide (45 mA, 1.5 hr). Next, the proteins were transferred to a PVDF membrane (0.13 A, 1 hr) and reacted with antibodies. Akt (Cell Signaling, 9272) or ERK (Santa Cruz, sc-94) was diluted at a ratio of 1000-fold and used as a primary antibody. An HRP-labeled anti-rabbit antibody (GE Healthcare, NA9340V) was diluted at a ratio of 12500-fold and used as a secondary antibody. Bands were detected using ImmunoStar reagent (Wako Pure Chemical Industries, Ltd., 291-55203) and an X-ray film. The results were quantified using GS-800 Calibrated Densitometer manufactured by Bio-Rad Laboratories, Inc., and band intensity was digitized by multiplying the absorbance of the Akt- or ERK-1&2-derived band by an area. The value of Akt was corrected with that of ERK for the digitization.

The Akt degradation inhibitory effect of each compound was determined as the rate of inhibition indicated in the numerical value of the compound + rhIGF-1-supplemented group relative to that of a group supplemented with neither compound nor rhIGF-1 (unsupplemented) as 100% and that of a group supplemented with only rhIGF-1 as 0%.

$$\text{Rate of inhibition (\%)} = [(\text{compound} + \text{rhIGF-1}) - (\text{rhIGF-1})] \div [(\text{unsupplemented}) - (\text{rhIGF-1})] \times 100.$$

The Akt protein degradation inhibitory effect of each compound measured by this method is shown in Table 3.

[Table 2]

| Example No. | Akt degradation inhibitory rate (%) |
|---|---|
| **19** | **97** |
| **25** | **140** |

Experimental Example 3

Cognitive function improving effect in novel object recognition test

Experimental procedures:

Eight- to 9-month-old female Tg2576 mice were used, and wild-type littermates were used as control mice. All the mice were raised in a room having a temperature of 23 ± 3°C and 12-hour light/dark cycles (light phase: 7 a.m. to 7 p.m.) and allowed to freely take food (Oriental Yeast Co., Ltd.) and water.

The body weights and blood glucose levels of the Tg2576 mice were measured, and these mice were divided (n = 11-15) so that there was no difference among groups. 10 mg/kg/day compound or vehicle (0.5% methylcellulose solution, Wako Pure Chemical Industries, Ltd.) was orally administered to each mouse for 3 consecutive weeks. The following novel object recognition test was conducted (acquisition trial and retention trial were carried under blind): 4 or 5 mice in one cage were placed in a box of 30 x 30 x 30 cm and acclimatized for 30 minutes in the absence of objects. On the next day, two identical objects were placed in diagonally opposite corners of the box. The mice were placed in the box, and the time spent exploring the objects for 5 minutes was recorded (acquisition trial). 24 hours later, one of the objects was in turn replaced by a novel one, and the same test as the acquisition trial was conducted (retention trial).

During the test, places were appropriately changed between the two objects to minimize environmental/behavioral psychological factors. The ratio of the time spent exploring the novel object to the whole exploring time was calculated as an index showing cognitive functions and used as preference index (PI). Furthermore, a recovery ratio was calculated according to the following formula:

[Recovery ratio] = [[PI of Tg2576 mice treated by each compound] - [PI of Tg2576 mice treated by vehicle]] / [[PI of wild type mice] - [PI of Tg2576 mice treated by vehicle]] × 100 (%).

In this context, individuals that explored neither of the objects in the acquisition trial and the retention trial were excluded from this test. The results are shown in Table 4.

[Table 3]

| Example No. | Preference index (% recovery) |
|---|---|
| 12 | 70.3 |
| 19 | 118.2 |
| 25 | 97.0 |
| 55 | 19.8 |

Preparation Example:

| | | |
|---|---|---|
| (1) | the compound described in Example 1 | 50 mg |
| (2) | lactose | 34 mg of |
| (3) | corn starch | 10.6 mg |
| (4) | corn starch paste | 5 mg |
| (5) | magnesium stearate | 0.4 mg |
| (6) | carboxymethylcellulose calcium | 20 mg |
| | Total: | 120 mg |

According to a standard method, these ingredients (1) to (6) can be mixed and compressed in a tableting machine to obtain a tablet.

Industrial Applicability

A compound of the present invention or a salt thereof exhibits an excellent neurogenesis promoting effect and neurogenesis promoting effect and is useful as, for example, an IGF-1 signal modulator, a protein kinase B activator, or an agent for the prevention or treatment of a central nervous system disease (e.g., Alzheimer's disease).

**Claims**

1. A compound represented by the formula (I):

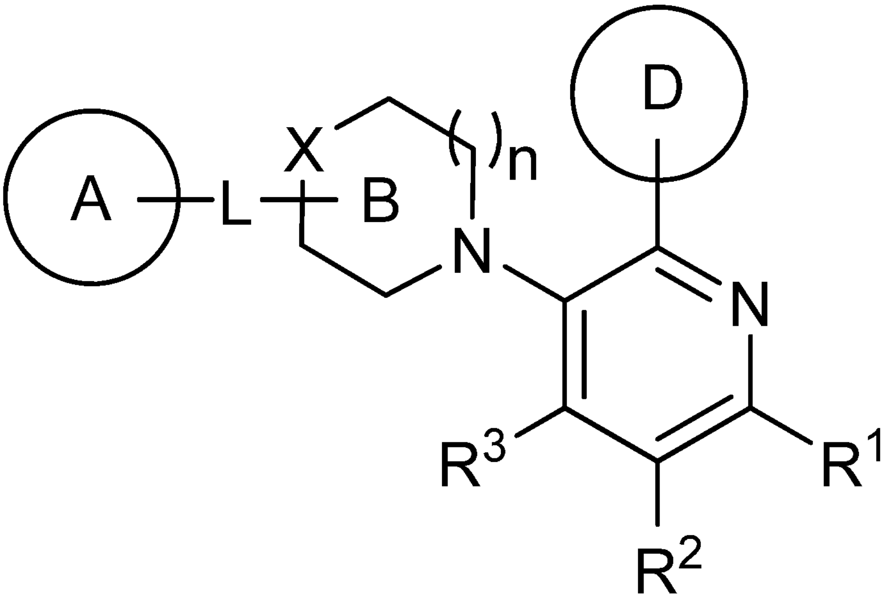

(I)

wherein

ring A represents a monocyclic aromatic hydrocarbon ring or a monocyclic aromatic heterocyclic ring, each of which may have a substituent(s);
ring B may further have a substituent(s);
ring D represents a monocyclic aromatic hydrocarbon ring or a monocyclic aromatic heterocyclic ring, each of which may have a substituent(s);
L represents a bond, or a methylene group or an ethylene group, each of which may have a substituent(s);
X represents -O-, -$NR^a$-, or a methylene group which may have a substituent(s), wherein $R^a$ represents a hydrogen atom or a substituent;
n represents 1 or 2; and
$R^1$ to $R^3$ are the same or different and each represent a hydrogen atom, a halogen atom, a cyano group, an acyl group, a $C_{1-6}$ alkyl group which may have a substituent(s), a $C_{1-6}$ alkoxy group which may have a substituent (s), a $C_{3-6}$ cycloalkyl group which may have a substituent(s), a $C_{3-6}$ cycloalkoxy group which may have a substituent(s), or an amino group which may have a substituent(s), wherein
ring A and ring D each may be condensed with another ring to form a condensed ring which may have a substituent(s), and ring A-L- in the formula (I) may be bonded to X,
provided that
when ring A is pyrazole which may be substituted and ring D is benzene which may be substituted, L is a bond;
or a salt thereof.

**2.** The compound according to claim 1 or a salt thereof, wherein in the formula (I), the following substructural formula:

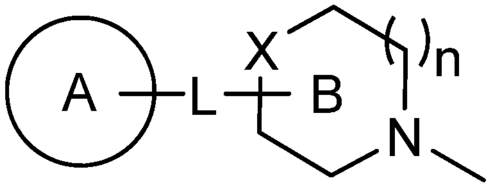

is

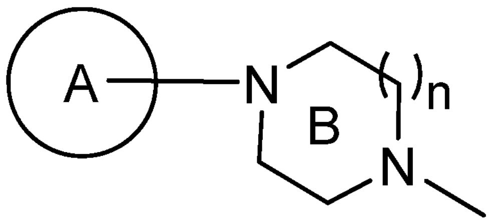

3. The compound according to claim 2 or a salt thereof, wherein in the formula,
ring A is benzene having a substituent(s) or pyridine having a substituent(s);
ring B may further have a substituent(s); and
n is 1 or 2.

4. The compound according to claim 1 or a salt thereof, wherein in the formula,
ring A is benzene having a substituent(s) or pyridine having a substituent(s);
ring B may further have a substituent(s);
ring D is benzene having a substituent(s), pyrazole having a substituent(s), furan, pyridine having a substituent(s), or indazole having a substituent(s);
L is a bond or methylene;
X is

(1) -O-,
(2) -$NR^a$-, or
(3) methylene having a substituent(s), wherein

$R^a$ is a hydrogen atom or a substituent;
n is 1 or 2;
$R^1$ is a $C_{1-6}$ alkoxy group or a $C_{1-6}$ alkyl group having a substituent(s);
$R^2$ is a hydrogen atom; and
$R^3$ is a hydrogen atom or a $C_{1-6}$ alkyl group, wherein
the substituent on ring D may be further bonded to ring D to form dihydrobenzofuran which may have a substituent (s), and ring A-L- in the formula (I) may be bonded to X.

5. The compound according to claim 1 or a salt thereof, wherein in the formula,
ring A is benzene having a substituent(s) selected from a halogen atom, $C_{1-6}$ alkyl which may be halogenated, and $C_{1-6}$ alkoxy, or pyridine substituted by $C_{1-6}$ alkyl;
ring B may further have a substituent(s) selected from oxo and $C_{1-6}$ alkyl;
ring D is benzene having a substituent(s) selected from a halogen atom, $C_{1-6}$ alkyl, $C_{1-6}$ alkoxy which may be halogenated, morpholinyl, and di-$C_{1-6}$ alkylamino, pyrazole substituted by $C_{1-6}$ alkyl, furan, pyridine substituted by $C_{1-6}$ alkoxy, or indazole substituted by $C_{1-6}$ alkoxy;
L is a bond or methylene;
X is

(1) -O-,
(2) -$NR^a$-, or
(3) methylene bonded to ring A-L- in the formula (I) and further substituted by hydroxy, wherein

$R^a$ is a hydrogen atom, $C_{1-6}$ alkyl, $C_{1-6}$ alkoxycarbonyl, or ring A-L- in the formula (I);
n is 1 or 2;
$R^1$ is a $C_{1-6}$ alkoxy group or $C_{1-6}$ alkyl having hydroxy;
$R^2$ is a hydrogen atom; and
$R^3$ is a hydrogen atom or $C_{1-6}$ alkyl, wherein
the substituent on ring D may be combined with ring D to form dihydrobenzofuran substituted by $C_{1-6}$ alkyl.

6. The compound according to claim 5 or a salt thereof, wherein in the formula,
ring A is benzene having a substituent(s) selected from a halogen atom, $C_{1-6}$ alkyl which may be halogenated, and $C_{1-6}$ alkoxy; and

ring D is benzene having a substituent(s) selected from a halogen atom, $C_{1-6}$ alkyl, $C_{1-6}$ alkoxy which may be halogenated, morpholinyl, and di-$C_{1-6}$ alkylamino.

**7.** The compound according to claim 1 or a salt thereof, wherein in the formula,
ring A is benzene having a substituent(s) selected from a halogen atom, $C_{1-6}$ alkyl which may be halogenated, and $C_{1-6}$ alkoxy;
ring B may be further substituted by $C_{1-6}$ alkyl;
ring D is benzene having a substituent(s) selected from $C_{1-6}$ alkoxy and di-$C_{1-6}$ alkylamino;
L is a bond;
X is -O- or -NR$^a$-, wherein
R$^a$ is ring A-L- in the formula (I);
n is 1 or 2;
$R^1$ is a $C_{1-6}$ alkoxy group; and
both of $R^2$ and $R^3$ are a hydrogen atom.

**8.** 4-{3-[4-(4-fluorophenyl)piperazin-1-yl]-6-methoxypyridin-2-yl}-N,N-dimethylaniline or a salt thereof.

**9.** 4-{6-methoxy-3-[4-(4-methoxyphenyl)piperazin-1-yl]pyridin-2-yl}-N,N-dimethylaniline or a salt thereof.

**10.** 4-{6-methoxy-3-[4-(4-methylphenyl)piperazin-1-yl]pyridin-2-yl}-N,N-dimethylaniline or a salt thereof.

**11.** 1-[2-(4-ethoxyphenyl)-6-methoxypyridin-3-yl]-4-(4-methoxyphenyl)piperazine or a salt thereof.

**12.** A medicament comprising a compound according to claim 1 or a salt thereof.

**13.** The medicament according to claim 12, wherein the medicament is a neuronal differentiation promoter, an IGF-1 signal modulator, or a protein kinase B activator.

**14.** A therapeutic or prophylactic agent for Alzheimer's disease, comprising a compound represented by the formula (Io) :

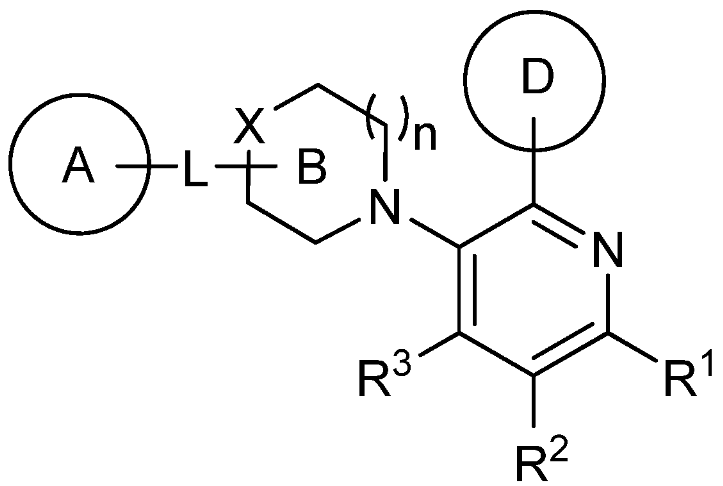

$(I_0)$

wherein

ring A represents a monocyclic aromatic hydrocarbon ring or a monocyclic aromatic heterocyclic ring, each of which may have a substituent(s);
ring B may further have a substituent(s);
ring D represents a monocyclic aromatic hydrocarbon ring or a monocyclic aromatic heterocyclic ring, each of which may have a substituent(s);
L represents a bond, or a methylene group or an ethylene group, each of which may have a substituent(s);
X represents -O-, -NR$^a$-, or a methylene group which may have a substituent(s), wherein
R$^a$ represents a hydrogen atom or a substituent;
n represents 1 or 2; and

$R^1$ to $R^3$ are the same or different and each represent a hydrogen atom, a halogen atom, a cyano group, an acyl group, a $C_{1-6}$ alkyl group which may have a substituent(s), a $C_{1-6}$ alkoxy group which may have a substituent (s), a $C_{3-6}$ cycloalkyl group which may have a substituent(s), a $C_{3-6}$ cycloalkoxy group which may have a substituent(s), or an amino group which may have a substituent(s), wherein
ring A and ring D each may be condensed with another ring to form a condensed ring which may have a substituent(s), and ring A-L- in the formula ($I_0$) may be bonded to X; or

a salt thereof.

**15.** A method for promoting neuronal differentiation, modulating an IGF-1 signal, or activating protein kinase B, comprising administering to a mammal an effective amount of a medicament according to claim 12.

**16.** A method for preventing or treating Alzheimer's disease, comprising administering to a mammal an effective amount of a compound represented by the formula ($I_0$) :

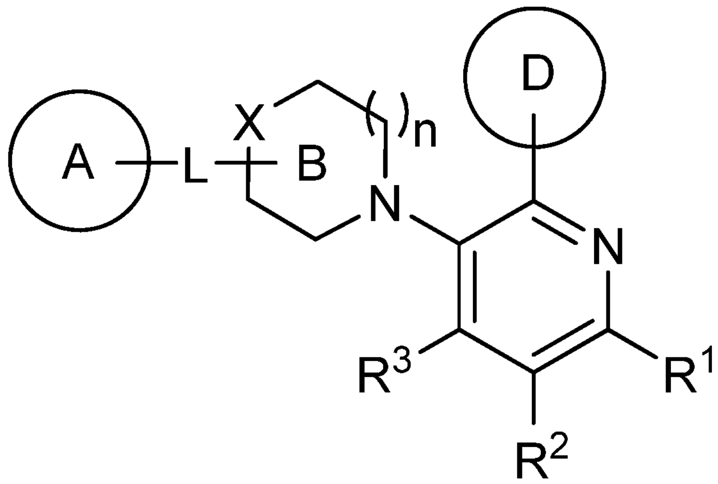

($I_0$)

wherein

ring A represents a monocyclic aromatic hydrocarbon ring or a monocyclic aromatic heterocyclic ring, each of which may have a substituent(s);
ring B may further have a substituent(s);
ring D represents a monocyclic aromatic hydrocarbon ring or a monocyclic aromatic heterocyclic ring, each of which may have a substituent(s);
L represents a bond, or a methylene group or an ethylene group, each of which may have a substituent(s);
X represents -O-, -$NR^a$-, or a methylene group which may have a substituent(s), wherein
$R^a$ represents a hydrogen atom or a substituent;
n represents 1 or 2; and
$R^1$ to $R^3$ are the same or different and each represent a hydrogen atom, a halogen atom, a cyano group, an acyl group, a $C_{1-6}$ alkyl group which may have a substituent(s), a $C_{1-6}$ alkoxy group which may have a substituent (s), a $C_{3-6}$ cycloalkyl group which may have a substituent(s), a $C_{3-6}$ cycloalkoxy group which may have a substituent(s), or an amino group which may have a substituent(s), wherein
ring A and ring D each may be condensed with another ring to form a condensed ring which may have a substituent(s), and ring A-L- in the formula ($I_0$) may be bonded to X; or

a salt thereof.

**17.** Use of a compound according to claim 1 or a salt thereof for manufacturing a neuronal differentiation promoter, an IGF-1 signal modulator, or a protein kinase B activator.

**18.** Use of a compound represented by the formula ($I_0$):

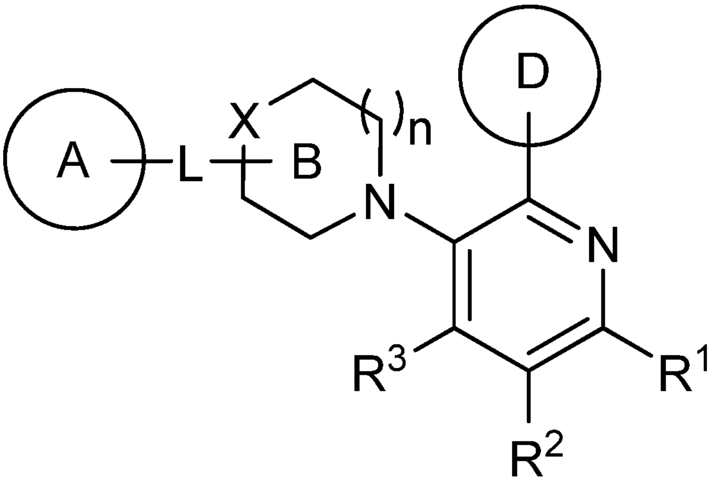

$(I_0)$

wherein

ring A represents a monocyclic aromatic hydrocarbon ring or a monocyclic aromatic heterocyclic ring, each of which may have a substituent(s);
ring B may further have a substituent(s);
ring D represents a monocyclic aromatic hydrocarbon ring or a monocyclic aromatic heterocyclic ring, each of which may have a substituent(s);
L represents a bond, or a methylene group or an ethylene group, each of which may have a substituent(s);
X represents -O-, -$NR^a$-, or a methylene group which may have a substituent(s), wherein
$R^a$ represents a hydrogen atom or a substituent;
n represents 1 or 2; and
$R^1$ to $R^3$ are the same or different and each represent a hydrogen atom, a halogen atom, a cyano group, an acyl group, a $C_{1-6}$ alkyl group which may have a substituent(s), a $C_{1-6}$ alkoxy group which may have a substituent (s), a $C_{3-6}$ cycloalkyl group which may have a substituent(s), a $C_{3-6}$ cycloalkoxy group which may have a substituent(s), or an amino group which may have a substituent(s), wherein
ring A and ring D each may be condensed with another ring to form a condensed ring which may have a substituent(s), and ring A-L- in the formula $(I_0)$ may be bonded to X; or

a salt thereof for manufacturing a therapeutic or prophylactic agent for Alzheimer's disease.

**19.** A compound represented by the formula $(I_0)$:

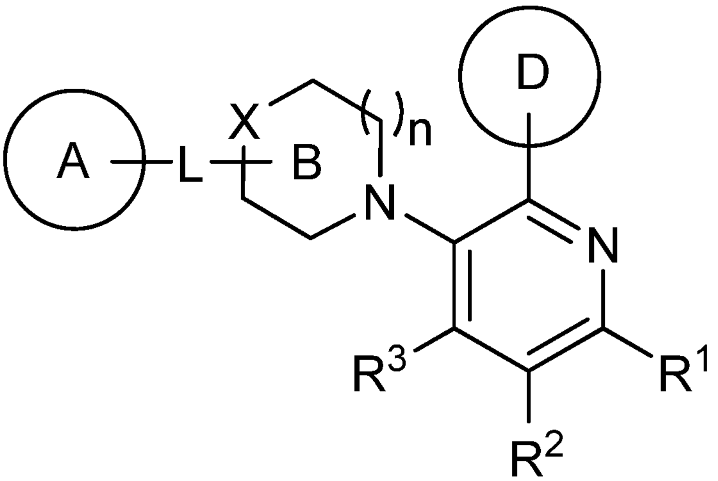

$(I_0)$

wherein

ring A represents a monocyclic aromatic hydrocarbon ring or a monocyclic aromatic heterocyclic ring, each of which may have a substituent(s);
ring B may further have a substituent(s);
ring D represents a monocyclic aromatic hydrocarbon ring or a monocyclic aromatic heterocyclic ring, each of which may have a substituent(s);
L represents a bond, or a methylene group or an ethylene group, each of which may have a substituent(s);
X represents -O-, -NR$^a$-, or a methylene group which may have a substituent(s), wherein
R$^a$ represents a hydrogen atom or a substituent;
n represents 1 or 2; and
$R^1$ to $R^3$ are the same or different and each represent a hydrogen atom, a halogen atom, a cyano group, an acyl group, a $C_{1-6}$ alkyl group which may have a substituent(s), a $C_{1-6}$ alkoxy group which may have a substituent (s), a $C_{3-6}$ cycloalkyl group which may have a substituent(s), a $C_{3-6}$ cycloalkoxy group which may have a substituent(s), or an amino group which may have a substituent(s), wherein
ring A and ring D each may be condensed with another ring to form a condensed ring which may have a substituent(s), and ring A-L- in the formula ($I_0$) may be bonded to X; or

a salt thereof for use in the prevention or treatment of Alzheimer's disease.

**INTERNATIONAL SEARCH REPORT**

International application No.
PCT/JP2010/070496

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D213/74, A61K31/496, A61K31/5377, A61K31/551, A61P3/10, A61P9/00, A61P9/10, A61P13/00, A61P25/00, A61P25/14, A61P25/16, A61P25/18, A61P25/22, A61P25/24, A61P25/28, A61P43/00, C07D401/04, C07D405/04, C07D409/04,

Documentation searched other than minimum documentation to the extent that such documents are included in the fields searched

Electronic data base consulted during the international search (name of data base and, where practicable, search terms used)
CAPLUS/REGISTRY(STN),

C. DOCUMENTS CONSIDERED TO BE RELEVANT

| Category* | Citation of document, with indication, where appropriate, of the relevant passages | Relevant to claim No. |
|---|---|---|
| X<br>A | WO 2008/141020 A1 (LILLY & CO. ELI), 20 November 2008 (20.11.2008), examples; claims & EP 2155717 A1 | 19<br>1-14,17,18 |
| A | WO 2008/099210 A2 (MERCK & CO. INC.), 21 August 2008 (21.08.2008), entire text & EP 2121633 A2 | 1-14,17-19 |
| A | WO 2008/156580 A1 (MERCK & CO. INC.), 24 December 2008 (24.12.2008), entire text (Family: none) | 1-14,17-19 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 December, 2010 (09.12.10) | 21 December, 2010 (21.12.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/070496

C (Continuation). DOCUMENTS CONSIDERED TO BE RELEVANT

| Category* | Citation of document, with indication, where appropriate, of the relevant passages | Relevant to claim No. |
|---|---|---|
| A | WO 2006/036015 A2 (MITSUBISHI PHARMA CORP.), 06 April 2006 (06.04.2006), entire text & EP 1805164 A2 & JP 2008-514587 A & US 2009/239864 A1 | 1-14,17-19 |

Form PCT/ISA/210 (continuation of second sheet) (July 2009)

**INTERNATIONAL SEARCH REPORT**

International application No.
PCT/JP2010/070496

Continuation of A. CLASSIFICATION OF SUBJECT MATTER
(International Patent Classification (IPC))

*C07D213/74*(2006.01)i, *A61K31/496*(2006.01)i, *A61K31/5377*(2006.01)i, *A61K31/551*(2006.01)i, *A61P3/10*(2006.01)i, *A61P9/00*(2006.01)i, *A61P9/10*(2006.01)i, *A61P13/00*(2006.01)i, *A61P25/00*(2006.01)i, *A61P25/14*(2006.01)i, *A61P25/16*(2006.01)i, *A61P25/18*(2006.01)i, *A61P25/22*(2006.01)i, *A61P25/24*(2006.01)i, *A61P25/28*(2006.01)i, *A61P43/00*(2006.01)i, *C07D401/04*(2006.01)i, *C07D405/04*(2006.01)i, *C07D409/04*(2006.01)i, *C07D413/04*(2006.01)i

(According to International Patent Classification (IPC) or to both national classification and IPC)

Continuation of B. FIELDS SEARCHED
Minimum documentation searched (International Patent Classification (IPC))

C07D413/04

Minimum documentation searched (classification system followed by classification symbols)

Form PCT/ISA/210 (extra sheet) (July 2009)

**INTERNATIONAL SEARCH REPORT**

International application No.
PCT/JP2010/070496

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 15,16
because they relate to subject matter not required to be searched by this Authority, namely:
Claims 15 and 16 pertain to "methods for treatment of the human body by therapy" and thus relate to a subject matter on which this International Searching Authority is not required to carry out a search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006008558 A
- WO 2008099210 A
- WO 2006036015 A
- US 5866562 A
- WO 2007130383 A
- WO 2008141020 A
- WO 200034262 A
- WO 200228850 A
- WO 2003004485 A
- WO 2010104194 A
- WO 1999514333 A
- WO 2001500852 A
- JP 9263545 A


### Non-patent literature cited in the description


- *Science,* 1989, vol. 245, 417-420
- *Neurobiology of Aging,* 1992, vol. 13, 587-590
- *Journal of Molecular Biology,* 1991, vol. 218, 149-163
- *Cell,* 1997, vol. 91, 231-241
- *J. Neurosci.,* 1991, vol. 11, 2552-2563
- *Science,* 1995, vol. 267, 2003-2006
- *J. Biol. Chem.,* 1997, vol. 272, 154-161
- *J. Biol. Chem.,* 1994, vol. 269, 3568-3573
- *Endocrinology,* 1989, vol. 125, 314-320
- *European Journal of Pharmacology,* 2004, vol. 490, 97-113
- *Journal of Neuroscience,* 2006, vol. 26 (2), 662-670
- **Greene ; Wuts.** Protective Groups in Organic Synthesis
- Development of Medicines. Molecular Design. Hirokawa Shoten Co., Ltd, 1990, vol. 7, 163-198
- *Japanese Pharmacopoeia*
- *Biochem. J.,* 1999, vol. 340 (1), 283-289
- *Journal of Clinical Oncology,* 1994, vol. 12, 213-225
- *British Journal of Cancer,* 1993, vol. 68, 314-318